# EUROPEAN PATENT SPECIFICATION

(11) **EP 3 104 878 B1**
(45) Date of publication and mention of the grant of the patent: **22.05.2019**
(21) Application number: 15709383.2
(22) Date of filing: 13.02.2015
(51) Int. Cl.: A61K 39/00

(54) **IMMUNOTHERAPY OF CANCER THROUGH COMBINATION OF LOCAL AND SYSTEMIC IMMUNE STIMULATION**
IMMUNTHERAPIE VON KREBS DURCH KOMBINATION VON LOKALER UND SYSTEMISCHER IMMUNSTIMULATION
IMMUNOTHÉRAPIE DU CANCER PAR COMBINAISON DE STIMULATION IMMUNITAIRE LOCALE ET SYSTÉMIQUE

(30) Priority: 14.02.2014 US 201461940109 P; 29.04.2014 US 201461985787 P; 08.07.2014 US 201462022070 P; 30.10.2014 US 201462072548 P; 08.01.2015 US 201562101335 P
(43) Date of publication of application: 21.12.2016
(60) Divisional application: 19168987.6
(73) Proprietor: Immune Design Corp., Seattle, WA 98102 (US)
(72) Inventor: TER MEULEN, Jan, Henrik, Mercer Island, WA 98040 (US); PAYA CUENCA, Carlos, V., San Francisco, CA 94114 (US)
(74) Representative: Mewburn Ellis LLP
(86) International application number: PCT/US2015/015767
(87) International publication number: WO 2015/123496

(56) References cited:
- WO-A1-2012/109203
- WO-A1-2012/112691
- WO-A1-2012/141984
- WO-A1-2013/172927
- XIAO HAIYAN ET AL: "Local Administration of TLR Ligands Rescues the Function of Tumor-Infiltrating CD8 T Cells and Enhances the Antitumor Effect of Lentivector Immunization", JOURNAL OF IMMUNOLOGY, vol. 190, no. 11, June 2013 (2013-06), pages 5866-5873, XP002739058, cited in the application
- M. B. DAVIS ET AL: "Intratumoral Administration of TLR4 Agonist Absorbed into a Cellular Vector Improves Antitumor Responses", CLINICAL CANCER RESEARCH, vol. 17, no. 12, 4 May 2011 (2011-05-04), pages 3984-3992, XP055148643, ISSN: 1078-0432, DOI: 10.1158/1078-0432.CCR-10-3262
- TREGONING JOHN S ET AL: "A "prime-pull" vaccine strategy has a modest effect on local and systemic antibody responses to HIV gp140 in mice.", PLOS ONE 2013, vol. 8, no. 11, 2013, page e80559, XP002739059, ISSN: 1932-6203
- TINA C ALBERSHARDT ET AL: "A prime-pull immunotherapy approach using a lentiviral vector and intratumoral TLR4 agonist redirects cytotoxic T cells", JOURNAL FOR IMMUNOTHERAPY OF CANCER, BIOMED CENTRAL LTD, LONDON, UK, vol. 2, no. Suppl 3, 6 November 2014 (2014-11-06), page P190, XP021202501, ISSN: 2051-1426, DOI: 10.1186/2051-1426-2-S3-P190

## Description

### BACKGROUND

### Technical Field

The present disclosure relates generally to methods for enhancing immunotherapy of cancer through combination of local and systemic immune stimulation.

### Description of the Related Art

The immune system of a host provides the means for quickly and specifically mounting a protective response to pathogenic microorganisms and also for contributing to rejection of malignant tumors. Immune responses have been generally described as including humoral responses, in which antibodies specific for antigens are produced by differentiated B lymphocytes, and cell mediated responses, in which various types of T lymphocytes eliminate antigens by a variety of mechanisms. For example, CD4 (also called CD4+) helper T cells that are capable of recognizing specific antigens may respond by releasing soluble mediators such as cytokines to recruit additional cells of the immune system to participate in an immune response. CD8 (also called CD8+) cytotoxic T cells are also capable of recognizing specific antigens and may bind to and destroy or damage an antigen-bearing cell or particle. In particular, cell mediated immune responses that include a cytotoxic T lymphocyte (CTL) response can be important for elimination of tumor cells and virus-infected cells.

Cancer includes a broad range of diseases and affects approximately one in four individuals worldwide. A CTL response is a key feature of effective cancer vaccines; effective CD4 T cell help is also likely to play a critical role in productive CD8 T cell activation and provide clinical benefit. The autologous dendritic cell (DC)-based vaccine Sipuleucel-T (PROVENGE®) was recently approved by the FDA for the treatment of metastatic, castrate-resistant prostate cancer though the survival benefit associated with this treatment is a modest 4.1 months, leaving significant need for improvement (see, e.g., Kantoff, et al., New Engl. J. Med. 363(5):411 (2010)). The poxvirus-vector based vaccine ProstVac® VF also shows a significant survival benefit in Phase II (see, e.g., Kantoff, et al., J. Clin. Oncol. 28(7):1099 (2010)). Active immune therapies such as Sipuleucel-T and ProstVac® VF have generally been better tolerated than the chemotherapeutic regimens that comprise the current standard of care for castrate-resistant disease (see, e.g., Petrylak, et al., N. Engl. J. Med. 351(15):1513 (2004); Sylwester, et al., J. Exp. Med. 202(5):673 (2005)). These clinical successes demonstrate that the immune response can be harnessed in a cancer setting to provide improved patient outcomes and extended survival.

Many foreign (that is, non-self) antigens are poorly immunogenic and require administration of an adjuvant to provide a satisfactory immune response to an antigen. Adjuvants may also be used to bias an immune response toward a humoral response or a cell-mediated response, and certain adjuvants may also be used to bias the antibody response to a particular antibody isotype or bias a cellular response toward a particular T-cell subset. The choice of adjuvant and/or mode of delivery of an adjuvant for inducing or enhancing the immune response to immunogens can be important aspects of therapeutic and prophylactic vaccine development.

Following surgical treatment and depending on its stage, non-muscle invasive bladder cancer (NMIBC) can be controlled by immunotherapy using the Bacillus Calmette Guerin (BCG) vaccine instilled repeatedly in the bladder, usually as a six-week course followed by yearly maintenance therapy. The application of the BCG vaccine in this context is a first line therapy and generally regarded as the oldest and to date most successful immunotherapy of cancer (Gandh, N.M., et. al., BJU Int., Aug;112(3):288-97 (2013)).

The anti-tumor effect of BCG is not fully understood, but involves infection of urothelial or bladder cancer cells and induction of both nonspecific inflammatory as well as specific anti-tumoral responses (Kawai, et al., Cancer Sci., Jan;104(1):22-7 (2013)). Immune system cell subsets that have potential roles in BCG therapy include CD4+ and CD8+ lymphocytes, natural killer cells, granulocytes, macrophages, and dendritic cells. Bladder cancer cells are killed through direct cytotoxicity by these cells, by secretion of soluble factors such as TRAIL (tumour necrosis factor-related apoptosis-inducing ligand), and, to some degree, by the direct action of BCG (Redelman-Sidi G., ET AL., Nat Rev Urol., Feb 4. [Epub ahead of print] (2014)). It should be noted that in this context, e.g., use of BCG in the treatment of bladder cancer, BCG functions as a TLR agonist. In particular, TLR2 and TLR4 appear to regulate distinct aspects of the host immune response against BCG (see J Leukoc Biol. 2003;74:277-86).

A significant limitation of current BCG treatment is the lack of response in a substantial number of patients. For example, depending on the endpoint studied, up to 50% of patients fail to respond and show progression of the cancer to muscle invasive disease. Additionally, apprimately one-third of patients that initially respond to therapy will show tumor recurrence (Sylvester, R.J., Int J Urol., Feb; 18(2): 113-20 (2011)). A recent study showed that patients with recurrence after BCG therapy may present with urothelial carcinoma of the upper urinary tract or the prostatic urethra, which is not accessible to BCG (Giannarini, G., et al., Eur Urol. Oct 9.[Epub ahead of print] 2013)). Also, patients with no measurable pre-existing T-cell immunity to BCG (due to previous BCG immunization or natural exposure to mycobacteria) have a lower recurrence-free survival rate (Biot, C., et al., Sci Transl Med., Jun 6;4(137):137ra72 (2012)). Finally, BCG immunotherapy may have severe side effects resulting from local or systemic infection with BCG (Miyazaki, J., et al., Jpn J Clin Oncol., Aug;43(8):827-34 (2013)).

Several avenues of research are currently being pursued to improve upon the current BCG therapy. For example, replacement of classical BCG with genetically engineered BCG expressing Th1-type cytokines or being replication incompetent may improve efficacy or reduce side effects (Kawai, et al., Cancer Sci., Jan;104(1):22-7 (2013)). Another example is the use of small molecule agonists of toll-like receptors (TLR) to stimulate an innate immune response in the bladder (Falke, J., et al., J Urol., Jun;189(6):2077-82 (2013)). Finally, viral vectors (e.g. adenovirus-based) are being used to induce "immunogenic cell death" in virus-induced cancer cells and express in addition immune-stimulatory factors, such a GM-CSF (Burke, J., Cytokine Growth Factor Rev.; 21(2-3):99-102 (2010)). However, none of the above strategies have to date progressed beyond early stage clinical trials and in the absence of good preclinical models for bladder cancer, their potential effectiveness is difficult to predict.

A recent study suggests that bladder cancer is associated with Kaposi's sarcoma-associated herpesvirus (KSHV), KSHV, also known as human herpesvirus 8. In particular, KSHV DNA was detected in 55 % of patients. (M. Paradžik et al., Tumor Biology, 2014, 35(1), pp 567-572). More generally, about 12% of all human cancers worldwide are caused by oncogenic virus infection (see e.g., Mesri et al., 2014, Cell Host & Microbe 15:266-282; Bouvard, V., et al. (2009). Lancet Oncol. 10, 321-322; Boyle, P., and Levin, B. (2008). World Cancer Report 2008. (Lyon: International Agency for Research on Cancer and World Health Organization Press); de Martel, C., et al., (2012). Lancet Oncol. 13, 607-615).

WO2013/172927 discloses a vaccination strategy that establishes protective immunity, including a protective memory T-cell population. The vaccination strategy includes the steps of parenterally administering to the subject at least one immunogen, wherein the at least one immunogen induces an immune response, and locally administering to the anatomic location of the subject at least one chemokine, wherein the chemokine recruits the immune response to the anatomic location. In particular, the chemokine may be selected from the group consisting of CXCL9, CXCL10 and CCL5.

The present invention provides an immunotherapeutic treatment of cancers, including virus-induced cancers. These and other advantages are provided in the present disclosure.

### BRIEF SUMMARY

The present invention, which is set out in the accompanying claims, provides a composition comprising:
a) a first composition comprising a vector particle optionally with an adjuvant, the vector particle comprising a recombinant expression vector wherein the recombinant expression vector comprises a polynucleotide encoding a cancer-associated antigen, and wherein the polynucleotide is operatively linked to at least one regulatory expression sequence, and
b) a second composition comprising a TLR4 agonist wherein the composition does not comprise antigen;
wherein the first composition and the second composition each further comprise a pharmaceutically suitable excipient,
for use in a method of increasing T cells in a tumor microenvironment in a subject,
where the first composition is administered systemically, thereby inducing an immune response against the cancer-associated antigen in the subject; and where the second composition is administered intratumorally or peritumorally to the subject; and where the first and second composition are administered concurrently or sequentially; thereby increasing T cells in the tumor microenvironment, wherein, when present, the adjuvant is glucopyranosyl lipid A (GLA).

Further aspects of the present invention are set out in the claims.

In addition the present disclosure also provides the following:

The present disclosure provides compositions and methods for enhancing immunotherapy of cancer, such as virus-induced cancer, through combination of local and systemic immune stimulation. In one embodiment of the present disclosure, a method of treating cancer is provided comprising a) inducing an immune response specific for an immunogen in a subject, comprising administering to the subject a first composition comprising a vector particle, the vector particle comprising a recombinant expression vector wherein the recombinant expression vector comprises a polynucleotide encoding the immunogen, and wherein the polynucleotide is operatively linked to at least one regulatory expression sequence; and b) administering locally to the subject a second composition comprising a pharmaceutically suitable adjuvant, wherein the second composition does not comprise or is substantially devoid of immunogen, wherein the first composition and the second composition each further comprise a pharmaceutically suitable excipient, and wherein the first composition and second composition are administered concurrently or sequentially. In certain embodiments, administration of the second composition induces expression of one or more cytokines capable of recruiting CD8 T-cells to the local site of administration.

In another embodiment, an aforementioned method is provided wherein the first composition and the second composition are administered sequentially, and wherein the first composition is administered to the subject at a first site and the second composition is administered to the subject at a second site, wherein the first site and the second site are different. In still another embodiment, an aforementioned method is provided wherein the first composition is administered at the first site via a first route and the second composition is administered at the second site via a second route.

In another embodiment of the present disclosure, an aforementioned method is provided wherein the first route and the second route are different and each is selected from parenteral, enteral, oral, intramuscular, intradermal, subcutaneous, intratumoral, intranodal, perinodal, intranasal, transdermal, inhalation, mucosal, intravesical and topical. In another embodiment, the first route is intramuscular, intradermal or subcutaneous and the second route is intravesical, intratumoral or intranodal.

In still another embodiment, an aforementioned method is provided wherein the first composition is administered prior to administration of the second composition. In another embodiment, an aforementioned method is provided wherein the second composition is administered prior to administration of the first composition. In another embodiment, the first composition and the second composition are administered concurrently but at different sites. In yet another embodiment, an aforementioned method is provided wherein the first composition and/or the second composition are administered 2, 3, 4, 5, 6, 7, 8, 9 or 10 times.

In another embodiment of the present disclosure, an aforementioned method is provided wherein the recombinant expression vector is selected from a retroviral vector genome, a lentiviral vector genome, poxvirus vector genome, vaccinia virus vector genome, adenovirus vector genome, adenovirus-associated virus vector genome, herpes virus vector genome, alpha virus vector genome, plasmid DNA and RNA. In another embodiment, the vector particle is a lentiviral vector particle that comprises the lentiviral vector genome; a poxvirus vector particle that comprises the poxvirus vector genome; a vaccinia virus vector particle that comprises the vaccinia virus vector genome; an adenovirus vector particle that comprises the adenovirus vector genome; an adenovirus-associated virus vector particle that comprises the adenovirus-associated virus vector genome; a herpes virus vector particle that comprises the herpes virus vector genome; or an alpha virus vector particle that comprises the alpha virus vector genome. In still another embodiment, the vector particle is the lentiviral vector particle and comprises the lentiviral vector genome. In still another embodiment, the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein.

In another embodiment, an aforementioned method is provided wherein the vector particle delivers the recombinant expression vector to an antigen-presenting cell. In one embodiment, the antigen-presenting cell is a dendritic cell.

In still another embodiment, an aforementioned method is provided wherein the immunogen is a viral antigen from an oncogenic virus. Exemplary oncogenic viruses include, but are not limited to, EBV, HPV, HBV, HCV, HTLV, and KSHV. Exemplary viral antigens from oncogenic viruses that can be used herein include but are not limited to, EBV: EBNA-1, LMP-1, LMP-2A; HPV: E6, E7, E5; HBV: HBx; HCV: Core, NS3, Ns5A; HTLV: Tax, HBZ; KSHV: vFLIP, LANA, vGPCR, vIRF-1.

In another embodiment, the immunogen is a tumor-associated antigen. In another embodiment, the tumor associated antigen is selected from p53, Ras, c-Myc, A-Raf, B-Raf, C-Raf, cyclin-dependent kinases, CTA, NY-ESO-1, LAGE-1, MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A, BK T-antigen, MAGE-A2, MAGE-A6, MAGE-A12, MART-1, DAM-6, -10, GAGE-1, -2, -8, GAGE-3, -4, -5, -6, -7B, NA88-A, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, β-catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RARa, Tumor-associated calcium signal transducer 1 (TACSTD1) TACSTD2, receptor tyrosine kinases, Epidermal Growth Factor receptor (EGFR), EGFRvIII, platelet derived growth factor receptor (PDGFR), vascular endothelial growth factor receptor (VEGFR), cytoplasmic tyrosine kinases, src-family, syk-ZAP70, integrin-linked kinase (ILK), signal transducers and activators of transcription STAT3, STAT5, and STAT6, hypoxia inducible factors, HIF-1α and HIF-2α, Nuclear Factor-Kappa B (NF-κB), Notch receptors, Notch1-4, c-Met, mammalian targets of rapamycin (mTOR), WNT, extracellular signal-regulated kinases (ERKs), PMSA, PR-3, MDM2, Mesothelin, renal cell carcinoma - 5T4, SM22-alpha, carbonic anhydrases I (CAI) and IX (CAIX), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGs5, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, and fos related antigen 1.

In another embodiment, a method is provided herein wherein the tumor-associated antigen is selected from a bladder cancer antigen. In one embodiment, the bladder cancer antigen is selected from CTA, NY-ESO-1, LAGE-1,MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A, and BK T-antigen.

In yet another embodiment of the present disclosure, an aforementioned method is provided wherein the induced immune response comprises a cytotoxic T lymphocyte immune response. In certain embodiments, a method as described herein is provided wherein the induced immune response comprises antigen-specific CD4 and/or CD8 T cells, effector memory T cells (T_{EM}), central memory T cells (T_{CM}) and/or tissue-resident memory T cells (T_{RM}). In certain embodiments of the methods provided herein, one or more of these populations of cells is recruited to a local site of interest (e.g., the mucosa, intratumoral, intranodal). In another embodiment, an aforementioned method is provided wherein the induced immune response comprises production of an immunogen specific antibody.

In still another embodiment, an aforementioned method is provided wherein the adjuvant is a non-toxic lipid A-related adjuvant. In one embodiment, the non-toxic lipid A-related adjuvant is glucopyranosyl lipid A (GLA). In another embodiment, the GLA is formulated in a stable oil-in-water emulsion. In still another embodiment, the GLA has the formula: 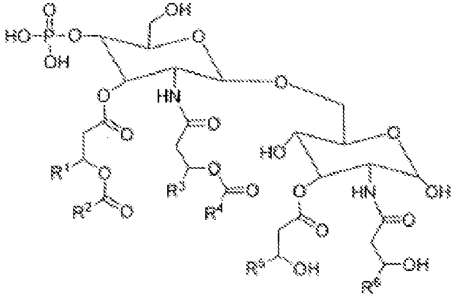 where: R¹, R³, R⁵ and R⁶ are C₁₁-C₂₀ alkyl; and R² and R⁴ are C₁₂-C₂₀ alkyl. In yet another embodiment, R¹, R³, R⁵ and R⁶ equal to undecyl, and R² and R⁴ equal to tridecyl.

In certain embodiments of the methods herein, the method further comprises administering a composition comprising BCG. In another embodiment of the present disclosure, a method is provided wherein the adjuvant composition further comprises BCG.

In another embodiment, an aforementioned method is provided wherein the first composition further comprises an adjuvant.

In yet another embodiment, a method of treating virus-induced cancer is provided comprising a) inducing an immune response specific for an immunogen associated with a virus-induced cancer (e.g., a viral antigen derived from the virus associated with the cancer) in a subject, comprising administering to the subject a first composition comprising a lentivector particle, the lentivector particle comprising a recombinant expression vector, wherein the recombinant expression vector comprises a polynucleotide encoding the immunogen, wherein the polynucleotide is operatively linked to at least one regulatory expression sequence, wherein the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein; and b) inducing expression of one or more cytokines capable of recruiting CD8 T-cells to the tumor comprising administering to the subject a second composition comprising a pharmaceutically suitable adjuvant, wherein the adjuvant is a non-toxic lipid A-related adjuvant is glucopyranosyl lipid A (GLA) formulated in a stable oil-in-water emulsion, wherein the GLA has the formula: 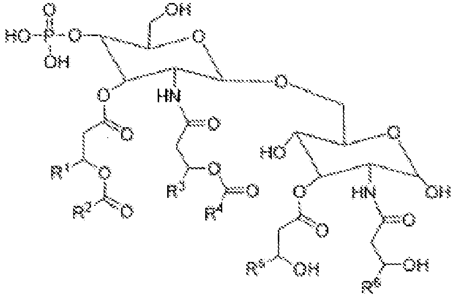 where: R¹, R³, R⁵ and R⁶ equal to undecyl, and R² and R⁴ equal to tridecyl; wherein the cytokines are selected from the group consisting of Cxcl9, Cxcl10, CCL2, CCL3, MCP-1, TNFa, IFNc and IP-10; wherein the immunogen associated with a virus-induced cancer is a viral antigen selected from EBNA-1, LMP-1, LMP-2A; E6, E7, E5; HBx; HCV Core, NS3, Ns5A; Tax, HBZ; vFLIP, LANA, vGPCR, vIRF-1; wherein the first composition and the second composition each further comprise a pharmaceutically suitable excipient, wherein the first composition and second composition are administered concurrently or sequentially, wherein the first composition is administered intramuscularly, intradermally or subcutaneously and the second composition is administered intratumorally or intravesically; and wherein the second composition optionally comprises BCG.

In another embodiment of the present disclosure, a kit is provided comprising a first composition and a second composition according to any one of the previous methods, above or described herein.

Another aspect of the present disclosure provides a method of treating an oncogenic virus-induced cancer comprising: a) inducing an immune response specific for an oncogenic virus antigen in a subject, comprising administering to the subject a first composition comprising a vector particle, the vector particle comprising a recombinant expression vector wherein the recombinant expression vector comprises a polynucleotide encoding the oncogenic virus antigen, and wherein the polynucleotide is operatively linked to at least one regulatory expression sequence; and b) administering to the subject at a local site a second composition comprising a pharmaceutically suitable adjuvant, wherein the first composition and the second composition each further comprise a pharmaceutically suitable excipient, and wherein the first composition and second composition are administered concurrently or sequentially. In one embodiment, administration of the second composition at the local site induces expression of one or more cytokines capable of recruiting T cells to the local site of administration. In certain embodiments, the T cells are antigen-specific T cells, such as antigen-specific CD4 and/or CD8 T cells, effector memory T cells (T_{EM}), central memory T cells (T_{CM}) and/or tissue-resident memory T cells (T_{RM}). In one embodiment of the method, the local site of local administration is intratumoral, intranodal, intravesical or mucosal. In another embodiment, the expression vector further comprises a polynucleotide encoding a tumor-associated antigen. In a further embodiment, the second composition further comprises the tumor-associated antigen.

One specific aspect of the present disclosure provides a method for treating an oncogenic virus-induced cancer in a subject comprising: a) inducing an immune response specific for an antigen derived from the oncogenic virus in the subject, comprising administering to the subject a first composition comprising a vector particle, the vector particle comprising a recombinant expression vector wherein the recombinant expression vector comprises a polynucleotide encoding the antigen derived from the oncogenic virus, and wherein the polynucleotide is operatively linked to at least one regulatory expression sequence; and b) administering to the subject intratumorally a second composition comprising a pharmaceutically suitable adjuvant, wherein the first composition and the second composition each further comprise a pharmaceutically suitable excipient, and wherein the first composition and the second composition are administered concurrently or sequentially. In one embodiment, administering the second composition intratumorally induces expression of one or more cytokines or chemokines capable of recruiting T cells, in particular antigen-specific T cells (e.g., CD4 and/or CD8 T cells, effector memory T cells (T_{EM}), central memory T cells (T_{CM}) and/or tissue-resident memory T cells (T_{RM})) to the tumor.

Another aspect of the disclosure provides a method of treating a cancer comprising: a) inducing an immune response specific for a cancer-associated antigen in a subject, comprising administering to the subject a first composition comprising a vector particle, the vector particle comprising a recombinant expression vector wherein the recombinant expression vector comprises a polynucleotide encoding the cancer-associated antigen, and wherein the polynucleotide is operatively linked to at least one regulatory expression sequence; and b) administering intra-tumorally to the subject a second composition comprising a pharmaceutically suitable adjuvant; wherein the first composition and the second composition each further comprise a pharmaceutically suitable excipient, and wherein the first composition and second composition are administered concurrently or sequentially.

In certain embodiments of the methods of treating cancer, including oncovirus-induced cancers, the first composition and/or the second composition are administered 2, 3, 4, 5, 6, 7, 8, 9 or 10 times. In other embodiments, the recombinant expression vector is selected from a retroviral vector genome, a lentiviral vector genome, poxvirus vector genome, vaccinia virus vector genome, adenovirus vector genome, adenovirus-associated virus vector genome, herpes virus vector genome, alpha virus vector genome, plasmid DNA and RNA. In yet a further embodiment of the methods herein, the vector particle is a retroviral vector particle that comprises the retroviral vector genome; a lentiviral vector particle that comprises the lentiviral vector genome; a poxvirus vector particle that comprises the poxvirus vector genome; a vaccinia virus vector particle that comprises the vaccinia virus vector genome; an adenovirus vector particle that comprises the adenovirus vector genome; an adenovirus-associated virus vector particle that comprises the adenovirus-associated virus vector genome; a herpes virus vector particle that comprises the herpes virus vector genome; or an alpha virus vector particle that comprises the alpha virus vector genome. In one embodiment, the vector particle is the lentiviral vector particle and comprises the lentiviral vector genome. In one particular embodiment, the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein. In another embodiment of the methods herein, the vector particle delivers the recombinant expression vector to a dendritic cell. In certain embodiments of the methods described herein the immunogen is a tumor-associated antigen and may be selected from CTA, NY-ESO-1, LAGE-1, MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A, and BK T-antigen, p53, Ras, c-Myc, A-Raf, B-Raf, C-Raf, cyclin-dependent kinases, MAGE-A2, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, β-catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RARa, Tumor-associated calcium signal transducer 1 (TACSTD1) TACSTD2, receptor tyrosine kinases, Epidermal Growth Factor receptor (EGFR), EGFRvIII, platelet derived growth factor receptor (PDGFR), vascular endothelial growth factor receptor (VEGFR), cytoplasmic tyrosine kinases, src-family, syk-ZAP70, integrin-linked kinase (ILK), signal transducers and activators of transcription STAT3, STAT5, and STAT6, hypoxia inducible factors, HIF-1α and HIF-2α, Nuclear Factor-Kappa B (NF-κB), Notch receptors, Notch1-4, c-Met, mammalian targets of rapamycin (mTOR), WNT, extracellular signal-regulated kinases (ERKs), PMSA, PR-3, MDM2, Mesothelin, renal cell carcinoma - 5T4, SM22-alpha, carbonic anhydrases I (CAI) and IX (CAIX), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGs5, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, and fos related antigen 1.

In another embodiment of the methods described herein, the bladder cancer antigen is selected from CTA, NY-ESO-1, LAGE-1,MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A, and BK T-antigen.

In one embodiment of the methods described herein for treating cancer, the induced immune response comprises a cytotoxic T lymphocyte immune response or production of an immunogen specific antibody.

In certain embodiments of the methods described herein for treating cancer, the adjuvant is a non-toxic lipid A-related adjuvant. In particular, the non-toxic lipid A-related adjuvant is glucopyranosyl lipid A (GLA). In certain embodiments, the GLA is formulated in a stable oil-in-water emulsion. In one embodiment, the GLA has the formula: 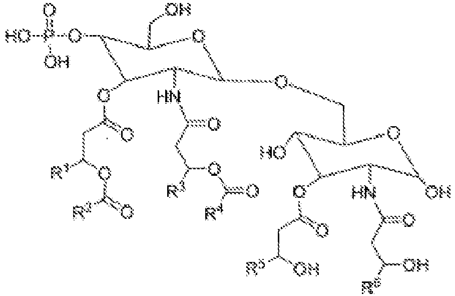 where: R¹, R³, R⁵ and R⁶ are C11-C20 alkyl; and R² and R⁴ are C12-C20 alkyl. In another embodiment, R¹, R³, R⁵ and R⁶ equal to undecyl, and R² and R⁴ equal to tridecyl.

Another aspect of the present disclosure provides a method of increasing T cells in the tumor microenvironment comprising, a) administering to a subject having a tumor a first composition comprising a vector particle, the vector particle comprising a recombinant expression vector wherein the recombinant expression vector comprises a polynucleotide encoding a cancer-associated antigen, and wherein the polynucleotide is operatively linked to at least one regulatory expression sequence, thereby inducing an immune response against the cancer-associated antigen in the subject; and b) administering intratumorally or peritumorally to the subject a second composition comprising a TLR4 agonist wherein the composition does not comprise antigen; wherein the first composition and the second composition each further comprise a pharmaceutically suitable excipient, and wherein the first composition and second composition are administered concurrently or sequentially; thereby increasing T cells in the tumor microenvironment. In certain embodiments, the vector particle is a lentiviral vector particle that comprises the lentiviral vector genome; a poxvirus vector particle that comprises the poxvirus vector genome; a vaccinia virus vector particle that comprises the vaccinia virus vector genome; an adenovirus vector particle that comprises the adenovirus vector genome; an adenovirus-associated virus vector particle that comprises the adenovirus-associated virus vector genome; a herpes virus vector particle that comprises the herpes virus vector genome; or an alpha virus vector particle that comprises the alpha virus vector genome. In another embodiment, the vector particle is the lentiviral vector particle and comprises the lentiviral vector genome. In additional embodiments, the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein and in some embodiments, delivers the recombinant expression vector to a dendritic cell. In some embodiments of the method, the TLR4 agonist is a non-toxic lipid A-related adjuvant such as GLA described herein and may be formulated in stable oil-in-water emulsion.

### BRIEF DESCRIPTION OF THE DRAWINGS

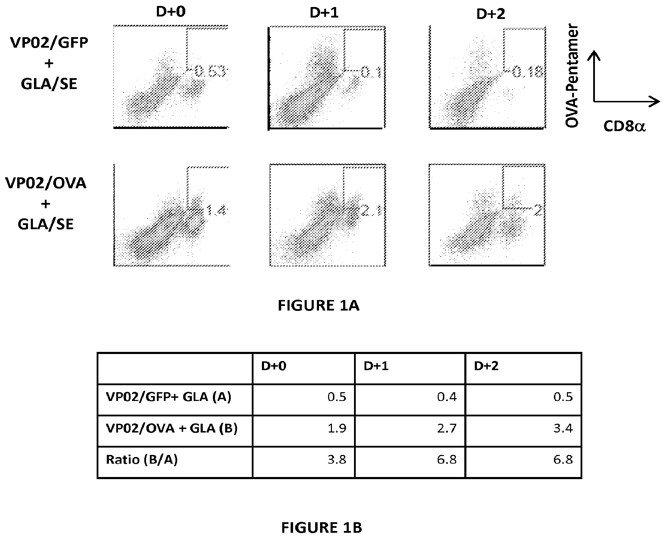

**Figure 1****:** Prime-Pull Vaccination Strategy. C57BL/6 mice (5 mice per group) were inoculated with B16F10-OVA cells on Day 0. Ten days post-tumor inoculation, tumor-bearing mice were immunized with VP02/OVA or VP02/GFP (control vector) at the tail base. Twenty-one days post-tumor-inoculation, immunized mice were given intra-tumoral administration of GLA/SE. Tumors were harvested immediately prior to (D+0), one day post-(D+1), or two days post- (D+2) GLA/SE administration. Harvested cells were stained for flow cytometry analysis. **Figure 1 (A)** Representative dot plots of the analyzed time points. Red gate include OVA-specific CD3ε + CD8α+ cells. **Figure 1 (B)** Average percent of OVA-specific CD3 ε + CD8α+ cells of the analyzed time points.

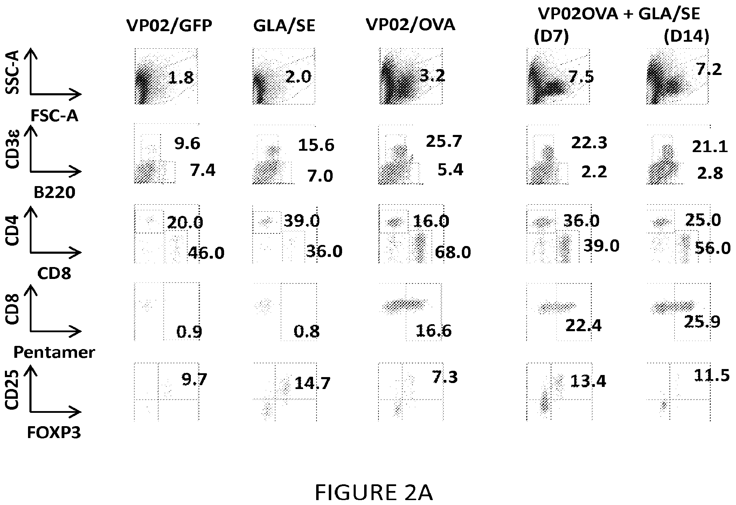

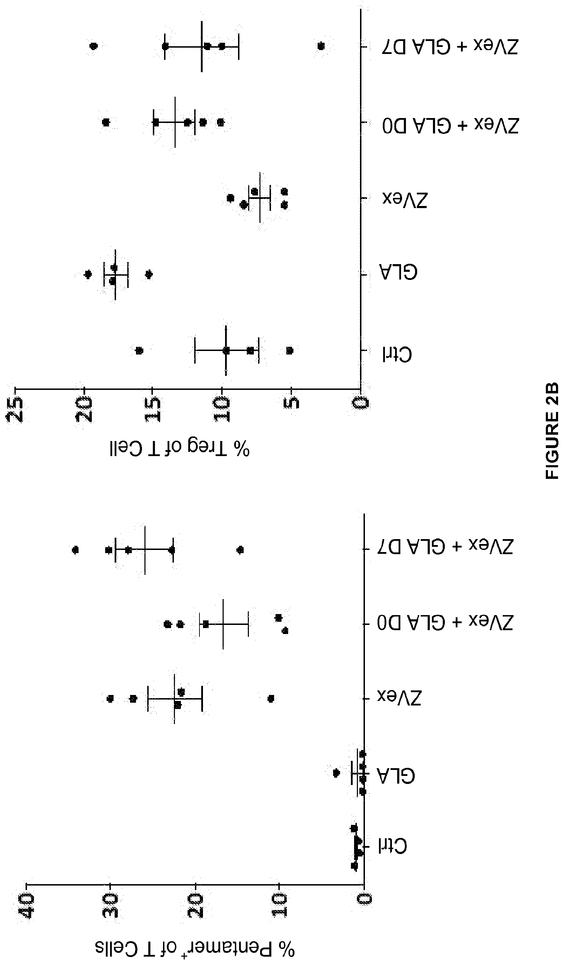

**Figure 2****:** Recruitment of Lymphocytes to the Tumor by the Prime-Pull Vaccination Strategy. C57BL/6 mice (5 mice per group) were inoculated with 1 x 10⁵ B16F10-OVA cells, s.c. in the flank, on Day 0. Seven days post-tumor inoculation, tumor-bearing mice were immunized with VP02/OVA or VP02/GFP (control vector) at the tail base. Starting at Day 7 (D7) or Day 14 (D14) post-tumor-challenge, immunized mice were given intra-tumoral administration of 5 µg GLA/2% SE every 3-4 days. On Day 18, tumors were harvested. Tumor-infiltrating lymphocytes were isolated on a 1:1 cushion of Histopaque®-1083 and stained with MHC-I/SIINFEKL pentamers, CD3, CD8, CD4, CD25, and FOXP3 antibodies for flow cytometry analysis. **Figure 2 (A)** Reresentative dot plots for gating strategy are shown, with % group averages denoted. **Figure 2 (B)** Scatter plots for % pentamer⁺ cells and % regulatory T cells are shown for each group. Error bars represent mean ± SEM.

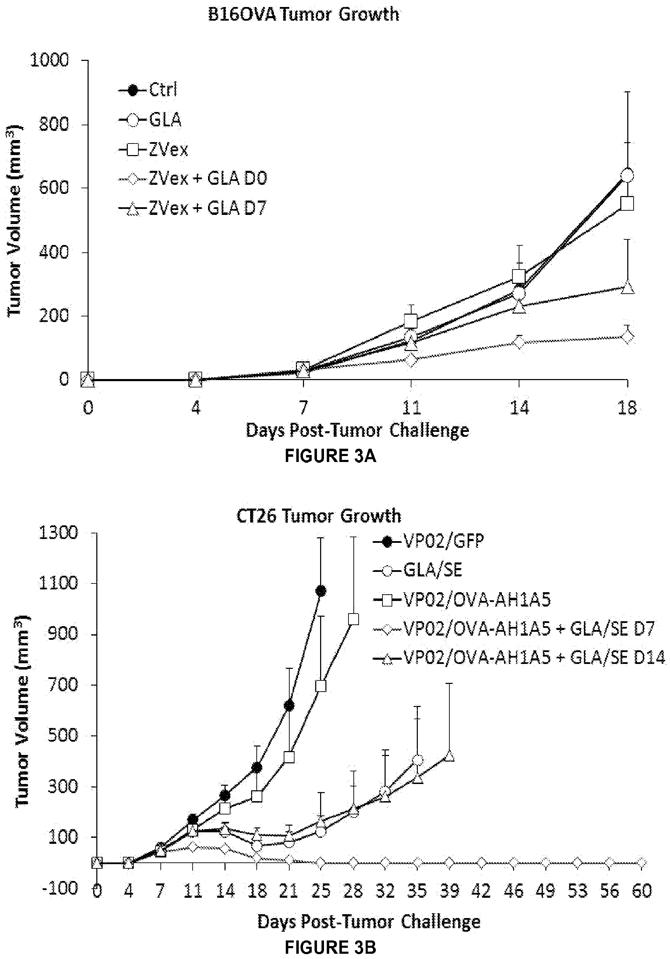

**Figure 3****:** Therapeutic Efficacy of the Prime-Pull Vaccination Strategy. **Figure 3** (A) C57BL/6 mice (5 mice per group) were inoculated with 1 x 10⁵ B16F10-OVA cells, s.c. in the flank, on Day 0. Seven days post-tumor inoculation, tumor-bearing mice were immunized with VP02/OVA or VP02/GFP (control vector) at the tail base. Starting at Day 7 (D7) or Day 14 (D14) post-tumor-challenge, immunized mice were given intra-tumoral administration of 5 µg GLA/2% SE every 3-4 days. **Figure 3 (B)** BALB/c mice (5 mice per group) were inoculated with 1 x 10⁵ CT26 cells, s.c. in the flank, on Day 0. Seven days post-tumor inoculation, tumor-bearing mice were immunized with VP02/AH1A5 or VP02/GFP (control vector) at the tail base. Starting at Day 7 (D7) or Day 14 (D14) post-tumor-challenge, immunized mice were given intra-tumoral administration of 5 µg GLA/2% SE every 3-4 days. Tumor growth was measured 2-3 times per week. Tumor volume was calculated based on a modified ellipsoid formula: length x width x *π*/6. Error bars represent mean ± SEM.

### DETAILED DESCRIPTION

The present invention provides, *inter alia,* an integrated immunotherapy for cancers, such as virally-induced cancers, comprising a composition for use as set out in the claims.

The present invention could be used in the setting of any cancer, including virally-induced cancer, such as Merkel cell carcinoma (Merkel polyoma virus; large T antigen), head and Neck cancers (HPV; E6 and E7 antigens), cervical cancer (HPV; E6 and E7 antigens), liver cancer (hepatitis B- large, medium and small S antigens; hepatitis C virus), nasopharyngeal cancer (EBV; EB nuclear antigen 1 (EBNA1), latent membrane protein-1 and -2 (LMP-1), LMP-2), Kaposi's sarcoma (HHV8) and glioblastoma (human Cytomegalovirus, pp65, IE1, us28).

Without being bound by theory, the use of expression vectors, including viral vectors, expressing one or several viral antigens or one or several tumor antigens in combination with intratumoral application of a TLR4 agonist, such as a GLA described herein, in a novel application (prime/pull), results in the local production in or around the tumor of the cytokines, including cxcl9 and cxcl10, which will direct systemically or locally induced tumor specific cytotoxic T cells to the site of the cancer. Again, without being bound by theory, through this mechanism and the immune-stimulatory effect of a TLR4 agonist such as GLA (*e*.*g*. enhanced antigen presentation by dendritic cells, as well as their maturation, through TLR4 receptor stimulation) the efficacy of immunotherapy of cancers, including virus-induced cancers, will be greatly enhanced.

The first report of a prime-pull using a systemic lentivector prime to induce CD8 T-cells, followed by intratumoral injection of a toll-like receptor ligands to enhance therapeutic efficacy was published in 2013 by Xiao et al. (J. Immunotherapy, 2013 June 1; 190(11): 5866-5873). In these experiments, a VSV-pseudotyped lentivector was used for immunization, and the toll like receptor agonists 3 (poly-I:C) and 9 (CpG) were injected intratumorally (i.t.). While the investigators did observe a therapeutic effect, unlike the present disclosure, i.t injection of TLR3/9 ligands decreased the absolute numbers of CD8 and CD4 TILs compared to lentiviral vector immunization alone (see Xiao et al., Fig. 2A), indicating that enhancement of the antitumor effect by injection of TLR3/9 ligands was not due to an increase in the numbers of CD8 and CD4 TILs. Therefore, the skilled person would not have been led to use i.t. or local administration of TLR agonists for recruitment of T cells to the tumor site in view of this study.

In one aspect, in order to address the unmet need of the existing therapies for virus-induced cancers, (including but not limited to, bladder cancer, Merkel cell carcinoma, Kaposi's sarcoma Burkitt's lymphoma, non-Hodgkin's lymphoma, post-transplant lymphoproliferative disorder, nasopharynx cancer, cervical carcinoma, head and neck cancer, hepatocellular carcinoma, adult T cell leukemia/lymphoma), the present disclosure provides compositions and methods for inducing and/or enhancing a specific anti-viral CD8 T-cell response which is re-directed to the tumor. To this end, immunization with, for example, a recombinant expression vector, e.g., a dendritic cell-targeting lentivector as described herein, expressing a specific viral antigen, or in some embodiments, a cancer associated tumor antigen(s) or both (i.e., "immunogen"), followed by local administration (e.g., intratumoral, intranodal, mucosal or intravesical treatment) with a TLR agonist such as TLR4 agonist glucopyranosly lipid A (GLA) as described herein. This results in the induction of viral antigen-specific CD8 T-cells and their re-direction, or recruitment or homing, to the tumor, resulting in a therapeutic effect superior to current immunotherapies. In this regard, and without being bound by theory, tumor associated viral antigens such as those described herein (e.g. HPV-E6/E7) are foreign antigens and as such are more immunogenic than endogenous tumor associated antigens, which will increase the efficacy of the immunotherapy.

In certain embodiments, (e.g., for bladder cancer) the local administration may optionally including BCG as described herein, and in certain embodiments, the antigen-specific CD8 T cell response is re-directed to the mucosa of the bladder.

The present disclosure, therefore, provides in one embodiment a "prime-pull" immunization wherein a first "prime" or in certain embodiments a "prime/boost" composition comprising, for example, a recombinant expression vector expressing a specific immunogen (e.g., one or more tumor associated antigens; one or more viral antigen(s) where the virus is an oncogenic virus associated with cancer), in order to induce a CD8 T-cell response is followed by a "pull" composition comprising a TLR4 agonist such as GLA in order to recruit the CD8 T-cells to the tumor. As noted above, tumor associated viral antigens are more immunogenic than endogenous tumor associated antigens, which will increase the efficacy of the immunotherapy. However, in certain embodiments, the immunogen may be a cancer-associated antigen not associated with an oncogenic virus. According to various embodiments of the disclosure, the expression vector and the TLR4 agonist may be administered multiple times, at various sites, and using various routes, as described herein. Prime-pull immunization has also been described International Publication No. WO 2013/172927.

The present disclosure, in one embodiment, provides in one embodiment a first "prime" or "prime/boost" composition comprising, for example, a recombinant expression vector expressing a specific bladder cancer associated tumor antigen(s) or immunogen(s) in order to induce an immune response, including a CD8 T-cell response, and a "pull" composition comprising a TLR4 agonist such as GLA with or without BCG in order to recruit the antigen-specific T cells, including CD8 T-cells, to the mucosa of the bladder. According to various embodiments of the disclosure, the expression vector and the TLR agonist (with or without BCG) may be administered multiple times, at various sites, and using various routes, as described herein. Accordingly, the present disclosure provides an integrated first-line immunotherapy for established cancer of the bladder.

In one embodiment, the present disclosure provides a method comprising administering (i) a dendritic cell-targeting lentivector-based vaccine expressing one or more oncogenic virus antigens or immunogens (e.g., one or more antigens from a virus associated with inducing a cancer) applied intradermally, subcutaneously or intramuscularly as a priming immunization (ii) the same vaccine applied intratumorally as a boosting immunization (iii) the synthetic GLA applied repeatedly intratumorally alone or in some embodiments, in conjunction with the oncogenic virus antigen or a tumor-associated antigen, as a maintenance therapy and to direct systemically or locally primed cytotoxic T-cells to the tumor.

In one embodiment, the present disclosure provides a method comprising administering (i) a dendritic cell-targeting lentivector-based vaccine expressing one or more oncogenic virus antigens and one or more tumor associated antigens or immunogens applied intradermally, subcutaneously or intramuscularly as a priming immunization (ii) the same vaccine applied intravesically as a boosting immunization (iii) the synthetic GLA applied repeatedly mucosally, intratumorally or intravesically, alone or optionally in combination with BCG, as a maintenance therapy and to direct systemically or locally primed cytotoxic T-cells to the bladder mucosa. Such a method is used for the treatment of virus-induced cancers. In certain embodiments, the methods herein are used for the treatment of bladder cancer.

The use of local administration of GLA will result in the local production of cytokines, including but not limited to Cxcl9 and Cxcl10, in the tumor which will direct systemically or locally induced virus-specific cytotoxic T cells or tumor-specific cytotoxic T cells to the tumor where the viral antigen is expressed by tumor cells. Without being bound by theory, through the mechanism of TLR4 receptor stimulation by GLA, the efficacy of immunotherapy of virus-induced cancer will be greatly enhanced.

In another aspect of the present disclosure, the "prime" immune response may be achieved by adoptively transfering immune cells into the patient. In this regard, cells may be isolated from the subject, modified *ex vivo* and then transferred back into the patient. Local administration of a suitable composition, such as a TLR4 agonist (e.g., GLA) or other adjuvant composition as described herein, is then used to "pull" the adoptively transferred cells to the tumor or local site of administration. Immune cells can be modified *ex vivo* in a variety of ways known to the skilled person, such as the cells may be expanded by various technologies to generate antigen-specific or polyclonal T cells (see for example Ridell et al., J. Immunol. Meth. (1990) 128:189-201; Riddell et al., Science (1992) 257:238-241; US5,827,642; US6,040,177; US6,692,964; US6,352,694); genetically modified using chimeric antigen receptor technology (see for example, US Patents 8,906,682; 8,916,381; 7,741,465; 5,843,728; 7,446,190; 6,392,013; WO 2012/079000, chimeric TCR technology or other genetic modification (see for example, US8,741,814; US20130189309). In certain embodiments, cells may be isolated from a donor other than the subject or in other embodiments, the cells may be derived from a suitable cell line or artificial cell modified so as to be compatible with the subject.

Thus, another aspect of the present disclosure provides a method of treating a cancer in a subject by 1) administering to the subject a first composition comprising tumor-associated antigen-specific T cells that have been expanded or modified *ex vivo*, genetically modified CAR-T cells that have been modified genetically to recognize a tumor-associated antigen, or T cells genetically modified to express a specific chimeric T cell receptor (TCR) that recognizes a cancer-associated epitope in the context of MHC; 2) administering locally (e.g., intra-tumorally) to the subject a second composition comprising a pharmaceutically suitable adjuvant as described herein to pull the adoptively transferred cells to the tumor; thereby treating the cancer in the subject. In certain embodiments, the adoptively transferred cells may be boosted by administering to the subject an expression vector as described herein prior to the local administration of the second composition to pull the adoptively (and then boosted) cells to the local site (e.g., to a tumor).

### Cancers

The prime-pull regimens described herein may be useful for the treatment of a variety of cancers. In one embodiment, the methods described herein may be useful for the treatment of a variety of solid tumors, *i*.*e*., carcinomas, sarcomas, and lymphomas. In certain embodiments, the cancer is a primary solid tumor, and in certain other embodiments a cancer is a metastatic or secondary solid tumor. In certain related embodiments, the cancer is selected from melanoma, lung cancer, cervical cancer, ovarian cancer, uterine cancer, breast cancer, liver cancer, gastric cancer, colon cancer, prostate cancer, pancreatic cancer, kidney cancer, bladder cancer, brain cancer, fibrosarcoma, myxosarcoma, liposarcoma, chondrosarcoma, osteogenic sarcoma, chordoma, angiosarcoma, endotheliosarcoma, lymphangiosarcoma, pseudomyxoma petitonei, lymphangioendotheliosarcoma, synovioma, mesothelioma, Ewing's tumor, leiomyosarcoma, rhabdomyosarcoma, colon carcinoma, pancreatic cancer, squamous cell carcinoma, basal cell carcinoma, adenocarcinoma, Merkel cell carcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma and Wilms' tumor. In certain other related embodiments the cancer cell originates in a cancer that is selected from testicular tumor, lung carcinoma, small cell lung carcinoma, bladder carcinoma, epithelial carcinoma, glioma, glioblastoma multiforme, astrocytoma, plasmocytoma, medulloblastoma, craniopharyngioma, ependymoma, pinealoma, hemangioblastoma, acoustic neuroma, oliodendroglioma, meningioma, melanoma, neuroblastoma, retinoblastoma, leukemia, lymphoma, non-Hodgkin's lymphoma, multiple myeloma, Waldenstrom's macroglobulinemia or other cancers. Thus, the methods described herein include methods for the treatment of, ameliorating the symptoms of, and inhibiting metastasis of cancer comprising administering "prime-pull" immunization wherein a first "prime" or in certain embodiments a "prime/boost" composition comprising, for example, a recombinant expression vector expressing a specific immunogen (e.g., one or more tumor associated antigens), and a "pull" composition comprising a TLR agonist such as GLA in order to recruit immune cells to the tumor.

In certain embodiments, the prime-pull regimens described herein are useful for treating cancers in which the tumors are particularly immunogenic and in which the tumors are accessible, such as but not limited to melanoma, Merkel cell carcinoma (MCC), cervical cancer, head-and-neck cancers, bladder cancer, sarcomas, and esophageal cancers. In some embodiments immunogenicity can be determined based on mutational heterogeneity and/or expression of cancer testis antigens and/or expression of viral antigens (e.g., specific antigen/neo-antigen vaccination approach). See for example, Chen et al., Cancer Immunol Res. 2014 May;2(5):480-6; and Lawrence et al., 2013 Nature 499:214-218.

In particular embodiments, a virus-induced cancer is amenable to being treated or ameliorated by the methods described herein. Numerous cancers induced by oncogenic viruses are known in the art. Exemplary virus-induced cancers that can be treated or ameliorated by the methods described herein include, but are not limited to, bladder cancer, Merkel cell carcinoma, Kaposi's sarcoma, liver cancer, Burkitt's lymphoma, non-Hodgkin's lymphoma, post-transplant lymphoproliferative disorder, nasopharynx cancer, cervical carcinoma, head and neck cancer, hepatocellular carcinoma, and adult T cell leukemia/lymphoma (see *e*.*g*., Mesri et al., Cell Host & Microbe 15:266-282). Other cancers may be determined to be induced by particular viruses, known and unknown. Cancers that are not induced by oncogenic viruses may also be treated using the prime-pull methods described herein.

### Bladder Cancer

A recent study suggests that bladder cancer is associated with Kaposi's sarcoma-associated herpesvirus (KSHV), KSHV, also known as human herpesvirus 8. In particular, KSHV DNA was detected in 55 % of patients. (M. Paradžik et al., Tumor Biology, 2014, 35(1), pp 567-572). BCG induces a strong innate and inflammatory response in the bladder mucosa, as measured by the release of cytokines and infiltration with neutrophils and T-cells. In humans, the intravesical application of BCG has been shown to result in the induction of a large number of pro-inflammatory cytokines and chemokines such as IL-2, IL-6, IL-8, IL-12, TNF-α, IFN-γ, and GM-CSF (Kitamura, H., and Tsukamoto, T., Cancers (3): 3055-3072 (2011), and Agarwal, A., et al., Immunopharmacol Immunotoxicol 32(2): 384-356 (2010)). In mice, BCG significantly upregulated genes for T-helper type 1 (Th1) chemokines (Cxcl2, Cxcl9, Cxcl10, Xcl1), increased the expression of Th1/Th2 chemokines (RANTES, Ccl6 and Ccl7) and Th1 polarizing cytokines (IL-1β and TNF-α), and the genes for Fc γ -R1 and iNOS as early as after four weekly instillations. Most of these genes remained highly expressed after 6 weeks (Seow, S.W., et al., Immunology. 2008 Jul;124(3):419-27 (2008)). Additional disclosure on the use of BCG as an antitumor agent can be found, for example, in U.S. Patent No. 5,712,123.

GLA induces strong cytokine/chemokine responses in murine PBMC in vitro, including chemokines for TH1-type T cells (CXCL9 and CXCL10) and mononuclear leukocytes (CCL2, CCL3), as well as MCP-1, TNFa, IFNc and IP-10 in (Lambert, S.L., et al., PLoS One; 7(12) 2012)).

GLA applied mucosally with recombinant protein induces both local and systemic specific antibody responses and specific systemic CD4 cells, especially of the Th17 phenotype, as well as some CD8 cells (Arias, M.A., et al., PLoS One. 2012;7(7):e41144 (2012)).

Tumor infiltrating lymphocytes (TIL) have been identified as an independent prognostic factor in bladder cancer as early as 1978 and CD8 TIL were shown to be predictive of survival in musle-invasive carcinoma (Mostofi, F.K., and Sesterhenn, I., Natl Cancer Inst Monogr; 49: 133-141 (1978); Lopez-Beltran, a. et al., Urol Int; 44: 205-209 (1989); Morita, t., et al., Cancer Immunol Immunother; 32: 191-194 (1990); Ikemoto, S., et al., Br J Urol; 65: 333-338 (1990); Lipponen, P.K., et al., Eur J Cancer; 29: 69-75 (1993); and Sharma, P., et al., Proc Natl Acad Sci U S A., Mar 6;104(10):3967-72 (2007)). Importantly, both CD4 and CD8 cells are essential for BCG-mediated antitumor activity and a predominant Th1 immune response is associated with effective BCG therapy, while a Th2 response seems to be associated with failure (Ratliff, T.L., et al., J Urol; 150:1018 - 23 (1993); Riemensberger, J., et al., Clin Exp Immunol; 127:20 - 6 (2002); Saint, f. et al., J Urol; 167:364 - 7 (2002); de Reijke, t.M., et al. J Urol; 155:477 - 82 (1996); Nadler, R., et al., . Clin Exp Immunol; 131:206 - 16 (2003), Luo, Y., et al., Cytokine; 21:17 - 26 (2003); and Bockholt, N.A., et al., J Urol; 187:2228 - 35 (2012)). Dendritic cells (CD1a+, CD1) were also detected in transitional cell bladder cancer, however, they expressed classical activation markers only at a low levels (Troy, A.J., et al., J Urol., Jun;161(6):1962-7 (1999)).

The cancer-testis antigens (CTA) NY-ESO-1, LAGE-1,MAGE-A1, MAGE-A3, MAGE-A4, MAGE-A10, CT7, CT10, and GAGE, have been found to be expressed by bladder cancers, with at least one CTA being expressed in 77% of samples and two CTAs expressed in 61% of tumors (Sharma, P., et al., Cancer Immun., Dec 18;3:19 (2003); and Sharma, P., et al., Clin Cancer Res., Sep 15;12(18):5442-7 (2006)). Importantly, an investigation of 69 patients with urogenital carcinoma for the presence of intratumoral CD8 T cells, expression of MHC class I antigen and the expression of the NY-ESO-1, showed that patients with advanced cancers and higher numbers of CD8 TILs had better disease-free survival (P < 0.001) and overall survival (P = 0.018) than did patients with similar-staged cancer and fewer CD8 TILs (Sharma, P., et al., Clin Cancer Res., Sep 15;12(18):5442-7 (2006)).

Six of six and one of six patients with NY-ESO-1 expressing bladder cancer mounted CD4 and CD8 responses, respectively, upon intradermal immunization with recombinant NY-ESO-1 adjuvanted with GM-CSF and BCG (Sharma, P., et al., J Immunother., Nov-Dec;31(9):849-57 92008)).

In the mouse, antigen specific CD8 T-cells homing to the bladder and resulting in tumor regression could be induced by both subcutaneous and intranasal immunization with CpG adjuvanted peptides (Domingos-Pereira, S., et al., J Urol., Aug 13, 5347(13)05123-9 (2013)). However, it was also shown in mice that subcutaneous immunization followed by local application of the chemokines CXCL9 and CXCL10 to the mucosa of the genital tract resulted in recruitment of activated CD8 T-cells and establishing a memory T-cell pool in this anatomic location; for this type of immunization the term "prime-pull" was coined by the authors (Shin, H., et al., Nature, Nov 15;491(7424):463-7 (2012)). Thus, it has been demonstrated that both imprinting via route of immunization, as well as local action of chemokines, may result in recruitment of activated CD8 T-cells to the mucosa of the bladder.

### Recombinant Expression Vectors

In one embodiment, recombinant expression vectors are provided that comprise a polynucleotide sequence encoding at least one immunogen that induces an immune response to the immunogen. To obtain efficient transcription and translation of the immunogen, the encoding polynucleotide sequences in each vector should include at least one appropriate expression control sequence (also called a regulatory expression sequence or feature) (e.g., promoter, enhancer, leader), which are described in greater detail herein, that is operatively linked to the encoding polynucleotide sequence(s). These recombinant expression vectors are thus provided for directing expression of the immunogen or for directing co-expression of at least two immunogens in any appropriate host cell that has been transformed, transduced, or transfected with the recombinant expression vector or into which a vector particle containing the recombinant expression vector has been introduced.

The recombinant expression vectors described herein may encode one or more immunogens (i.e., at least one, at least two, at least three immunogens, etc.), which immunogens are described in greater detail elsewhere herein. In particular embodiments, at least one, two, or three, or more immunogens from an oncogenic virus (e.g., EBV, HPV, HBV, HCV, HTLV, and KSHV) may be encoded by a recombinant expression vector. Exemplary immunogens are oncogenic virus antigens, including but not limited to, EBV: EBNA-1, LMP-1, LMP-2A; HPV: E6, E7, E5; HBV: HBx, large, medium, small S antigens; HCV: Core, NS3, Ns5A; HTLV: Tax, HBZ; KSHV: vFLIP, LANA, vGPCR, vIRF-1; Merkel polyoma virus: large T antigen; hCMV: pp65, IE1, US28. In another specific embodiment, a recombinant expression vector described herein may encode at least one, two, three, or more tumor-associated antigens. These tumor associated antigens are described in greater detail herein and may be, for example, a tumor-associated antigen from a bladder cancer antigen, a Merkel cell carcinoma antigen, a renal cell carcinoma antigen, a prostate cancer antigen (e.g., prostatic acid phosphatase, prostate specific antigen, NKX3.1, and prostate specific membrane antigen), a mesothelioma antigen, a pancreatic cancer antigen, a melanoma antigen, a breast cancer antigen, a colorectal cancer antigen, a lung cancer antigen, an ovarian cancer antigen, or any cancer or tumor-associate antigen described herein and in the art.

Recombinant expression vectors may be used for expression of any one or more of the immunogens described herein. In particular embodiments, the recombinant expression vector is delivered to an appropriate cell (for example, an antigen-presenting cell i.e., a cell that displays a peptide/MHC complex on its cell surface, such as a dendritic cell) or tissue (e.g., lymphoid tissue) that will induce the desired immune response (i.e., a specific humoral response (i.e., B cell response) and/or induction of a specific cell-medicated immune response, which may include an immunogen-specific CTL response). The recombinant expression vectors may therefore also include, for example, lymphoid tissue-specific transcriptional regulatory elements (TRE) such as a B lymphocyte, T lymphocyte, or dendritic cell specific TRE. Lymphoid tissue specific TRE are known in the art (see, e.g., Thompson et al. (1992), Mol. Cell. Biol. 12, 1043-1053; Todd et al. (1993), J. Exp. Med. 177, 1663-1674; Penix et al. (1993), J. Exp. Med. 178, 1483-1496).

In a particular embodiment, the recombinant expression vector is plasmid DNA or cosmid DNA. Plasmid DNA or cosmid DNA containing one or more polynucleotides encoding an immunogen as described herein are readily constructed using standard techniques well known in the art. The vector may be typically constructed in a plasmid form that can then be transfected into a packaging or producer cell line. The plasmid generally comprises sequences useful for replication of the plasmid in bacteria. Such plasmids are well known in the art. In addition, vectors that include a prokaryotic origin of replication may also include a gene whose expression confers a detectable or selectable marker such as a drug resistance. Typical bacterial drug resistance products are those that confer resistance to ampicillin or tetracycline. For analysis to confirm that the correct nucleotide sequences are incorporated in plasmids, the plasmid may be replicated in E. coli, purified, and analyzed by restriction endonuclease digestion and/or its nucleotide sequence determined by conventional methods.

In other particular embodiments, the recombinant expression vector is a viral vector. Exemplary recombinant expression viral vectors include a retroviral vector, a lentiviral vector, poxvirus vector, vaccinia virus vector, adenovirus vector, adenovirus-associated virus vector, herpes virus vector, and alpha virus vector. Viral vectors may be live, attenuated, replication conditional or replication deficient, and typically is a non-pathogenic (defective), replication competent viral vector.

By way of example, in a specific embodiment, when the viral vector comprises a vaccinia virus vector, the polynucleotide encoding an immunogen of interest may be inserted into a non-essential site of a vaccinia viral vector genome. Such non- essential sites are described, for example, in Perkus et al., Virology 152:285 (1986); Hruby et al., Proc. Natl. Acad. Sci. USA 80:3411 (1983); Weir et al., J. Virol. 46:530 (1983)). Suitable promoters for use with vaccinia viruses include but are not limited to P7.5 (see, e.g., Cochran et al., J. Virol. 54:30 (1985); P11 (see, e.g., Bertholet, et al., Proc. Natl. Acad. Sci. USA 82:2096 (1985)); and CAE-1 (see, e.g., Patel et al., Proc. Natl. Acad. Sci. USA 85:9431 (1988)). Highly attenuated strains of vaccinia are more acceptable for use in humans and include Lister, NYVAC, which contains specific genome deletions (see, e.g., Guerra et al., J. Virol. 80:985-98 (2006); Tartaglia et al., AIDS Research and Human Retroviruses 8:1445-47 (1992)), or MVA (see, e.g., Gheradi et al., J. Gen. Virol. 86:2925-36 (2005); Mayr et al., Infection 3:6-14 (1975)). See also Hu et al. (J. Virol. 75:10300-308 (2001), describing use of a Yaba-Like disease virus as a vector for cancer therapy); U.S. Patent Nos. 5,698,530 and 6,998,252. See also, e.g., U.S. Patent No. 5,443,964. See also U.S. Patent Nos. 7,247,615 and 7,368,116.

In certain embodiments, an adenovirus vector or adenovirus-associated virus vector may be used for expressing an immunogen of interest. Several adenovirus vector systems and methods for administering the vectors have been described (see, e.g., Molin et al., J. Virol. 72:8358-61 (1998); Narumi et al., Am J. Respir. Cell Mol. Biol. 19:936-41 (1998); Mercier et al., Proc. Natl. Acad. Sci. USA 101:6188-93 (2004); U.S. Patent Nos. 6,143,290; 6,596,535; 6,855,317; 6,936,257; 7,125,717; 7,378,087; 7,550,296).

Retroviral vectors may include those based upon murine leukemia virus (MuLV), gibbon ape leukemia virus (GaLV), ecotropic retroviruses, simian immunodeficiency virus (SIV), human immunodeficiency virus (HIV), and combinations (see, e.g., Buchscher et al., J. Virol. 66:2731-39 (1992); Johann et al., J. Virol. 66:1635-40 (1992); Sommerfelt et al., Virology 176:58-59 (1990); Wilson et al., J. Virol. 63:2374-78 (1989); Miller et al., J. Virol. 65:2220-24 (1991); Miller et al., Mol. Cell Biol. 10:4239 (1990); Kolberg, NIH Res. 4:43 1992; Cornetta et al., Hum. Gene Ther. 2:215 (1991)).

In a more specific embodiment, the recombinant expression vector is a viral vector. In certain embodiments, the viral vector is a retroviral vector and in another embodiment, the retroviral vector is a lentiviral vector. As would be understood by the skilled person, a viral vector, such as a lentiviral vector, generally refers to a viral vector particle that comprises the viral vector genome. For example, a lentiviral vector particle may comprise a lentiviral vector genome. With resepect to lentiviral vectors, the vector genome can be derived from any of a large number of suitable, available lentiviral genome based vectors, including those identified for human gene therapy applications (see, e.g., Pfeifer et al., Annu. Rev. Genomics Hum. Genet. 2:177-211 (2001)). Suitable lentiviral vector genomes include those based on Human Immunodeficiency Virus (HIV-1), HIV-2, feline immunodeficiency virus (FIV), equine infectious anemia virus, Simian Immunodeficiency Virus (SIV), and maedi/visna virus. A desirable characteristic of lentiviruses is that they are able to infect both dividing and non-dividing cells, although target cells need not be dividing cells or be stimulated to divide. Generally, the genome and envelope glycoproteins will be based on different viruses, such that the resulting viral vector particle is pseudotyped. Safety features of the viral vector are desirably incorporated. Safety features include self-inactivating LTR and integration deficiency as described in more detail herein. In certain embodiments integration deficiency may be conferred by elements of the vector genome but may also derive from elements of the packaging system (e.g., a non functional integrase protein that may not be part of the vector genome but supplied in trans). Exemplary vectors contain a packaging signal (psi), a Rev-responsive element (RRE), splice donor, splice acceptor, optionally a central poly-purine tract (cPPT), and WPRE element. In certain exemplary embodiments, the viral vector genome comprises sequences from a lentivirus genome, such as the HIV-1 genome or the SIV genome. The viral genome construct may comprise sequences from the 5' and 3' LTRs of a lentivirus, and in particular may comprise the R and U5 sequences from the 5' LTR of a lentivirus and an inactivated or self-inactivating 3' LTR from a lentivirus. The LTR sequences may be LTR sequences from any lentivirus from any species. For example, they may be LTR sequences from HIV, SIV, FIV or BIV. Typically, the LTR sequences are HIV LTR sequences.

The vector genome may comprise an inactivated or self-inactivating 3' LTR (see, e.g., Zufferey et al., J. Virol. 72: 9873, 1998; Miyoshi et al., J. Virol. 72:8150, 1998; both of which are incorporated in their entirety). A self-inactivating vector generally has a deletion of the enhancer and promoter sequences from the 3' long terminal repeat (LTR), which is copied over into the 5' LTR during vector integration. In one instance, the U3 element of the 3' LTR contains a deletion of its enhancer sequence, the TATA box, Sp1 and NF-kappa B sites. As a result of the self-inactivating 3' LTR, the provirus that is generated following entry and reverse transcription will comprise an inactivated 5' LTR. The rationale is to improve safety by reducing the risk of mobilization of the vector genome and the influence of the LTR on nearby cellular promoters. The self-inactivating 3' LTR may be constructed by any method known in the art.

Optionally, the U3 sequence from the lentiviral 5' LTR may be replaced with a promoter sequence in the viral construct, such as a heterologous promoter sequence. This can increase the titer of virus recovered from the packaging cell line. An enhancer sequence may also be included. Any enhancer/promoter combination that increases expression of the viral RNA genome in the packaging cell line may be used. In one example, the CMV enhancer/promoter sequence is used (see, e.g., U.S. Patent Nos. 5,385,839 and 5,168,062).

In certain embodiments, the risk of insertional mutagenesis is minimized by constructing the lentiviral vector to be integration defective. A variety of approaches can be pursued to produce a non-integrating vector genome. These approaches entail engineering a mutation(s) into the integrase enzyme component of the pol gene, such that it encodes a protein with an inactive integrase. The vector genome itself can be modified to prevent integration by, for example, mutating or deleting one or both attachment sites, or making the 3' LTR-proximal polypurine tract (PPT) non-functional through deletion or modification. In addition, non-genetic approaches are available; these include pharmacological agents that inhibit one or more functions of integrase. The approaches are not mutually exclusive, that is, more than one of them can be used at a time. For example, both the integrase and attachment sites can be non-functional, or the integrase and PPT site can be non-functional, or the attachment sites and PPT site can be non-functional, or all of them can be non-functional.

Integrase is involved in cleavage of viral double-stranded blunt-ended DNA and joining the ends to 5'-phosphates in the two strands of a chromosomal target site. Integrase has three functional domains: N-terminal domain, which contains a zinc-binding motif (HHCC); the central domain core, which contains the catalytic core and a conserved DD35E motif (D64, D116, E152 in HIV-1); and a C-terminal domain, which has DNA binding properties. Point mutations introduced into integrase are sufficient to disrupt normal function. Many integrase mutations have been constructed and characterized (see, e.g., Philpott and Thrasher, Human Gene Therapy 18:483, 2007; Apolonia, Thesis submitted to University College London, April 2009, pp, 82-97; Engelman et al., J. Virol. 69: 2729, 1995; Nightingale et al., Mol. Therapy, 13: 1121, 2006). The sequence encoding the integrase protein can be deleted or mutated to render the protein inactive, preferably without significantly impairing reverse transcriptase activity or nuclear targeting, thereby only preventing integration of the provirus into the target cell genome. Acceptable mutations can reduce integrase catalysis, strand transfer, binding to att sites, binding to host chromosomal DNA, and other functions. For example, a single aspartic acid to asparagine substitution at residue 35 of HIV or SIV integrase completely abolishes viral DNA integration. Deletions of integrase will generally be confined to the C-terminal domain. Deletion of coding sequence for residues 235-288 result in a useful non-functional integrase (see, e.g., Engelman et al., J. Virol. 69:2729, 1995). As further examples, mutations can be generated, for example, Asp64 (residue numbers are given for HIV-1, corresponding residue numbers for integrase from other lentiviruses or retroviruses can be readily determined by one of ordinary skill) (e.g., D64E, D64V), Asp116 (e.g., D116N), Asn120 (e.g., N120K), Glu152, Gln148 (e.g., Q148A), Lys156, Lys159, Trp235 (e.g., W235E), Lys264 (e.g., K264R), Lys266 (e.g., K266R), Lys273 (e.g., K273R). Other mutations can be constructed and tested for integration, transgene expression, and any other desirable parameter. Assays for these functions are well known. Mutations can be generated by any of a variety of techniques, including site-directed mutagenesis and chemical synthesis of nucleic acid sequence. One mutation may be made or more than one of these mutations can be present in integrase. For example, an integrase may have mutations at two amino acids, three amino acids, four amino acids, and so on.

Alternatively or in combination with the use of integrase mutant(s), the attachment sites (att) in U3 and U5 can also be mutated. Integrase binds to these sites and the 3'-terminal dinucleotide is cleaved at both ends of the vector genome. A CA dinucleotide is located at the recessed 3' end; the CA is required for processing, mutation of the nucleotides blocks integration into the host chromosome. The A of the CA dinucleotide is the most critical nucleotide for integration, and mutations at both ends of the genome will give the best results (see, e.g., Brown et al., J. Virol. 73:9011 (1999)). In one exemplification, the CA at each end is changed to TG. In other exemplifications, the CA at each end is changed to TG at one end and GT at the other end. In other exemplifications, the CA at each end is deleted; in other exemplifications, the A of the CA is deleted at each end.

Integration can also be inhibited by mutation or deletion of polypurine tract (PPT) (see, e.g., WO 2009/076524), located proximally to the 3' LTR. The PPT is a polypurine sequence of about 15 nucleotides that can serve as a primer binding site for plus-strand DNA synthesis. In this instance, mutations or deletions of PPT targets the reverse transcription process. Without wishing to be held to a particular mechanism, by mutating or deleting PPT, production of linear DNA is radically reduced, and essentially only 1-LTR DNA circles are produced. Integration requires a linear double-stranded DNA vector genome, and integration is essentially eliminated without it. As stated herein, a PPT can be made non-functional by mutation or by deletion. Typically, the entire about 15 nt PPT is deleted, although in some embodiments, shorter deletions of 14 nt, 13, nt, 12 nt, 11 nt, 10 nt, 9 nt, 8 nt, 7 nt, 6 nt, 5 nt, 4 nt, 3 nt and 2 nt may be made. When mutations are made, typically multiple mutations are made, especially in the 5' half of the PPT (see, e.g., McWilliams et al., J. Virol. 77:11150, 2003), although single and double mutations in the first four bases still reduce transcription. Mutations made at the 3' end of PPT generally have a more dramatic effect (see, e.g., Powell et al., J. Virol. 70:5288, 1996).

These different approaches to make a vector genome non-integrating can be used individually or in combination. Using more than one approach may be used to build a fail-safe vector through redundant mechanisms. Thus, PPT mutations or deletions can be combined with att site mutations or deletions or with Integrase mutations or PPT mutations or deletions can be combined with both att site mutations or deletions and Integrase mutations. Similarly, att site mutations or deletions and Integrase mutations may be combined with each other or with PPT mutations or deletions.

As described herein, lentiviral vector constructs contain a promoter for expression in mammalian cells. Promoters, which are discussed in greater detail herein, include, for example, the human ubiquitin C promoter (UbiC), the cytomegalovirus immediate early promoter (CMV), and the Rous sarcoma virus (RSV) promoter. The U3 region may comprise a PPT (polypurine tract) sequence immediately upstream. In certain specific embodiments, any one of at least three different U3 regions (at the 3' end) may be included in the lentiviral vector (see SEQ ID NOS:21-23). The constructs contain deletions in the U3 regions. The SIN construct has a deletion of about 130 nucleotides in the U3 (see, e.g., Miyoshi, et al. J. Virol. 72: 8150, 1998; Yu et al., Proc. Natl. Acad. Sci. USA 83: 3194, 1986), which removes the TATA box, thereby abolishing LTR promoter activity. The deletions in constructs 703 and 704 increase expression from lentivirus vectors (see, e.g., Bayer et al., Mol. Therapy 16: 1968, 2008). In addition, construct 704 contains a deletion of the 3' PPT, which decreases integration of the vector (see, e.g., WO 2009/076524). See also U.S. Patent Application No. 12/842,609 and International Patent Application No. PCT/US 10/042870.

### Regulatory Expression Sequences

As described herein, the recombinant expression vector comprises at least one regulatory expression sequence. In certain embodiments, when the recombinant expression vector comprises a viral vector genome, expression of the at least one immunogen is desired in particular target cells. Typically, for example, in a lentiviral vector the polynucleotide sequence encoding the immunogen is located between the 5' LTR and 3' LTR sequences. Further, the encoding nucleotide sequence(s) is preferably operatively linked in a functional relationship with other genetic or regulatory sequences or features, for example transcription regulatory sequences including promoters or enhancers, that regulate expression of the immunogen in a particular manner. In certain instances, the useful transcriptional regulatory sequences are those that are highly regulated with respect to activity, both temporally and spatially. Expression control elements that may be used for regulating the expression of the encoded polypeptides are known in the art and include, but are not limited to, inducible promoters, constitutive promoters, secretion signals, enhancers, and other regulatory sequences.

The polynucleotide encoding the immunogen and any other expressible sequence is typically in a functional relationship with internal promoter/enhancer regulatory sequences. With respect to lentiviral vector constructs, an "internal" promoter/enhancer is one that is located between the 5' LTR and the 3' LTR sequences in the viral vector and is operatively linked to the encoding polynucleotide sequence of interest. The internal promoter/enhancer may be any promoter, enhancer or promoter/enhancer combination known to increase expression of a gene with which it is in a functional relationship. A "functional relationship" and "operatively linked" mean, without limitation, that the sequence is in the correct location and orientation with respect to the promoter and/or enhancer such that the sequence of interest will be expressed when the promoter and/or enhancer is contacted with the appropriate molecules.

The choice of an internal promoter/enhancer is based on the desired expression pattern of the immunogen and the specific properties of known promoters/enhancers. Thus, the internal promoter may be constitutively active. Non-limiting examples of constitutive promoters that may be used include the promoter for ubiquitin (see, e.g., U.S. Patent No. 5510474; WO 98/32869); CMV (see, e.g., Thomsen et al., Proc. Natl. Acad. Sci. USA 81:659, 1984; U.S. Patent No. 5168062); beta-actin (Gunning et al. 1989 Proc. Natl. Acad. Sci. USA 84:4831-4835); and pgk (see, for example, Adra et al. 1987 Gene 60:65-74; Singer-Sam et al. 1984 Gene 32:409-417; and Dobson et al. 1982 Nucleic Acids Res. 10:2635-2637).

Alternatively, the promoter may be a tissue specific promoter. In some embodiments, the promoter is a target cell-specific promoter. For example, the promoter can be from any product expressed by dendritic cells, including CD11c, CD103, TLRs, DC-SIGN, BDCA-3, DEC-205, DCIR2, mannose receptor, Dectin-1, Clec9A, MHC class II. In addition, promoters may be selected to allow for inducible expression of the immunogen. A number of systems for inducible expression are known in the art, including the tetracycline responsive system, the lac operator-repressor system, as well as promoters responsive to a variety of environmental or physiological changes, including heat shock, metal ions, such as metallothionein promoter, interferons, hypoxia, steroids, such as progesterone or glucocorticoid receptor promoter, radiation, such as VEGF promoter. A combination of promoters may also be used to obtain the desired expression of each of the immunogen-encoding polynucleotide sequences. The artisan of ordinary skill will be able to select a promoter based on the desired expression pattern of the polynucleotide sequence in the organism or the target cell of interest.

A recombinant expression vector, including a viral vector genome, may comprise at least one RNA Polymerase II or III responsive promoter. This promoter can be operatively linked to the polynucleotide sequence of interest and can also be linked to a termination sequence. In addition, more than one RNA Polymerase II or III promoter may be incorporated. RNA polymerase II and III promoters are well known to persons of skill in the art. A suitable range of RNA polymerase III promoters can be found, for example, in Paule and White, Nucleic Acids Res., Vol. 28, pp 1283-1298 (2000). RNA polymerase II or III promoters also include any synthetic or engineered DNA fragment that can direct RNA polymerase II or III to transcribe downstream RNA coding sequences. Further, the RNA polymerase II or III (Pol II or III) promoter or promoters used as part of the viral vector genome can be inducible. Any suitable inducible Pol II or III promoter can be used with the methods described herein. Particularly suited Pol II or III promoters include the tetracycline responsive promoters provided in Ohkawa and Taira, Human Gene Therapy, Vol. 11, pp 577-585 (2000) and in Meissner et al., Nucleic Acids Research, Vol. 29, pp 1672-1682 (2001).

An internal enhancer may also be present in the recombinant expression vector, including a viral vector genome, to increase expression of the polynucleotide sequence of interest. For example, the CMV enhancer (see, e.g., Boshart et al., Cell 41:521, 1985) may be used. Many enhancers in viral genomes, such as HIV, CMV, and in mammalian genomes have been identified and characterized (see, e.g., publically available databases such as GenBank). An enhancer can be used in combination with a heterologous promoter. One of ordinary skill in the art will be able to select the appropriate enhancer based on the desired expression pattern.

When targeting delivery of a recombinant expression vector, including a viral vector genome, to a particular target cell, the vector genome will usually contain a promoter that is recognized by the target cell and that is operatively linked to the sequence of interest, viral components (when the vector is a viral vector), and other sequences discussed herein. A promoter is an expression control element formed by a nucleic acid sequence that permits binding of RNA polymerase and transcription to occur. Promoters may be inducible, constitutive, temporally active or tissue specific. The activity of inducible promoters is induced by the presence or absence of biotic or abiotic factors. Inducible promoters can be a useful tool in genetic engineering because the expression of genes to which they are operatively linked can be turned on or off at certain stages of development of an organism, its manufacture, or in a particular tissue. Inducible promoters can be grouped as chemically-regulated promoters, and physically-regulated promoters. Typical chemically-regulated promoters include, not are not limited to, alcohol-regulated promoters (e.g., alcohol dehydrogenase I (alcA) gene promoter), tetracycline-regulated promoters (e.g., tetracycline-responsive promoter), steroid-regulated promoter (e.g., rat glucocorticoid receptor (GR)-based promoter, human estrogen receptor (ER)-based promoter, moth ecdysone receptor-based promoter, and the promoters based on the steroid/retinoid/thyroid receptor superfamily), metal-regulated promoters (e.g., metallothionein gene-based promoters), and pathogenesis-related promoters (e.g., Arabidopsis and maize pathogen-related (PR) protein-based promoters). Typical physically-regulated promoters include, but are not limited to, temperature-regulated promoters (e.g., heat shock promoters), and light-regulated promoters (e.g., soybean SSU promoter). Other exemplary promoters are described elsewhere, for example, in patents and published patent applications that can be identified by searching the U.S. Patent and Trademark Office databases.

One of skill in the art will be able to select an appropriate promoter based on the specific circumstances. Many different promoters are well known in the art, as are methods for operatively linking the promoter to the polynucleotide sequence to be expressed. Both native promoter sequences and many heterologous promoters may be used to direct expression in the packaging cell and target cell. Heterologous promoters are typically used because they generally permit greater transcription and higher yields of the desired protein as compared to the native promoter.

The promoter may be obtained, for example, from the genomes of viruses such as polyoma virus, fowlpox virus, adenovirus, bovine papilloma virus, avian sarcoma virus, cytomegalovirus, a retrovirus, hepatitis-B virus and Simian Virus 40 (SV40). The promoter may also be, for example, a heterologous mammalian promoter, for example, the actin promoter or an immunoglobulin promoter, a heat-shock promoter, or the promoter normally associated with the native sequence, provided such promoters are compatible with the target cell. In one embodiment, the promoter is the naturally occurring viral promoter in a viral expression system. In some embodiments, the promoter is a dendritic cell-specific promoter. The dendritic cell-specific promoter can be, for example, CD11c promoter.

Transcription may be increased by inserting an enhancer sequence into the vector(s). Enhancers are typically cis-acting elements of DNA, usually about 10 to 300 bp in length, that act on a promoter to increase its transcription. Many enhancer sequences are now known from mammalian genes (globin, elastase, albumin, alpha-fetoprotein, and insulin) and from eukaryotic cell viruses. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the antigen-specific polynucleotide sequence, but is preferably located at a site 5' from the promoter.

Expression vectors may also contain sequences necessary for the termination of transcription and for stabilizing the mRNA. These sequences are often found in the 5' and, occasionally 3', untranslated regions of eukaryotic or viral DNAs or cDNAs and are well known in the art.

A recombinant expression construction, including a viral vector genome, may also contain additional genetic elements. The types of elements that may be included in the construct are not limited in any way and may be chosen to achieve a particular result. For example, a signal that facilitates nuclear entry of the recombinant expression vector or viral genome in the target cell may be included. An example of such a signal is the HIV-1 flap signal. Additional regulatory sequences may be included that facilitate the characterization of the provirus integration site in the target cell. For example, a tRNA amber suppressor sequence may be included in the construct. An insulator sequence, for example from chicken β-globin, may also be included in the viral genome construct. This element reduces the chance of silencing an integrated provirus in the target cell due to methylation and heterochromatinization effects. In addition, the insulator may shield the internal enhancer, promoter and exogenous polynucleotide sequences from positive or negative positional effects from surrounding DNA at the integration site on the chromosome. In addition, the recombinant construct, including the vector genome, may contain one or more genetic elements designed to enhance expression of the gene of interest. For example, a woodchuck hepatitis virus responsive element (WRE) may be placed into the construct (see, e.g., Zufferey et al. 1999. J. Virol. 74:3668-3681; Deglon et al., 2000. Hum. Gene Ther. 11:179-190).

When the recombinant expression vector is a viral vector genome, the viral vector genome is typically constructed in a plasmid form that may be transfected into a packaging or producer cell line for production of the viral vector genome construct. The plasmid generally comprises sequences useful for replication of the plasmid in bacteria. Such plasmids are well known in the art. In addition, vectors that include a prokaryotic origin of replication may also include a gene whose expression confers a detectable or selectable marker such as a drug resistance. Typical bacterial drug resistance products are those that confer resistance to ampicillin or tetracycline.

In certain configurations, recombinant expression vectors contain polynucleotide sequences that encode dendritic cell (DC) maturation / stimulatory factors. Exemplary stimulatory molecules include GM-CSF, IL-2, IL-4, IL-6, IL-7, IL-15, IL-21, IL-23, TNFα, B7.1, B7.2, 4-1BB, CD40 ligand (CD40L), drug-inducible CD40 (iCD40), and the like. These polynucleotides are typically under the control of one or more regulatory elements that direct the expression of the coding sequences in dendritic cells. Maturation of dendritic cells contributes to successful vaccination (see, e.g., Banchereau et al., Nat. Rev. Immunol. 5:296-306 (2005); Schuler et al., Curr. Opin. Immunol. 15:138-147 (2003); Figdor et al., Nat. Med. 10:475-480 (2004)). Maturation can transform DCs from cells actively involved in antigen capture into cells specialized for T cell priming. For example, engagement of CD40 by CD40L on CD4-helper T cells is a critical signal for DC maturation, resulting in potent activation of CD8+ T cells. Such stimulatory molecules are also referred to as maturation factors or maturation stimulatory factors.

Immune checkpoints represent significant barriers to activation of functional cellular immunity in cancer, and antagonistic antibodies specific for inhibitory ligands on T cells including CTLA4 and programmed death-1 (PD-1) are examples of targeted agents being evaluated in the clinics. A significant tolerance mechanism in chronic infections and cancer is the functional exhaustion of antigen-specific T cells that express high levels of PD-1. As the potency of therapeutic immunization has been shown to be significantly enhanced by combination with immune checkpoint control, as a non-limiting example, it can be appreciated by those of ordinary skill in the art that an alternative approach to inhibiting immune checkpoint is to inhibit the expression of programmed death (PD) ligands one and two (PD-L1/L2). One way to accomplish inhibition is by the expression of RNA molecules such as those described herein, which repress the expression of PD-L1/L2 in the DCs transduced with a viral vector genome, such as the lentivirus vector genome, encoding one or more of the relevant molecules. Maturation of DCs or expression of particular elements such as immune checkpoints, for example PD-1 ligands, can be characterized by flow cytometry analysis of up-regulation of surface marker such as MHC II, and by profiling expressed chemokines and cytokines, for example, by performing techniques and methods described herein.

In another embodiment, for retroviral vectors described herein, such as lentiviral vectors described herein, the lentiviral vector may comprise a nucleic acid which encodes an immunogen and the same or a separate lentiviral vector may comprise a nucleic acid which encodes a single chain antibody (e.g., scFV) which binds to and blocks the activity of a checkpoint inhibitor. In another embodiment, the lentiviral vectors described herein which target dendritic cells may comprise a nucleic acid which encodes an immunogen and the same or a separate lentiviral vector may comprise a nucleic acid which downregulates or otherwise inhibits expression of an immune checkpoint molecule expressed by the dendritic cell.

Illustrative immune checkpoint molecules that may be targeted for blocking or inhibition include, but are not limited to, CTLA-4, 4-1BB (CD137), 4-1BBL (CD137L), PDL1, PDL2, PD1, B7-H3, B7-H4, BTLA, HVEM, TIM3, GAL9, LAG3, TIM3, B7H3, B7H4, VISTA, KIR, 2B4 (belongs to the CD2 family of molecules and is expressed on all NK, γδ, and memory CD8⁺ (αβ) T cells), CD160 (also referred to as BY55) and CGEN-15049. Immune checkpoint inhibitors include antibodies, or antigen binding fragments thereof, or other binding proteins, that bind to and block or inhibit the activity of one or more of CTLA-4, PDL1, PDL2, PD1, B7-H3, B7-H4, BTLA, HVEM, TIM3, GAL9, LAG3, TIM3, B7H3, B7H4, VISTA, KIR, 2B4, CD160 and CGEN-15049. Illustrative immune checkpoint inhibitors include Tremelimumab (CTLA-4 blocking antibody), anti-OX40, PD-L1 monoclonal Antibody (Anti-B7-H1; MEDI4736), ipilimumab, MK-3475 (PD-1 blocker), Nivolumamb (anti-PD1 antibody), CT-011 (anti-PD1 antibody), BY55 monoclonal antibody, AMP224 (anti-PDLl antibody), BMS-936559 (anti-PDLl antibody), MPLDL3280A (anti-PDL1 antibody), MSB0010718C (anti-PDLl antibody) and Yervoy/ipilimumab (anti-CTLA-4 checkpoint inhibitor).

A sequence encoding a detectable product, usually a protein, can be included to allow for identification of cells that are expressing the desired immunogen. For example, a fluorescent marker protein, such as green fluorescent protein (GFP), is incorporated into the recombinant expression construct along with a polynucleotide sequence of interest (i.e., encoding an at least one immunogen). In other instances, the protein may be detectable by an antibody, or the protein may be an enzyme that acts on a substrate to yield a detectable product, or may be a protein product that allows selection of a transfected or transduced target cell, for example confers drug resistance, such as hygromycin resistance. Typical selection genes encode proteins that confer resistance to antibiotics or other toxins suitable for use in eukaryotic cells, for example, neomycin, methotrexate, blasticidine, among others known in the art, or complement auxotrophic deficiencies, or supply critical nutrients withheld from the media. The selectable marker can optionally be present on a separate plasmid and introduced by co-transfection.

With respect to vector particles described herein, one or more multicistronic expression units may be used that include two or more of a polynucleotide sequence encoding an immunogen, and a sequence encoding an envelope molecule as described herein or one or more DC maturation factors necessary for production of the desired vector particle in packaging cells. The use of multicistronic vectors reduces the total number of nucleic acid molecules required and thus may avoid the possible difficulties associated with coordinating expression from multiple vector genomes. In a multicistronic vector the various elements to be expressed are operatively linked to one or more promoters (and other expression control elements as necessary). In some configurations, a multicistronic vector comprises a sequence encoding an at least one immunogen (i.e., one or more) of interest, a sequence encoding a reporter product, and a sequence encoding one or more vector particle components. In certain embodiments in which the recombinant construct comprises a polynucleotide that encodes an immunogen, the construct optionally encodes a DC maturation factor. In certain embodiments, the recombinant construct comprises a polynucleotide that encodes an oncogenic viral antigen and a tumor associated antigen. In certain other embodiments, a multicistronic vector comprises a polynucleotide sequences that encode each of an immunogen, a DC maturation factor, and optionally viral components when the expression vector is a viral expression vector.

Each component to be expressed in a multicistronic expression vector may be separated, for example, by an internal ribosome entry site (IRES) element or a viral 2A element, to allow for separate expression of the various proteins from the same promoter. IRES elements and 2A elements are known in the art (see, e.g., U.S. Pat. No. 4,937,190; de Felipe et al. 2004. Traffic 5: 616-626). In one embodiment, oligonucleotides such as furin cleavage site sequences (RAKR) (see, e.g., Fang et al. 2005 Nat. Biotech. 23: 584-590) linked with 2A-like sequences from foot-and-mouth diseases virus (FMDV); equine rhinitis A virus (ERAV); and thosea asigna virus (TaV) (see, e.g., Szymczak et al. 2004 Nat. Biotechnol. 22: 589-594) are used to separate genetic elements in a multicistronic vector. The efficacy of a particular multicistronic vector can readily be tested by detecting expression of each of the genes using standard protocols.

In a specific exemplification, a viral vector genome comprises: a cytomegalovirus (CMV) enhancer/promoter sequence; the R and U5 sequences from the HIV 5' LTR; a packaging sequence (ψ); optionally the HIV-1 flap signal; an internal enhancer; an internal promoter; a gene of interest; the woodchuck hepatitis virus responsive element; a tRNA amber suppressor sequence; a U3 element with a deletion of its enhancer sequence; the chicken β-globin insulator; and the R and U5 sequences of the 3' HIV LTR. In some exemplifications, the vector genome comprises an intact lentiviral 5' LTR and a self-inactivating 3' LTR (see, e.g., Iwakuma et al. Virology 15:120, 1999).

Construction of the vector genome can be accomplished using any suitable genetic engineering techniques known in the art, including, without limitation, the standard techniques of restriction endonuclease digestion, ligation, transformation, plasmid purification, and DNA sequencing, for example as described in Sambrook et al. (1989 and 2001 editions; Molecular Cloning: A Laboratory Manual, Cold Spring Harbor Laboratory Press, NY); Coffin et al. (Retroviruses. Cold Spring Harbor Laboratory Press, N.Y. (1997)); and "RNA Viruses: A Practical Approach" (Alan J. Cann, Ed., Oxford University Press, (2000).

Vectors constructed for transient expression in mammalian cells may also be used. Transient expression involves the use of an expression vector that is able to replicate efficiently in a host cell, such that the host cell accumulates many copies of the expression vector and, in turn, synthesizes high levels of a the polypeptide encoded by the immunogen-specific polynucleotide in the expression vector. See Sambrook et al., supra, pp. 16.17-16.22, 1989. Other vectors and methods suitable for adaptation to the expression of polypeptides are well known in the art and are readily adapted to the specific circumstances.

By using the teachings provided herein and the knowledge in the art, a person skilled in the art will recognize that the efficacy of a particular expression system can be tested by transfecting packaging cells with a vector comprising a polynucleotide sequence encoding a reporter protein and measuring the expression using a suitable technique, for example, measuring fluorescence from a green fluorescent protein conjugate. Other suitable reporter genes are well known in the art.

A recombinant expression vector that comprises a polynucleotide sequence that encodes an immunogen may be used for production of the immunogen. Recombinant expression vectors include at least one regulatory expression sequence, such as a promoter or enhancer that is operatively linked to the polynucleotide encoding the immunogen. Each of the expression vectors may be used to transform, transducer, or transfect an appropriate host cell for recombinant production of a respective immunogen. Suitable host cells for production of the immunogen include prokaryotes, yeast and higher eukaryotic cells (e.g., CHO and COS). The immunogen may each be isolated from the respective host cell or host cell culture using any one of a variety of isolation methods (e.g., filtration, diafiltration, chromatography (including affinity chromatography, high pressure liquid chromatography), and preparative electrophoresis) known and routinely practiced in the protein art. In certain embodiments, as described herein, the isolated immunogen may then be formulated with a pharmaceutically suitable excipient to provide an immunogenic composition.

Particular methods for producing polypeptides recombinantly are generally well known and routinely used. For example, molecular biology procedures are described by Sambrook et al. (Molecular Cloning, A Laboratory Manual, 2nd ed., Cold Spring Harbor Laboratory, New York, 1989; see also Sambrook et al., 3rd ed., Cold Spring Harbor Laboratory, New York, (2001)). DNA sequencing can be performed as described in Sanger et al. (Proc. Natl. Acad. Sci. USA 74:5463 (1977)) and the Amersham International plc sequencing handbook and including improvements thereto.

### Vector Particles

In another embodiment, vector particles are provided. A vector particle comprises any one of the recombinant expression vectors described herein that comprise a polynucleotide sequence encoding at least one immunogen. In certain other embodiments, a vector particle comprises a recombinant expression system that comprises one recombinant expression vector (also called a first recombinant expression vector) comprising a polynucleotide sequence encoding at least one immunogen that induces a specific immune response. Also provided herein are methods for delivering a polynucleotide encoding at least one immunogen (as described herein) to a target cell. In particular embodiments, the target cell is an immune cell that is an antigen-presenting cell; in more specific embodiments and as described herein, the target cell is a dendritic cell. Such methods comprise contacting (i.e., permitting interaction) of the target cell with a vehicle that delivers the polynucleotide. In particular embodiments, described in detail herein, methods for delivering the polynucleotide comprise contacting the cell by administering to a subject a vector particle that comprises a recombinant expression vector that contains a polynucleotide sequence that encodes the immunogen. The vector particles, recombinant expression vectors, polynucleotides, and immunogens are discussed in greater detail herein.

Dendritic cells (DCs) are essential antigen presenting cells for the initiation and control of immune responses. DCs can develop along two pathways: one pathway is independent of monocytes and the second pathway is derived from monocytes (Mo-DCs). Blood monocytes, upon culture with GM-CSF and IL-4 acquire a dendritic morphology and strong capacities to initiate adaptive immunity (see, e.g., Bender, et al., J. Immunol. Methods 196(2), 121 (1996); Sallusto et al., J. Exp. Med. 179(4), 1109 (1994), including in vivo in humans (see, e.g., Dhodapkar, et al., J. Clin. Invest 104(2), 173 (1999); Schuler-Thurner, et al., J. Immunol. 165(6), 3492 (2000)). A more effective immunogen-specific T cell responses may be achieved by using a vector particle vaccine, in particular a lentiviral vector particle system that efficiently delivers immunogens directly to Mo-DCs in vivo, without the need for ex vivo cellular manipulation. Human Mo-DCs express high levels of two C-type lectin receptors, mannose receptor (MMR) and DC-specific intercellular adhesion molecule-3-grabbing non-integrin (DC-SIGN). As described in greater detail herein, expression of immunogens may be targeted to Mo-DCs using a recombinant lentiviral vector engineered to target DC-SIGN.

A DC-SIGN-targeting envelope, SVGmu, consisting of an engineered Sindbis virus (SIN) glycoprotein that selectively binds DC-SIGN has been modified as described (see description herein and U.S. Patent Application No. 12/842,609 and International Patent Application No. PCT/US10/042870). The lentiviral vector induced highly functional CD8 T cell immune responses after a single immunization in mice (see, e.g., Dai, et al., Proc. Natl. Acad. Sci. U.S.A (2009); Yang, et al., Nat. Biotechnol. 26(3), 326 (2008)). This prototype has been significantly advanced by two major modifications. This lentiviral vector described herein comprises a glycoprotein envelope (termed SINvar1) based on native SIN, an arbovirus known to infect dermal DCs via the DC-SIGN receptor (see, e.g., Gardner, et al., J. Virol. 74(24), 11849 (2000); Klimstra, et al., J. Virol. 77(22), 12022 (2003)) that is modified to prevent binding to ubiquitous heparan sulfate receptors (see, e.g., Klimstra et al., J. Virol. 72(9), 7357 (1998)). The SINvar1 envelope confers both increased productivity and in vivo function compared with the parental SVGmu envelope. The vector is also redundantly integration incompetent through the combination of a mutant Integrase (polD64V), rendering it non-functional (see, e.g., Apolonia, et al., Mol. Ther. 15(11), 1947 (2007)), and a vector backbone deleted of the U3 region of the LTR (up to att) and the 3' LTR poly-purine tract (PPT). Thus, in addition to a disabled Integrase, the composition of the vector backbone prevents transcription of the full-length vector genome (self-inactivating mutation) resulting in single-LTR reverse transcribed episomal dsDNA circles in the infected DC, which are not a template for chromosomal integration (see, e.g., Bayer, et al., Mol. Ther. 16(12), 1968 (2008); Breckpot et al., J. Virol. (2010); Ma et al., Mol. Ther. 10(1): 139 (2004)). Approximately 75% of the parental HIV genome has been removed from DC-NILV, including all of the regulatory and accessory proteins except for Rev. After a single injection, DC-NILV induces highly robust tumor antigen-specific CD8 T cell response. The potency of lentivector vaccination is dependent at least in part on engagement of TLR3 and TLR7 pattern recognition receptors (see, e.g., Beignon et al., J. Virol. (2009); Breckpot et al., supra). DC-NILV can induce immune responses of equivalent magnitude to its integrating counterpart and can be used in a homologous prime-boost regimen.

In certain embodiments, the vector particle is a viral vector particle and in other certain embodiments, the vector particle is a particle derived from a bacteria such as, for example, Listeria monocytogenes, Salmonella spp., Mycobacterium bovis, Escherichia coli, Shigella spp., and Yersinia spp. (see, e.g., Paterson, Semin. Immunol (2010) 22:183; Loessner, Expert Opin. Biol. Ther. (2004) 4:157; Daudel, Expert Rev. Vaccines (2007) 6:97). Exemplary viral vector particles include a lentiviral vector particle that comprises a lentiviral vector genome; a poxvirus vector particle that comprises a poxvirus vector genome; a vaccinia virus vector particle that comprises a vaccinia virus vector genome; an adenovirus vector particle that comprises a adenovirus vector genome; an adenovirus-associated virus vector particle that comprises a adenovirus-associated virus vector genome; a herpes virus vector particle that comprises a herpes virus vector genome (e.g., Herpes simplex virus I or II); or an alpha virus vector particle that comprises an alpha virus vector genome.

In a more particular embodiment, the vector particle is a lentiviral vector particle that comprises a lentiviral vector genome (which is described in detail above). Methods and compositions are provided herein for targeting cells and targeting dendritic cells (DCs) in particular by using a lentiviral vector particle (which may also be called a virion, a lentivirus particle) for delivering a sequence that encodes at least one immunogen to DCs. The lentiviral vector particle comprises an envelope glycoprotein variant derived from Sindbis virus E2, and a recombinant expression construct that comprises the genome that includes the sequences of interest, and optionally other components. The glycoprotein variant exhibits reduced binding to heparan sulfate compared to the glycoprotein from HR, a reference Sindbis virus strain. The envelope glycoprotein facilitates infection of dendritic cells by the lentiviral vector particles. "Facilitates" infection, as used herein, is the same as facilitates transduction and refers to the role of the envelope glycoprotein, acting alone or in concert with other molecules, in promoting or enhancing receptor-mediated entry of a pseudotyped retrovirus or lentivirus particle into a target cell.

In general, the lentiviral vector particles are produced by a cell line that contains one or more plasmid vectors and/or integrated elements that together encode the components necessary to generate functional vector particles. These lentiviral vector particles are typically not replication-competent, i.e., they are only capable of a single round of infection. Most often, multiple plasmid vectors or individual expression cassettes integrated stably into the producer cell chromosome are utilized to separate the various genetic components that generate the lentiviral vector particles; however, a single plasmid vector having all of the lentiviral components can be used. In one exemplification, the packaging cell line is transfected with one or more plasmids containing the viral vector genome, including LTRs, a cis-acting packaging sequence, and the sequences of interest (i.e., at least a nucleotide sequence encoding one immunogen), at least one plasmid encoding the virus enzymatic and structural components (e.g., gag and pol), and at least one plasmid encoding an Arbovirus envelope glycoprotein. Viral particles bud through the cell membrane and comprise a core that includes typically two RNA genomes containing the sequences of interest and an Arbovirus envelope glycoprotein that targets dendritic cells. In certain embodiments, the Arbovirus glycoprotein is a Sindbis virus E2 glycoprotein, and the glycoprotein is engineered to have reduced binding to heparan sulfate compared to E2 from the reference strain HR. This usually involves at least one amino acid change compared to the HR E2 glycoprotein sequence. As well, the E2 glycoprotein may be engineered to increase targeting specificity to dendritic cells.

Without wishing to be bound by theory, binding of the viral particle to a cell surface is believed to induce endocytosis, bringing the virus into an endosome, triggering membrane fusion, and allowing the virus core to enter the cytosol. For certain embodiments, which utilize integrating lentiviral vector particles, following reverse transcription and migration of the product to the nucleus, the genome of the virus integrates into the target cell genome, incorporating the sequences of interest into the genome of the target cell. To reduce the chance of insertional mutagenesis and to promote transient expression of a designated immunogen(s), however, other embodiments utilize non-integrating lentiviral vector particles (i.e., those which do not integrate into the target cell genome), but instead express the sequences of interest from an episome. In either instance, the infected DC then expresses the sequences of interest (e.g., an immunogen and optionally a stimulatory molecule). The immunogen can then be processed by dendritic cells and presented to T and B cells, generating an antigen-specific immune response. The specific pathway described above is not required so long as the dendritic cell is able to stimulate an antigen-specific immune response.

The viral particles can be administered to a subject in order to provide a prophylactic or therapeutic effect. Following infection of dendritic cells and expression of the immunogen product, an immune response is generated to the products.

### Viral Vector Envelope

Arthropod-borne viruses (Arboviruses) are viruses that are transmitted to a host, such as humans, horses, or birds by an infected arthropod vector such as a mosquito. Arboviruses are further divided into sub-families of viruses including alphaviruses and flaviviruses, which have a single-stranded RNA genome of positive polarity and a glycoprotein-containing envelope. For example, dengue fever virus, yellow fever virus and West Nile virus belong to the flavivirus family, and Sindbis virus, Semliki Forest virus and Venezuelan Equine Encephalitis virus, are members of the alphavirus family (see, e.g., Wang et al., J. Virol. 66, 4992 (1992)). The envelope of Sindbis virus includes two transmembrane glycoproteins (see, e.g., Mukhopadhyay et al. Nature Rev. Microbiol. 3, 13 (2005)): E1, believed to be responsible for fusion, and E2, believed to be responsible for cell binding. Sindbis virus envelope glycoproteins are known to pseudotype other retroviruses, including oncoretroviruses and lentiviruses.

As discussed herein, an arbovirus envelope glycoprotein can be used to pseudotype a lentiviral-based vector genome. A "pseudotyped" lentivirus is a lentiviral particle having one or more envelope glycoproteins that are encoded by a virus that is distinct from the lentiviral genome. The envelope glycoprotein may be modified, mutated or engineered as described herein. Thus, lentiviral vector particles described herein include lentivirus pseudotyped with an arbovirus envelope glycoprotein, e.g. an alphavirus or flavivirus envelope glycoprotein, e.g. an E2 glycoprotein that may be modified, mutated or engineered. Lentiviruses may also be pseudotyped with other envelopes, including VSV-G, influenza virus, arenavirus, rhabdovirus, orthomyxovirus, HIV1, HIV2 and SIV.

The envelope of Sindbis virus and other alphaviruses incorporates into the lipid bilayer of the viral particle membrane, and typically includes multiple copies of two glycoproteins, E1 and E2. Each glycoprotein has membrane-spanning regions; E2 has an about 33 residue cytoplasmic domain whereas the cytoplasmic tail of E1 is very short (about 2 residues). Both E1 and E2 have palmitic acids attached in or near the membrane-spanning regions. E2 is initially synthesized as a precursor protein that is cleaved by furin or other Ca2+-dependent serine proteinase into E2 and a small glycoprotein called E3. Located between sequences encoding E2 and E1 is a sequence encoding a protein called 6K. E3 and 6K are signal sequences which serve to translocate the E2 and E1 glycoproteins, respectively, into the membrane. In the Sindbis virus genome, the coding region for Sindbis envelope proteins includes sequence encoding E3, E2, 6K, and E1. As used herein, "envelope" of an arbovirus virus includes at least E2, and may also include E1, 6K, and E3. An exemplary sequence of envelope glycoproteins of Sindbis virus, strain HR, is presented as SEQ ID NO: 17. Sequences of envelope glycoproteins for other arboviruses can be found in publically available databases, such as GenBank. For example, sequences encoding Dengue virus glycoproteins can be found in Accession GQ252677.1 (among others in GenBank) and in the virus variation database at NCBI and an exemplary sequence encoding Venezuelan equine encephalitis virus envelope glycoproteins in Accession NP_040824.1.

Although the cellular receptor(s) on dendritic cells for alphaviruses, and Sindbis virus in particular, have not been definitively identified to date, one receptor appears to be DC-SIGN (see, e.g., Klimstra et al., J. Virol. 77:12022, 2003). The use of the terms "attachment," "binding," "targeting" and the like are used interchangeably and are not meant to indicate a mechanism of the interaction between Sindbis virus envelope glycoprotein and a cellular component. DC-SIGN (Dendritic Cell Specific ICAM-3 (Intracellular Adhesion Molecules 3)-Grabbing Nonintegrin; also known as CD209) is a C-type lectin-like receptor capable of rapid binding and endocytosis of materials (see, e.g., Geijtenbeek et al. Annu. Rev. Immunol. 22: 33-54, 2004). E2 appears to target virus to dendritic cells through DC-SIGN. As shown herein, cells expressing DC-SIGN are transduced by viral vector particles pseudotyped with Sindbis virus E2 better (at least 2-fold, at least 3-fold, at least 4-fold, at least 5-fold, at least 6-fold, at least 7-fold, at least 8-fold, at least 9-fold, or at least 10-fold better) than isogenic cells that do not express DC-SIGN. The mechanism of how E2 glycoprotein facilitates viral infection appears to involve DC-SIGN, possibly through direct binding to DC-SIGN or causing a change in conformation or some other mechanism. Regardless of the actual mechanism, the targeting by E2 is preferential for cells expressing DC-SIGN, namely dendritic cells.

Sindbis virus also appears to bind to cells via heparan sulfate (see, e.g., Klimstra et al., J. Virol. 72: 7357, 1998; Burmes et al., J. Virol. 72: 7349, 1998). Because heparan sulfate and other cell surface glycosaminoglycans are found on the surface of most cell types, it is desirable to reduce interaction between heparan sulfate and Sindbis envelope glycoproteins. This can be accomplished by diminishing the binding of Sindbis virus envelope to heparan sulfate or increasing the binding, e.g., increasing avidity, of Sindbis virus envelope to dendritic cells or both. As a result, nonspecific binding to other molecules, which may be expressed by other cell types and which may occur even if the envelope is specific for DC-SIGN, is reduced, and the improved specificity may serve to avoid undesired side effects, such as side effects that may reduce the desired immune response, or side effects associated with off-target transduction of other cell types. Alternatively or in addition to the advantages of relatively specific transduction of cells expressing DC-SIGN, viral particles pseudo-typed with Sindbis virus envelope E2 glycoprotein may offer other advantages over viral particles pseudo-typed with glycoproteins such as VSV-G. Examples of such advantages include reduced complement-mediated lysis and/or reduced neuronal cell targeting, both of which are believed to associate with administration of VSV-G pseudo-typed viral particles.

In various exemplifications, the lentiviral vector particles specifically bind to cells expressing DC-SIGN and have reduced or abrogated binding to heparan sulfate. That is, a Sindbis virus envelope E2 glycoprotein may be modified to preferentially direct the virus to dendritic cells that express DC-SIGN relative to other cell types. Such envelope glycoproteins preferentially bind dendritic cells, especially dendritic cells expressing DC-SIGN, relative to other cell types that ubiquitously express heparin sulfate such as myeloid or lymphoid cells. As described below, this preferential binding ideally results in preferential infection of dendritic cells, especially those expressing DC-SIGN. Based on information obtained from structural studies and molecular modeling among other studies, variant sequences of envelope proteins, especially E2 and E1 glycoproteins, are designed and generated such that the glycoproteins maintain their functions as envelope proteins, but have the desired binding specificity, avidity, or level of binding. Candidate variant sequences may be created for each glycoprotein and assayed using the methods described below, or other methods known in the art, to identify envelope glycoproteins with the most desirable characteristics.

Certain variant sequences of Sindbis E2 have at least one amino acid alteration at residue 160 as compared to SEQ ID NO:1. Residue 160 is deleted or changed to an amino acid other than glutamic acid. An alteration is most commonly a substitution of at least one amino acid, but alternatively can be an addition or deletion of one or more amino acids. Preferably, any additional amino acids are few in number and do not comprise an antigenic epitope (e.g., hemagglutinin tag sequence), which may compromise safety. When there are two or more alterations, they can both be of the same type (e.g., substitution) or differing types (e.g., a substitution and a deletion). Multiple alterations can be scattered or located contiguously in the protein sequence.

By way of example, variant sequences comprise at least one amino acid alteration in the region of about residue 50 to about residue 180 of SEQ ID NO:1. Within this region are amino acids that are involved with binding to heparan sulfate. By reducing the net positive charge of E2, electrostatic interaction with heparan sulfate can be reduced, resulting in decreased binding to heparan sulfate. Candidate positively charged amino acids in this region include lysines at residues 63, 70, 76, 84, 97, 104, 129, 131, 133, 139, 148, 149, 159 and arginine at residues 65, 92, 128, 137, 157, 170, 172 (see, e.g., Bear et al., Virology 347: 183-190, 2006) (see SEQ ID NO:1). At least several of these amino acids are directly implicated in E2 binding to heparan sulfate. Net positive charge can be reduced by deletion of lysine or arginine or substitution of lysine or arginine with a neutral or negatively charged amino acid. For example, one or more of these lysines and arginines may be replaced with glutamic or aspartic acid. Certain embodiments have at least one substitution of lysine 70, 76 or 159. Exemplary amino acid sequences of the E2 glycoprotein are set forth in SEQ ID NOS:3 (e.g., residues 66 to 488), 4 (e.g., residues 66 to 488), and 5 (e.g., residues 66 to 486). In cases where E2 is expressed as a polyprotein with E3, the lysine located adjacent to the natural E3/E2 cleavage site is maintained - that is, the recognition sequence and cleavage site is unaltered. Alternatively, the native endopeptidase cleavage site sequence is replaced with a recognition sequence for a different endopeptidase.

Certain variants of E2 are also modified in a way that positively impacts binding to dendritic cells. Alteration of the glutamic acid found at residue 160 in the reference HR sequence can improve binding to dendritic cells (see, e.g., Gardner et al., J. Virol. 74, 11849, 2000). Alterations, such as a deletion of residue 160 or substitution of residue 160 are found in certain variants. In particular variants, a non-charged amino acid is substituted for Glu, in other variants, a non-acidic amino acid is substituted for Glu. Typically, Glu160 is replaced with one of the small or aliphatic amino acids, including glycine, alanine, valine, leucine or isoleucine.

Other variants comprise two or more amino acid alterations. Typically in these variants one of the alterations is Glu160 and the remaining alteration(s) are changes of one or more of the lysines and arginines in the region spanning residue about 50 to about 180 of SEQ ID NO:1. Certain of the variants comprise an alteration of Glu160 to a non-acidic residue or deletion and one or more alterations of lysine 70, lysine 76, or lysine 159 with a non-basic amino acid. Some specific variants comprise a Glu160 to Gly, Lys 70 to Glu, and Lys 159 to Glu; a Glu 160 to Gly, Lys 70, 76 and 159 to Glu; a deletion of Glu 160 and Lys 70 and 159 to Glu; and a deletion of Glu 160 and Lys 70, 76, and 159 to Glu. It is understood that the numbering of positions used herein is done with reference to SEQ ID NO: 1, such that, e.g. if an amino acid is deleted or inserted, the numbering adjusts accordingly. For example, if residue 1 is absent, then position 160 refers to position 159.

In certain embodiments, E2 protein is first expressed as a polyprotein in fusion with at least E3 or in fusion with a leader sequence. Regardless of whether the leader sequence is E3 or another sequence, E2 in the viral envelope should be free of the E3 or other leader sequence. In other words, E2 is preferably not an E3/E2 fusion protein (e.g., the E3/E2 fusion protein called SVGmu). In certain embodiments, E2 is expressed as part of E3-E2-6K-E1 polyprotein. Sindbis virus naturally expresses E2 as part of a polyprotein and the junction regions for E3/E2, E2/6K, and 6K/E1 have sequences recognized and cleaved by endopeptidases. Normally, the E3/E2 junction is cleaved by furin or a furin-like serine endopeptidase between residues 65 and 66. Furin has specificity for paired arginine residues that are separated by two amino acids. To maintain E3/E2 cleavage by furin, residues 62-66 (RSKRS; SEQ ID NO: 26) should maintain the two arginine residues with two amino acid separation and the serine residue. Alternatively, a different cleavage sequence can be used in place of the E3/E2 furin cleavage sequence or any of the other cleavage sequences. Recognition and cleavage sites can be incorporated for endopeptidases, including, without limitation, aspartic endopeptidases (e.g., cathepsin D, chymosin, HIV protease), cysteine endopeptidases (bromelains, papain, calpain), metalloendopeptidases, (e.g., collagenase, thermolysin), serine endopeptidases (e.g., chymotrypsin, factor IXa, factor X, thrombin, trypsin), streptokinases. The recognition and cleavage site sequences for these enzymes are well known.

Amino acids in E2, other than those already mentioned, may also be altered. Generally, a variant E2 sequence will have at least 80% sequence amino acid identity to the reference E2 sequence, or it may have at least 82%, at least 85%, at least 87%, at least 90%, at least 92%, at least 95%, or at least 98% sequence identity. Thus, any of the variant E2 glycoproteins described above, with any of the mutations described above (including but not limited to the mutation at position 160, or at one or more of the lysines and arginines in the region spanning residue about 50 to about 180, e.g., 70, 76 and/or 159), may have at least 80% sequence amino acid identity to any of the reference E2 sequences, e.g. the mature E2 glycoprotein sequence of SEQ ID NO: 1, 3 (e.g., residues 66 to 488), 4 (e.g., residues 66 to 488) or 5 (e.g., residues 66 to 486). The variant glycoprotein should exhibit biological function, such as the ability to facilitate infection of dendritic cells by a viral particle having an envelope comprising E2. Experiments have identified regions of envelope glycoproteins that appear to have an important role in various aspects of viral assembly, attachment to cell surface, and infection. When making variants, the following information can be used as guidelines. The cytoplasmic tail of E2 - approximately residues 408 to 415 - is important for virus assembly (see, e.g., West et al. J. Virol. 80: 4458-4468, 2006; incorporated in its entirety). Other regions are involved in forming secondary structure (approximately residues 33-53), and involved in transport and protein stability (approximately residues 86-119) (see, e.g., Navaratmarajah et al., J. Virol. 363:124-147, 2007; incorporated in its entirety). The variant may retain hydrophobic character of a region that spans the membrane, approximately residues 370-380. The variant may retain one or both N-linked glycosylation sites residues NIT (residues 196-198) and NFT (residues 318-320) and may retain one or more of the sites that are palmitoylated (C-396, C416 and C417) (see, e.g., Strauss et al., Microbiol. Rev. 58, 491-562, 1994; pp. 499-509). On the other hand, many regions of E2 may be altered without deleterious event. For example, insertions of transposons at many different locations in E2 still resulted in viable virus (see, e.g., Navaratmarajah, supra).

In certain embodiments, a tag peptide may be incorporated into E3, 6K, or E1 proteins. For some purposes, a tag may be incorporated into E2, but a tag is not desirable for use in a product for administration to human patients. A tag peptide, which is a short sequence (e.g., 5-30 amino acids), can be used to facilitate detection of envelope expression and its presence in viral particles. For detection purposes, a tag sequence will typically be detectable by antibodies or chemicals. Another use for a tag is to facilitate purification of viral particles. A substrate containing a binding partner for the tag can be used to absorb virus. Elution of the virus can be accomplished by treatment with a moiety that displaces the tag from the binding partner or when the tag sequence is in linkage with a cleavable sequence, treatment with the appropriate endopeptidase will conveniently allow release of virus. (See, for example, QiaGEN® catalog, Factor Xa Protease System). Removal of the tag peptide is generally desirable for safety purposes of the virus particles use in animal subjects. If the tag is not removed, an immune response to the tag may occur.

Suitable tags include, without limitation, FLAG (DYKDDDDK) (SEQ ID NO:35) (U.S. Patent No. 4,703,004, incorporated in its entirety), for which antibodies are commercially available, chitin binding protein, maltose binding protein, glutathione-S-transferase, poly(His) (U.S. Patent No. 4,569,794, incorporated in its entirety), thioredoxiin, HA (hemagglutinin)-tag, among others. Poly(His) can be adsorbed onto affinity media containing bound metal ions, such as, nickel or cobalt, and eluted with a low pH medium.

The vector particles may be evaluated to determine the specificity of the envelope glycoprotein incorporated into the virus that targets dendritic cells. For example, a mixed population of bone marrow cells can be obtained from a subject and cultured in vitro. Alternatively, isogenic cells lines that express or do not express DC-SIGN can be obtained and used. The recombinant virus can be administered to the mixed population of bone marrow cells or isogenic cell lines, and expression of a reporter gene incorporated into the virus can be assayed in the cultured cells. Certain embodiments may employ a limiting dilution analysis, in which the mixed population of cells is split into separate parts, which are then separately incubated with decreasing amounts of virus (e.g., 2-fold, 5-fold, 10-fold less virus in each part). In some embodiments, at least about 50%, or at least about 60%, 70%, 80% or 90%, or at least about 95% of infected cells in the mixed cell population are dendritic cells that express DC-SIGN. In certain embodiments, the ratio of infected dendritic cells to infected non-dendritic cells (or non DC-SIGN expressing cells) is at least about 2:1, at least about 3:1, at least about 4:1, at least about 5:1, at least about 6:1, at least about 7:1, at least about 8:1, at least about 9:1, at least about 10:1, at least about 20:1, at least about 30:1, at least about 40:1, at least about 50:1, at least about 100:1, at least about 200:1, at least about 500:1, at least about 1000:1, at least about 5000:1, at least about 10,000:1, or more. For limiting dilution, greater selectivity is typically seen at higher dilutions (i.e., lower amounts) of input virus.

Activity of pseudotyped viral particles can be determined by any of a variety of techniques. For example, a preferred method to measure infectivity efficiency (IU, infectious units) is by administering viral particles to cells and measuring expression of a product encoded in the vector genome. Any product that can be assayed may be used. One convenient type of product is a fluorescent protein, such as green fluorescent protein (GFP). Other products that can be used include proteins expressed on a cell surface (e.g., detection by antibody binding), enzymes, and the like. If the product is an antigen and cells are dendritic cells, infectivity / activity can be assessed by determining an immune response. Furthermore, it is possible to ascertain side effects in a mammal. The ability to specifically target dendritic cells can also be tested directly, for example, in cell culture as described below.

Vector particles, which include the viral particles described herein can also be prepared and tested for their selectivity and/or their ability to facilitate penetration of the target cell membrane. Viral particles that have an envelope with unmodified glycoproteins can be used as controls for comparison. Briefly, cells expressing a receptor for an envelope glycoprotein are infected by the virus using a standard infection assay. After a specified time, for example 48 hours post-infection, cells can be collected and the percentage of cells infected by the virus can be determined by flow cytometry, for example. Selectivity can be scored by calculating the percentage of cells infected by virus. Similarly, the effect of a variant envelope glycoprotein on viral titer can be quantified by dividing the percentage of cells infected by virus comprising a variant envelope by the percentage of cells infected by virus comprising the corresponding wild type (unmodified) envelope glycoprotein. A particularly suitable variant will have the best combination of selectivity and infectious titer. Once a variant is selected, viral concentration assays may be performed to confirm that these viruses can be concentrated without compromising activity. Viral supernatants are collected and concentrated by ultracentrifugation. The titers of viruses can be determined by limited dilution of viral stock solution and infection of cells expressing the receptor for the envelope glycoprotein, measuring the expression of a product expressed by the viruses as described above.

The entry of a lentiviral vector particle into a target cell is another type of evaluation of activity. BlaM-Vpr (beta-lactamase Vpr) fusion protein has been used to evaluate HIV-1 viral penetration; a fusion of BlaM and a Sindbis virus envelope glycoprotein, such as E1 or an E2/E1 fusion protein can be used to assess the efficacy of an envelope protein in facilitating fusion and penetration into a target cell. Viral particles may be prepared, for example, by transient transfection of packaging cells with one or more vectors comprising the viral elements, BlaM-Vpr, and the variant envelope of interest (and an affinity molecule if appropriate). The resulting viruses can be used to infect cells expressing a molecule the targeting molecule (or affinity molecule) specifically binds in the absence or presence of the free inhibitor of binding (such as an antibody). Cells can then be washed with CO2-independent medium and loaded with CCF2 dye (Aurora Bioscience). After incubation at room temperature to allow completion of the cleavage reaction, the cells can be fixed by paraformaldehyde and analyzed by flow cytometry and microscopy. The presence of blue cells indicates the penetration of viruses into the cytoplasm; fewer blue cells would be expected when blocking antibody is added (see, e.g., Cavrois et al., Nat. Biotechnol. 20:1151-54, 2002).

To investigate whether penetration is dependent upon a low pH, and to identify envelope glycoproteins with the desired pH dependence, NH4Cl or other compound that alters pH can be added at the infection step (NH4Cl will neutralize the acidic compartments of endosomes). In the case of NH4Cl, the disappearance of blue cells will indicate that penetration of viruses is low pH-dependent. In addition, to confirm that the activity is pH-dependent, lysosomotropic agents, such as ammonium chloride, chloroquine, concanamycin, bafilomycin Al, monensin, nigericin, etc., may be added into the incubation buffer. These agents elevate the pH within the endosomal compartments (see, e.g., Drose et al., J. Exp. Biol. 200, 1-8, 1997). The inhibitory effect of these agents will reveal the role of pH for viral fusion and entry. The different entry kinetics between viruses displaying different fusogenic molecules may be compared and the most suitable selected for a particular application.

PCR-based entry assays can be utilized to monitor reverse transcription and measure kinetics of viral DNA synthesis as an indication of the kinetics of viral entry. For example, viral particles comprising a particular envelope protein molecule are incubated with target cells, such as 293T cells, DCs, or any other cells that have been engineered to express, or which naturally express, the appropriate binding partner (receptor) for the envelope protein molecule. Either immediately, or after a time increment (to allow infection to occur), unbound viruses are removed and aliquots of the cells are analyzed for viral nucleic acids. DNA is extracted from these aliquots and subjected to amplification analysis, generally in a semi-quantitative assay, primed with LTR-specific primers. The appearance of LTR-specific DNA products indicates the success of viral entry.

Following viral infection with the viral vector particle, the immunogen is expressed by the target dendritic cells. If contacted ex vivo, the target dendritic cells are then transferred back to the patient, for example by injection, where they interact with immune cells that are capable of generating an immune response against the desired antigen. In preferred embodiments, the recombinant virus is injected into the patient where it transduces the targeted dendritic cells in situ. The dendritic cells then express the particular antigen associated with a disease or disorder to be treated, and the patient is able to mount an effective immune response against the disease or disorder.

The viral vector genome may contain a polynucleotide sequence encoding more than one immunogen, and upon transduction of a target dendritic cell, generates immune responses to each immunogen delivered to the cell. In some embodiments, the immunogens are related to a single disease or disorder. In other embodiments, the immunogens are related to multiple diseases or disorders.

In some of the vector particles, DC maturation factors that activate and/or stimulate maturation of the DCs are delivered in conjunction with the immunogen-encoding sequence of interest. In certain alternative embodiments, the DCs are activated by delivery of DC maturation factors prior to, simultaneously with, or after delivery of the vector particles. DC maturation factors may be provided separately from administration of the vector particles.

As described herein, one or more immune modulation or DC maturation factors can be encoded by one or more sequences that are contained in the vector particle and expressed after the particle enters or infects a dendritic cell. The sequences encoding immune modulation factors can also be provided in a separate vector that is co-transfected with the vector particle encoding one or more immunogens in a packaging cell line.

The methods described herein may be used for adoptive immunotherapy in a subject. As described above, an immunogen against which an immune response is desired is identified. A polynucleotide encoding the desired immunogen(s) is obtained and packaged into a vector particle. Target dendritic cells are obtained from the patient and transduced with the vector particle containing a polynucleotide that encodes the desired immunogen. The dendritic cells are then transferred back into the patient.

The vector particles (e.g., the viral vector particles described herein) may be injected in vivo, where the particles infect DCs and deliver the immunogen-encoding nucleotide sequence of interest. The amount of viral particles is at least 3X106 infectious units (IU), and can be at least 1X107 IU, at least 3X107 IU, at least 1X108 IU, at least 3X108 IU, at least 1X109 IU, or at least 3X109 IU. At selected intervals, DCs from the recipient's lymphoid organs may be used to measure expression, for example, by observing marker expression, such as GFP or luciferase if co-expressed by a polynucleotide sequence present in the recombinant expression vector included in the vector particle. Nucleic acid monitoring techniques and measurements of reverse transcriptase (RT) activity can also be used to analyze the biodistribution of vector particles when the vector particle is a lentiviral vector particle. T cells from peripheral blood mononuclear cells, lymph nodes, spleens, or malignant or target pathogen-infected tissue of vector particle (including lentiviral vector particle) treated recipients may be measured from the magnitude and durability of response to antigen stimulation. Tissue cells other than DCs, such as epithelial cells and lymphoid cells, may be analyzed for the specificity of in vivo gene delivery.

### Adjuvants and Adjuvant Compositions

The methods described herein comprise administering to a subject at least one composition comprising an adjuvant that is intended to "pull" the primed immune cells to a local site of administration, in particular at the site of a tumor. In another embodiment, compositions comprising the adjuvant further comprise an immunogen, in certain embodiments the immunogen of the "prime". Such compositions comprising an adjuvant may not comprise an immunogen and thus, in certain embodiments, compositions comprising the adjuvant are substantially devoid of immunogen. In certain embodiments, the adjuvant enhances (or improve, augment) the immune response to the immunogen (i.e., increase the level of the specific immune response to the immunogen in a statistically, biologically, or clinically significant manner compared with the level of the specific immune response in the absence of administering the adjuvant). Methods and techniques for determining the level of an immune response are discussed in greater detail herein and are routinely practiced in the art.

Exemplary adjuvants that may be used in the methods described herein include, but are not necessarily limited to, the following. Adjuvants that may be used in the methods described herein include adjuvants that may be useful for enhancing the CTL response to the immunogen (or to the immunogen-containing cell or particle) and/or enhancing the memory CD4 T cell response. In certain embodiments, the adjuvants for use in the methods herein induce a cytokine response so as to recruit T cells, in particular, antigen-specific T cells to a local administration site. In another embodiment, the adjuvants for use in the methods herein augment the response to the immunogen without causing conformational changes in the immunogen that might adversely affect the qualitative form of the response. Suitable adjuvants include aluminum salts, such as alum (potassium aluminum sulfate), or other aluminum containing adjuvants; nontoxic lipid A-related adjuvants such as, by way of non-limiting example, nontoxic monophosphoryl lipid A (see, e.g., Tomai et al., J. Biol. Response Mod. 6:99-107 (1987)), GLA described herein; 3 De-O-acylated monophosphoryl lipid A (MPL) (see, e.g., United Kingdom Patent Application No. GB 2220211); adjuvants such as QS21 and QuilA, that comprise a triterpene glycoside or saponin isolated from the bark of the Quillaja saponaria Molina tree found in South America (see, e.g., Kensil et al., in Vaccine Design: The Subunit and Adjuvant Approach (eds. Powell and Newman, Plenum Press, NY, 1995); U.S. Pat. No. 5,057,540). Other suitable adjuvants include oil in water emulsions (such as squalene or peanut oil), optionally in combination with immune stimulants, such as monophosphoryl lipid A (see, e.g., Stoute et al., N. Engl. J. Med. 336, 86-91 (1997)). Another suitable adjuvant is CpG (Bioworld Today, Nov. 15, 1998). Other suitable adjuvants include Toll-like receptor agonists and lipid A mimetics such as amino-akyl glucosaminide 4-phosphates (AGPs) (see, e.g., RC527 described in Baldridge et al., Expert Opin. Biol. Ther., 4(7): 1129-1138 (2004), and RC-544 described in Persing et al., Trends in Microbiology, 10(10) (suppl.):S32-S37 (2002)).

As described herein, a suitable adjuvant is an aluminum salt, such as aluminum hydroxide, aluminum phosphate, aluminum sulfate. Such adjuvants can be used with or without other specific immunostimulating agents such as MPL or 3-DMP, QS21, polymeric or monomeric amino acids such as polyglutamic acid or polylysine. Another class of suitable adjuvants is oil-in-water emulsion formulations (also called herein stable oil in water emulsions). Such adjuvants can be optionally used with other specific immunostimulating agents such as muramyl peptides (e.g., N-acetylmuramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'dipalmitoyl-sn--glycero-3-hydroxyphosphoryloxy)-ethylamine (MTP-PE), N-acetylglucsaminyl-N-acetylmuramyl-L-Al-D-isoglu-L-Ala-dipalmitoxy propylamide (DTP-DPP) theramide™), or other bacterial cell wall components. Oil-in-water emulsions include (1) MF59 (WO 90/14837), containing 5% Squalene, 0.5% Tween 80, and 0.5% Span 85 (optionally containing various amounts of MTP-PE) formulated into submicron particles using a microfluidizer such as Model 110Y microfluidizer (Microfluidics, Newton Mass.); (2) SAF, containing 10% Squalane, 0.4% Tween 80, 5% pluronic-blocked polymer L121, and thr-MDP, either microfluidized into a submicron emulsion or vortexed to generate a larger particle size emulsion, and (3) Ribi adjuvant system (RAS), (Ribi Immunochem, Hamilton, MT) containing 2% squalene, 0.2% Tween 80, and one or more bacterial cell wall components from the group consisting of monophosphorylipid A (MPL), trehalose dimycolate (TDM), and cell wall skeleton (CWS), preferably MPL+CWS (Detox™). Also as described above, suitable adjuvants include saponin adjuvants, such as Stimulon™ (QS21, Aquila, Worcester, Mass.) or particles generated therefrom such as ISCOMs (immunostimulating complexes) and ISCOMATRIX. Other adjuvants include Complete Freund's Adjuvant (CFA) (which is suitable for non-human use but not for human use) and Incomplete Freund's Adjuvant (IFA). Other adjuvants include cytokines, such as interleukins (IL-1, IL-2, and IL-12), macrophage colony stimulating factor (M-CSF), and tumor necrosis factor (TNF).

Another adjuvant that may be used in the compositions described herein is identified by chemical formula (I) and referred to as glucopyranosyl lipid A (GLA): 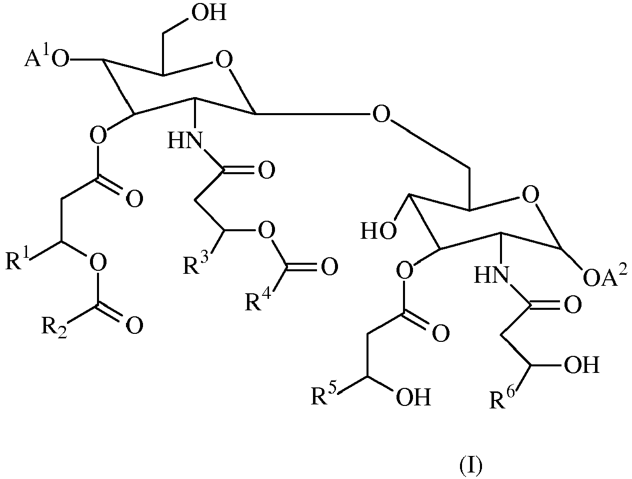 wherein the moieties A1 and A2 are independently selected from the group of hydrogen, phosphate, and phosphate salts. Sodium and potassium are exemplary counterions for the phosphate salts. The moieties R1, R2, R3, R4, R5, and R6 are independently selected from the group of hydrocarbyl having 3 to 23 carbons, represented by C3-C23. For added clarity it will be explained that when a moiety is "independently selected from" a specified group having multiple members, it should be understood that the member chosen for the first moiety does not in any way impact or limit the choice of the member selected for the second moiety. The carbon atoms to which R1, R3, R5 and R6 are joined are asymmetric, and thus may exist in either the R or S stereochemistry. In one embodiment all of those carbon atoms are in the R stereochemistry, while in another embodiment all of those carbon atoms are in the S stereochemistry.

"Hydrocarbyl" refers to a chemical moiety formed entirely from hydrogen and carbon, where the arrangement of the carbon atoms may be straight chain or branched, noncyclic or cyclic, and the bonding between adjacent carbon atoms maybe entirely single bonds, i.e., to provide a saturated hydrocarbyl, or there may be double or triple bonds present between any two adjacent carbon atoms, i.e., to provide an unsaturated hydrocarbyl, and the number of carbon atoms in the hydrocarbyl group is between 3 and 24 carbon atoms. The hydrocarbyl may be an alkyl, where representative straight chain alkyls include methyl, ethyl, n-propyl, n-butyl, n-pentyl, n-hexyl, and the like, including undecyl, dodecyl, tridecyl, tetradecyl, pentadecyl, hexadecyl, heptadecyl, octadecyl, etc.; while branched alkyls include isopropyl, sec-butyl, isobutyl, tert-butyl, isopentyl, and the like. Representative saturated cyclic hydrocarbyls include cyclopropyl, cyclobutyl, cyclopentyl, cyclohexyl, and the like; while unsaturated cyclic hydrocarbyls include cyclopentenyl and cyclohexenyl, and the like. Unsaturated hydrocarbyls contain at least one double or triple bond between adjacent carbon atoms (referred to as an "alkenyl" or "alkynyl", respectively, if the hydrocarbyl is non-cyclic, and cycloalkeny and cycloalkynyl, respectively, if the hydrocarbyl is at least partially cyclic). Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like; while representative straight chain and branched alkynyls include acetylenyl, propynyl, 1-butynyl, 2-butynyl, 1-pentynyl, 2-pentynyl, 3-methyl-1-butynyl, and the like.

The adjuvant of formula (I) may be obtained by synthetic methods known in the art, for example, the synthetic methodology disclosed in PCT International Publication No. WO 2009/035528, as well as the publications identified in WO 2009/035528.

Certain of the adjuvants may also be obtained commercially. A preferred adjuvant is Product No. 699800 as identified in the catalog of Avanti Polar Lipids, Alabaster AL (see E1 in combination with E10, below).

In various embodiments of the invention, the adjuvant has the chemical structure of formula (I) but the moieties A1, A2, R1, R2, R3, R4, R5, and R6 are selected from subsets of the options previously provided for these moieties, wherein these subsets are identified below by E1, E2, etc.
E1: A1 is phosphate or phosphate salt and A2 is hydrogen.
E2: R1, R3, R5 and R6 are C3-C21 alkyl; and R2 and R4 are C5-C23 hydrocarbyl.
E3: R1, R3, R5 and R6 are C5-C17 alkyl; and R2 and R4 are C7-C19 hydrocarbyl.
E4: R1, R3, R5 and R6 are C7-C15 alkyl; and R2 and R4 are C9-C17 hydrocarbyl.
E5: R1, R3, R5 and R6 are C9-C13 alkyl; and R2 and R4 are C11-C15 hydrocarbyl.
E6: R1, R3, R5 and R6 are C9-C15 alkyl; and R2 and R4 are C11-C17 hydrocarbyl.
E7: R1, R3, R5 and R6 are C7-C13 alkyl; and R2 and R4 are C9-C15 hydrocarbyl.
E8: R1, R3, R5 and R6 are C11-C20 alkyl; and R2 and R4 are C12-C20 hydrocarbyl.
E9: R1, R3, R5 and R6 are C11 alkyl; and R2 and R4 are C13 hydrocarbyl.
E10: R1, R3, R5 and R6 are undecyl and R2 and R4 are tridecyl.

In certain embodiments, E1 may be combined with each of E2 through E10. The hydrocarbyl groups of E2 through E9 may be alkyl groups, preferably straight chain alkyl groups. In certain embodiments, E1 is combined with each of E2 through E10 and each of E2 through E9 are alkyl groups. In certain embodiments, E1 is combined with each of E2 through E10 and each of E2 through E9 are straight chain alkyl groups. The adjuvant of formula (I) may be formulated into a pharmaceutical composition, optionally with a co-adjuvant, each as discussed below. In this regard reference is made to U.S. Patent Publication No. 2008/0131466 that provides formulations, such as aqueous formulation (AF) and stable emulsion formulations (SE) for GLA adjuvant, wherein these formulations may be used for any of the adjuvants of formula (I).

In another embodiment, the adjuvant is a synthetic lipid A type adjuvant as described in PCT International publication No. WO 2010/141861, and has a structure selected from the following chemical formula (II): 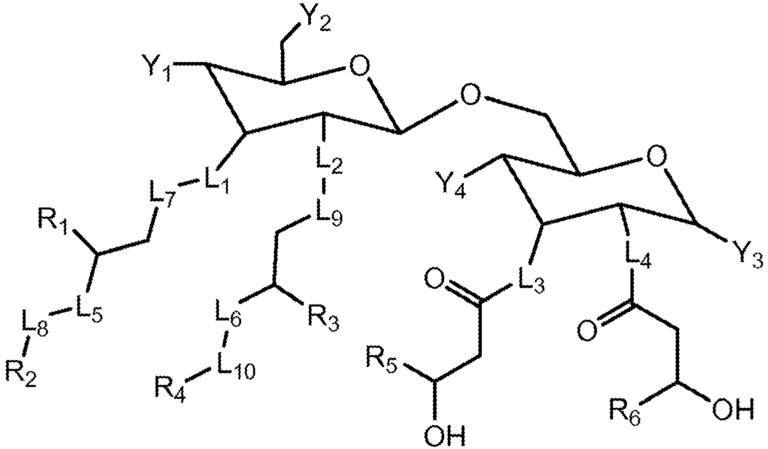 or a pharmaceutically acceptable salt thereof, wherein: L1, L2, L3, L4, L5 and L6 are the same or different and are independently selected from -O-, -NH-, and -(CH2)-; L7, L8, L9 and L10 are the same or different, and at any occurrence may be either absent or -C(=O)-; Y1 is an acid functional group; Y2 and Y3 are the same or different and are each independently selected from -OH, -SH, and an acid functional group; Y4 is -OH or -SH; R1, R3, R5 and R6 are the same or different and are each independently selected from the group of C8-C13 alkyl; and R2 and R4 are the same or different and are each independently selected from the group of C6-C11 alkyl.

Optionally, as described in greater detail below and herein, two or more different adjuvants can be used simultaneously, such as by way of non-limiting example, an aluminum salt with MPL, an aluminum salt with QS21, MPL with QS21, and alumna aluminum salt, QS21, and MPL together. Also, Incomplete Freund's adjuvant can be used (see, e.g., Chang et al., Advanced Drug Delivery Reviews 32, 173-186 (1998)), optionally in combination with any of an aluminum salt, QS21, and MPL and all combinations thereof.

In certain embodiments, the adjuvant of formula (I) may be formulated into a pharmaceutical (or adjuvant composition), optionally with a co-adjuvant as described above, each as discussed below or any other adjuvant described herein or available in the art. In this regard reference is made to U.S. Patent Publication No. 2008/0131466 that provides formulations, such as aqueous formulation (AF) and stable emulsion formulations (SE) for GLA adjuvant, which formulations may be used with respect to any of the adjuvants of formula (I).

As provided herein the adjuvant of formula I may be used in combination with a second adjuvant, referred to herein as a co-adjuvant. In three exemplary embodiments, the co-adjuvant may be a delivery system, or it may be an immunopotentiator, or it may be a composition that functions as both a delivery system and an immunopotentiator (see, e.g., O'Hagan et al., Pharm. Res. 21(9):1519-30 (2004)). The co-adjuvant may be an immunopotentiator that operates via a member of the Toll-like receptor family biomolecules. For example, the co-adjuvant may be selected for its primary mode of action, as either a TLR4 agonist, or a TLR8 agonist, or a TLR9 agonist. Alternatively, or in supplement, the co-adjuvant may be selected for its carrier properties; for example, the co-adjuvant may be an emulsion, a liposome, a microparticle, or alum.

In one embodiment, the co-adjuvant is alum, where this term refers to aluminum salts, such as aluminum phosphate (A1P04) and aluminum hydroxide (A1(OH)3). When alum is used as the co-adjuvant, the alum may be present in a dose of vaccine in an amount of about 100 to 1,000 µg, or 200 to 800 µg, or 300 to 700 µg or 400 to 600 µg. The adjuvant of formula (1) is typically present in an amount less than the amount of alum, and in various specific embodiments the adjuvant of formula (1), on a weight basis, is present at 0.1-1%, or 1-5%, or 1-10%, or 1-100% relative to the weight of alum.

In one particular embodiment, the adjuvant is an emulsion having vaccine adjuvanting properties. Such emulsions include oil-in-water emulsions. Freund's incomplete adjuvant (IFA) is one such adjuvant. Another suitable oil-in-water emulsion is MF-59™ adjuvant, which contains squalene, polyoxyethylene sorbitan monooleate (also known as Tween™ 80 surfactant), and sorbitan trioleate. Squalene is a natural organic compound originally obtained from shark liver oil, although also available from plant sources (primarily vegetable oils), including amaranth seed, rice bran, wheat germ, and olives. Other suitable adjuvants are Montanide™ adjuvants (Seppic Inc., Fairfield NJ) including Montanide™ ISA 50V, which is a mineral oil-based adjuvant; Montanide™ ISA 206; and Montanide™ IMS 1312. While mineral oil may be present in the co-adjuvant, in one embodiment the oil component(s) of the vaccine compositions of the present invention are all metabolizable oils.

Examples of immunopotentiators that may be used in the practice of the methods described herein as co-adjuvants include: MPL™; MDP and derivatives; oligonucleotides; double-stranded RNA; alternative pathogen-associated molecular patterns (PAMPS); saponins; small-molecule immune potentiators (SMIPs); cytokines; and chemokines.

In one embodiment, the co-adjuvant is MPL™ adjuvant, which is commercially available from GlaxoSmithKline (originally developed by Ribi ImmunoChem Research, Inc. Hamilton, MT). See, e.g., Ulrich and Myers, Chapter 21 from Vaccine Design: The Subunit and Adjuvant Approach, Powell and Newman, eds. Plenum Press, New York (1995). Related to MPL™ adjuvant, and also suitable as co-adjuvants for use in the compositions and methods described herein, are AS02™ adjuvant and AS04™ adjuvant. AS02™ adjuvant is an oil-in-water emulsion that contains both MPL™ adjuvant and QS-21™ adjuvant (a saponin adjuvant discussed elsewhere herein). AS04™ adjuvant contains MPL™ adjuvant and alum. MPL™ adjuvant is prepared from lipopolysaccharide (LPS) of Salmonella minnesota R595 by treating LPS with mild acid and base hydrolysis followed by purification of the modified LPS.

In another embodiment, the co-adjuvant is a saponin such as those derived from the bark of the Quillaja saponaria tree species, or a modified saponin (see, e.g., U.S. Patent Nos. 5,057,540; 5,273,965; 5,352,449; 5,443,829; and 5,560,398). The product QS-21™ adjuvant sold by Antigenics, Inc. Lexington, MA is an exemplary saponin-containing co-adjuvant that may be used with the adjuvant of formula (1). An alternative co-adjuvant, related to the saponins, is the ISCOM™ family of adjuvants, originally developed by Iscotec (Sweden) and typically formed from saponins derived from Quillaja saponaria or synthetic analogs, cholesterol, and phospholipid, all formed into a honeycomb-like structure.

In yet another embodiment, the co-adjuvant is a cytokine that functions as a co-adjuvant (see, e.g., Lin et al., Clin. Infect. Dis. 21(6):1439-49 (1995); Taylor, Infect. Immun. 63(9):3241-44 (1995); and Egilmez, Chap. 14 in Vaccine Adjuvants and Delivery Systems, John Wiley & Sons, Inc. (2007)). In various embodiments, the cytokine may be, for example, granulocyte-macrophage colony-stimulating factor (GM-CSF) (see, e.g., Change et al., Hematology 9(3):207-15 (2004); Dranoff, Immunol. Rev. 188:147-54 (2002); and U.S. Patent 5,679,356); or an interferon, such as a type I interferon (e.g., interferon-α (IFN-α) or interferon-β (IFN-β)), or a type II interferon (e.g., interferon-γ (IFN-γ) (see, e.g., Boehm et al., Ann. Rev. Immunol. 15:749-95 (1997); and Theofilopoulos et al., Ann. Rev. Immunol. 23:307-36 (2005)); an interleukin, specifically including interleukin-1α (IL-1α), interleukin-1β (IL-Iβ), interleukin-2 (IL-2) (see, e.g., Nelson, J. Immunol. 172(7):3983-88 (2004); interleukin-4 (IL-4), interleukin-7 (IL-7), interleukin-12 (IL-12) (see, e.g., Portielje et al., Cancer Immunol. Immunother. 52(3): 133-44 (2003); and Trinchieri, Nat. Rev. Immunol. 3(2):133-46 (2003)); interleukin-15 (11-15), interleukin-18 (IL-18); fetal liver tyrosine kinase 3 ligand (Flt3L), or tumor necrosis factor α (TNFα). The adjuvant of formula (I) may be co-formulated with the cytokine prior to combination with the vaccine antigen, or the antigen, adjuvant of formula (I) and cytokine co-adjuvant may be formulated separately and then combined.

In certain embodiments, as provided herein the adjuvants described herein in Formula I or II may be used in combination with another therapeutic agent. In one embodiment, the adjuvant compositions herein are administered, in particular embodiments at a local site such as at the site of a tumor, in combination with one or more immune checkpoint inhibitors. Immune checkpoints refer to a variety of inhibitory pathways of the immune system that are crucial for maintaining self-tolerance and for modulating the duration and amplitude of an immune responses. Tumors use certain immune-checkpoint pathways as a major mechanism of immune resistance, particularly against T cells that are specific for tumor antigens. (see., *e.g*., Pardoll, 2012 Nature 12:252; Chen and Mellman 2013 Immunity 39:1). The present disclosure provides immune checkpoint inhibitors that can be administered in combination with the adjuvant compositions, in particular without antigen. Such combination therapies work in concert to enhance an anti-cancer immune response. Certain viruses have also developed mechanisms to co-opt immune checkpoint pathways. Therefore, in certain embodiments, such combination therapy may be used to enhance an anti-viral immune response.

Immune checkpoint inhibitors include any agent that blocks or inhibits in a statistically, clinically, or biologically significant manner, the inhibitory pathways of the immune system. Such inhibitors may include small molecule inhibitors or may include antibodies, or antigen binding fragments thereof, that bind to and block or inhibit immune checkpoint receptors or antibodies that bind to and block or inhibit immune checkpoint receptor ligands. Illustrative immune checkpoint molecules that may be targeted for blocking or inhibition include, but are not limited to, CTLA-4, 4-1BB (CD137), 4-1BBL (CD137L), PDL1, PDL2, PD1, B7-H3, B7-H4, BTLA, HVEM, TIM3, GAL9, LAG3, TIM3, B7H3, B7H4, VISTA, KIR, 2B4 (belongs to the CD2 family of molecules and is expressed on all NK, γδ, and memory CD8⁺ (αβ) T cells), CD160 (also referred to as BY55) and CGEN-15049. Immune checkpoint inhibitors include antibodies, or antigen binding fragments thereof, or other binding proteins, that bind to and block or inhibit the activity of one or more of CTLA-4, PDL1, PDL2, PD1, B7-H3, B7-H4, BTLA, HVEM, TIM3, GAL9, LAG3, TIM3, B7H3, B7H4, VISTA, KIR, 2B4, CD160 and CGEN-15049. Illustrative immune checkpoint inhibitors include Tremelimumab (CTLA-4 blocking antibody), anti-OX40, PD-L1 monoclonal Antibody (Anti-B7-H1; MEDI4736), MK-3475 (PD-1 blocker), Nivolumab (anti-PD1 antibody), CT-011 (anti-PD1 antibody), BY55 monoclonal antibody, AMP224 (anti-PDLl antibody), BMS-936559 (anti-PDLl antibody), MPLDL3280A (anti-PDLl antibody), MSB0010718C (anti-PDLl antibody) and Yervoy/ipilimumab (anti-CTLA-4 checkpoint inhibitor).

In an additional embodiment, the adjuvant compositions herein are administered in combination with a cytokine. By "cytokine" is meant a generic term for proteins released by one cell population that act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha, beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL). As used herein, the term cytokine includes proteins from natural sources or from recombinant cell culture, and biologically active equivalents of the native sequence cytokines.

In certain embodiments, the compositions comprising adjuvants as described herein may be administered in combination with chloroquine or hydroxychloroquine, a lysosomotropic agent that prevents endosomal acidification and which inhibits autophagy induced by tumor cells to survive accelerated cell growth and nutrient deprivation. More generally, the compositions comprising adjuvants as described herein may be administered in combination with therapeutic agents that act as autophagy inhibitors, radiosensitizers or chemosensitizers, such as chloroquine, misonidazole, metronidazole, and hypoxic cytotoxins, such as tirapazamine. In this regard, such combinations with chloroquine or other radio or chemo sensitizer, or autophagy inhibitor, can be used in further combination with other cancer therapeutic agents or with radiation therapy.

In another embodiment, the compositions comprising adjuvants as described herein may be administered in combination with small molecule drugs which are known to result in killing of tumor cells with concomitant activation of immune responses, termed "immunogenic cell death", such as cyclophosphamide, doxorubicin, oxaliplatin and mitoxantrone. Furthermore, combinations with drugs known to enhance the immunogenicity of tumor cells such as patupilone (epothilone B), epidermal-growth factor receptor (EGFR)-targeting monoclonal antibody 7A7.27, histone deacetylase inhibitors (e.g., vorinostat, romidepsin, panobinostat, belinostat, and entinostat), the n3-polyunsaturated fatty acid docosahexaenoic acid, furthermore proteasome inhibitors (e.g. bortezomib), shikonin (the major constituent of the root of Lithospermum erythrorhizon,) and oncolytic viruses, such as TVec (talimogene laherparepvec). In other embodiments, the compositions comprising adjuvants as described herein may be administered in combination with epigenetic therapies, such as DNA methyltransferase inhbitors (*e.g*. Decitabine, 5-aza-2'-deoxycytidine) which may be administered locally or systemically.

In another embodiment, the adjuvant compositions as described herein may be administered in combination with one or more antibodies that increase ADCC uptake of tumor by DCs. Thus, the present invention contemplates combining compositions comprising a GLA with any molecule that induces or enhances the ingestion of a tumor cell or its fragments by an antigen presenting cell and subsequent presentation of tumor antigens to the immune system. These molecules include agents that induce receptor binding (such as Fc or mannose receptors) and transport into the antigen presenting cell such as antibodies, antibody-like molecules, multi-specific multivalent molecules and polymers. Such molecules may either be administered intratumorally with the composition comprising GLA, or administered by a different route. For example, a composition comprising GLA as described herein may be administered intratumorally in conjunction with intratumoral injection of rituximab, cetuximab, trastuzumab, Campath, panitumumab, ofatumumab, brentuximab, pertuzumab, Ado-trastuzumab emtansine, Obinutuzumab, anti-HER1, -HER2, or -HER3 antibodies (e.g., MEHD7945A; MM-111; MM-151; MM-121; AMG888), anti-EGFR antibodies (e.g. Nimotuzumab, ABT-806), or other like antibodies. Any multivalent scaffold that is capable of engaging Fc receptors and other receptors that can induce internalization may be used in the combination therapies described herein- e.g. peptides and/or proteins capable of binding targets that are linked to Fc fragments or polymers capable of engaging receptors.

In certain embodiments, the combination of adjuvants with such antibodies may be further combined with an antibody that promotes a co-stimulatory signal (e.g., by blocking inhibitory pathways), such as anti-CTLA-4, or that activates co-stimulatory pathways such as an anti-CD40, anti-CD28, anti-ICOS, anti-OX40, anti-CD27 antibodies and the like.

The compositions comprising adjuvants may be administered alone or in combination with other known cancer treatments, such as radiation therapy, immune checkpoint inhibitors, chemotherapy or other cancer therapeutic agents, transplantation, immunotherapy, hormone therapy, photodynamic therapy, etc. The compositions may also be administered in combination with antibiotics.

In the combination embodiments described herein, the therapeutic agent being used in combination with the adjuvants described herein may be administered as a separate composition or may be administered in the same composition as the adjuvants described herein. In certain embodiments, the combination therapies are administered concurrently at the same site but may be administered at a different time (before or after the adjuvant composition) and in certain cases at a different site.

A composition comprising the immunogen or a composition comprising a recombinant expression vector that encodes the immunogen or a vector particle comprising the vector are packaged and supplied in separate vials than those containing the adjuvant. Appropriate labels are typically packaged with each composition indicating the intended therapeutic application. The choice of an adjuvant and/or the excipient depends on the stability of the immunogen, recombinant expression vector, and/or vector particle; the route of administration; the dosing schedule; and the efficacy of the adjuvant for the species being vaccinated. For administration in humans, a pharmaceutically acceptable adjuvant is one that has been approved or is approvable for human administration by pertinent regulatory bodies. For example, as discussed herein and known in the art, Complete Freund's adjuvant is not suitable for human administration.

Adjuvants useful for use in the methods described herein are physiologically or pharmaceutically suitable adjuvants for the subject to whom the adjuvant is administered. Adjuvant compositions comprise at least one adjuvant (i.e., one or more adjuvants) and, optionally, at least one physiologically or pharmaceutically suitable (or acceptable) excipient. Any physiological or pharmaceutically suitable excipient or carrier (i.e., a non-toxic material that does not interfere with the activity of the active ingredient) known to those of ordinary skill in the art for use in pharmaceutical compositions may be employed in the adjuvant compositions described herein. Exemplary excipients include diluents and carriers that maintain stability and integrity of the component(s) of the adjuvant. Excipients for therapeutic use are well known, and are described, for example, in Remington: The Science and Practice of Pharmacy (Gennaro, 21st Ed. Mack Pub. Co., Easton, PA (2005)), and are described in greater detail herein.

### Immunogens and Immunogenic Compositions

An immunogen that may be used in these methods includes any immunogen for which induction of a specific immune response is desired. The immunogen is capable of inducing a humoral response (i.e., a B cell response) or a cell-mediated response (including a cytotoxic T cell response) or both. In particular, an immunogen contemplated for use in the methods herein comprise one or more oncogenic virus antigens. In certain embodiments, the immunogen comprises one or more oncogenic virus antigens and one or more tumor-associated antigens. In certain embodiments, one or more oncogenic virus antigen is used as the immunogen for the prime and the pull immunizations. In another embodiment, one or more oncogenic virus antigens is the immunogen in the prime and one or more tumor-associated antigens is the immunogen for the locally administered pull. In certain embodiments, one or more oncogenic virus antigens and one or more tumor-associated antigen are used as the immunogen in the prime and one or more oncogenic virus antigens and one or more tumor-associated antigen are used as the locally administered immunogen for the pull. In particular embodiments, the adjuvant composition (the "pull" composition) does not comprise or is substantially devoid of immunogen.

A cell-mediated immune response includes a helper T cell response (e.g., a CD4 T cell response) or a cytotoxic T lymphocyte response (e.g., a CD8 T cell response), or both, which response may destroy or damage a cell (e.g., a tumor cell, bacterial cell, virus, parasite, or fungal cell) or infectious particle (e.g., a virus particle) that produces or expresses the immunogen. Any antigen from an oncogenic virus that is associated with induction of cancer for which a humoral response or cell-mediated immune response or both is beneficial to the immunized subject can be used as an immunogen. Oncogenic virus antigens are known in the art. An antigen may be a previously identified antigen from an oncogenic virus, or may be identified by any method known in the art. For example, an antigen from an oncogenic virus associated with a type of cancer from which a patient is suffering may be known, or may be identified from the oncogenic virus or from the tumor itself by any of a variety of methods known in the art. One or more such oncogenic virus antigens may be useful for the immunotherapeutic treatment of virus-induced cancers as described herein. Exemplary oncogenic viruses include, but are not limited to, EBV, HPV, HBV, HCV, HTLV, and KSHV. Exemplary viral antigens from oncogenic viruses that can be used herein include but are not limited to, EBV: EBNA-1, LMP-1, LMP-2A; HPV: E6, E7, E5; HBV: HBx; HCV: Core, NS3, Ns5A; HTLV: Tax, HBZ; KSHV: vFLIP, LANA, vGPCR, vIRF-1. In particular embodiments, these immunogens may be delivered to antigen presenting cells, particularly dendritic cells, using the vector particles described herein that comprise a recombinant expression vector.

In certain embodiments, the immunogen comprises an antigen from an oncogenic virus and a tumor-associated antigen. Antigens associated with cancers, such as bladder cancer or Merkel cell carcinoma or other virus-induced cancers as described herein, are well known in the art. Such an antigen may be previously known to be associated with the cancer, or may be identified by any method known in the art. For example, an antigen associated with a type of cancer from which a patient is suffering may be known, such as a tumor-associated antigen, or may be identified from the tumor itself by any of a variety of methods known in the art. In certain embodiments, the immunogen is a tumor-associated antigen (also called herein a tumor antigen) derived from a cancer cell (i.e., tumor cell), and one or more such tumor antigens may be useful for the immunotherapeutic treatment of virus-induced cancer.

Tumor antigens that may be useful as immunogens as described herein and therefore useful for treating any cancer, including bladder cancer or other urogenital cancers include tumor antigens derived from cancers that are characterized by tumor associated antigen expression, such as HER-2/neu expression. Tumor associated antigens that may be used as immunogens include lineage-specific tumor antigens such as the melanocyte-melanoma lineage antigens MART-1/Melan-A, gp100, gp75, mda-7, tyrosinase and tyrosinase-related protein. Illustrative tumor-associated antigens include, but are not limited to, tumor antigens derived from or comprising any one or more of, NY-ESO-1, LAGE-1, CT7, CT10, MAGE 1, 3, and MAGE 4 (or other MAGE antigens such as those disclosed in International Patent Application Publication No. WO99/40188); PRAME; BAGE; RAGE, Lage (also known as NY ESO 1); SAGE; and HAGE (see, e.g., International Patent Application Publication No. WO 99/53061) or GAGE, p53, Ras, c-Myc, cytoplasmic serine/threonine kinases (e.g., A-Raf, B-Raf, and C-Raf, cyclin-dependent kinases), MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, MPHOSPH1 (M phase phosphoprotein-1), DEPDC1 (DEP domain containing-1 protein), decorin, oncofetal proteins IMP3, the polyomavirus BK T-antigen, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, β-catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RARa, Tumor-associated calcium signal transducer 1 (TACSTD1) TACSTD2, receptor tyrosine kinases (e.g., Epidermal Growth Factor receptor (EGFR) (in particular, EGFRvIII), platelet derived growth factor receptor (PDGFR), vascular endothelial growth factor receptor (VEGFR)), cytoplasmic tyrosine kinases (e.g., src-family, syk-ZAP70 family), integrin-linked kinase (ILK), signal transducers and activators of transcription STAT3, STAT5, and STAT6, hypoxia inducible factors (e.g., HIF-1α and HIF-2α), Nuclear Factor-Kappa B (NF-κB), Notch receptors (e.g., Notch1-4), c-Met, mammalian targets of rapamycin (mTOR), WNT, extracellular signal-regulated kinases (ERKs), and their regulatory subunits, PMSA, PR-3, MDM2, Mesothelin, renal cell carcinoma - 5T4, SM22-alpha, carbonic anhydrases I (CAI) and IX (CAIX) (also known as G250), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGs5, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, fos related antigen 1, and idiotype.

Exemplary tumor or tumor cell-derived bladder cancer antigens include cancer-testis antigens (CTA), NY-ESO-1, LAGE-1, MAGE-A1, MAGE-A3, MAGE-A4, MAGE-A10, CT7, CT10, and GAGE. MAGE 1, 3, and MAGE 4 (or other MAGE antigens such as those disclosed in International Patent Application Publication No. WO99/40188); PRAME; BAGE; RAGE, Lage (also known as NY ESO 1); SAGE; and HAGE (see, e.g., International Patent Application Publication No. WO 99/53061) or GAGE (Robbins et al., Curr. Opin. Immunol. 8:628-36 (1996); Van den Eynde et al., Int. J. Clin. Lab. Res. 27:81-86 (1997); Van den Eynde et al., Curr. Opin. Immunol. 9:648-93 (1997); Correale et al. J. Natl. Cancer Inst. 89: 293 (1997)). Furthermore, the recently identified oncoproteins MPHOSPH1 (M phase phosphoprotein-1) and DEPDC1 (DEP domain containing-1 protein), which are highly expressed in bladder cancers and can induce CTL responses in patients (Obara, W., et al., Jpn J Clin Oncol. 2012 Jul;42(7):591-600), and decorin, which has been implicated in invasiveness of bladder cancer cells (El Behi, M., et al., EMBO Mol Med. 2013 Dec;5(12):1835-51). Furthermore, the oncofetal proteins IMP3 and MAGE-A (Xylinas, E., et al., J Urol. 2013 Aug 28. pii: S0022-5347(13)05275-0.) and the polyomavirus BK T-antigen (Alexiev, B.A., et al., Hum Pathol. 2013 May;44(5):908-17).

Immunogens also include tumor antigens that comprise epitopic regions or epitopic peptides derived from genes mutated in tumor cells or from genes transcribed at different levels in tumor cells compared to normal cells, such as telomerase enzyme, survivin, mesothelin, mutated ras, bcr/abl rearrangement, Her2/neu, mutated or wild-type p53, cytochrome P450 1B1, and abnormally expressed intron sequences such as N-acetylglucosaminyltransferase-V; clonal rearrangements of immunoglobulin genes generating unique idiotypes in myeloma and B-cell lymphomas; tumor antigens that comprise epitopic regions or epitopic peptides derived from oncoviral processes, such as human papilloma virus proteins E6 and E7; Epstein bar virus protein LMP2; nonmutated oncofetal proteins with a tumor-selective expression, such as carcinoembryonic antigen and alpha-fetoprotein. See also Boon et al., Ann. Rev. Immunol. 12:337-65 (1994); Renkvist et al., Cancer Immunol. Immunother. 50:3-15 (2001).

Immunogens also include oncogenic virus antigens that comprise or consist of at least one immunogenic fragment of an oncogenic virus protein.

Polypeptides that comprise at least one immunogenic fragment of an immunogenic polypeptide may be used as immunogens to be administered with the adjuvants as described herein and/or encoded by the recombinant expression vectors described herein. An immunogenic fragment comprises at least one T cell epitope or at least one B cell epitope. The immunogenic fragment may consist of at least 8, 9, 10, 15, 20, 30, 40, 50, 60, 70, 80, 90, 100, or more contiguous amino acids of an immunogenic polypeptide. The immunogenic fragments may comprise a sufficient number of contiguous amino acids that form a linear epitope or may comprise a sufficient number of contiguous amino acids that permit the fragment to fold in the same (or sufficiently similar) three-dimensional conformation as the full-length polypeptide from which the fragment is derived and to present a non-linear epitope or epitopes (also referred to in the art as conformational epitopes). The three-dimensional conformation of a polypeptide fragment is sufficiently similar to the full-length polypeptide when the capability to bind and the level of binding of an antibody that specifically binds to the full-length polypeptide is substantially the same for the fragment as for the full-length polypeptide.

Assays for assessing whether the immunogenic fragment folds into a conformation comparable to the full-length polypeptide include, for example, the ability of the protein to react with mono- or polyclonal antibodies that are specific for native or unfolded epitopes, the retention of other ligand-binding functions, and the sensitivity or resistance of the polypeptide fragment to digestion with proteases (see, e.g., Sambrook et al., Molecular Cloning: A Laboratory Manual, 3d ed., Cold Spring Harbor Laboratory Press, NY (2001)). Additional methods for identifying epitopic regions include methods described in Hoffmeister et al., Methods 29:270-281 (2003); Maecker et al., J. Immunol. Methods 255:27-40 (2001). Assays for identifying epitopes are described herein and known to the skilled artisan and include, for example, those described in Current Protocols in Immunology, Coligan et al. (Eds), John Wiley & Sons, New York, NY (1991).

Identifying an immunogenic region and/or epitope of an immunogen of interest can be readily determined empirically by a person skilled in the art or by computer analysis and computer modeling, using methods and techniques that are routinely practiced by persons skilled in the art. Empirical methods include, by way of example, synthesizing polypeptide fragments comprising a particular length of contiguous amino acids of a protein, or generating fragments by use of one or more proteases and then determining the immunogenicity of the fragments using any one of numerous binding assays or immunoassay methods routinely practiced in the art. Exemplary methods for determining the capability of an antibody (polyclonal, monoclonal, or antigen-binding fragment thereof) to specifically bind to a fragment include, but are not limited to, ELISA, radioimmunoassay, immunoblot, competitive binding assays, fluorescence activated cell sorter analysis (FACS), and surface plasmon resonance.

Determination of the three-dimensional structures of a polypeptide, or immunogenic fragment thereof, of interest may be performed by routine methodologies to determine whether the immunogenic fragment retains the spatial positioning of the amino acids as found in the full-length polypeptide. See, for instance, Bradley et al., Science 309: 1868-1871 (2005); Schueler-Furman et al., Science 310:638 (2005); Dietz et al., Proc. Nat. Acad. Sci. USA 103:1244 (2006); Dodson et al., Nature 450:176 (2007); Qian et al., Nature 450:259 (2007). Also available in the art are software tools, for example, PSORT or PSORT II, and Spscan (Wisconsin Sequence Analysis Package, Genetics Computer Group) that are useful for predicting transmembrane segments and membrane topology of polypeptides that are known or believed to traverse a cellular membrane (see, for example, Nakai et al., Trends Biochem. Sci. 24:34-36 (1999)).

Separately, or in combination with the above-described techniques, and given an exemplary amino acid sequence of a polypeptide of interest, a person skilled in the art can identify potential epitopes of the polypeptide (see, e.g., Jameson and Wolf, Comput. Appl. Biosci. 4:181-86 (1988)). By way of another example, Hopp and Woods describe the hydrophilicity method, which is based on empirical demonstrations of the close correlation between the hydrophilicity of polypeptide regions and their antigenicity (see, e.g., Hopp, Pept. Res. 6:183-90 (1993); Hofmann et al., Biomed. Biochim. Acta 46:855-66 (1987)). Computer programs are also available for identifying B cell or T cell epitopes. A BASIC program called EPIPLOT predicts B-cell antigenic sites in proteins from their primary structures by calculating and plotting flexibility, hydrophilicity, and antigenicity profiles using 13 different scales (see, for example, Menendez et al., Comput. Appl. Biosci. 6:101-105 (1990)). See also, such as, Van Regenmortel, Methods: a companion to Methods in Enzymology, 9: 465-472 (1996); Pellequer et al., "Epitope predictions from the primary structure of proteins," In Peptide antigens: a practical approach (ed. G.B. Wisdom), pp. 7-25; Oxford University Press, Oxford (1994); Van Regenmortel, "Molecular dissection of protein antigens" In Structure of antigens (ed. M.H.V. Van Regenmortel), Vol. 1, pp. 1-27. CRC Press, Boca Raton (1992).

T-cell epitopes of an immunogen may also be identified using a peptide motif searching program based on algorithms developed by Rammensee, et al. (Immunogenetics 50: 213-219 (1999)); by Parker, et al. (supra), or by using methods such as those described by Doytchinova and Flower in Immunol. Cell Biol. 80(3):270-9 (2002); Blythe et al., Bioinformatics 18:434-439 (2002); Guan et al., Applied Bioinformatics 2:63-66 (2003); Flower et al., Applied Bioinformatics 1:167-176 (2002); Mallios, Bioinformatics 17: 942-48 (2001); Schirle et al., J. Immunol. Meth. 257:1-16 (2001). T cell epitopes may also be identified using screening assays known in the art, for example using pools of overlapping peptides to stimulate T cells *in vitro* and measuring cell proliferation (proliferation assays) and/or cytotoxic killing (CTL assays).

Epitopic regions of tumor or oncogenic virus antigens that may be used as immunogens in the methods described herein are also described in the art. See by way of example, Lamb et al., Rev. Infect. Dis. Mar - Apr: Suppl 2:s443 - 447 (1989); Lamb et al., EMBO J. 6:1245-49 (1987); Lamb et al., Lepr. Rev. Suppl 2:131-137 (1986); Mehra et al., Proc. Natl. Acad. Sci. USA 83:7013 -27 (1986); Horsfall et al., Immunol. Today 12:211-13 (1991); Rothbard et al., Curr. Top. Microbiol. Immunol. 155:143-52 (1990); Singh et al., Bioinformatics 17:1236-37 (2001); DeGroot et al., Vaccine 19:4385-95 (2001); DeLalla et al., J. Immunol. 163:1725-29 (1999); Cochlovius et al., J. Immunol. 165:4731- 41 (2000); Consogno et al., Blood 101:1039-44 (2003); Roberts et al., AIDS Res. Hum. Retrovir. 12:593-610 (1996); Kwok et al., Trends Immunol. 22:583-88 (2001); Novak et al., J. Immunol. 166:6665-70 (2001).

In certain instances when antigen-specific T-cell lines or clones are available, for example tumor-infiltrating lymphocytes (TIL), virus-specific or bacteria-specific cytotoxic T lymphocytes (CTL), these cells may be used to screen for the presence of relevant epitopes using target cells prepared with specific antigens. Such targets can be prepared using random, or selected, synthetic peptide libraries, which would be used to sensitize the target cells for lysis by the CTL. Another approach to identify a relevant epitope when T cell lines or clones are available is to use recombinant DNA methodologies. Gene or cDNA libraries from CTL-susceptible targets are first prepared and transfected into non-susceptible target cells. This allows the identification and cloning of the gene encoding the protein precursor of the peptide containing the CTL epitope. The second step in this process is to prepare truncated genes from the relevant cloned gene, in order to narrow down the region that encodes for the at least one CTL epitope. This step is optional if the gene is not too large. The third step is to prepare synthetic peptides of, for example, approximately 10-20 amino acids in length, overlapping by 5-10 residues, which are used to sensitize targets for the CTL. When a peptide, or peptides, is shown to contain the relevant epitope, and if desired, smaller peptides can be prepared to establish the peptide of minimal size that contains the epitope. These epitopes are typically, but not necessarily, contained within 9-10 residues for CTL epitopes and up to 20 or 30 residues for helper T lymphocyte (HTL) epitopes.

Alternatively, epitopes may be defined by direct elution of peptides that are non-covalently bound by particular major histocompatibility complex (MHC) molecules followed by amino acid sequencing of the eluted peptides (see, for example, Engelhard et al., Cancer J. 2000 May;6 Suppl 3:S272-80). Briefly, the eluted peptides are separated using a purification method such as HPLC, and individual fractions are tested for their capacity to sensitize targets for CTL lysis or to induce proliferation of cytokine secretion in HTL. When a fraction has been identified as containing the peptide, it is further purified and submitted to sequence analysis. The peptide sequence can also be determined using tandem mass spectrometry. A synthetic peptide is then prepared and tested with the CTL or HTL to corroborate that the correct sequence and peptide have been identified.

Epitopes may also be identified using computer analysis, such as the Tsites program (see, e.g., Rothbard and Taylor, EMBO J. 7:93-100, 1988; Deavin et al., Mol. Immunol. 33:145-155, 1996), which searches for peptide motifs that have the potential to elicit Th responses. CTL peptides with motifs appropriate for binding to murine and human class I or class II MHC may be identified according to BIMAS (Parker et al., J. Immunol. 152:163, 1994) and other HLA peptide binding prediction analyses. Briefly, the protein sequences, for example from microbial components or antigens, or tumor cell components or tumor antigens, are examined for the presence of MHC-binding motifs. These binding motifs, which exist for each MHC allele, are conserved amino acid residues, usually at positions 2 (or 3) and 9 (or 10) for MHC class I binding peptides that are typically 9-10 residues long. Synthetic peptides are then prepared that comprise those sequences bearing the MHC binding motifs, and subsequently such peptides are tested for their ability to bind to MHC molecules. The MHC binding assay can be carried out either using cells which express high numbers of empty (unoccupied) MHC molecules (cellular binding assay), or using purified MHC molecules. Lastly, the MHC binding peptides are then tested for their capacity to induce a CTL response in naive individuals, either in vitro using human lymphocytes, or in vivo using HLA-transgenic animals. These CTL are tested using peptide-sensitized target cells, and targets that naturally process the antigen, such as viral infected cells or tumor cells. To further confirm immunogenicity, a peptide may be tested using an HLA A2 transgenic mouse model and/or any of a variety of in vitro stimulation assays.

In certain embodiments, an immunogen (i.e., an oncogenic virus antigen or a tumor associated antigen) includes polypeptide species that have one or more amino acid substitutions, insertions, or deletions in an amino acid sequence that is known and available in the art for the respective immunogen. Conservative substitutions of amino acids are well known and may occur naturally in the polypeptide or may be introduced when the polypeptide is recombinantly produced. Amino acid substitutions, deletions, and additions may be introduced into a polypeptide using well-known and routinely practiced mutagenesis methods (see, e.g., Sambrook et al. Molecular Cloning: A Laboratory Manual, 3d ed., Cold Spring Harbor Laboratory Press, NY 2001)). Oligonucleotide-directed site-specific (or segment specific) mutagenesis procedures may be employed to provide an altered polynucleotide that has particular codons altered according to the substitution, deletion, or insertion desired. Deletion or truncation variants of immunogens may also be constructed by using convenient restriction endonuclease sites adjacent to the desired deletion. Subsequent to restriction, overhangs may be filled in and the DNA re-ligated. Alternatively, random mutagenesis techniques, such as alanine scanning mutagenesis, error prone polymerase chain reaction mutagenesis, and oligonucleotide-directed mutagenesis may be used to prepare immunogen polypeptide variants (see, e.g., Sambrook et al., supra). Variants of a particular immunogen (or polypeptide fragment thereof) include a polypeptide immunogen that has at least 85%, 90%, 95%, or 99% amino acid sequence identity to any of the exemplary amino acid sequences known in the art.

These polypeptide immunogen variants retain, in a statistically, clinically, or biologically significant manner, the capability to induce an immune response (e.g., a humoral response (i.e., B cell response), cell-mediated response (i.e., T cell response (including a cytotoxic T lymphocyte response)) or both a humoral and cell-mediated response in a subject. Given the many molecular biology, protein expression, and protein isolation techniques and methods routinely practiced in the art for introducing mutations in a polypeptide, preparing polypeptide fragments, isolating the fragments and variants, and analyzing same, immunogen polypeptide variants and fragments having the desired ability to induce an immune response can be made readily and without undue experimentation.

A variety of criteria known to persons skilled in the art indicate whether an amino acid that is substituted at a particular position in a peptide or polypeptide is conservative (or similar). For example, a similar amino acid or a conservative amino acid substitution is one in which an amino acid residue is replaced with an amino acid residue having a similar side chain. Similar amino acids may be included in the following categories: amino acids with basic side chains (e.g., lysine, arginine, histidine); amino acids with acidic side chains (e.g., aspartic acid, glutamic acid); amino acids with uncharged polar side chains (e.g., glycine, asparagine, glutamine, serine, threonine, tyrosine, cysteine, histidine); amino acids with nonpolar side chains (e.g., alanine, valine, leucine, isoleucine, proline, phenylalanine, methionine, tryptophan); amino acids with beta-branched side chains (e.g., threonine, valine, isoleucine), and amino acids with aromatic side chains (e.g., tyrosine, phenylalanine, tryptophan). Proline, which is considered more difficult to classify, shares properties with amino acids that have aliphatic side chains (e.g., leucine, valine, isoleucine, and alanine). In certain circumstances, substitution of glutamine for glutamic acid or asparagine for aspartic acid may be considered a similar substitution in that glutamine and asparagine are amide derivatives of glutamic acid and aspartic acid, respectively. As understood in the art "similarity" between two polypeptides is determined by comparing the amino acid sequence and conserved amino acid substitutes thereto of the polypeptide to the sequence of a second polypeptide (e.g., using GENEWORKS, Align, the BLAST algorithm, or other algorithms described herein and practiced in the art).

As described herein for immunogenic fragments, assays for assessing whether a respective variant folds into a conformation comparable to the non-variant polypeptide or fragment include, for example, the ability of the protein to react with mono- or polyclonal antibodies that are specific for native or unfolded epitopes, the retention of ligand-binding functions, and the sensitivity or resistance of the mutant protein to digestion with proteases (see Sambrook et al., supra). Such variants can be identified, characterized, and/or made according to methods described herein or other methods known in the art, which are routinely practiced by persons skilled in the art.

Immunogenic compositions that are administered to a subject according to the methods described herein may include at least one pharmaceutically (or physiologically) suitable excipient. Any physiological or pharmaceutically suitable excipient or carrier (i.e., a non-toxic material that does not interfere with the activity of the active ingredient) known to those of ordinary skill in the art for use in pharmaceutical compositions may be employed in the immunogenic compositions described herein. Exemplary excipients include diluents and carriers that maintain stability and integrity of proteins. Excipients for therapeutic use are well known, and are described, for example, in Remington: The Science and Practice of Pharmacy (Gennaro, 21st Ed. Mack Pub. Co., Easton, PA (2005)), and are described in greater detail herein.

### Methods and Compositions for Treating Cancer

Any of the compositions described herein can be used in a method of treatment of a human or animal subject. The method may comprise administering the compositions to the subject as described herein.

In one embodiment, methods are provided herein for treating cancer by inducing an immune response specific for a cancer-associated antigen optionally in combination with one or more other antigens. In particular, the methods provided herein comprise inducing an immune response specific for an immunogen by administering to a subject in need thereof a composition comprising a vector particle comprising a recombinant expression vector, which recombinant expression vector comprises a polynucleotide encoding the immunogen (also called herein a first composition or an immunogenic composition), wherein the polynucleotide is operatively linked to at least one regulatory expression sequence. Concurrently or sequentially, a second composition comprising a pharmaceutically suitable adjuvant (also called herein an adjuvant composition) substantially devoid of antigen is administered to the subject. In certain embodiments, the second composition comprising a pharmaceutically suitable adjuvant may also comprise an immunogen. Generally, the first "prime" composition is administered systemically (e.g., by intradermal, intramuscular or subcutaneous injection) and the second composition (e.g. an adjuvant composition) is administered locally, such as intratumorally, peritumorally, intranodally, perinodally, mucosally or intravesically.

In one embodiment, methods are provided herein for treating virus-induced cancer by inducing an immune response specific for an oncogenic virus antigen optionally in combination with a tumor associated antigen where the cancer is associated with the oncogenic virus. In particular, the methods provided herein comprise inducing an immune response specific for an immunogen by administering to a subject in need thereof a composition comprising a vector particle comprising a recombinant expression vector, which recombinant expression vector comprises a polynucleotide encoding the immunogen (also called herein a first composition or an immunogenic composition), wherein the polynucleotide is operatively linked to at least one regulatory expression sequence. Concurrently or sequentially, a second composition comprising a pharmaceutically suitable adjuvant (also called herein an adjuvant composition) is administered to the subject. In certain embodiments, the second composition comprising a pharmaceutically suitable adjuvant may also comprise an immunogen. In certain embodiments, the first composition comprises an oncogenic virus antigen and is administered systemically (e.g., by intradermal, intramuscular or subcutaneous injection). The second composition (e.g. an adjuvant composition) is, in certain embodiments, administered locally, such as intratumorally or intravesically, and may optionally comprise the oncogenic virus antigen or antigens administered in the first composition and or a tumor associated antigen as described herein.

In one embodiment, methods are provided herein for treating bladder cancer by inducing an immune response specific for an immunogen by administering to a subject in need thereof a composition comprising a vector particle comprising a recombinant expression vector, which recombinant expression vector comprises a polynucleotide encoding the immunogen (also called herein a first composition or an immunogenic composition), wherein the polynucleotide is operatively linked to at least one regulatory expression sequence. Concurrently or sequentially, a second composition comprising a pharmaceutically suitable adjuvant (also called herein an adjuvant composition) is administered to the subject.

In the methods described herein, the adjuvant composition may or may not include BCG, according to various embodiments of the present disclosure. The adjuvant composition may be administered at a time and in a manner sufficient that the adjuvant enhances the immune response to the immunogen; that is, the level of the immune response specific for the immunogen (i.e., humoral response, cell-mediated response, or both a humoral response and cell-mediated response) is greater (or increased) in a statistically, clinically, and/or biologically significant manner compared with the level of the immune response specific to the immunogen when the immunogen is administered in the absence of the adjuvant. In those instances when an antigen is incapable of inducing a detectable specific immune response in the absence of an adjuvant, induction of a detectable, specific immune response represents enhancement of the immune response. In other embodiments, the adjuvant composition is administered at a time and in a manner sufficient that the adjuvant recruits T-cells (e.g., the CD8 T-cells induced following administration of the first immunogenic composition) to the local administration site (e.g., the tumor or the mucosa of the bladder).

According to various embodiments of the present disclosure, the first immunogenic composition acts as a "priming" composition or optionally a "prime/boosting" composition (see for example, WO2012/141984 which describes prime-boost regimens useful herein). The first immunogenic composition comprises a vector particle and/or a recombinant expression vector and, optionally, an adjuvant such as a TLR agonist described herein. As described herein, the vector particle/expression vector and optionally an adjuvant may be in the same or different compositions, and may be administered at the same or different sites, times and routes. In certain embodiments, the second composition, or adjuvant composition, comprises an adjuvant and, optionally, BCG. The adjuvant and BCG, according to various embodiments, may be in the same or different compositions, and may be admininstered at the same or different sites, times and routes. In certain embodiments, the second composition, or adjuvant composition, comprises an adjuvant and, optionally, BCG and/or a tumor-associated antigen, e.g., an antigen associated with bladder cancer.

Inducing an immune response using the prime-pull immunization methods and immunogenic compositions described herein may be accomplished by employing a variety of different regimens. An exemplary, nonexhaustive list of immunization regimens comprise administering:
a) a first composition comprising an expression vector that encodes and expresses an immunogen; and/or
b) a first composition comprising an expression vector that encodes and expresses an immunogen and also comprising an adjuvant;
c) a first composition comprising autologous or heterologous tumor antigen-specific T cells that have been expanded *ex vivo,* genetically modified with a chimeric antigen receptor to recognize a tumor-associated antigen (CAR-T cells), or T cells genetically modified to express a specific chimeric T cell receptor (CAR-TCR cells) that recognizes a cancer-associated epitope in the context of MHC;
   wherein either a) or b) or c) is administered multiple times, in any order (with respect to each other), and may be administered concurrently, sequentially and at different or the same administration sites and routes as the following second compositions:
   i) a composition comprising an adjuvant without an immunogen;
   ii) a composition comprising an adjuvant with BCG;
   iii) a composition comprising an adjuvant with an immunogen; and/or
   iv) a composition comprising an adjuvant with an immunogen and BCG.

Although exemplary regimens described herein are described as "prime-pull" regimens, one will appreciate that the compositions provided above may be administered multiple times, in any order, and may be administered concurrently, sequentially and at different or the same administration sites and routes. As one non-limiting example, the present disclosure contemplates a "priming" administration with either composition a) or b) or c), above, optionally, one or more"boosting" administration with either composition a) or b), or c) above, and a "pulling" administration with either composition i) - iv), above. The priming, boosting and pulling administrations/compositions may be administered multiple times, in any order, and may be administered concurrently, sequentially and at different or the same administration sites and routes. The "boosting" composition in certain embodiments comprises an adjuvant as described herein in combination with an immunogen, in particular embodiments, the same immunogen expressed by the expression vector in the first composition (prime) that encodes and expresses an immunogen.

In certain embodiments, the methods described herein comprise administering the adjuvant and the recombinant expression vector in the same composition. In other embodiments, the methods described herein comprise administering the adjuvant and the recombinant expression vector in two separate compositions and either at different times, at different sites, and/or via different routes of administration. In certain embodiments, the vector particle comprising the recombinant expression vector encoding the immunogen(s) of interest and the adjuvant are not combined together in a single composition. In other words, the composition comprising the vector particle that comprises the recombinant expression vector that encodes an immunogen (i.e., immunogenic composition) lacks an adjuvant, and the composition comprising the adjuvant lacks the recombinant expression vector or lacks the vector particle comprising the vector that encodes the immunogen of interest. In other particular embodiments of the methods described herein, when a composition comprising the immunogen-encoding vector and a composition comprising the adjuvant are administered at the same time (i.e., concurrently), each composition is administered at a different site. When each composition is administered at a different site, each composition may be administered via the same or different routes. Alternatively, each composition may be administered via a different route at the same site. In further embodiments, the adjuvant and the recombinant expression vector are administered in the same composition.

In certain embodiments, the "pull" composition (e.g. a composition comprising a TLR4 agonist) may be administered at one, two, three, four, five, six, seven, eight, nine, ten, or more local sites, either concurrently or sequentially (either concurrently or sequentially to eachother or concurrently or sequentially to the "prime" administration; or both). As one example, an antigen-specific immune response generated by the "prime" administration can be "pulled" to one or more tumors, such as at a primary tumor site and/or one or more metastatic tumor sites by local (e.g., by peritumoral or intratumoral) administration of a composition comprising a suitable adjuvant (e.g., a TLR4 agonist such as GLA described herein). As an additional non-limiting example, in certain embodiments, a primary tumor site may not be accessible for administration of the "pull" composition, but one or more metastatic sites may be accessible. In certain embodiments, administration at one or more local tumor sites causes infiltration of antigen-specific T cells to the tumor where the "pull" composition was administered and in some embodiments, an abscopal effect is also observed whereby administration at one local tumor site also results in infiltration of T cells at a distant tumor site and reduction in the size of the distant tumor.

In certain embodiments, the immunogenic "prime" composition (e.g., a composition comprising a lentiviral vector encoding a cancer-associated antigen, and optionally including one or more other antigens; or a composition comprising adoptively transferred T cells, antigen-specific or genetically modified CAR-T cells) and the "pull" adjuvant composition (e.g., a composition comprising a TLR4 agonist) are administered concurrently to the subject. When the "prime" and "pull" compositions are administered concurrently, the compositions are administered within 5 to 120 minutes of eachother. In certain embodiments, the immunogenic composition (the "prime") is administered within one hour of the adjuvant composition (the "pull"). Generally, concurrent administration is within a short a period of time, as is feasible for safe and acceptable administration for the patient and the clinical staff. In embodiments where the prime composition comprises adoptively transferred T cells, administration of the composition comprising T cells may be over a certain period of time (e.g., infusion of T cells may be over the course of one hour, two hours or three hours) and the concurrent administration of the pull composition follows completion of the infusion of cells but is generally within 5 to 120 minutes following the infusion or as is safe for the patient and determined by the skilled clinician.

In embodiments wherein the prime and the pull compositions are administered concurrently, the immunogenic composition (prime) may be administered via one route (i.e., a first route) and the adjuvant composition is administered via a second, different route. In one embodiment, the prime composition is administered systemically (e.g., by intradermal, intramuscular or subcutaneous injection) and the pull composition is administered locally (e.g., intratumorally, peritumorally, intranodally, intravesically). Routes of administration from which the first and second routes may be independently selected include, but are not limited to, topical, oral, enteral, nasal (i.e., intranasal), inhalation, intrathecal, rectal, vaginal, intraocular, subconjunctival, sublingual, intradermal, transdermal, or parenteral administration, including subcutaneous, percutaneous, intravenous, intramuscular, intratumoral, intranodal, intrasternal, intracavernous, intravesical, intrameatal or intraurethral injection or infusion. In certain more particular embodiments, the first and second routes are different and each selected from parenteral, oral, enteral, sublingual, intranasal, intramuscular, intradermal, subcutaneous, intratumoral, intranodal, percutaneous, transdermal, intravesical and topical. In a more specific embodiment, the first and second routes are different and selected from intramuscular, subcutaneous, percutaneous, intratumoral, intranodal, intranasal, intravesical and oral. In still another embodiment, the first immunogenic composition is administered intradermally, intramuscularly or subcutaneously without adjuvant, and the adjuvant composition is administered locally at the site of the cancer, e.g. intratumorally or intravesically, with or without an immunogen, and in certain embodiments, such as for the treatment of bladder cancer, with or without BCG. The immunogenic and adjuvant compositions are formulated appropriately for delivery by different routes as described in the art and discussed in greater detail herein.

In other embodiments described herein, when the "prime" immunogenic composition and the adjuvant composition are each administered concurrently to the subject, the compositions may be administered at different sites of the subject. The different sites are sufficiently physically separated from each other to permit induction or enhancement of the immune response to the immunogen. When each composition is administered at a different site, the compositions may be administered via the same route or may be administered by different routes. By way of example and for purposes of illustration only, the immunogenic composition may be administered subcutaneously or intramuscularly to an extremity (e.g., an arm) of the subject and the adjuvant composition may be administered subcutaneously or intramuscularly, respectively, to a different extremity (e.g., a leg) of the subject. By way of an additional example, administration of each composition concurrently at different sites but by the same route may include administration of the immunogenic composition to an extremity (e.g., an arm or a leg) and administration of the adjuvant composition to another (or second) extremity of the same type. In other particular embodiments, when the immunogenic composition and the adjuvant composition are administered concurrently at the same site, the routes of administration for each of the immunogenic and adjuvant compositions are different and each is selected from oral, enteral, parenteral, intramuscular, intradermal, subcutaneous, intratumoral, intranodal, percutaneous, transdermal, sublingual, intravesical and topical. By way of example, one composition may be delivered orally to be ingested, and the second composition may be delivered sublingually. As another example, one composition may be delivered intramuscularly at a site, and the second composition delivered subcutaneously or percutaneously at approximately the same site (e.g., the same arm or the same leg).

Selection of a route of administration will depend upon a number of factors, including the composition delivered, the age of the subject, and the body mass of the subject. The route of administration and the site of administration are typically chosen to maximize the amount of an active ingredient in a composition delivered to the subject in the safest manner. Typical sites for intramuscular administration of an immunogenic composition and/or adjuvant composition include the anterolateral thigh muscle and the deltoid muscle. In humans, intramuscular injection of the deltoid muscle is typically used by persons skilled in the vaccine art to deliver a vaccine to adults, and in certain instances to children and teens and toddlers between 1 and 2 years old. The vastus lateralis muscle in the anterolateral thigh is typically recommended for intramuscular injection of infants (i.e., less than one year of age) and may also be the site of intramuscular delivery in older children and adults. Alternatively, the site of an intramuscular injection in humans may be the ventrogluteal area. Those skilled in the art appreciate that suboptimal delivery of a vaccine may occur if the immunogenic composition is delivered to a dorsogluteal site or upper outer quadrant of the buttock.

By way of example, intravesical administration of an adjuvant composition described herein includes administration of the composition directly into the bladder (e.g., through a catheter) rather than giving it by mouth or injecting it into a vein. Compositions given this way primarily affect the cells lining the bladder (mucosa). Multiple administrations of the adjuvant composition may be neccessary since the permeability of the urothelial layer is very low and instilled drug solutions may become diluted with urine and get washed out of the bladder during voiding,. In various embodiments, permeation enhancers, such as chitosan and dimethylsulphoxide, may be used to temporarily disrupt the tight packing of the urothelium and nanocarriers such as liposomes, gelatin nanoparticles, polymeric nanoparticles and magnetic particles can be used to enhance local drug concentrations in the bladder as well as target diseased cells. In other embodiments, intravesical drug carriers are improved by using mucoadhesive biomaterials which strongly adhere to the urothelial cell lining, thus preventing the carrier from being washed away during urine voiding. Polymeric hydrogels, such as the temperature sensitive PEG-PLGA-PEG polymer, have been used to develop *in situ* gelling systems to deliver drugs into the bladder cavity (GuhaSarkar, S., et al., J Control Release. 2010 Dec 1;148(2):147-59).

By way of example, typical sites for subcutaneous administration of an immunogenic or an adjuvant composition include fatty tissue over the anterolateral thigh muscle or fatty tissue over the triceps. The thigh muscle is the preferred site for subcutaneous administration of a composition to a human infant. Percutaneous administration may be at the deltoid muscle or at the anterolateral thigh muscle.

In another embodiment, the first composition comprising a vector particle comprising a recombinant expression vector that comprises a polynucleotide encoding at least one immunogen and the second composition comprising an adjuvant are administered sequentially. In certain embodiments, the immunogenic composition is administered prior to the adjuvant composition (i.e., the adjuvant composition is administered subsequent to administration of the immunogenic composition). In other certain embodiments, the adjuvant composition is administered prior to administration of the immunogenic composition (i.e., the immunogenic composition is administered subsequent to administration of the adjuvant composition).

In certain embodiments where the "prime" composition comprises adoptively transferred T cells, the prime composition and the suitable "pull" composition, such as a TLR4 agonist (*e.g*., GLA) or other adjuvant composition as described herein, are administered at the same time or may be administered sequentially. Multiple administrations of adoptively transferred cells may be administered prior to administration of a "pull" composition.

Thus, in certain embodiments, the present disclosure provides a method of treating a cancer in a subject by 1) administering to the subject a first composition comprising tumor-associated antigen-specific T cells that have been expanded or modified *ex vivo*, genetically modified CAR-T cells that have been modified genetically to recognize a tumor-associated antigen, or T cells genetically modified to express a specific chimeric T cell receptor (TCR) that recognizes a cancer-associated epitope in the context of MHC; 2) administering locally or intra-tumorally, either concurrently or subsequently, to the subject a second composition comprising a pharmaceutically suitable adjuvant as described herein to pull the adoptively transferred cells to the tumor; thereby treating the cancer in the subject. In certain embodiments, the adoptively transferred cells may be boosted by administering to the subject an expression vector as described herein prior to the local administration of the second composition to pull the adoptively (and then boosted) cells to the local site (e.g., to a tumor).

In embodiments described herein where the immunogenic composition and the adjuvant composition are each administered sequentially to the subject in need thereof, each administration is separated by hours or by days. Where the adjuvant composition is administered sequentially to the immunogenic composition, the adjuvant composition is administered at a time interval such that the immune response recruited to the site of administration by the adjuvant composition is detectable. In certain embodiments, the immunogenic composition is administered at least one, at least two, at least three, at least four, at least five, at least six, least seven, at least eight, at least nine, at least ten, or at least 12 hours, or at least 18 hours prior to administration of the adjuvant composition. In other particular embodiments, the immunogenic composition is administered at least one, at least two, at least three, at least four, at least five, at least six, or at least seven days prior to administration of the adjuvant composition. In other embodiments, the immunogenic composition is administered between one and 36 days prior to administration of the adjuvant composition. The immunogenic composition may be administered 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 days prior to administration of the adjuvant composition. In yet other certain embodiments, the adjuvant composition is administered prior to the immunogenic composition and is administered at least one, at least two, at least three, at least four, at least five, at least six, least seven, at least eight, at least nine, at least ten, or at least 12 hours, or at least 18 hours prior to administration of the immunogenic composition. In still other embodiments described herein, the adjuvant composition is administered prior to the immunogenic composition and is administered at least one, at least two, at least three, at least four, at least five, at least six, or at least seven days prior to administration of the immunogenic composition. In other embodiments, the adjuvant composition is administered between one and 36 days prior to administration of the immunogenic composition. The adjuvant composition may be administered 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 days prior to administration of the immunogenic composition. The optimum time interval between administrations of each composition may be determined by a person skilled in the art by appropriately designed pre-clinical and clinical trial studies. The optimum time interval may depend on the immunogen of interest and/or the particular adjuvant administered.

In still other embodiments, the immunogenic composition and the adjuvant composition are each administered sequentially to the subject in need thereof, more than once (e.g., twice, three times, or four times). In a specific embodiment, the adjuvant composition may be administered the same number of times as the immunogenic composition. In yet another embodiment, the adjuvant composition may be administered a lesser number of times than the immunogenic composition. By way of non-limiting example, when the immunogenic composition is administered more than once, the adjuvant composition may be administered only prior to or subsequent to administration of the first administration of the immunogenic composition and not prior to or subsequent to any subsequent (i.e., second, third, or fourth) administration of the immunogenic composition. In yet another specific embodiment, the adjuvant composition may be administered more times than the immunogenic composition is administered.

In other embodiments, the adjuvant composition may be administered at least one time more than the immunogenic composition. For example, the adjuvant is capable of inducing an innate (or nonspecific) immune response and may be administered at a sufficient time interval prior to or after administration of the immunogenic composition to induce or stimulate an innate immune response. Without being bound by theory, this response includes local induction of cytokines and chemokines that recruit antigen-sepcific T cells to the site of administration. The adjuvant composition may then also be administered concurrently (which concurrent administration may be at a different site or via a different route) or sequentially with the first administration of the immunogenic composition.

When the adjuvant composition and the immunogenic composition are administered sequentially, each of the compositions may be administered via the same route or may be administered by different routes. Routes of administration for delivery of each of the adjuvant and immunogenic compositions may be independently selected include, but are not limited to, topical, oral, enteral, nasal (i.e., intranasal), inhalation, intrathecal, rectal, vaginal, intraocular, subconjunctival, sublingual, intradermal, intratumoral, peritumoral, intranodal, perinodal, transdermal, or parenteral administration, including subcutaneous, percutaneous, intravenous, intramuscular, intrasternal, intracavernous, intravesical, intrameatal or intraurethral injection or infusion. In certain embodiments, the first and second routes are different and each is selected from parenteral, oral, enteral, sublingual, intranasal, intramuscular, intradermal, intratumoral, intranodal, subcutaneous, percutaneous, transdermal, and topical.

When the immunogenic composition and the adjuvant composition are administered to the subject by the same route, each of the compositions may be administered at the same site on the subject or may be administered at different sites on the subject. In other specific embodiments, when the adjuvant composition and the immunogenic composition are administered sequentially, each composition may be delivered by a different route but at sites sufficiently proximal to be considered the same site. By way of non-limiting example, an immunogenic composition may be administered intramuscularly into an extremity of the subject (e.g., an arm (deltoid muscle) or a leg (thigh muscle)), and prior to or subsequent to administration of the immunogenic composition, the adjuvant composition is administered subcutaneously at the same site on the extremity of the subject (e.g., the same arm (deltoid muscle) or the same leg (thigh muscle), respectively). By way of a second non-limiting example, an adjuvant composition may be administered intramuscularly into an extremity of the subject (e.g., an arm (deltoid muscle) or a leg (thigh muscle)), and prior to or subsequent to administration of the adjuvant composition, the immunogenic composition is administered subcutaneously at the same site on the extremity of the subject (e.g., the same arm (deltoid muscle) or the same leg (thigh muscle), respectively).

As discussed herein, the choice of site and route of administration may depend on factors including but not necessarily limited to the age, health status, and/or size of the subject to be immunized; the recombinant expression vector or vector particle comprising the vector; the immunogen(s) encoded by the recombinant expression vector; and/or the adjuvant. The condition, disease, or disorder to be treated or prevented by administration of an immunogenic composition and the type of immune response desired may be additional factors considered by a person skilled in the art in determining the route of administration and the site of administration for maximizing the therapeutic and/or prophylactic benefit of the immunogenic and adjuvant compositions.

During the course of an immunization protocol or regimen, the level of the immune response to the immunogen may be monitored. Thus, the number of times the immunogenic composition and the adjuvant composition are each administered to the subject may be determined by monitoring the level of the immune response to the immunogen after each administration of the immunogenic composition. Techniques and methods for monitoring an immune response are routinely practiced in the art and are described herein and in the art.

### Immune Response

As described herein, methods are provided for inducing an immune response to an immunogen and in particular embodiments, for attracting or recruiting the induced cells to a site of interest (e.g., to a tumor; mucosal site of infection). Cells of the immune system that are involved in an immune response are referred to, generally, as immune cells and include a lymphocyte and a non-lymphoid cell such as accessory cell. Lymphocytes are cells that specifically recognize and respond to foreign antigens, and accessory cells are those that are not specific for certain antigens but are involved in the cognitive and activation phases of immune responses. For example, mononuclear phagocytes (macrophages), other leukocytes (e.g., granulocytes, including neutrophils, eosinophils, basophils), and dendritic cells function as accessory cells in the induction of an immune response. The activation of lymphocytes by a foreign antigen leads to induction or elicitation of numerous effector mechanisms that function to eliminate the antigen. Accessory cells such as mononuclear phagocytes that affect or are involved with the effector mechanisms are also called effector cells.

Major classes of lymphocytes include B lymphocytes (B cells), T lymphocytes (T cells), and natural killer (NK) cells, which are large granular lymphocytes. B cells are capable of producing antibodies. T lymphocytes are further subdivided into helper T cells (CD4+ (also referred to herein and in the art as CD4)) and cytolytic or cytotoxic T cells (CD8+ (also referred to herein and in the art as CD8)). T cells are also described in the art as antigen-specific CD4 and/or CD8 T cells, effector memory T cells (T_{EM}), central memory T cells (T_{CM}) and/or tissue-resident memory T cells (T_{RM}). Helper cells secrete cytokines that promote proliferation and differentiation of the T cells and other cells, including B cells and macrophages, and recruit and activate inflammatory leukocytes. Another subgroup of T cells, called regulatory T cells or suppressor T cells actively suppress activation of the immune system and prevent pathological self-reactivity, that is, autoimmune disease.

The methods described herein for inducing an immune response may induce a humoral response, also called a B cell response herein and in the art, or may induce a cell-mediated immune response involving various types of T cells (i.e., T lymphocytes). A humoral response includes production of antibodies that specifically bind to an antigen (or immunogen). Antibodies are produced by differentiated B lymphocytes known as plasma cells. In a cell mediated response, the various types of T lymphocytes act to eliminate an antigen by a number of mechanisms. For example, helper T cells that are capable of recognizing specific antigens may respond by releasing soluble mediators such as cytokines to recruit additional cells of the immune system to participate in an immune response. Also, cytotoxic T cells are capable of specifically recognizing an antigen and may respond by binding to and destroying or damaging an antigen-bearing cell or particle.

An immune response in a host or subject may be determined by any number of well-known immunological methods described herein and with which those having ordinary skill in the art will be familiar. As described herein, methods and techniques for determining the presence and level of an immune response include, for example, fluorescence resonance energy transfer, fluorescence polarization, time-resolved fluorescence resonance energy transfer, scintillation proximity assays, reporter gene assays, fluorescence quenched enzyme substrate, chromogenic enzyme substrate and electrochemiluminescence, immunoassays, (such as enzyme-linked immunosorbant assays (ELISA), radioimmunoassay, immunoblotting, immunohistochemistry, and the like), surface plasmon resonance, cell-based assays such as those that use reporter genes, and functional assays (e.g., assays that measure immune function and immunoresponsiveness).

Such assays include, but need not be limited to, in vivo or in vitro determination of the presence and level of soluble antibodies, soluble mediators such as cytokines (e.g., IFN-γ, IL-2, IL-4, IL-10, IL-12, IL-6, IL-23, TNF-α, and TGF-β), lymphokines, chemokines, hormones, growth factors, and the like, as well as other soluble small peptide, carbohydrate, nucleotide and/or lipid mediators. Immunoassays also include determining cellular activation state changes by analyzing altered functional or structural properties of cells of the immune system, for example, cell proliferation, altered motility, induction of specialized activities such as specific gene expression or cytolytic behavior; cell maturation, such as maturation of dendritic cells in response to a stimulus; alteration in relationship between a Th1 response and a Th2 response; cellular differentiation by cells of the immune system, including altered surface antigen expression profiles or the onset of apoptosis (programmed cell death). Other methods are also available for measuring cell surface markers to identify various populations of immune cells, such as but not limited to antigen-specific CD4 and/or CD8 T cells, effector memory T cells (T_{EM}), central memory T cells (T_{CM}) and/or tissue-resident memory T cells (T_{RM}). Procedures for performing these and similar assays are may be found, for example, in Lefkovits (Immunology Methods Manual: The Comprehensive Sourcebook of Techniques, 1998). See also Current Protocols in Immunology; Weir, Handbook of Experimental Immunology, Blackwell Scientific, Boston, MA (1986); Mishell and Shigii (eds.) Selected Methods in Cellular Immunology, Freeman Publishing, San Francisco, CA (1979); Green and Reed, Science 281:1309 (1998) and references cited therein).

Determining the presence and/or level of antibodies that specifically bind to an immunogen of interest may be determined using any one of several immunoassays routinely practiced in the art, including but not limited to, ELISAs, immunoprecipitation, immunoblotting, countercurrent immunoelectrophoresis, radioimmunoassays, dot blot assays, inhibition or competition assays, and the like (see, e.g., U.S. Patent Nos. 4,376,110 and 4,486,530; Harlow et al., Antibodies: A Laboratory Manual, Cold Spring Harbor Laboratory (1988)). Immunoassays may also be performed to determine the class and isotype of an antibody that specifically binds to an immunogen. Antibodies (polyclonal and/or monoclonal or antigen-binding fragments thereof) that specifically bind to an immunogen and which may be used as controls in immunoassays detecting an antibody-specific immune response in an immunized subject, may generally be prepared by any of a variety of techniques known to persons having ordinary skill in the art. See, e.g., Harlow et al., Antibodies: A Laboratory Manual, Cold Spring Harbor Laboratory (1988); Peterson, ILAR J. 46:314-19 (2005); (Kohler et al., Nature, 256:495-97 (1976); Kohler et al., Eur. J. Immunol. 6:511-19 (1975); Coligan et al. (eds.), Current Protocols in Immunology, 1:2.5.1-2.6.7 (John Wiley & Sons 1991); U.S. Patent Nos. 4,902,614, 4,543,439, and 4,411,993; Monoclonal Antibodies, Hybridomas: A New Dimension in Biological Analyses, Plenum Press, Kennett et al. (eds.) (1980); Antibodies: A Laboratory Manual, Harlow and Lane (eds.), Cold Spring Harbor Laboratory Press (1988); see also, e.g., Brand et al., Planta Med. 70:986-92 (2004); Pasqualini et al., Proc. Natl. Acad. Sci. USA 101:257-59 (2004). The immunogen, or immunogenic fragments thereof, or a cell or particle bearing the immunogen or immunogenic fragment thereof may be used for immunizing an animal for production of either polyclonal antibodies or monoclonal antibodies.

Levels of cytokines may be determined according to methods described herein and practiced in the art, including for example, ELISA, ELISPOT, intracellular cytokine staining, and flow cytometry and combinations thereof (e.g., intracellular cytokine staining and flow cytometry). Immune cell proliferation and clonal expansion resulting from an antigen-specific elicitation or stimulation of an immune response may be determined by isolating lymphocytes, such as spleen cells or cells from peripheral blood, apheresis samples, lymph nodes, stimulating the cells with antigen, and measuring cell surface markers, cytokine production, cell proliferation and/or cell viability, such as by incorporation of tritiated thymidine or non-radioactive assays, such as MTT assays and the like. The effect of an immunogen described herein on the balance between a Th1 immune response and a Th2 immune response may be examined, for example, by determining levels of Th1 cytokines, such as IFN-γ, IL-12, IL-2, and TNF-β, and Type 2 cytokines, such as IL-4, IL-5, IL-9, IL-10, and IL-13.

The level of an antigen-specific T cells response, e.g., the level of a CTL immune response and/or the level of a memory CD4 T cell response, may be determined by any one of numerous immunological methods described herein and routinely practiced in the art. The level of a CTL immune response may be determined prior to administration of any one of the compositions, vectors, or vector particles described herein and then used for comparison with the level of CTL immune response at an appropriate time point and/or location after one or more administrations of the compositions, vectors, or vector particles that provide memory CD4 T cell help. Cytotoxicity assays for determining CTL activity may be performed using any one of several techniques and methods routinely practiced in the art (see, e.g., Henkart et al., "Cytotoxic T-Lymphocytes" in Fundamental Immunology, Paul (ed.) (2003 Lippincott Williams & Wilkins, Philadelphia, PA), pages 1127-50, and references cited therein).

For example, when the prime composition comprising the vector particles containing the polynucleotide encoding the immunogen and the pull composition comprising an adjuvant composition are administered according to the methods described herein, the level of the CTL immune response, or CD8+ T cell response, is enhanced (improved), in particular at the site of administration of the pull composition comprising an adjuvant. For example, a 50% improvement may be observed when measured by functional T cell assays such as intracellular cytokine staining; ELISPOT; or measurement of soluble cytokine secretion by Luminex for 1-4 weeks after administration of both compositions. In this regard, the improvement is generally as compared to an appropriate control, such as in the absence of administering a composition comprising an adjuvant as described herein.

In particular embodiments, a 2-50 fold increase in locally infiltrating antigen-specific T cells is observed following the methods described herein. In certain embodiments, a 2-40 fold increase, a 2-30 fold increase, a 2-20 fold increase, a 2-10 fold increase, 3-8 fold increase, a 4-7 fold increase, or a 5-6 fold increase in locally infiltrating (e.g., tumor-inflitrating) antigen-specific T cells is observed. Generally, the increase in locally infiltrating antigen-specific T cells is as compared to the number of locally infiltrating antigen-specific T cells present in the absence of a "pull" administration or as compared to an appropriate control administration. Generally, the methods herein provide an increase in a statistically, biologically, and/or clinically significant manner of the locally infiltrating antigen-specific T cells as compared to an appropriate control in the absence of administering the adjuvant.

As used herein, binding partner or an antibody is said to be "immunospecific," "specific for" or to "specifically bind" an immunogen of interest if the antibody reacts at a detectable level with the immunogen or immunogenic fragment thereof, preferably with an affinity constant, Ka, of greater than or equal to about 10⁴ M⁻¹, or greater than or equal to about 10⁵ M⁻¹, greater than or equal to about 10⁶ M⁻¹, greater than or equal to about 10⁷ M⁻¹, or greater than or equal to 10⁸ M⁻¹. Affinity of an antibody for its cognate antigen is also commonly expressed as a dissociation constant KD, and an antibody specifically binds to the immunogen of interest if it binds with a KD of less than or equal to 10⁻⁴ M, less than or equal to about 10⁻⁵ M, less than or equal to about 10⁻⁶ M, less than or equal to 10⁻⁷ M, or less than or equal to 10⁻⁸ M.

Affinities of binding partners or antibodies can be readily determined using conventional techniques, for example, those described by Scatchard et al. (Ann. N.Y. Acad. Sci. USA 51:660 (1949)) and by surface plasmon resonance (SPR; BIAcore™, Biosensor, Piscataway, NJ). For surface plasmon resonance, target molecules are immobilized on a solid phase and exposed to a binding partner (or ligand) in a mobile phase running along a flow cell. If ligand binding to the immobilized target occurs, the local refractive index changes, leading to a change in SPR angle, which can be monitored in real time by detecting changes in the intensity of the reflected light. The rates of change of the SPR signal can be analyzed to yield apparent rate constants for the association and dissociation phases of the binding reaction. The ratio of these values gives the apparent equilibrium constant (affinity) (see, e.g., Wolff et al., Cancer Res. 53:2560-2565 (1993)).

A biological sample may be obtained from the subject for determining the presence and level of an immune response to an immunogen in the subject who has received a composition comprising a vector particle that contains a recombinant expression vector comprising a polynucleotide encoding at least one immunogen and a composition comprising an adjuvant according to the methods described herein. A "biological sample" as used herein may be a blood sample (from which serum or plasma may be prepared), apheresis sample, biopsy specimen, body fluids (e.g., lung lavage, ascites, mucosal washings, synovial fluid), bone marrow, lymph nodes, tissue explant, organ culture, or any other tissue or cell preparation from the subject or a biological source.

With respect to all immunoassays and methods described herein for determining an immune response, a person skilled in the art will also readily appreciate and understand which controls are appropriately included when practicing these methods. Concentrations of reaction components, buffers, temperature, and time period sufficient to permit interaction of the reaction components can be determined and/or adjusted according to methods described herein and with which persons skilled in the art are familiar.

### Methods of Use and Compositions

Once an antigen has been identified and selected as an immunogen for inducing an immune response, a polynucleotide sequence that encodes the desired immunogen is identified and selected. The recombinant expression vector comprising the polynucleotide sequence or a vector particle comprising the vector is then formulated in an immunogenic composition with at least one pharmaceutically suitable excipient or carrier. As described herein, an adjuvant is formulated with at least one pharmaceutically suitable excipient or carrier. Both the immunogenic composition and adjuvant composition are formulated in a manner appropriate for the immunogen and adjuvant, respectively, and for the route (or mode) of administration.

As described herein, at least one immunogen or immunogenic composition is administered to a subject in need thereof in an amount sufficient to induce an effective immune response, which may be an effective humoral response and/or an effective cell-mediated immune response (which may include a cytotoxic T cell response). Without being bound by theory, as described herein the adjuvant or adjuvant composition administered to the subject induces the expression of cytokines (e.g., Cxcl9, Cxc110, CCL2, CCL3, MCP-1, TNFa, IFNc and IP-10) which recruit CD8 T-cells (e.g., to the local site of administration, such as at the site of a tumor or the bladder) and/or enhances or improves the immune response to the at least one immunogen.

The immunogenic compositions, adjuvant compositions, recombinant expression vectors, and vector particles may therefore be useful in methods for preventing (i.e., reducing the likelihood of occurrence or recurrence of) and/or treating a disease or disorder, for example, a cancer, in particular virus-induced cancers. The immunogen is an oncogenic virus antigen or a tumor-associated antigen that is believed to be or known to be expressed by one or more of the particular cancer cells.

The present invention provides compositions for use in methods for preventing, ameliorating or treating a cancer by the prime-pull administration regimens described herein wherein the method comprises administration of a first prime composition comprising a recombinant expression vector expressing an immunogen (e.g., one or more cancer-associated antigens), followed by administration of a pull composition comprising a TLR4 agonist, such as GLA, in the absence of antigen (but optionally with one or more tumor associated antigens), administered locally at the site of the cancer or tumor (e.g., intratumorally, peritumorally, intranodally, perinodally, mucosally, intravesically).

In certain embodiments, the methods described herein are useful for preventing, ameliorating or treating a cancer where the cancer comprises one or more tumors that are accessible to allow for direct injection either into or around the tumor of the pull composition comprising a TLR4 agonist as described herein. In certain embodiments, the cancer comprises a solid tumor. In some embodiments, the solid tumor is a carcinoma, a sarcoma or a lymphoma. In this regard, while lymphomas are generally considered liquid tumors, accessible "solid" tumors may form in the lymph node and thus may be treated using the prime-pull regimens described herein.

The present invention provides compositions for use in methods for preventing, ameliorating or treating a virus-induced cancer by the prime-pull administration regimens described herein wherein the method comprises administration of a first prime composition comprising a recombinant expression vector expressing an immunogen (e.g., one or more viral antigen(s) where the virus is an oncogenic virus associated with cancer), followed by administration of a pull composition comprising a TLR4 agonist such as GLA, optionally with one or more viral antigens and/or one or more tumor associated antigens, administered locally at the site of the cancer or tumor (e.g., intratumorally, mucosally, intravesically). Exemplary virus-induced cancers that can be treated or ameliorated by the methods described herein include, but are not limited to, bladder cancer, Merkel cell carcinoma, Kaposi's sarcoma, liver cancer, glioblastoma, Burkitt's lymphoma, non-Hodgkin's lymphoma, post-transplant lymphoproliferative disorder, nasopharynx cancer, cervical carcinoma, head and neck cancer, hepatocellular carcinoma, and adult T cell leukemia/lymphoma.

In certain embodiments, the prime pull administration regimens described herein are administered in conjuction with one or more other therapeutic agents. Thus, in certain embodiments, also contemplated is the administration of compositions comprising a GLA of this disclosure in combination with one or more other therapeutic agents (e.g. other anti-cancer agents, or other palliative or adjunctive therapy). In certain embodiments, such therapeutic agents may be accepted in the art as a standard treatment for a particular cancer as described herein. Exemplary therapeutic agents contemplated include cytokines, growth factors, steroids, NSAIDs, DMARDs, anti-inflammatories, immune checkpoint inhibitors, chemotherapeutics, radiotherapeutics, or other active and ancillary agents.

In one embodiment, the methods described herein are used in conjunction with one or more cancer therapeutic agents, including one or more chemotherapeutic agents. Examples of cancer therapeutic agents include alkylating agents such as thiotepa and cyclophosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, *e.g.* paclitaxel (TAXOL®, Bristol-Myers Squibb Oncology, Princeton, N.J.) and doxetaxel (TAXOTERE®., Rhne-Poulenc Rorer, Antony, France); chlorambucil; gemcitabine; 6-thioguanine; mercaptopurine; methotrexate; platinum analogs such as cisplatin and carboplatin; vinblastine; trastuzumab, docetaxel, platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid derivatives such asTargretin™ (bexarotene),
Panretin™ (alitretinoin) ; ONTAK™ (denileukin diftitox) ; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above. Further cancer therapeutic agents include sorafenib and other protein kinase inhibitors such as afatinib, axitinib, bevacizumab, cetuximab, crizotinib, dasatinib, erlotinib, fostamatinib, gefitinib, imatinib, lapatinib, lenvatinib, mubritinib, nilotinib, panitumumab, pazopanib, pegaptanib, ranibizumab, ruxolitinib, trastuzumab, vandetanib, vemurafenib, and sunitinib; sirolimus (rapamycin), everolimus and other mTOR inhibitors.

In another embodiment, the methods herein are administered in combination with another immunostimulatory agent. Such immunostimulatory agents include, but are not limited to, N-acetylmuramyl-L-alanine-D-isoglutamine (MDP), glucan, IL-12, GM-CSF, interferon-y and anti-CD40 antibodies or other antibodies that bind to and activate co-stimulatory pathways (*e.g*., CD28, ICOS, OX40, CD27 and the like).

In one embodiment, the methods herein are used in combination with one or more immune checkpoint inhibitors. Immune checkpoints refer to a variety of inhibitory pathways of the immune system that are crucial for maintaining self-tolerance and for modulating the duration and amplitude of an immune responses. Tumors use certain immune-checkpoint pathways as a major mechanism of immune resistance, particularly against T cells that are specific for tumor antigens. (see., *e.g.,* Pardoll, 2012 Nature 12:252; Chen and Mellman 2013 Immunity 39:1). The present disclosure provides immune checkpoint inhibitors that can be administered in combination with the GLA compositions without antigen. Such combination therapies work in concert to enhance an anti-cancer immune response. Certain viruses have also developed mechanisms to co-opt immune checkpoint pathways. Therefore, in certain embodiments, such combination therapy may be used to enhance an anti-viral immune response.

Immune checkpoint inhibitors include any agent that blocks or inhibits in a statistically, clinically, or biologically relevant manner, the inhibitory pathways of the immune system. Such inhibitors may include small molecule inhibitors or may include antibodies, or antigen binding fragments thereof, that bind to and block or inhibit immune checkpoint receptors or antibodies that bind to and block or inhibit immune checkpoint receptor ligands. Illustrative immune checkpoint molecules that may be targeted for blocking or inhibition include, but are not limited to, CTLA-4, 4-1BB (CD137), 4-1BBL (CD137L), PDL1, PDL2, PD1, B7-H3, B7-H4, BTLA, HVEM, TIM3, GAL9, LAG3, TIM3, B7H3, B7H4, VISTA, KIR, 2B4 (belongs to the CD2 family of molecules and is expressed on all NK, γδ, and memory CD8⁺ (αβ) T cells), CD160 (also referred to as BY55) and CGEN-15049. Immune checkpoint inhibitors include antibodies, or antigen binding fragments thereof, or other binding proteins, that bind to and block or inhibit the activity of one or more of CTLA-4, PDL1, PDL2, PD1, B7-H3, B7-H4, BTLA, HVEM, TIM3, GAL9, LAG3, TIM3, B7H3, B7H4, VISTA, KIR, 2B4, CD160 and CGEN-15049. Illustrative immune checkpoint inhibitors include Tremelimumab (CTLA-4 blocking antibody), anti-OX40, PD-L1 monoclonal Antibody (Anti-B7-H1; MEDI4736), MK-3475 (PD-1 blocker), Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and Yervoy/ipilimumab (anti-CTLA-4 checkpoint inhibitor).

In a further embodiment, the prime pull administration methods herein are used in conjunction with other TLR4 agonists, or a TLR8 agonist, or a TLR9 agonist. Such an agonist may be selected from peptidoglycan, polyI:C, CpG, 3M003, flagellin, and *Leishmania* homolog of eukaryotic ribosomal elongation and initiation factor 4a (LeIF).

In an additional embodiment, the prime pull administration regimens herein are used in conjunction with other with one or more cytokines. By "cytokine" is meant a generic term for proteins released by one cell population that act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha, beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL). As used herein, the term cytokine includes proteins from natural sources or from recombinant cell culture, and biologically active equivalents of the native sequence cytokines.

In certain embodiments, the prime pull administration methods herein are used in conjunction with chloroquine, a lysosomotropic agent that prevents endosomal acidification and which inhibits autophagy induced by tumor cells to survive accelerated cell growth and nutrient deprivation. More generally, the compositions comprising GLA as described herein may be administered in combination with therapeutic agents that act as autophagy inhibitors, radiosensitizers or chemosensitizers, such as chloroquine, misonidazole, metronidazole, and hypoxic cytotoxins, such as tirapazamine. In this regard, such combinations of a GLA with chloroquine or other radio or chemo sensitizer, or autophagy inhibitor, can be used in further combination with other cancer therapeutic agents or with radiation therapy.

In another embodiment, the prime pull administration methods herein are used in conjunction with other small molecule drugs which are known to result in killing of tumor cells with concomitant activation of immune responses, termed "immunogenic cell death", such as cyclophosphamide, doxorubicin, oxaliplatin and mitoxantrone. Furthermore, combinations with drugs known to enhance the immunogenicity of tumor cells such as patupilone (epothilone B), epidermal-growth factor receptor (EGFR)-targeting monoclonal antibody 7A7.27, histone deacetylase inhibitors (*e.g*., vorinostat, romidepsin, panobinostat, belinostat, and entinostat), the n3-polyunsaturated fatty acid docosahexaenoic acid, furthermore proteasome inhibitors (e.g. bortezomib), shikonin (the major constituent of the root of Lithospermum erythrorhizon,) and oncolytic viruses, such as TVec (talimogene laherparepvec). In other embodiments, the compositions comprising GLA as described herein may be administered in combination with epigenetic therapies, such as DNA methyltransferase inhbitors (*e.g*. Decitabine, 5-aza-2'-deoxycytidine) which may be administered locally or systemically.

In another embodiment, the prime pull administration methods herein are used in conjunction with other one or more antibodies that increase ADCC uptake of tumor by DCs. Thus, the present invention contemplates combining compositions comprising a GLA with any molecule that induces or enhances the ingestion of a tumor cell or its fragments by an antigen presenting cell and subsequent presentation of tumor antigens to the immune system. These molecules include agents that induce receptor binding (such as Fc or mannose receptors) and transport into the antigen presenting cell such as antibodies, antibody-like molecules, multi-specific multivalent molecules and polymers. Such molecules may either be administered intratumorally with the composition comprising GLA, or administered by a different route. For example, a composition comprising GLA as described herein may be administered intratumorally in conjunction with intratumoral injection of rituximab, cetuximab, trastuzumab, Campath, panitumumab, ofatumumab, brentuximab, pertuzumab, Ado-trastuzumab emtansine, Obinutuzumab, anti-HER1, -HER2, or -HER3 antibodies (*e.g*., MEHD7945A; MM-111; MM-151; MM-121; AMG888), anti-EGFR antibodies (e.g. Nimotuzumab, ABT-806), or other like antibodies. Any multivalent scaffold that is capable of engaging Fc receptors and other receptors that can induce internalization may be used in the combination therapies described herein- *e.g*. peptides and/or proteins capable of binding targets that are linked to Fc fragments or polymers capable of engaging receptors.

In certain embodiments, the prime pull administration methods herein are used in conjunction with other an antibody that promotes a co-stimulatory signal (e.g., by blocking inhibitory pathways), such as anti-CTLA-4, or that activates co-stimulatory pathways such as an anti-CD40, anti-CD28, anti-ICOS, anti-OX40, anti-CD27 antibodies and the like.

The prime pull administration methods herein may be used alone or in combination with other known cancer treatments, such as radiation therapy, immune checkpoint inhibitors, chemotherapy or other cancer therapeutic agents, transplantation, immunotherapy, hormone therapy, photodynamic therapy, etc. The compositions may also be administered in combination with antibiotics.

As understood by a person skilled in the medical art, the terms, "treat" and "treatment," refer to medical management of a disease, disorder, or condition of a subject (i.e., patient) (see, e.g., Stedman's Medical Dictionary). In general, an appropriate dose and treatment regimen provide the immunogen and adjuvant in an amount sufficient to provide therapeutic and/or prophylactic benefit. Therapeutic and/or prophylactic benefit includes, for example, an improved clinical outcome, both therapeutic treatment and prophylactic or preventative measures, wherein the object is to prevent or slow or retard (lessen) an undesired physiological change or disorder, or to prevent or slow or retard (lessen) the expansion or severity of such disease or disorder. Beneficial or desired clinical results from treating a subject include, but are not limited to, abatement, lessening, or alleviation of symptoms that result from or are associated the disease or disorder to be treated; decreased occurrence of symptoms; improved quality of life; longer disease-free status (i.e., decreasing the likelihood or the propensity that a subject will present symptoms on the basis of which a diagnosis of a disease is made); diminishment of extent of disease; stabilized (i.e., not worsening) state of disease; delay or slowing of disease progression; amelioration or palliation of the disease state; and remission (whether partial or total), whether detectable or undetectable; and/or overall survival. "Treatment" can also mean prolonging survival when compared to expected survival if a subject were not receiving treatment. Subjects in need of treatment include those who already have the disease or disorder as well as subjects prone to have or at risk of developing the disease or disorder. Subjects in need of prophylactic treatment include subjects in whom the disease, condition, or disorder is to be prevented (i.e., decreasing the likelihood of occurrence or recurrence of the disease or disorder).

Recombinant expression vectors and vector particles may be administered to a subject in a pharmaceutically or physiologically acceptable or suitable excipient or carrier. Pharmaceutically acceptable excipients are biologically compatible vehicles, e.g., physiological saline, which are described in greater detail herein, that are suitable for administration to a human or other non-human subject including a non-human mammalian subject. A therapeutically effective amount provides an amount of the polynucleotide which is capable of producing a medically desirable result (i.e., a sufficient amount of the immunogen is expressed to induce or enhance the immune response specific for the immunogen (humoral and/or cell-mediated response, including a cytotoxic T cell response) in a statistically, biologically, and/or significant manner) in a treated human or non-human animal. As is well known in the medical arts, the dosage for any one patient depends upon many factors, including the patient's size, body surface area, age, the particular compound to be administered, sex, time and route of administration, general health, and other drugs being administered concurrently. Doses will vary, but a preferred dose for administration of a vector particle comprising a recombinant expression vector is sufficient to provide approximately 10⁶ to 10¹² copies of the vector polynucleotide molecule.

Pharmaceutical compositions, including immunogenic and adjuvant compositions described herein, may be administered in a manner appropriate to the disease to be treated (or prevented) as determined by persons skilled in the medical art. An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (such as described herein, including an improved clinical outcome, such as more frequent complete or partial remissions, or longer disease-free and/or overall survival, or a lessening of symptom severity). For prophylactic use, a dose should be sufficient to prevent, delay the onset of, or diminish the severity of a disease associated with disease or disorder.

In general, the amount of an immunogen, including fusion polypeptides as described herein, present in a dose, or produced in situ by an encoding polynucleotide present in a dose, ranges from about 0.01 µg to about 1000 µg per kg of host. The use of the minimum dosage that is sufficient to provide effective therapy is usually preferred. Patients may generally be monitored for therapeutic or prophylactic effectiveness using assays suitable for the condition being treated or prevented, which assays will be familiar to those having ordinary skill in the art and which are described herein. When administered in a liquid form, suitable dose sizes will vary with the size of the patient, but will typically range from about 1 ml to about 500 ml (comprising from about 0.01 µg to about 1000 µg per kg) for a 10-60 kg subject. Optimal doses may generally be determined using experimental models and/or clinical trials. The optimal dose may depend upon the body mass, weight, or blood volume of the subject. As described herein, the appropriate dose may also depend upon the patient's (e.g., human) condition, that is, stage of the disease, general health status, as well as age, gender, and weight, and other factors familiar to a person skilled in the medical art.

Pharmaceutical compositions may be formulated for any appropriate manner of administration, including, for example, topical, oral, enteral, nasal (i.e., intranasal), inhalation, intrathecal, rectal, vaginal, intraocular, subconjunctival, sublingual, intradermal, intranodal, intratumoral, transdermal, or parenteral administration, including subcutaneous, percutaneous, intravenous, intramuscular, intrasternal, intracavernous, intrameatal or intraurethral injection or infusion. Methods of administration are described in greater detail herein.

For parenteral administration, the carrier preferably comprises water, saline, alcohol, a fat, a wax or a buffer. For oral administration, any of the above excipients or a solid excipient or carrier, such as mannitol, lactose, starch, magnesium stearate, sodium saccharine, talcum, cellulose, kaolin, glycerin, starch dextrins, sodium alginate, carboxymethylcellulose, ethyl cellulose, glucose, sucrose and/or magnesium carbonate, may be employed.

An immunogenic composition and a composition comprising the recombinant vector construct or the vector particle may be formulated for delivery by any route that provides an effective dose of the immunogen. Such administrations methods include oral administration or delivery by injection and may be in the form of a liquid. A liquid pharmaceutical composition may include, for example, one or more of the following: a sterile diluent such as water for injection, saline solution, preferably physiological saline, Ringer's solution, isotonic sodium chloride, fixed oils that may serve as the solvent or suspending medium, polyethylene glycols, glycerin, propylene glycol or other solvents; antibacterial agents; antioxidants; chelating agents; buffers and agents for the adjustment of tonicity such as sodium chloride or dextrose. A parenteral preparation can be enclosed in ampoules, disposable syringes or multiple dose vials made of glass or plastic. The use of physiological saline is preferred, and an injectable pharmaceutical composition is preferably sterile.

For pharmaceutical compositions comprising a nucleic acid molecule such as the recombinant expression vectors described herein, the nucleic acid molecule may be present within any of a variety of delivery systems known to those of ordinary skill in the art, including nucleic acid, and bacterial, viral and mammalian expression systems such as, for example, vector particles and recombinant expression constructs as provided herein. Techniques for incorporating a polynucleotide (e.g., DNA) into such expression systems are well known to those of ordinary skill in the art. In other certain embodiments, the DNA may also be "naked," as described, for example, in Ulmer et al., Science 259:1745-49, 1993 and reviewed by Cohen, Science 259:1691-1692, 1993. The uptake of naked DNA may be increased by coating the DNA onto biodegradable beads, which are efficiently transported into the cells.

Nucleic acid molecules may be delivered into a cell according to any one of several methods described in the art (see, e.g., Akhtar et al., Trends Cell Bio. 2:139 (1992); Delivery Strategies for Antisense Oligonucleotide Therapeutics, ed. Akhtar, 1995, Maurer et al., Mol. Membr. Biol. 16:129-40 (1999); Hofland and Huang, Handb. Exp. Pharmacol. 137:165-92 (1999); Lee et al., ACS Symp. Ser. 752:184-92 (2000); U.S. Patent No. 6,395,713; International Patent Application Publication No. WO 94/02595); Selbo et al., Int. J. Cancer 87:853-59 (2000); Selbo et al., Tumour Biol. 23:103-12 (2002); U.S. Patent Application Publication Nos. 2001/0007666, and 2003/077829). Such delivery methods known to persons having skill in the art, include, but are not restricted to, encapsulation in liposomes, by iontophoresis, or by incorporation into other vehicles, such as biodegradable polymers; hydrogels; cyclodextrins (see, e.g., Gonzalez et al., Bioconjug. Chem. 10:1068-74 (1999); Wang et al., International Application Publication Nos. WO 03/47518 and WO 03/46185); poly(lactic-co-glycolic)acid (PLGA) and PLCA microspheres (also useful for delivery of peptides and polypeptides and other substances) (see, e.g., U.S. Patent No. 6,447,796; U.S. Patent Application Publication No. 2002/130430); biodegradable nanocapsules; and bioadhesive microspheres, or by proteinaceous vectors (International Application Publication No. WO 00/53722). In another embodiment, the nucleic acid molecules can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives (see also, e.g., U.S. Patent Application Publication No. 2003/0077829).

In particular embodiments of the methods described herein, the subject is a human or non-human animal. A subject in need of the treatments described herein may exhibit symptoms or sequelae of a disease, disorder, or condition described herein or may be at risk of developing the disease, disorder, or condition. Non-human animals that may be treated include mammals, for example, non-human primates (e.g., monkey, chimpanzee, gorilla, and the like), rodents (e.g., rats, mice, gerbils, hamsters, ferrets, rabbits), lagomorphs, swine (e.g., pig, miniature pig), equine, canine, feline, bovine, and other domestic, farm, and zoo animals.

The compositions provided herein can be in various forms, e.g., in solid, liquid, powder, aqueous, or lyophilized form. Examples of suitable pharmaceutical excipients and carriers for administering a vector particle, including a viral vector particle and a bacterial vector particle, immunogenic compositions, and recombinant expression vectors are known in the art. Such excipients, carriers, and/or additives can be formulated by conventional methods and can be administered to the subject at a suitable dose. Stabilizing agents such as lipids, nuclease inhibitors, polymers, and chelating agents that may be included in the compositions described herein can assist preservation of the compositions and components of the compositions from degradation within the body.

The vector particles, including a viral vector particle and a bacterial vector particle, immunogenic compositions, adjuvant compositions, and recombinant expression vectors provided herein can be packaged as kits. Kits can optionally include one or more components such as instructions for use, devices, and additional reagents, and components, such as tubes, containers, e.g. vials, and syringes for practice of the methods. Exemplary kits can optionally include instructions for use, a device or reagents for detecting a vector particle, the recombination expression vector, or the immunogen in a subject, and a device for administering the composition or compositions to a subject.

Kits comprising polynucleotides encoding an immunogen are also contemplated herein. Such a kit may also include at least one plasmid that encodes virus packaging components and a vector encoding Sindbis virus E2 glycoprotein variant. Some kits will contain at least one plasmid encoding virus packaging components, a vector encoding Sindbis virus E2 glycoprotein variant, and a vector encoding at least one DC maturation factor.

Kits comprising a viral vector encoding a sequence of interest (typically encoding an antigen or immunogen) and optionally, a polynucleotide sequence encoding a DC maturation factor are also contemplated herein. In some kits, the kit includes at least one plasmid encoding virus packaging components and a vector encoding Sindbis virus E2 glycoprotein variant.

A kit may also contain instructions. Instructions typically describe methods for administration, including methods for determining the proper state of the subject, the proper dosage amount, and the proper administration method, for administering the composition. Instructions can also include guidance for monitoring the subject over the duration of the treatment time.

Kits provided herein also can include a device for administering an immunogenic composition comprising a vector particle that comprises a recombinant expression vector, and/or an adjuvant composition to a subject. Any of a variety of devices known in the art for administering medications or vaccines can be included in the kits provided herein. Exemplary devices include, but are not limited to, a hypodermic needle, an intravenous needle, a catheter, a needle-less injection device, an inhaler, and a liquid dispenser, such as an eyedropper. Typically, the device for administering a composition is compatible with the active components of the kit. For example, a needle-less injection device such as a high pressure injection device can be included in kits with vector particles, polynucleotides, and polypeptides not damaged by high pressure injection, but is typically not included in kits that include vector particles, polynucleotides, and polypeptides that may be damaged by high pressure injection.

Other embodiments and uses will be apparent to one skilled in the art in light of the present disclosures. The following examples are provided merely as illustrative of various embodiments and shall not be construed to limit the invention in any way.

### EXAMPLES

### Example 1: Recruitment of CD8 T-cell to the Bladder

Studies are performed in mice to demonstrate that CD8 T-cells generated by subcutaneous immunization can be recruited to the bladder. Groups of BALB/C mice (including appropriate control groups; 5 mice per group) are immunized via the tail base with different doses (10E8, 10E9, 10E10) of a recombinant lentivector as described herein at up to three time points (e.g. 0, 2, 4 weeks). One week after the last immunization, mice are instilled either weekly with BCG (strain TICE) at 1 mg/ml, with 5-8 x 10E7 CFU/mg, for 6 instillations, following established procedures, or twice weekly with 5µg of GLA formulated as 2% stable emulsion. One week after the last BCG/GLA instillation the mice are sacrificed and their bladders removed. From each bladder, histological sections are prepared, fixated and stained with the appropriate antibodies for detection and typing of immune cells such as NK cells, dendritic cells, T-cells, B-cells etc. following established protocols. In addition, a portion of each bladder cells is subjected to collagenase digestion, T-cells are isolated using magnetic beads, stained with pentamers for NY-ESO-1 epitope specific CD8 T-cells and analyzed in Flow, following established protocols.

An established orthotopic mouse bladder cancer model based on the mouse tumor cell line MB-49 is used to determine the immunological and clinical efficacy of the treatment (Durek 2002; and Kang, M. R., et al., J. Vis. Exp. (65), e4207, (2012)). Using an integrating recombinant lentivector as described herein coding for NY-ESO-1, recombinant clones of MB-49 expressing NY-ESO-1 are generated and tested for tumor growth kinetics after implantation the bladder. Mice with established tumors are subjected to the treatments outlined in (1), and clinical effect on the tumors is monitored through appropriate methods in vivo (e.g. ultrasound imaging), morphometric measurements after sacrificing of the mice at predefined time points, and analysis of tumor infiltrating lymphocytes (Watkins, S. K., et al., J. Vis. Exp. (64), e3952, (2012)).

### Example 2: Lentiviral Vector Prime Followed by Intra-tumoral GLA Administration Effectively "Pulls" Antigen-Specific CD8 T Cells to Tumor

This Example demonstrates that intratumoral administration of GLA post-immunization with vector vaccine "pulls" vector-induced antigen-specific CD8 T cells to the tumor.

In this study, on Day 0, C57BL/6 mice (5 mice per group) were inoculated with 1 X 10⁶ B16F10-OVA cells, subcutaneously in the footpad. On Day 10, mice were immunized with 2 X 10¹⁰ genomes of VP02/OVA (or control vector, VP02/GFP) via tail base administration. On Day 21, mice were given intra-tumoral administration of 5.0 µg GLA/2% SE. Tumors were harvested and analyzed for tumor-infiltrating lymphocytes via flow cytometry immediately prior to (D+0), one day post- (D+1), or two days post- (D+2) GLA administration.

As expected, few OVA-specific CD8 T cells were found in tumors of mice immunized with the control vector, VP02/GFP (Fig. 1A, top panels). On Day 0, an average of 1.9% OVA-specific CD8 T cells were found in tumors of mice immunized with VP02/OVA indicating that some CD8-positive antigen-specific cells primed by the vector were infiltrating the tumor. Day 1 and 2 post-GLA administration, an average of 2.7% and 3.4%, respectively, OVA-specific CD8 T cells were found in tumors of mice immunized with VP02/OVA, compared to the 1.9% prior to GLA administration (Fig. 1B) indicating that intra-tumoral administration of GLA resulted in pulling of CD8 antigen-specific T cells into the tumor. Within 48 hours of GLA administration, there was an average of a 7-fold increase in percent Ag-specific CD8 T cells in treated tumors compared to non-treated tumors (n=5). The ratio of % OVA-specific CD8 T cells found in tumors of vector-immunized mice over non-immunized mice increased 1.8-fold day 1 and 2 post-GLA administration.

The above data demonstrate that once antigen-specific CD8 T cells are systemically generated ("prime"), local administration of an adjuvant at the tumor site "pulled" these CD8 T cells to the tumor, mediating protective immunity. These data support the use of "Prime-pull" as a promising vaccine strategy for cancer immunotherapy.

### Example 3: Evaluate Therapeutic Efficacy of Prime-Pull Strategy

This Example demonstrates that intra-tumoral administration of GLA post-immunization with vector vaccine "pulls" vector-induced antigen-specific CD8 T cells to the tumor and further demonstrates the therapeutic efficacy of the prime-pull strategy.

In this study, on Day 0, C57BL/6 or BALB/c female mice (5 mice per group) were inoculated with 1 X 10⁵ B16F10-OVA cells or CT26 cells, respectively, subcutaneously in the flank. On Day 7, mice were immunized with 2 X 10¹⁰ genomes of VP02/OVA (C57BL/6) or VP02/AH1A5 (BALB/C) (or control vector, VP02/GFP) via tail base administration. Starting on Day 7 or 14, mice were given intra-tumoral administration of 5.0 µg GLA/2% SE every 3-4 days. On Day 18 (one day post-last GLA/SE treatment), B16F10-OVA tumors were harvested and analyzed for tumor-infiltrating lymphocytes via flow cytometry.

Mice immunized with the control vector (VP02/GFP) had 1.8% lymphocytes that infiltrated the tumor (Fig. 2A). GLA/SE alone did not increase percent lymphocyte infiltration. VP02/OVA alone increased % lymphocyte infiltration to 3.2%, whereas VP02/OVA + GLA/SE further increased % lymphocyte infiltration to 7.5%, for a total of 4-fold increase above baseline in tumor-infiltrating lymphocytes. Of the lymphocytes found in the tumor, VP02/OVA alone generated 16.6% OVA-specific CD8 T cells compared to 0.9% from control mice, whereas GLA/SE alone generated 14.7% regulatory T cells compared to 9.7% from control mice. These findings indicate that the vector-generated antigen-specific CD8 T cells trafficked to the tumor, and that in the absence of an antigen-specific prime, GLA/SE pulled mainly regulatory T cells to the site. In the presence of an antigen-specific prime, however, while GLA/SE continued to pull regulatory T cells to the tumor, it also pulled more antigen-specific CD8 T cells into the tumor (Fig. 2B).

In both B16-OVA and CT26 tumor models, the prime-pull strategy achieved the best tumor control in mice (Fig. 3). Surprisingly, the prime-pull strategy achieved complete regression in the CT26 model out at least as far as 60 days post-tumor inoculation (experiment still ongoing) (Fig. 3B, open diamonds).

The above data demonstrate that once antigen-specific CD8 T cells are systemically generated ("prime"), local administration of a TLR4 agonist at the tumor site "pulled" these CD8 T cells to the tumor, mediating protective immunity against tumor growth. These data support the use of "Prime-pull" as a promising vaccine strategy for cancer immunotherapy.

### Example 4: Investigation of the Kinetics of the Prime-Pull Strategy.

A study is currently ongoing to investigate the kinetics of the prime-pull strategy. In this study, on Day 0, C57BL/6 mice (n=5 per group) were inoculated with 1 x 10⁵ B16OVA cells, subcutaneously in the right flank. When tumors measured > 4 mm² (Day 7), mice were immunized once with VP02/OVA or vehicle control at the base of tail. Intratumoral administration of 5 µg GLA/2% SE alone were started when tumors measured > 4 mm² (Day 7) or on Day 21 and continued every 3-4 days thereafter until the end of the study. On Day 22, 23, and 24, splenocytes and tumors are harvested to determine which time point (1, 2, or 3 days post-last GLA/SE administration) has the highest percentage of tumor-infiltrating lymphocytes.

### Example 5: Determine the phenotype of tumor-infiltrating lymphocytes resulting from the GLA pull, compared to baseline tumor-infiltrating lymphocytes.

Methods: Mice are inoculated with tumor cells. Tumor-bearing mice are then be immunized with VP02/A via tail base administration, followed by intra-tumoral administration of GLA. Tumors are harvested and analyzed for phenotyping of tumor-infiltrating lymphocytes via flow cytometry and analysis post-GLA administration. Marker phenotyping is done on pentamer-positive cells whereas peptide restimulation is used to assess multipotency and response balance phenotype.

### Example 6: Determine whether the GLA pull is specific for antigen-specific CD8 T cells.

Methods: CFSE-labeled splenocytes are adoptively transferred from non-immunized or VP02/OVA-immunized mice into naive mice inoculated with B16/OVA tumor, followed by intra-tumoral administration of GLA. Tumors are harvested and analyzed for antigen A-specific tumor-infiltrating lymphocytes via flow cytometry post-GLA administration. To increase the signal and to study the pull effect on CD8 and CD4 cells, OT-I (CD8) or OT-II (CD4) mice are immunized and used as donors in the adoptive transfer experiment.

### Example 7: Evaluate whether the GLA pull is antigen-dependent.

Methods: Mice are inoculated with tumor cells (expressing or not expressing antigen A). Tumor-bearing mice will then be immunized with VP02/A (containing antigen A) via tail base administration. Immunized mice are then given intra-tumoral administration of GLA. Tumors are harvested and analyzed for antigen A-specific tumor-infiltrating lymphocytes via flow cytometry post-GLA administration.

### Example 8: Monitor the magnitude and kinetics of the GLA pull.

Methods: OT-I-luc mice are transgenic mice that have OVA-specific CD8 T cells that will luminesce in the presence of luciferase. These mice therefore do not need to be immunized with VP02/OVA to generate OVA-specific CD8 T cells, however immunization is generally required to activate the T cells. The mice are inoculated with tumor cells, followed by immunization with VP02/OVA and then given intra-tumoral administration of GLA. A control arm of the study is mock-primed with VP02/IrrAg. The mice are inoculated with tumor cells, followed by intra-tumoral administration of GLA. Tumor-bearing animals are imaged with the IVIS system to assess bioluminescent signal within the tumor. Because only the OT-I-luc CD8 T cells have bioluminescence properties, the magnitude of the signal directly correlates with the number of OVA-specific CD8 T cells in the tumor. For kinetics studies, tumor inoculation is optional.

### Example 9: Investigate the diversity of the T cell receptor repertoire of the prime-pull, compared to "prime" only, "pull" only, or untreated mice

Methods: Mice are inoculated with tumor cells. Tumor-bearing mice are then (1) immunized with VP02/A via tail base administration, followed by intra-tumoral administration of GLA, or (2) just immunized with VP02/A, or (3) just treated by intratumoral GLA injection, or (4) left untreated. Tumors and spleens are harvested and analyzed by T cell receptor analysis of tumor-infiltrating lymphocytes and splenocytes via deep sequencing. It is anticipated that mice treated with prime-pull show the greatest clonal diversity both in splenic lymphocytes and TIL.

### Examples of Aspects

1. A method of treating a cancer comprising:
   a) administering to a subject a first composition comprising a viral vector particle, the viral vector particle comprising a recombinant expression vector wherein the recombinant expression vector comprises a polynucleotide encoding a cancer-associated antigen, and wherein the polynucleotide is operatively linked to at least one regulatory expression sequence, thereby inducing an immune response against the cancer-associated antigen in the subject; and
   b) administering locally to the subject a second composition comprising a pharmaceutically suitable adjuvant wherein the composition does not comprise antigen;
   wherein the first composition and the second composition each further comprise a pharmaceutically suitable excipient, and wherein the first composition and second composition are administered concurrently or sequentially.
2. The method of embodiment 1, wherein the cancer is an oncogenic virus-induced cancer.
3. The method of any of the precending embodiments, wherein the cancer-associated antigen is an antigen derived from the oncogenic virus.
4. The method of any of the precending embodiments, wherein the first composition is administered systemically via a first route and the second composition is administered locally via a second route.
5. The method of any of the precending embodiments wherein the first route is intramuscular, intradermal or subcutaneous and the second route is intratumoral, intranodal, mucosal or intravesical.
6. The method of any of the precending embodiments wherein the pharmaceutically suitable adjuvant is a TLR4 agonist.
7. The method of embodiment 6 wherein the TLR4 agonist is a non-toxic lipid A-related adjuvant.
8. The method of any of the precending embodiments, wherein the non-toxic lipid A-related adjuvant is glucopyranosyl lipid A (GLA).
9. The method of any of the precending embodiments, wherein GLA is formulated in a stable oil-in-water emulsion.
10. The method of any one of the previous embodiments wherein the GLA has the formula: 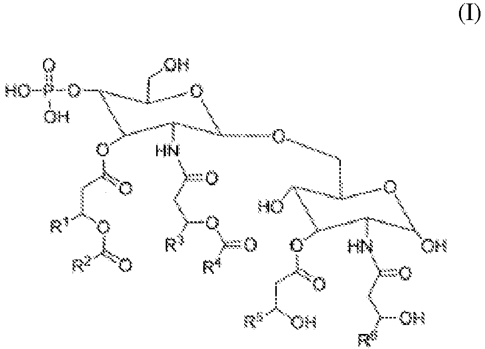 where: R¹, R³, R⁵ and R⁶ are C₁₁-C₂₀ alkyl; and R² and R⁴ are C12-C20 alkyl.
11. The method of embodiment 10 wherein R¹, R³, R⁵ and R⁶ equal to undecyl, and R² and R⁴ equal to tridecyl.
12. The method of any of the precending embodiments wherein the GLA has the formula: 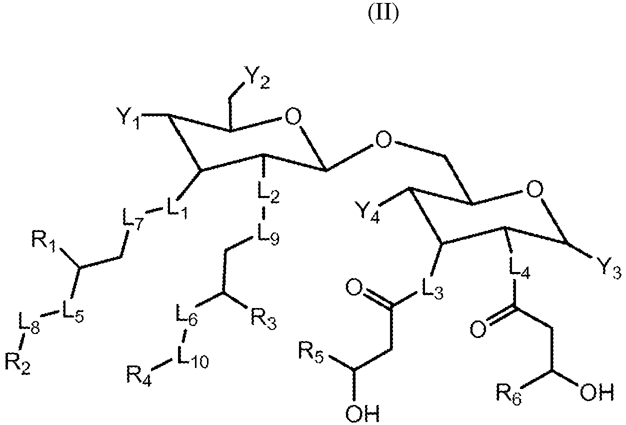 or a pharmaceutically acceptable salt thereof, wherein: L1, L2, L3, L4, L5 and L6 are the same or different and are independently selected from -O-, -NH-, and -(CH2)-; L7, L8, L9 and L10 are the same or different, and at any occurrence may be either absent or -C(=O)-; Y1 is an acid functional group; Y2 and Y3 are the same or different and are each independently selected from -OH, -SH, and an acid functional group; Y4 is -OH or -SH; R1, R3, R5 and R6 are the same or different and are each independently selected from the group of C8-C13 alkyl; and R2 and R4 are the same or different and are each independently selected from the group of C6-C11 alkyl.
13. The method of any one of the previous embodiments, wherein the first composition is administered prior to administration of the second composition.
14. The method of any one of the previous embodiments wherein the first composition and/or the second composition are administered 2, 3, 4, 5, 6, 7, 8, 9 or 10 times.
15. The method of any one of the previous embodiments, wherein the recombinant expression vector is selected from a retroviral vector genome, a lentiviral vector genome, poxvirus vector genome, vaccinia virus vector genome, adenovirus vector genome, adenovirus-associated virus vector genome, herpes virus vector genome, alpha virus vector genome, plasmid DNA and RNA.
16. The method of any one of the previous embodiments, wherein the viral vector particle is a lentiviral vector particle that comprises the lentiviral vector genome; a poxvirus vector particle that comprises the poxvirus vector genome; a vaccinia virus vector particle that comprises the vaccinia virus vector genome; an adenovirus vector particle that comprises the adenovirus vector genome; an adenovirus-associated virus vector particle that comprises the adenovirus-associated virus vector genome; a herpes virus vector particle that comprises the herpes virus vector genome; or an alpha virus vector particle that comprises the alpha virus vector genome.
17. The method of any one of the previous embodiments, wherein the viral vector particle is the lentiviral vector particle and comprises the lentiviral vector genome.
18. The method of previous embodiments, wherein the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein.
19. The method of any one of the previous embodiments, wherein the viral vector particle delivers the recombinant expression vector to a dendritic cell.
20. The method of any one of the previous embodiments, wherein the cancer associated antigen is selected from p53, Ras, c-Myc, A-Raf, B-Raf, C-Raf, cyclin-dependent kinases, MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, β-catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RARα, Tumor-associated calcium signal transducer 1 (TACSTD1) TACSTD2, receptor tyrosine kinases, Epidermal Growth Factor receptor (EGFR), EGFRvIII, platelet derived growth factor receptor (PDGFR), vascular endothelial growth factor receptor (VEGFR), cytoplasmic tyrosine kinases, src-family, syk-ZAP70, integrin-linked kinase (ILK), signal transducers and activators of transcription STAT3, STAT5, and STAT6, hypoxia inducible factors, HIF-1α and HIF-2α, Nuclear Factor-Kappa B (NF-κB), Notch receptors, Notch1-4, c-Met, mammalian targets of rapamycin (mTOR), WNT, extracellular signal-regulated kinases (ERKs), PMSA, PR-3, MDM2, Mesothelin, renal cell carcinoma - 5T4, SM22-alpha, carbonic anhydrases I (CAI) and IX (CAIX), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGs5, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, and fos related antigen 1.
21. The method of any one of the previous embodiments, wherein the cancer associated antigen is selected from CTA, NY-ESO-1, LAGE-1,MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A, and BK T-antigen.
22. The method of any one of the previous embodiments, wherein the cancer is bladder cancer.
23. The method of any one of the previous embodiments, wherein the second composition comprising a pharmaceutically suitable adjuvant further comprises BCG.
24. The method of any one of the previous embodiments, wherein the second composition is administered in combination with a composition comprising one or more therapeutic agents selected from the group consisting of a checkpoint inhibitor (e.g., anti-PD1, anti-PDL1, anti-CTLA4 and the like), a cytokine, chloroquine, an antibody that increase ADCC, an antibody that promotes a co-stimulatory signal, or an anti-CD40 antibody.
25. The method of any one of the previous embodiments, wherein the first composition further comprises an adjuvant.
26. The method of any one of the previous embodiments, further comprising administering a boosting composition comprising an adjuvant in combination with a polypeptide, wherein the polypeptide comprises the cancer-associated antigen, or an immunogenic fragment thereof.
27. The method of any one of the previous embodiments, wherein the induced immune response comprises a cytotoxic T lymphocyte immune response.
28. The method of any one of the previous embodiments, wherein administering locally the second composition induces an increase in CD8+ T cells at the local site of administration.
29. A kit comprising a first composition and a second composition according to any one of the preceding embodiments.
30. A method of treating cancer comprising:
   a) inducing an immune response specific for an immunogen associated with the cancer in a subject, comprising administering to the subject a first composition comprising a lentiviral vector particle, the lentiviral vector particle comprising a recombinant expression vector, wherein the recombinant expression vector comprises a polynucleotide encoding the immunogen, wherein the polynucleotide is operatively linked to at least one regulatory expression sequence, wherein the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein; and
   b) administering intratumorally a second composition comprising a pharmaceutically suitable adjuvant, wherein the second composition does not comprise an immunogen, and wherein the adjuvant is a non-toxic lipid A-related adjuvant is glucopyranosyl lipid A (GLA) formulated in a stable oil-in-water emulsion, wherein the GLA has the formula: 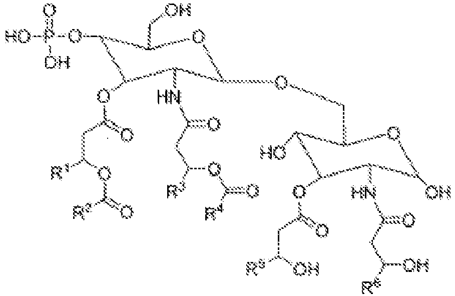
      where: R¹, R³, R⁵ and R⁶ equal to undecyl, and R² and R⁴ equal to tridecyl;
      wherein the immunogen associated with the cancer is selected from CTA, NY-ESO-1, LAGE-1, MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A;
      wherein the first composition and the second composition each further comprise a pharmaceutically suitable excipient, wherein the first composition and second composition are administered concurrently or sequentially, wherein the first composition is administered intramuscularly, intradermally or subcutaneously.
31. A method of treating a cancer in a subject comprising:
   a) administering to the subject a first composition comprising
      i. antigen-specific T cells specific for a cancer-associated antigen, said antigen-specific T cells having been expanded *ex vivo*,
      ii. T cells that have been genetically modified to express a chimeric antigen receptor (CAR) that recognizes the cancer-associated antigen, or
      iii. T cells that have been genetically modified to express a specific chimeric T cell receptor (TCR) that recognizes the cancer-associated antigen in the context of MHC; and
   b) administering intratumorally to the subject a second composition comprising a pharmaceutically suitable adjuvant wherein the second composition does not comprise antigen; or optionally, comprises an antigen, such as a cancer-associated antigen listed in any of the preceding embodiments;
   wherein the first composition and the second composition each further comprise a pharmaceutically suitable excipient, and wherein the first composition and second composition are administered concurrently or sequentially.
32. The method of embodiment 31, wherein the adjuvant is a non-toxic lipid A-related adjuvant.
33. The method of embodiment 31, wherein the adjuvant is glucopyranosyl lipid A (GLA).
34. The method of embodiment 33, wherein GLA is formulated in a stable oil-in-water emulsion.
35. The method of embodiment 33 or embodiment 34 wherein the GLA has the formula: 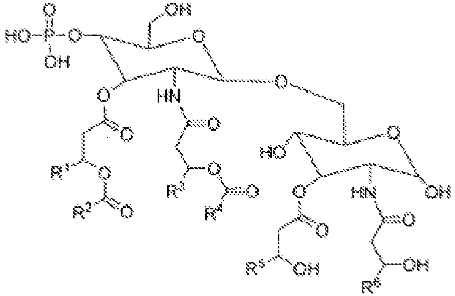 where: R¹, R³, R⁵ and R⁶ are C₁₁-C₂₀ alkyl; and R² and R⁴ are C₁₂-C₂₀ alkyl.
36. The method of embodiment 35 wherein R¹, R³, R⁵ and R⁶ equal to undecyl, and R² and R⁴ equal to tridecyl.
37. A composition comprising
   a) a first composition comprising a viral vector particle, the viral vector particle comprising a recombinant expression vector wherein the recombinant expression vector comprises a polynucleotide encoding a cancer-associated antigen, and wherein the polynucleotide is operatively linked to at least one regulatory expression sequence, thereby inducing an immune response against the cancer-associated antigen in the subject; and
   b) a second composition comprising a pharmaceutically suitable adjuvant; wherein the second composition does not comprise antigen;
   wherein the first composition and the second composition each further comprise a pharmaceutically suitable excipient, for use in a method of treating a cancer wherein the first composition is administered systemically to the subject; and wherein the second composition is administered locally to the subject; and wherein the first composition and second composition are administered concurrently or sequentially.
38. The composition of embodiment 37, wherein the cancer is an oncogenic virus-induced cancer.
39. The composition of embodiment 38, wherein the cancer-associated antigen is an antigen derived from the oncogenic virus.
40. The composition of embodiment 37, wherein the first composition is administered systemically via a first route and the second composition is administered locally via a second route.
41. The composition of embodiment 40 wherein the first route is intramuscular, intradermal or subcutaneous and the second route is intratumoral, intranodal, mucosal or intravesical.
42. The composition of embodiment 37 wherein the pharmaceutically suitable adjuvant is a TLR4 agonist.
43. The composition of embodiment 42 wherein the TLR4 agonist is a non-toxic lipid A-related adjuvant.
44. The composition of embodiment 43, wherein the non-toxic lipid A-related adjuvant is glucopyranosyl lipid A (GLA).
45. The composition of embodiment 44, wherein GLA is formulated in a stable oil-in-water emulsion.
46. The composition of any one of the preceding embodiments wherein the GLA has the formula: 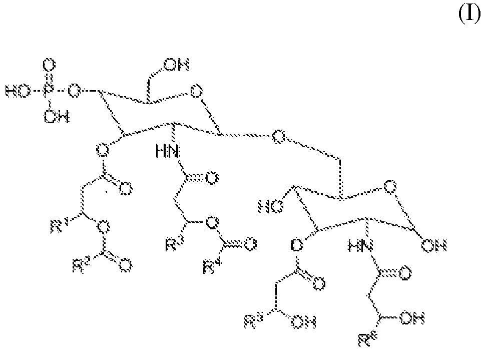 where: R¹, R³, R⁵ and R⁶ are C₁₁-C₂₀ alkyl; and R² and R⁴ are C₁₂-C₂₀ alkyl.
47. The composition of embodiment 46 wherein R¹, R³, R⁵ and R⁶ equal to undecyl, and R² and R⁴ equal to tridecyl.
48. The composition of any one of embodiments 44 - 45 wherein the GLA has the formula: 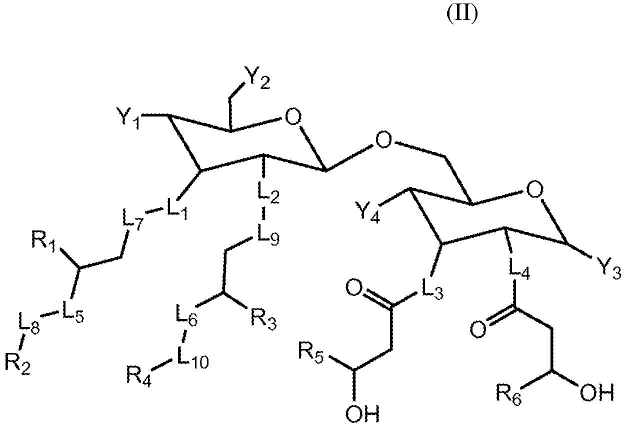 or a pharmaceutically acceptable salt thereof, wherein: L1, L2, L3, L4, L5 and L6 are the same or different and are independently selected from -O-, -NH-, and -(CH2)-; L7, L8, L9 and L10 are the same or different, and at any occurrence may be either absent or -C(=O)-; Y1 is an acid functional group; Y2 and Y3 are the same or different and are each independently selected from -OH, -SH, and an acid functional group; Y4 is -OH or -SH; R1, R3, R5 and R6 are the same or different and are each independently selected from the group of C8-C13 alkyl; and R2 and R4 are the same or different and are each independently selected from the group of C6-C11 alkyl.
49. The composition of any one of embodiments 37-45, wherein the first composition is administered prior to administration of the second composition.
50. The composition of any one of embodiments 37-45 wherein the first composition and/or the second composition are administered 2, 3, 4, 5, 6, 7, 8, 9 or 10 times.
51. The composition of any one of the previous embodiments, wherein the recombinant expression vector is selected from a retroviral vector genome, a lentiviral vector genome, poxvirus vector genome, vaccinia virus vector genome, adenovirus vector genome, adenovirus-associated virus vector genome, herpes virus vector genome, alpha virus vector genome, plasmid DNA and RNA.
52. The composition of any one of the previous embodiments, wherein the viral vector particle is a lentiviral vector particle that comprises the lentiviral vector genome; a poxvirus vector particle that comprises the poxvirus vector genome; a vaccinia virus vector particle that comprises the vaccinia virus vector genome; an adenovirus vector particle that comprises the adenovirus vector genome; an adenovirus-associated virus vector particle that comprises the adenovirus-associated virus vector genome; a herpes virus vector particle that comprises the herpes virus vector genome; or an alpha virus vector particle that comprises the alpha virus vector genome.
53. The composition of any one of the previous embodiments, wherein the viral vector particle is the lentiviral vector particle and comprises the lentiviral vector genome.
54. The composition of any one of the previous embodiments, wherein the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein.
55. The composition of any one of the previous embodiments, wherein the viral vector particle delivers the recombinant expression vector to a dendritic cell.
56. The composition of any one of the previous embodiments, wherein the cancer associated antigen is selected from CTA, NY-ESO-1, LAGE-1,MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 MAGE-A, BK T-antigen, p53, Ras, c-Myc, A-Raf, B-Raf, C-Raf, cyclin-dependent kinases, MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, β-catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RARα, Tumor-associated calcium signal transducer 1 (TACSTD1) TACSTD2, receptor tyrosine kinases, Epidermal Growth Factor receptor (EGFR), EGFRvIII, platelet derived growth factor receptor (PDGFR), vascular endothelial growth factor receptor (VEGFR), cytoplasmic tyrosine kinases, src-family, syk-ZAP70, integrin-linked kinase (ILK), signal transducers and activators of transcription STAT3, STAT5, and STAT6, hypoxia inducible factors, HIF-1α and HIF-2α, Nuclear Factor-Kappa B (NF-κB), Notch receptors, Notch1-4, c-Met, mammalian targets of rapamycin (mTOR), WNT, extracellular signal-regulated kinases (ERKs), PMSA, PR-3, MDM2, Mesothelin, renal cell carcinoma - 5T4, SM22-alpha, carbonic anhydrases I (CAI) and IX (CAIX), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGs5, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, and fos related antigen 1.
58. The composition of any one of the previous embodiments, wherein the cancer is bladder cancer.
59. The composition of any one of the previous embodiments, wherein the second composition comprising a pharmaceutically suitable adjuvant further comprises BCG.
60. The composition of any one of the previous embodiments, wherein the second composition is administered in combination with a composition comprising a therapeutic agent selected from the group consisting of a checkpoint inhibitor, a cytokine, chloroquine, an antibody that increase ADCC, an antibody that promotes a co-stimulatory signal, an anti-CD40 antibody.
61. The composition of any one of the previous embodiments, wherein the first composition further comprises an adjuvant.
62. The composition of any one of the previous embodiments, further comprising administering a boosting composition comprising an adjuvant in combination with a polypeptide, wherein the polypeptide comprises the cancer-associated antigen, or an immunogenic fragment thereof.
63. The composition of any one of the previous embodiments, wherein the induced immune response comprises a cytotoxic T lymphocyte immune response.
64. The composition of any one of the previous embodiments, wherein administering locally the second composition induces an increase in CD8+ T cells at the local site of administration.
65. A kit comprising a first composition and a second composition according to any one of the previous embodiments.
66. A composition comprising:
   a) a first composition comprising a lentiviral vector particle, the lentiviral vector particle comprising a recombinant expression vector, wherein the recombinant expression vector comprises a polynucleotide encoding an immunogen associated with cancer, wherein the polynucleotide is operatively linked to at least one regulatory expression sequence, wherein the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein; and
   b) a second composition comprising a pharmaceutically suitable adjuvant, wherein the second composition does not comprise an immunogen; wherein the adjuvant is a non-toxic lipid A-related adjuvant is glucopyranosyl lipid A (GLA) formulated in a stable oil-in-water emulsion, wherein the GLA has the formula: 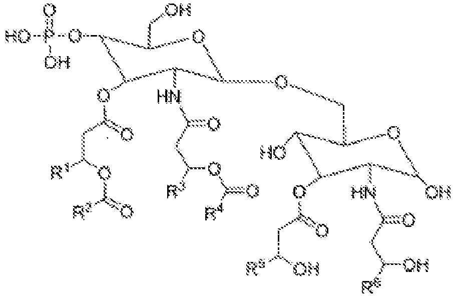
      where: R¹, R³, R⁵ and R⁶ equal to undecyl, and R² and R⁴ equal to tridecyl;
      wherein the immunogen associated with cancer is selected from CTA, NY-ESO-1, LAGE-1, MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3 and MAGE-A;
      wherein the first composition and the second composition each further comprise a pharmaceutically suitable excipient, for use in a method of treating a cancer in a subject, wherein the first composition is administered intramuscularly, intradermally or subcutaneously and induces an immune response specific for the immunogen associated with the cancer in the subject; wherein the second composition is administered intratumorally; and wherein the first composition and second composition are administered concurrently or sequentially.
67. A composition comprising:
   a) a first composition comprising
      i. autologous or heterologous antigen-specific T cells specific for a cancer-associated antigen, said antigen-specific T cells having been expanded *ex vivo,*
      ii. autologous or heterologous T cells that have been genetically modified to express a chimeric antigen receptor (CAR) that recognizes the cancer-associated antigen, or
      iii. T cells that have been genetically modified to express a specific chimeric T cell receptor (TCR) that recognizes the cancer-associated antigen in the context of MHC; and
   b) a second composition comprising a pharmaceutically suitable adjuvant; wherein the second composition does not comprise an immunogen;
   wherein the first composition and the second composition each further comprise a pharmaceutically suitable excipient;
   for use in a method of treating a cancer in a subject wherein the first composition is administered systemically to the subject; and the second composition is administered intratumorally and wherein the first composition and second composition are administered concurrently or sequentially.
68. The method of any one of the previous embodiments, wherein the adjuvant is a non-toxic lipid A-related adjuvant.
69. The method of any one of the previous embodiments, wherein the adjuvant is glucopyranosyl lipid A (GLA).
70. The method of any one of the previous embodiments, wherein GLA is formulated in a stable oil-in-water emulsion.
71. The method of any one of the previous embodiments wherein the GLA has the formula: 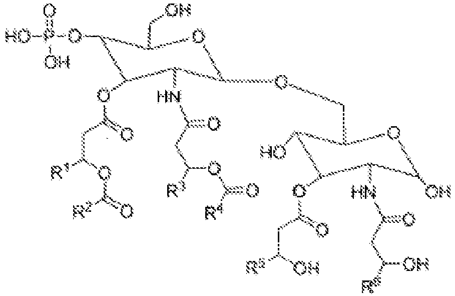 where: R¹, R³, R⁵ and R⁶ are C11-C20 alkyl; and R² and R⁴ are C12-C20 alkyl.
72. The method of any one of the previous embodiments wherein R¹, R³, R⁵ and R⁶ equal to undecyl, and R² and R⁴ equal to tridecyl.
73. A method of increasing T cells in the tumor microenvironment comprising,
   a) administering to a subject having a tumor a first composition comprising a vector particle, the vector particle comprising a recombinant expression vector wherein the recombinant expression vector comprises a polynucleotide encoding a cancer-associated antigen, and wherein the polynucleotide is operatively linked to at least one regulatory expression sequence, thereby inducing an immune response against the cancer-associated antigen in the subject; and
   b) administering intratumorally or peritumorally to the subject a second composition comprising a TLR4 agonist wherein the composition does not comprise antigen;
   wherein the first composition and the second composition each further comprise a pharmaceutically suitable excipient, and wherein the first composition and second composition are administered concurrently or sequentially;
   thereby increasing T cells in the tumor microenvironment.

## Claims

1. A kit comprising:
a) a first composition comprising a vector particle optionally with an adjuvant, the vector particle comprising a recombinant expression vector wherein the recombinant expression vector comprises a polynucleotide encoding a cancer-associated antigen, and wherein the polynucleotide is operatively linked to at least one regulatory expression sequence, and
b) a second composition comprising a TLR4 agonist wherein the composition does not comprise antigen;
wherein the first composition and the second composition each further comprise a pharmaceutically suitable excipient,
for use in a method of increasing T cells in a tumor microenvironment in a subject,
where the first composition is administered systemically, thereby inducing an immune response against the cancer-associated antigen in the subject; and where the second composition is administered intratumorally or peritumorally to the subject; and where the first and second composition are administered concurrently or sequentially; thereby increasing T cells in the tumor microenvironment, wherein, when present, the adjuvant is glucopyranosyl lipid A (GLA).

2. The kit for use according to claim 1 wherein the TLR4 agonist is glucopyranosyl lipid A (GLA).

3. The kit for use according to claim 1 or 2, wherein the cancer is an oncogenic virus-induced cancer, or wherein the cancer associated antigen is selected from CTA, NY-ESO-1, LAGE-1,MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3, MAGE-A, BK T-antigen, p53, Ras, c-Myc, A-Raf, B-Raf, C-Raf, cyclin-dependent kinases, MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, β-catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RARα, Tumor-associated calcium signal transducer 1 (TACSTD1) TACSTD2, receptor tyrosine kinases, Epidermal Growth Factor receptor (EGFR), EGFRvIII, platelet derived growth factor receptor (PDGFR), vascular endothelial growth factor receptor (VEGFR), cytoplasmic tyrosine kinases, src-family, syk-ZAP70, integrin-linked kinase (ILK), signal transducers and activators of transcription STAT3, STAT5, and STAT6, hypoxia inducible factors, HIF-1α and HIF-2α, Nuclear Factor-Kappa B (NF-κB), Notch receptors, Notch1-4, c-Met, mammalian targets of rapamycin (mTOR), WNT, extracellular signal-regulated kinases (ERKs), PMSA, PR-3, MDM2, Mesothelin, renal cell carcinoma - 5T4, SM22-alpha, carbonic anhydrases I (CAI) and IX (CAIX), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGs5, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, and fos related antigen 1.

4. The kit for use according to any one of the preceding claims, wherein the recombinant expression vector is selected from a retroviral vector genome, a lentiviral vector genome, poxvirus vector genome, vaccinia virus vector genome, adenovirus vector genome, adenovirus-associated virus vector genome, herpes virus vector genome, alpha virus vector genome, plasmid DNA and RNA.

5. The kit for use according to claim 4, wherein the vector particle is a lentiviral vector particle and comprises a lentiviral vector genome.

6. The kit for use according to claim 4 or 5, wherein the lentiviral vector particle further comprises an envelope comprising a Sindbis virus E2 glycoprotein having at least one amino acid change compared to SEQ ID NO:1, wherein residue 160 is either absent or an amino acid other than glutamic acid, and wherein E2 glycoprotein is not part of a fusion protein with Sindbis virus E3 protein, and preferably wherein the lentiviral vector particle delivers the recombinant expression vector to a dendritic cell.

7. The kit for use according to any one of the preceding claims, wherein the second composition is administered in combination with a composition comprising a therapeutic agent selected from the group consisting of a checkpoint inhibitor, a cytokine, chloroquine, an antibody that increase ADCC, an antibody that promotes a co-stimulatory signal, an anti-CD40 antibody.

8. The kit for use according to any one of the preceding claims, further comprising administering a boosting composition comprising an adjuvant in combination with a polypeptide, wherein the polypeptide comprises the cancer-associated antigen, or an immunogenic fragment thereof.

9. The kit for use according to any preceding claim wherein the GLA is formulated in a stable oil-in-water emulsion, and/or wherein the GLA has the formula: (I) 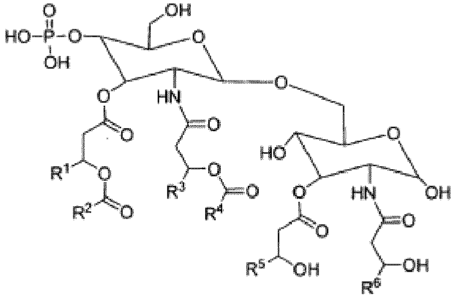 where: R¹, R³, R⁵ and R⁶ are C₁₁-C₂₀ alkyl; and R² and R⁴ are C₁₂-C₂₀ alkyl.

10. The kit for use according to any preceding claim wherein the GLA has the formula: (I) 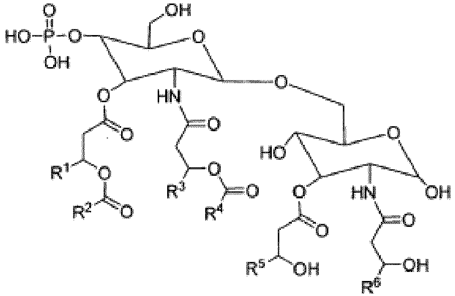 wherein R¹, R³, R⁵ and R⁶ equal to undecyl, and R² and R⁴ equal to tridecyl.

11. The kit for use according to any preceding claim, wherein the GLA has the formula: (II) 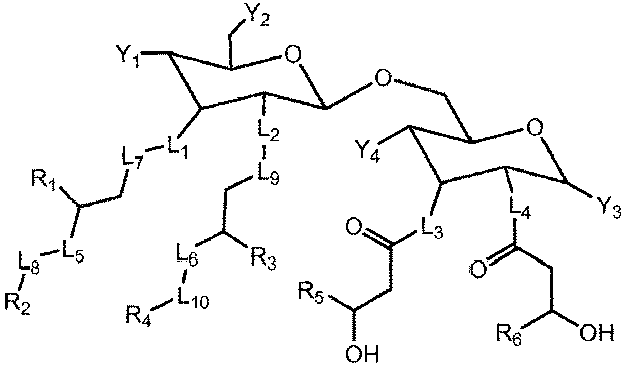 or a pharmaceutically acceptable salt thereof, wherein: L1, L2, L3, L4, L5 and L6 are the same or different and are independently selected from -O-, -NH-, and -(CH2)-; L7, L8, L9 and L10 are the same or different, and at any occurrence may be either absent or -C(=O)-; Y1 is an acid functional group; Y2 and Y3 are the same or different and are each independently selected from -OH, -SH, and an acid functional group; Y4 is -OH or -SH; R1, R3, R5 and R6 are the same or different and are each independently selected from the group of C8-C13 alkyl; and R2 and R4 are the same or different and are each independently selected from the group of C6-C11 alkyl.

## Patentansprüche

1. Set, das Folgendes umfasst:
a) eine erste Zusammensetzung, die ein Vektorpartikel, gegebenenfalls mit einem Adjuvans, umfasst, wobei das Vektorpartikel einen rekombinanten Expressionsvektor umfasst, wobei der rekombinante Expressionsvektor ein Polynucleotid umfasst, das für ein Krebs-assoziiertes Antigen kodiert, und wobei das Polynucleotid mit zumindest einer Expressionsregulationssequenz operabel verbunden ist, und
b) eine zweite Zusammensetzung, die einen TLR4-Agonisten umfasst, wobei die Zusammensetzung kein Antigen umfasst;
wobei die erste Zusammensetzung und die zweite Zusammensetzung jeweils weiters einen pharmazeutisch annehmbaren Exzipienten umfassen,
zur Verwendung in einem Verfahren zur Steigerung der T-Zellen in einer Tumormikroumgebung bei einem Individuum,
wobei die erste Zusammensetzung systemisch verabreicht wird, wodurch eine Immunreaktion gegen das Krebs-assoziierte Antigen bei dem Individuum induziert wird; und wobei die zweite Zusammensetzung intratumoral oder peritumoral an das Individuum verabreicht wird; und wobei die erste und die zweite Zusammensetzung gleichzeitig oder nacheinander verabreicht werden; wodurch die T-Zellen in der Tumormikroumgebung zunehmen, wobei das Adjuvans, wenn es vorhanden ist, Glucopyranosyl-Lipid A (GLA) ist.

2. Set zur Verwendung nach Anspruch 1, wobei der TLR4-Agonist Glucopyranosyl-Lipid A (GLA) ist.

3. Set zur Verwendung nach Anspruch 1 oder 2, wobei der Krebs ein Krebs ist, der von einem onkogenen Virus induziert ist, oder wobei das Krebs-assoziierte Antigen aus CTA, NY-ESO-1, LAGE-1, MAGE-A1, MAGE-A3, MAGE-A4, MAGE-A10, CT7, CT10, GAGE, PRAME; BAGE; RAGE, SAGE, HAGE, MPHOSPH1, DEPDC1, IMP3, MAGE-A, BK-T-Antigen, p53, Ras, c-Myc, A-Raf, B-Raf, C-Raf, Cyclin-abhängigen Kinasen, MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositid-3-Kinasen (PI3Ks), TRK-Rezeptoren, PRAME, P15, RU1, RU2, SART-1, SART-3, Wilms-Tumorantigen (WT1), AFP, β-Catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, Interferon-regulierendem Faktor 4 (IRF4), ETV6/AML, LDLR/FUT, Pml/RARα, Tumor-assoziiertem Calciumsignalwandler 1 (TACSTD1), TACSTD2, Rezeptor-Tyrosin-Kinasen, Epidermis-Wachstumsfaktor-Rezeptor (EGFR), EGFRvIII, Blutplättchen-Wachstumsfaktor-Rezeptor (PDGFR), Gefäßendothel-Wachstumsfaktor-Rezeptor (VEGFR), zytoplasmatischen Tyrosin-Kinasen, src-Familie, syk-ZAP70, integringekoppelter Kinase (ILK), Signalwandler und Aktivatoren der Transkription STAT3, STAT5 und STAT6, durch Hypoxie induzierbaren Faktoren, HIF-1α und HIF-2α, Nuklearfaktor κB (NF-κB), Notch-Rezeptoren, Notch1-4, c-Met, Ziel des Rapamycins im Säugetier (mTOR), WNT, extrazellulären Signal-regulierten Kinasen (ERKs), PMSA, PR-3, MDM2, Mesothelin, Nierenzellkarzinom - 5T4, SM22-α, Carboanhydrasen I (CAI) und IX (CAIX), STEAD, TEL/AML1, GD2, Proteinase3, hTERT, Sarkom-Translokationsbruchstellen, EphA2, ML-IAP, EpCAM, ERG (TMPRSS2-ETS-Fusionsgen), NA17, PAX3, ALK, Androgen-Rezeptor, Cyclin-B1, Polysialinsäure, MYCN, RhoC, GD3, Fucosyl-GM1, Mesothel, PSCA, sLe, PLACI, GM3, BORIS, Tn, GLoboH, NY-BR-1, RGs5, SART3, STn, PAX5, OY-TES1, Sperma-Protein 17, LCK, HMWMAA, AKAP-4, SSX2, XAGE 1, B7H3, Legumain, TIE2, Page4, MAD-CT-1, FAP, MAD-CT-2 und fos-verwandtem Antigen 1 ausgewählt ist.

4. Set zur Verwendung nach einem der vorangegangenen Ansprüche, wobei der rekombinante Expressionsvektor aus einem retroviralen Vektorgenom, einem lentiviralen Vektorgenom, Pockenvirusvektorgenom, Vacciniavirusvektorgenom, Adenovirusvektorgenom, adenovirusassoziierten Virusvektorgenom, Herpesvirusvektorgenom, Alphavirusvektorgenom, Plasmid-DNA und -RNA ausgewählt ist.

5. Set zur Verwendung nach Anspruch 4, wobei das Vektorpartikel ein lentivirales Vektorpartikel ist und ein lentivirales Vektorgenom umfasst.

6. Set zur Verwendung nach Anspruch 4 oder 5, wobei das lentivirale Vektorpartikel weiters eine Hülle umfasst, die ein Sindbis-Virus-E2-Glykoprotein mit zumindest einer Aminosäureänderung zu SEQ ID No: 1 aufweist, wobei Rest 160 entweder fehlt oder eine andere Aminosäure als Glutaminsäure ist und wobei E2-Glykoprotein nicht Teil eines Fusionsproteins mit Sindbis-Virus-E3-Protein ist, und wobei das lentivirale Vektorpartikel vorzugsweise den rekombinanten Expressionsvektor an eine dendritische Zelle liefert.

7. Set zur Verwendung nach einem der vorangegangenen Ansprüche, wobei die zweite Zusammensetzung in Kombination mit einer Zusammensetzung verabreicht wird, die ein Therapeutikum umfasst, das aus der aus einem Checkpoint-Hemmer, einem Zytokin, Chloroquin, einem ADCC steigernden Antikörper, einem ein kostimulatorisches Signal fördernden Antikörper und einem Anti-CD40-Antikörper bestehenden Gruppe ausgewählt ist.

8. Set zur Verwendung nach einem der vorangegangenen Ansprüche, das weiters das Verabreichen einer Verstärkungszusammensetzung, umfassend ein Adjuvans in Kombination mit einem Polypeptid, umfasst, wobei das Polypeptid das Krebs-assoziierte Antigen oder ein immunogenes Fragment davon umfasst.

9. Set zur Verwendung nach einem der vorangegangenen Ansprüche, wobei das GLA in einer stabilen ÖI-in-Wasser-Emulsion formuliert ist und/oder wobei das GLA die folgende Formel (I) aufweist: 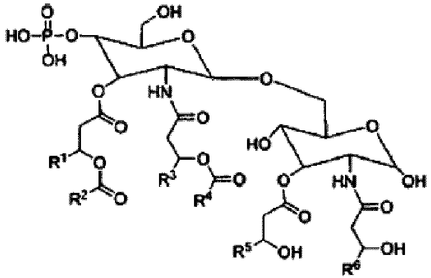 worin R¹, R³, R⁵ und R⁶ C₁₁-C₂₀-Alkyl sind und R² und R⁴ C₁₂-C₂₀-Alkyl sind.

10. Set zur Verwendung nach einem der vorangegangenen Ansprüche, wobei das GLA die folgende Formel (I) aufweist: 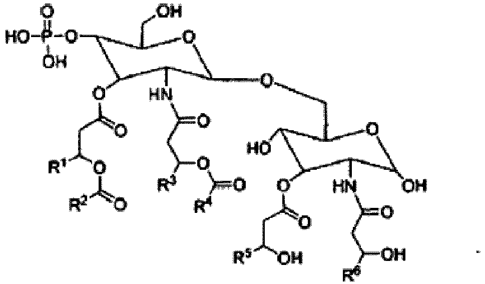 worin R¹, R³, R⁵ und R⁶ gleich wie Undecyl sind und R² und R⁴ gleich wie Tridecyl sind.

11. Set zur Verwendung nach einem der vorangegangenen Ansprüche, wobei GLA die folgende Formel (II) aufweist: 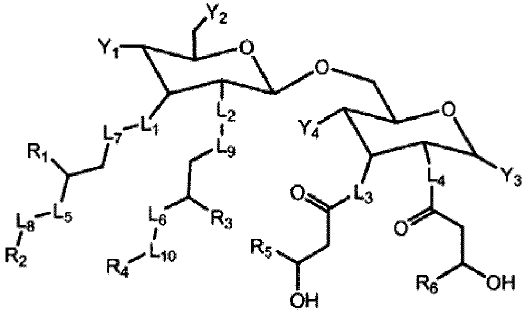 oder ein pharmazeutisch annehmbares Salz davon, wobei L₁, L₂, L₃, L₄, L₅ und L₆ gleich oder unterschiedlich sind und unabhängig aus -O-, -NH- und -(CH₂)- ausgewählt sind; L₇, L₈, L₉ und L₁₀ gleich oder unterschiedlich sind und bei jedem Auftreten entweder nicht vorhanden oder -C(=O)- sein können; Y₁ eine funktionelle Säuregruppe ist; Y₂ und Y₃ gleich oder unterschiedlich sind und jeweils unabhängig aus -OH, -SH und einer funktionellen Säuregruppe ausgewählt sind; Y₄ -OH oder -SH ist; R₁, R³, R⁵ und R⁶ gleich oder unterschiedlich sind und jeweils unabhängig aus der Gruppe C₈-C₁₃-Alkyl ausgewählt sind; und R₂ und R₄ gleich oder unterschiedlich sind und jeweils unabhängig aus der Gruppe C₆-C₁₁-Alkyl ausgewählt sind.

## Revendications

1. Kit comprenant :
a) une première composition comprenant une particule de vecteur facultativement avec un adjuvant, la particule de vecteur comprenant un vecteur d'expression recombinant, où le vecteur d'expression recombinant comprend un polynucléotide codant pour un antigène associé au cancer, et où le polynucléotide est en liaison fonctionnelle avec au moins une séquence régulatrice de l'expression, et
b) une seconde composition comprenant un agoniste de TLR4, où la composition ne comprend pas d'antigène ;
dans lequel la première composition et la seconde composition comprennent en outre chacune un excipient pharmaceutiquement approprié,
pour une utilisation dans un procédé d'augmentation des cellules T dans le microenvironnement d'une tumeur chez un sujet,
où la première composition est administrée par voie systémique, en induisant ainsi une réponse immunitaire contre l'antigène associé au cancer chez le sujet ; et où la seconde composition est administrée par voie intratumorale ou par voie péritumorale au sujet ; et où la première et la seconde composition sont administrées de manière concomitante ou de manière séquentielle ; en augmentant ainsi les cellules T dans le microenvironnement d'une tumeur, dans lequel, lorsque présent, l'adjuvant est le glucopyranosyl-lipide A (GLA).

2. Kit destiné à être utilisé selon la revendication 1, dans lequel l'agoniste de TLR4 est le glucopyranosyl-lipide A (GLA).

3. Kit destiné à être utilisé selon la revendication 1 ou 2, dans lequel le cancer est un cancer induit par un virus oncogène, ou dans lequel l'antigène associé au cancer est sélectionné parmi CTA, NY-ESO-1, LAGE-1, MAGE-A1, MAGE-A3, MAGE-A4, MAGE-A10, CT7, CT10, GAGE, PRAME ; BAGE ; RAGE, SAGE, HAGE, MPHOSPH1, DEPDC1, IMP3, MAGE-A, l'antigène T du virus BK, p53, Ras, c-Myc, A-Raf, B-Raf, C-Raf, les kinases cycline-dépendantes, MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, tyrosinase, TRP-1, TRP-2, ART-4, CAMEL, CEA, Cyp-B, hTERT, hTRT, iCE, MUC1, MUC2, les phosphoinositide 3-kinases (PI3K), les récepteurs TRK, PRAME, P15, RU1, RU2, SART-1, SART-3, l'antigène de la tumeur de Wilms (WT1), l'AFP, la β-caténine/m, la caspase-8/m, CEA, CDK-4/m, ELF2M, Gnt-V, G250, HSP70-2M, HST-2, KIAA0205, MUM-1, MUM-2, MUM-3, la myosine/m, RAGE, SART-2, TRP-2/INT2, 707-AP, l'annexine II, CDC27/m, TPI/mbcr-abl, BCR-ABL, le facteur de régulation de l'interféron 4 (IRF4), ETV6/AML, LDLR/FUT, Pml/RARα, le transducteur 1 du signal du calcium associé aux tumeurs (TACSTD1), TACSTD2, les récepteurs tyrosine kinases, le récepteur du facteur de croissance épidermique (EGFR), l'EGFRvIII, le récepteur du facteur de croissance dérivé des plaquettes (PDGFR), le récepteur du facteur de croissance de l'endothélium vasculaire (VEGFR), des tyrosine kinases cytoplasmiques, la famille src, syk-ZAP70, la kinase liée aux intégrines (ILK), les transducteurs du signal et activateurs de la transcription STAT3, STAT5, et STAT6, les facteurs inductibles par l'hypoxie, HIF-1α et HIF-2α, le facteur nucléaire kappa B (NF-κB), les récepteurs Notch, Notch1-4, c-Met, les cibles de la rapamycine chez les mammifères (mTOR), WNT, les kinases régulées par un signal extracellulaire (ERK), PMSA, PR-3, MDM2, la mésothéline, 5T4 du carcinome à cellules rénales, SM22-alpha, les anhydrases carboniques I (CAI) et IX (CAIX), STEAD, TEL/AML1, GD2, la protéinase 3, hTERT, les points de cassure des translocations des sarcomes, EphA2, ML-IAP, EpCAM, ERG (gène de fusion TMPRSS2 ETS), NA17, PAX3, ALK, le récepteur des androgènes, la cycline B1, l'acide polysialique, MYCN, RhoC, GD3, le fucosyl-GM1, la mésothéline, PSCA, sLe, PLAC1, GM3, BORIS, Tn, GLoboH, NY-BR-1, RGs5, SART3, STn, PAX5, OY-TES1, la protéine du sperme 17, LCK, HMWMAA, AKAP-4, SSX2, XAGE 1, B7H3, la légumaïne, TIE2, Page4, MAD-CT-1, FAP, MAD-CT-2, et l'antigène 1 apparenté à Fos.

4. Kit destiné à être utilisé selon l'une quelconque des revendications précédentes, dans lequel le vecteur d'expression recombinant est sélectionné parmi un génome de vecteur rétroviral, un génome de vecteur lentiviral, un génome de vecteur poxvirus, un génome de vecteur de virus de la vaccine, un génome de vecteur adénovirus, un génome de vecteur de virus adéno-associé, un génome de vecteur herpèsvirus, un génome de vecteur alphavirus, un ADN et un ARN plasmidique.

5. Kit destiné à être utilisé selon la revendication 4, dans lequel la particule de vecteur est une particule de vecteur lentiviral et comprend un génome de vecteur lentiviral.

6. Kit destiné à être utilisé selon la revendication 4 ou 5, dans lequel la particule de vecteur lentiviral comprend en outre une enveloppe comprenant une glycoprotéine E2 du virus Sindbis comportant au moins une modification d'acide aminé par comparaison à SEQ ID NO: 1, dans laquelle le résidu 160 est absent ou est un acide aminé autre que l'acide glutamique, et dans lequel la glycoprotéine E2 ne fait pas partie d'une protéine de fusion avec la protéine E3 du virus Sindbis, et de préférence dans lequel la particule de vecteur lentiviral délivre le vecteur d'expression recombinant à une cellule dendritique.

7. Kit destiné à être utilisé selon l'une quelconque des revendications précédentes, dans lequel la seconde composition est administrée en combinaison avec une composition comprenant un agent thérapeutique sélectionné dans le groupe consistant en un inhibiteur de point de contrôle, une cytokine, une chloroquine, un anticorps qui augmente l'ADCC, un anticorps qui favorise un signal co-stimulateur, un anticorps anti-CD40.

8. Kit destiné à être utilisé selon l'une quelconque des revendications précédentes, comprenant en outre l'administration d'une composition de rappel comprenant un adjuvant en combinaison avec un polypeptide, où le polypeptide comprend l'antigène associé au cancer, ou un fragment immunogène de celui-ci.

9. Kit destiné à être utilisé selon une quelconque revendication précédente, dans lequel le GLA est formulé dans une émulsion huile dans eau stable, et/ou dans lequel le GLA possède la formule : (I) 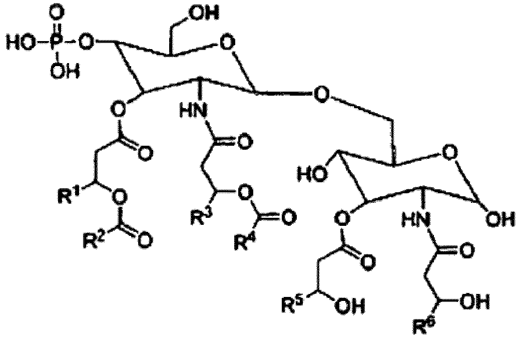 dans laquelle : R¹, R³, R⁵ et R⁶ sont alkyle en C₁₁-C₂₀ ; et R² et R₄ sont alkyle en C₁₂-C₂₀.

10. Kit destiné à être utilisé selon une quelconque revendication précédente, dans lequel le GLA possède la formule : (I) 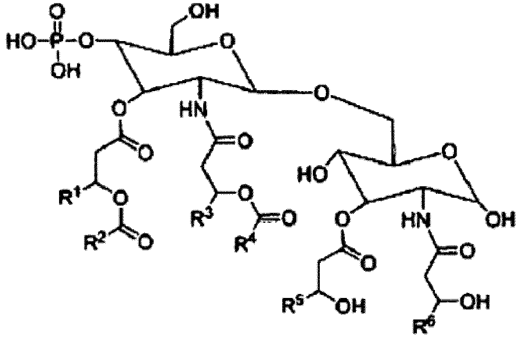 dans laquelle R¹, R³, R⁵ et R⁶ sont équivalents à undécyle, et R² et R⁴ sont équivalents à tridécyle.

11. Kit destiné à être utilisé selon une quelconque revendication précédente, dans lequel le GLA possède la formule : (II) 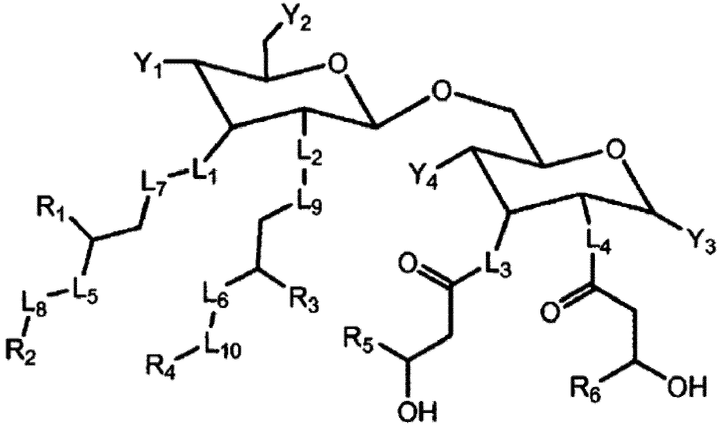 ou un sel de celui-ci pharmaceutiquement acceptable, dans laquelle : L1, L2, L3, L4, L5 et L6 sont identiques ou différents et sont indépendamment sélectionnés parmi -O-, - NH-, et -(CH2)- ; L7, L8, L9 et L10 sont identiques ou différents et, à une quelconque occurrence, peuvent être absents ou -C(=O)- ; Y1 est un groupe fonctionnel acide ; Y2 et Y3 sont identiques ou différents et sont chacun indépendamment sélectionnés parmi -OH, -SH, et un groupe fonctionnel acide ; Y4 est -OH ou -SH ; R1, R3, R5 et R6 sont identiques ou différents et sont chacun indépendamment sélectionnés dans le groupe des alkyles en C8-C13 ; et R2 et R4 sont identiques ou différents et sont chacun indépendamment sélectionnés dans le groupe des alkyles en C6-C11.


### SEQUENCE LISTING

<110> IMMUNE DESIGN CORP
<120> IMMUNOTHERAPY OF CANCER THROUGH COMBINATION OF LOCAL AND SYSTEMIC IMMUNE STIMULATION
<130> 31943/48357
<150> US-61/940,109
   <151> 2014-02-14
<150> US-61/985,787
   <151> 2014-04-29
<150> US-62/022,070
   <151> 2014-07-08
<150> US-62/072,548
   <151> 2014-10-30
<150> US-62/101,335
   <151> 2015-01-08
<160> 45
<170> PatentIn version 3.5
<210> 1
   <211> 423
   <212> PRT
   <213> Sindbis virus
<220>
   <221> misc_feature
   <222> (160) .. (160)
   <223> Xaa can be any naturally occurring amino acid
<400> 1
   Ser Val Ile Asp Asp Phe Thr Leu Thr Ser Pro Tyr Leu Gly Thr Cys
   1               5                   10                  15
   Ser Tyr Cys His His Thr Val Pro Cys Phe Ser Pro Val Lys Ile Glu
               20                  25                  30
   Gln Val Trp Asp Glu Ala Asp Asp Asn Thr Ile Arg Ile Gln Thr Ser
           35                  40                  45
   Ala Gln Phe Gly Tyr Asp Gln Ser Gly Ala Ala Ser Ala Asn Lys Tyr
       50                  55                  60
   Arg Tyr Met Ser Leu Lys Gln Asp His Thr Val Lys Glu Gly Thr Met
   65                  70                  75                  80
   Asp Asp Ile Lys Ile Ser Thr Ser Gly Pro Cys Arg Arg Leu Ser Tyr
                   85                  90                  95
   Lys Gly Tyr Phe Leu Leu Ala Lys Cys Pro Pro Gly Asp Ser Val Thr
               100                 105                 110
   Val Ser Ile Val Ser Ser Asn Ser Ala Thr Ser Cys Thr Leu Ala Arg
           115                 120                 125
   Lys Ile Lys Pro Lys Phe Val Gly Arg Glu Lys Tyr Asp Leu Pro Pro
       130                 135                 140
   Val His Gly Lys Lys Ile Pro Cys Thr Val Tyr Asp Arg Leu Lys Xaa
   145                 150                 155                 160
   Thr Thr Ala Gly Tyr Ile Thr Met His Arg Pro Arg Pro His Ala Tyr
                   165                 170                 175
   Thr Ser Tyr Leu Glu Glu Ser Ser Gly Lys Val Tyr Ala Lys Pro Pro
               180                 185                 190
   Ser Gly Lys Asn Ile Thr Tyr Glu Cys Lys Cys Gly Asp Tyr Lys Thr
           195                 200                 205
   Gly Thr Val Ser Thr Arg Thr Glu Ile Thr Gly Cys Thr Ala Ile Lys
       210                 215                 220
   Gln Cys Val Ala Tyr Lys Ser Asp Gln Thr Lys Trp Val Phe Asn Ser
   225                 230                 235                 240
   Pro Asp Leu Ile Arg His Asp Asp His Thr Ala Gln Gly Lys Leu His
                   245                 250                 255
   Leu Pro Phe Lys Leu Ile Pro Ser Thr Cys Met Val Pro Val Ala His
               260                 265                 270
   Ala Pro Asn Val Ile His Gly Phe Lys His Ile Ser Leu Gln Leu Asp
           275                 280                 285
   Thr Asp His Leu Thr Leu Leu Thr Thr Arg Arg Leu Gly Ala Asn Pro
       290                 295                 300
   Glu Pro Thr Thr Glu Trp Ile Val Gly Lys Thr Val Arg Asn Phe Thr
   305                 310                 315                 320
   Val Asp Arg Asp Gly Leu Glu Tyr Ile Trp Gly Asn His Glu Pro Val
                   325                 330                 335
   Arg Val Tyr Ala Gln Glu Ser Ala Pro Gly Asp Pro His Gly Trp Pro
   340 345 350
   His Glu Ile Val Gln His Tyr Tyr His Arg His Pro Val Tyr Thr Ile
   355 360 365
   Leu Ala Val Ala Ser Ala Thr Val Ala Met Met Ile Gly Val Thr Val
   370 375 380
   Ala Val Leu Cys Ala Cys Lys Ala Arg Arg Glu Cys Leu Thr Pro Tyr
   385 390 395 400
   Ala Leu Ala Pro Asn Ala Val Ile Pro Thr Ser Leu Ala Leu Leu Cys
   405 410 415
   Cys Val Arg Ser Ala Asn Ala
   420
<210> 2
   <211> 986
   <212> PRT
   <213> Sindbis virus
<400> 2
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Ser Val Ile
       50                  55                  60
   Asp Asp Phe Thr Leu Thr Ser Pro Tyr Leu Gly Thr Cys Ser Tyr Cys
   65                  70                  75                  80
   His His Thr Val Pro Cys Phe Ser Pro Val Lys Ile Glu Gln Val Trp
                   85                  90                  95
   Asp Glu Ala Asp Asp Asn Thr Ile Arg Ile Gln Thr Ser Ala Gln Phe
               100                 105                 110
   Gly Tyr Asp Gln Ser Gly Ala Ala Ser Ala Asn Lys Tyr Arg Tyr Met
           115                 120                 125
   Ser Leu Lys Gln Met Tyr Pro Tyr Asp Val Pro Asp Tyr Ala Thr Val
   130 135 140
   Lys Glu Gly Thr Met Asp Asp Ile Lys Ile Ser Thr Ser Gly Pro Cys
   145 150 155 160
   Arg Arg Leu Ser Tyr Lys Gly Tyr Phe Leu Leu Ala Lys Cys Pro Pro
   165 170 175
   Gly Asp Ser Val Thr Val Ser Ile Val Ser Ser Asn Ser Ala Thr Ser
   180 185 190
   Cys Thr Leu Ala Arg Lys Ile Lys Pro Lys Phe Val Gly Arg Glu Lys
   195 200 205
   Tyr Asp Leu Pro Pro Val His Gly Lys Lys Ile Pro Cys Thr Val Tyr
   210 215 220
   Asp Arg Leu Ala Ala Thr Thr Ala Gly Tyr Ile Thr Met His Arg Pro
   225 230 235 240
   Arg Pro His Ala Tyr Thr Ser Tyr Leu Glu Glu Ser Ser Gly Lys Val
   245 250 255
   Tyr Ala Lys Pro Pro Ser Gly Lys Asn Ile Thr Tyr Glu Cys Lys Cys
   260 265 270
   Gly Asp Tyr Lys Thr Gly Thr Val Ser Thr Arg Thr Glu Ile Thr Gly
   275 280 285
   Cys Thr Ala Ile Lys Gln Cys Val Ala Tyr Lys Ser Asp Gln Thr Lys
   290 295 300
   Trp Val Phe Asn Ser Pro Asp Leu Ile Arg His Asp Asp His Thr Ala
   305 310 315 320
   Gln Gly Lys Leu His Leu Pro Phe Lys Leu Ile Pro Ser Thr Cys Met
   325 330 335
   Val Pro Val Ala His Ala Pro Asn Val Ile His Gly Phe Lys His Ile
   340 345 350
   Ser Leu Gln Leu Asp Thr Asp His Leu Thr Leu Leu Thr Thr Arg Arg
   355 360 365
   Leu Gly Ala Asn Pro Glu Pro Thr Thr Glu Trp Ile Val Gly Lys Thr
   370 375 380
   Val Arg Asn Phe Thr Val Asp Arg Asp Gly Leu Glu Tyr Ile Trp Gly
   385                 390                 395                 400
   Asn His Glu Pro Val Arg Val Tyr Ala Gln Glu Ser Ala Pro Gly Asp
                   405                 410                 415
   Pro His Gly Trp Pro His Glu Ile Val Gln His Tyr Tyr His Arg His
               420                 425                 430
   Pro Val Tyr Thr Ile Leu Ala Val Ala Ser Ala Thr Val Ala Met Met
           435                 440                 445
   Ile Gly Val Thr Val Ala Val Leu Cys Ala Cys Lys Ala Arg Arg Glu
       450                 455                 460
   Cys Leu Thr Pro Tyr Ala Leu Ala Pro Asn Ala Val Ile Pro Thr Ser
   465                 470                 475                 480
   Leu Ala Leu Leu Cys Cys Val Arg Ser Ala Asn Ala Glu Thr Phe Thr
                   485                 490                 495
   Glu Thr Met Ser Tyr Leu Trp Ser Asn Ser Gln Pro Phe Phe Trp Val
               500                 505                 510
   Gln Leu Cys Ile Pro Leu Ala Ala Phe Ile Val Leu Met Arg Cys Cys
           515                 520                 525
   Ser Cys Cys Leu Pro Phe Leu Val Val Ala Gly Ala Tyr Leu Ala Lys
       530                 535                 540
   Val Asp Ala Tyr Glu His Ala Thr Thr Val Pro Asn Val Pro Gln Ile
   545                 550                 555                 560
   Pro Tyr Lys Ala Leu Val Glu Arg Ala Gly Tyr Ala Pro Leu Asn Leu
                   565                 570                 575
   Glu Ile Thr Val Met Ser Ser Glu Val Leu Pro Ser Thr Asn Gln Glu
               580                 585                 590
   Tyr Ile Thr Cys Lys Phe Thr Thr Val Val Pro Ser Pro Lys Ile Lys
           595                 600                 605
   Cys Cys Gly Ser Leu Glu Cys Gln Pro Ala Ala His Ala Gly Tyr Thr
       610                 615                 620
   Cys Lys Val Phe Gly Gly Val Tyr Pro Phe Met Trp Gly Gly Ala Gln
   625                 630                 635                 640
   Cys Phe Cys Asp Ser Glu Asn Ser Gln Met Ser Glu Ala Tyr Val Glu
                   645                 650                 655
   Leu Ser Ala Asp Cys Ala Ser Asp His Ala Gln Ala Ile Lys Val His
               660                 665                 670
   Thr Ala Ala Met Lys Val Gly Leu Arg Ile Val Tyr Gly Asn Thr Thr
           675                 680                 685
   Ser Phe Leu Asp Val Tyr Val Asn Gly Val Thr Pro Gly Thr Ser Lys
       690                 695                 700
   Asp Leu Lys Val Ile Ala Gly Pro Ile Ser Ala Ser Phe Thr Pro Phe
   705                 710                 715                 720
   Asp His Lys Val Val Ile His Arg Gly Leu Val Tyr Asn Tyr Asp Phe
                   725                 730                 735
   Pro Glu Tyr Gly Ala Met Lys Pro Gly Ala Phe Gly Asp Ile Gln Ala
               740                 745                 750
   Thr Ser Leu Thr Ser Lys Asp Leu Ile Ala Ser Thr Asp Ile Arg Leu
           755                 760                 765
   Leu Lys Pro Ser Ala Lys Asn Val His Val Pro Tyr Thr Gln Ala Ser
       770                 775                 780
   Ser Gly Phe Glu Met Trp Lys Asn Asn Ser Gly Arg Pro Leu Gln Glu
   785                 790                 795                 800
   Thr Ala Pro Phe Gly Cys Lys Ile Ala Val Asn Pro Leu Arg Ala Val
                   805                 810                 815
   Asp Cys Ser Tyr Gly Asn Ile Pro Ile Ser Ile Asp Ile Pro Asn Ala
               820                 825                 830
   Ala Phe Ile Arg Thr Ser Asp Ala Pro Leu Val Ser Thr Val Lys Cys
           835                 840                 845
   Glu Val Ser Glu Cys Thr Tyr Ser Ala Asp Phe Gly Gly Met Ala Thr
       850                 855                 860
   Leu Gln Tyr Val Ser Asp Arg Glu Gly Gln Cys Pro Val His Ser His
   865                 870                 875                 880
   Ser Ser Thr Ala Thr Leu Gln Glu Ser Thr Val His Val Leu Glu Lys
                   885                 890                 895
   Gly Ala Val Thr Val His Phe Ser Thr Ala Ser Pro Gln Ala Asn Phe
   900 905 910
   Ile Val Ser Leu Cys Gly Lys Lys Thr Thr Cys Asn Ala Glu Cys Lys
   915 920 925
   Pro Pro Ala Asp His Ile Val Ser Thr Pro His Lys Asn Asp Gln Glu
   930 935 940
   Phe Gln Ala Ala Ile Ser Lys Thr Ser Trp Ser Trp Leu Phe Ala Leu
   945 950 955 960
   Phe Gly Gly Ala Ser Ser Leu Leu Ile Ile Gly Leu Met Ile Phe Ala
   965 970 975
   Cys Ser Met Met Leu Thr Ser Thr Arg Arg
   980 985
<210> 3
   <211> 982
   <212> PRT
   <213> Sindbis virus
<400> 3
   Met Ser Ala Ala Pro Leu Val Thr Ala Met Cys Leu Leu Gly Asn Val
   1 5 10 15
   Ser Phe Pro Cys Asp Arg Pro Pro Thr Cys Tyr Thr Arg Glu Pro Ser
   20 25 30
   Arg Ala Leu Asp Ile Leu Glu Glu Asn Val Asn His Glu Ala Tyr Asp
   35 40 45
   Thr Leu Leu Asn Ala Ile Leu Arg Cys Gly Ser Ser Gly Arg Ser Lys
   50 55 60
   Arg Ser Val Ile Asp Asp Phe Thr Leu Thr Ser Pro Tyr Leu Gly Thr
   65 70 75 80
   Cys Ser Tyr Cys His His Thr Val Pro Cys Phe Ser Pro Val Lys Ile
   85 90 95
   Glu Gln Val Trp Asp Glu Ala Asp Asp Asn Thr Ile Arg Ile Gln Thr
   100 105 110
   Ser Ala Gln Phe Gly Tyr Asp Gln Ser Gly Ala Ala Ser Ala Asn Lys
   115 120 125
   Tyr Arg Tyr Met Ser Leu Glu Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Glu
       210                 215                 220
   Gly Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
                   325                 330                 335
   His Ala Pro Asn Val Ile His Gly Phe Lys His Ile Ser Leu Gln Leu
               340                 345                 350
   Asp Thr Asp His Leu Thr Leu Leu Thr Thr Arg Arg Leu Gly Ala Asn
           355                 360                 365
   Pro Glu Pro Thr Thr Glu Trp Ile Val Gly Lys Thr Val Arg Asn Phe
   370 375 380
   Thr Val Asp Arg Asp Gly Leu Glu Tyr Ile Trp Gly Asn His Glu Pro
   385 390 395 400
   Val Arg Val Tyr Ala Gln Glu Ser Ala Pro Gly Asp Pro His Gly Trp
   405 410 415
   Pro His Glu Ile Val Gln His Tyr Tyr His Arg His Pro Val Tyr Thr
   420 425 430
   Ile Leu Ala Val Ala Ser Ala Thr Val Ala Met Met Ile Gly Val Thr
   435 440 445
   Val Ala Val Leu Cys Ala Cys Lys Ala Arg Arg Glu Cys Leu Thr Pro
   450 455 460
   Tyr Ala Leu Ala Pro Asn Ala Val Ile Pro Thr Ser Leu Ala Leu Leu
   465 470 475 480
   Cys Cys Val Arg Ser Ala Asn Ala Glu Thr Phe Thr Glu Thr Met Ser
   485 490 495
   Tyr Leu Trp Ser Asn Ser Gln Pro Phe Phe Trp Val Gln Leu Cys Ile
   500 505 510
   Pro Leu Ala Ala Phe Ile Val Leu Met Arg Cys Cys Ser Cys Cys Leu
   515 520 525
   Pro Phe Leu Val Val Ala Gly Ala Tyr Leu Ala Lys Val Asp Ala Tyr
   530 535 540
   Glu His Ala Thr Thr Val Pro Asn Val Pro Gln Ile Pro Tyr Lys Ala
   545 550 555 560
   Leu Val Glu Arg Ala Gly Tyr Ala Pro Leu Asn Leu Glu Ile Thr Val
   565 570 575
   Met Ser Ser Glu Val Leu Pro Ser Thr Asn Gln Glu Tyr Ile Thr Cys
   580 585 590
   Lys Phe Thr Thr Val Val Pro Ser Pro Lys Ile Lys Cys Cys Gly Ser
   595 600 605
   Leu Glu Cys Gln Pro Ala Ala His Ala Asp Tyr Thr Cys Lys Val Phe
   610 615 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705                 710                 715                 720
   Val Ile His Arg Gly Leu Val Tyr Asn Tyr Asp Phe Pro Glu Tyr Gly
                   725                 730                 735
   Ala Met Lys Pro Gly Ala Phe Gly Asp Ile Gln Ala Thr Ser Leu Thr
               740                 745                 750
   Ser Lys Asp Leu Ile Ala Ser Thr Asp Ile Arg Leu Leu Lys Pro Ser
           755                 760                 765
   Ala Lys Asn Val His Val Pro Tyr Thr Gln Ala Ser Ser Gly Phe Glu
       770                 775                 780
   Met Trp Lys Asn Asn Ser Gly Arg Pro Leu Gln Glu Thr Ala Pro Phe
   785                 790                 795                 800
   Gly Cys Lys Ile Ala Val Asn Pro Leu Arg Ala Val Asp Cys Ser Tyr
                   805                 810                 815
   Gly Asn Ile Pro Ile Ser Ile Asp Ile Pro Asn Ala Ala Phe Ile Arg
               820                 825                 830
   Thr Ser Asp Ala Pro Leu Val Ser Thr Val Lys Cys Glu Val Ser Glu
           835                 840                 845
   Cys Thr Tyr Ser Ala Asp Phe Gly Gly Met Ala Thr Leu Gln Tyr Val
       850                 855                 860
   Ser Asp Arg Glu Gly Gln Cys Pro Val His Ser His Ser Ser Thr Ala
   865                 870                 875                 880
   Thr Leu Gln Glu Ser Thr Val His Val Leu Glu Lys Gly Ala Val Thr
                   885                 890                 895
   Val His Phe Ser Thr Ala Ser Pro Gln Ala Asn Phe Ile Val Ser Leu
               900                 905                 910
   Cys Gly Lys Lys Thr Thr Cys Asn Ala Glu Cys Lys Pro Pro Ala Asp
           915                 920                 925
   His Ile Val Ser Thr Pro His Lys Asn Asp Gln Glu Phe Gln Ala Ala
       930                 935                 940
   Ile Ser Lys Thr Ser Trp Ser Trp Leu Phe Ala Leu Phe Gly Gly Ala
   945                 950                 955                 960
   Ser Ser Leu Leu Ile Ile Gly Leu Met Ile Phe Ala Cys Ser Met Met
                   965                 970                 975
   Leu Thr Ser Thr Arg Arg
               980
<210> 4
   <211> 982
   <212> PRT
   <213> Sindbis virus
<400> 4
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
   115 120 125
   Tyr Arg Tyr Met Ser Leu Glu Gln Asp His Thr Val Glu Glu Gly Thr
   130 135 140
   Met Asp Asp Ile Lys Ile Ser Thr Ser Gly Pro Cys Arg Arg Leu Ser
   145 150 155 160
   Tyr Lys Gly Tyr Phe Leu Leu Ala Lys Cys Pro Pro Gly Asp Ser Val
   165 170 175
   Thr Val Ser Ile Val Ser Ser Asn Ser Ala Thr Ser Cys Thr Leu Ala
   180 185 190
   Arg Lys Ile Lys Pro Lys Phe Val Gly Arg Glu Lys Tyr Asp Leu Pro
   195 200 205
   Pro Val His Gly Lys Lys Ile Pro Cys Thr Val Tyr Asp Arg Leu Glu
   210 215 220
   Gly Thr Thr Ala Gly Tyr Ile Thr Met His Arg Pro Arg Pro His Ala
   225 230 235 240
   Tyr Thr Ser Tyr Leu Glu Glu Ser Ser Gly Lys Val Tyr Ala Lys Pro
   245 250 255
   Pro Ser Gly Lys Asn Ile Thr Tyr Glu Cys Lys Cys Gly Asp Tyr Lys
   260 265 270
   Thr Gly Thr Val Ser Thr Arg Thr Glu Ile Thr Gly Cys Thr Ala Ile
   275 280 285
   Lys Gln Cys Val Ala Tyr Lys Ser Asp Gln Thr Lys Trp Val Phe Asn
   290 295 300
   Ser Pro Asp Leu Ile Arg His Asp Asp His Thr Ala Gln Gly Lys Leu
   305 310 315 320
   His Leu Pro Phe Lys Leu Ile Pro Ser Thr Cys Met Val Pro Val Ala
   325 330 335
   His Ala Pro Asn Val Ile His Gly Phe Lys His Ile Ser Leu Gln Leu
   340 345 350
   Asp Thr Asp His Leu Thr Leu Leu Thr Thr Arg Arg Leu Gly Ala Asn
   355 360 365
   Pro Glu Pro Thr Thr Glu Trp Ile Val Gly Lys Thr Val Arg Asn Phe
       370                 375                 380
   Thr Val Asp Arg Asp Gly Leu Glu Tyr Ile Trp Gly Asn His Glu Pro
   385                 390                 395                 400
   Val Arg Val Tyr Ala Gln Glu Ser Ala Pro Gly Asp Pro His Gly Trp
                   405                 410                 415
   Pro His Glu Ile Val Gln His Tyr Tyr His Arg His Pro Val Tyr Thr
               420                 425                 430
   Ile Leu Ala Val Ala Ser Ala Thr Val Ala Met Met Ile Gly Val Thr
           435                 440                 445
   Val Ala Val Leu Cys Ala Cys Lys Ala Arg Arg Glu Cys Leu Thr Pro
       450                 455                 460
   Tyr Ala Leu Ala Pro Asn Ala Val Ile Pro Thr Ser Leu Ala Leu Leu
   465                 470                 475                 480
   Cys Cys Val Arg Ser Ala Asn Ala Glu Thr Phe Thr Glu Thr Met Ser
                   485                 490                 495
   Tyr Leu Trp Ser Asn Ser Gln Pro Phe Phe Trp Val Gln Leu Cys Ile
               500                 505                 510
   Pro Leu Ala Ala Phe Ile Val Leu Met Arg Cys Cys Ser Cys Cys Leu
           515                 520                 525
   Pro Phe Leu Val Val Ala Gly Ala Tyr Leu Ala Lys Val Asp Ala Tyr
       530                 535                 540
   Glu His Ala Thr Thr Val Pro Asn Val Pro Gln Ile Pro Tyr Lys Ala
   545                 550                 555                 560
   Leu Val Glu Arg Ala Gly Tyr Ala Pro Leu Asn Leu Glu Ile Thr Val
                   565                 570                 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
   610 615 620
   Gly Gly Val Tyr Pro Phe Met Trp Gly Gly Ala Gln Cys Phe Cys Asp
   625 630 635 640
   Ser Glu Asn Ser Gln Met Ser Glu Ala Tyr Val Glu Leu Ser Ala Asp
   645 650 655
   Cys Ala Ser Asp His Ala Gln Ala Ile Lys Val His Thr Ala Ala Met
   660 665 670
   Lys Val Gly Leu Arg Ile Val Tyr Gly Asn Thr Thr Ser Phe Leu Asp
   675 680 685
   Val Tyr Val Asn Gly Val Thr Pro Gly Thr Ser Lys Asp Leu Lys Val
   690 695 700
   Ile Ala Gly Pro Ile Ser Ala Ser Phe Thr Pro Phe Asp His Lys Val
   705 710 715 720
   Val Ile His Arg Gly Leu Val Tyr Asn Tyr Asp Phe Pro Glu Tyr Gly
   725 730 735
   Ala Met Lys Pro Gly Ala Phe Gly Asp Ile Gln Ala Thr Ser Leu Thr
   740 745 750
   Ser Lys Asp Leu Ile Ala Ser Thr Asp Ile Arg Leu Leu Lys Pro Ser
   755 760 765
   Ala Lys Asn Val His Val Pro Tyr Thr Gln Ala Ser Ser Gly Phe Glu
   770 775 780
   Met Trp Lys Asn Asn Ser Gly Arg Pro Leu Gln Glu Thr Ala Pro Phe
   785 790 795 800
   Gly Cys Lys Ile Ala Val Asn Pro Leu Arg Ala Val Asp Cys Ser Tyr
   805 810 815
   Gly Asn Ile Pro Ile Ser Ile Asp Ile Pro Asn Ala Ala Phe Ile Arg
   820 825 830
   Thr Ser Asp Ala Pro Leu Val Ser Thr Val Lys Cys Glu Val Ser Glu
   835 840 845
   Cys Thr Tyr Ser Ala Asp Phe Gly Gly Met Ala Thr Leu Gln Tyr Val
   850 855 860
   Ser Asp Arg Glu Gly Gln Cys Pro Val His Ser His Ser Ser Thr Ala
   865                 870                 875                 880
   Thr Leu Gln Glu Ser Thr Val His Val Leu Glu Lys Gly Ala Val Thr
                   885                 890                 895
   Val His Phe Ser Thr Ala Ser Pro Gln Ala Asn Phe Ile Val Ser Leu
               900                 905                 910
   Cys Gly Lys Lys Thr Thr Cys Asn Ala Glu Cys Lys Pro Pro Ala Asp
           915                 920                 925
   His Ile Val Ser Thr Pro His Lys Asn Asp Gln Glu Phe Gln Ala Ala
       930                 935                 940
   Ile Ser Lys Thr Ser Trp Ser Trp Leu Phe Ala Leu Phe Gly Gly Ala
   945                 950                 955                 960
   Ser Ser Leu Leu Ile Ile Gly Leu Met Ile Phe Ala Cys Ser Met Met
                   965                 970                 975
   Leu Thr Ser Thr Arg Arg
               980
<210> 5
   <211> 980
   <212> PRT
   <213> Sindbis virus
<400> 5
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Glu Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Thr
       210                 215                 220
   Thr Ala Gly Tyr Ile Thr Met His Arg Pro Arg Pro His Ala Tyr Thr
   225                 230                 235                 240
   Ser Tyr Leu Glu Glu Ser Ser Gly Lys Val Tyr Ala Lys Pro Pro Ser
                   245                 250                 255
   Gly Lys Asn Ile Thr Tyr Glu Cys Lys Cys Gly Asp Tyr Lys Thr Gly
               260                 265                 270
   Thr Val Ser Thr Arg Thr Glu Ile Thr Gly Cys Thr Ala Ile Lys Gln
           275                 280                 285
   Cys Val Ala Tyr Lys Ser Asp Gln Thr Lys Trp Val Phe Asn Ser Pro
       290                 295                 300
   Asp Leu Ile Arg His Asp Asp His Thr Ala Gln Gly Lys Leu His Leu
   305                 310                 315                 320
   Pro Phe Lys Leu Ile Pro Ser Thr Cys Met Val Pro Val Ala His Ala
                   325                 330                 335
   Pro Asn Val Ile His Gly Phe Lys His Ile Ser Leu Gln Leu Asp Thr
               340                 345                 350
   Asp His Leu Thr Leu Leu Thr Thr Arg Arg Leu Gly Ala Asn Pro Glu
           355                 360                 365
   Pro Thr Thr Glu Trp Ile Val Gly Lys Thr Val Arg Asn Phe Thr Val
       370                 375                 380
   Asp Arg Asp Gly Leu Glu Tyr Ile Trp Gly Asn His Glu Pro Val Arg
   385                 390                 395                 400
   Val Tyr Ala Gln Glu Ser Ala Pro Gly Asp Pro His Gly Trp Pro His
                   405                 410                 415
   Glu Ile Val Gln His Tyr Tyr His Arg His Pro Val Tyr Thr Ile Leu
               420                 425                 430
   Ala Val Ala Ser Ala Thr Val Ala Met Met Ile Gly Val Thr Val Ala
           435                 440                 445
   Val Leu Cys Ala Cys Lys Ala Arg Arg Glu Cys Leu Thr Pro Tyr Ala
       450                 455                 460
   Leu Ala Pro Asn Ala Val Ile Pro Thr Ser Leu Ala Leu Leu Cys Cys
   465                 470                 475                 480
   Val Arg Ser Ala Asn Ala Glu Thr Phe Thr Glu Thr Met Ser Tyr Leu
                   485                 490                 495
   Trp Ser Asn Ser Gln Pro Phe Phe Trp Val Gln Leu Cys Ile Pro Leu
               500                 505                 510
   Ala Ala Phe Ile Val Leu Met Arg Cys Cys Ser Cys Cys Leu Pro Phe
           515                 520                 525
   Leu Val Val Ala Gly Ala Tyr Leu Ala Lys Val Asp Ala Tyr Glu His
       530                 535                 540
   Ala Thr Thr Val Pro Asn Val Pro Gln Ile Pro Tyr Lys Ala Leu Val
   545                 550                 555                 560
   Glu Arg Ala Gly Tyr Ala Pro Leu Asn Leu Glu Ile Thr Val Met Ser
                   565                 570                 575
   Ser Glu Val Leu Pro Ser Thr Asn Gln Glu Tyr Ile Thr Cys Lys Phe
               580                 585                 590
   Thr Thr Val Val Pro Ser Pro Lys Ile Lys Cys Cys Gly Ser Leu Glu
           595                 600                 605
   Cys Gln Pro Ala Ala His Ala Asp Tyr Thr Cys Lys Val Phe Gly Gly
   610 615 620
   Val Tyr Pro Phe Met Trp Gly Gly Ala Gln Cys Phe Cys Asp Ser Glu
   625 630 635 640
   Asn Ser Gln Met Ser Glu Ala Tyr Val Glu Leu Ser Ala Asp Cys Ala
   645 650 655
   Ser Asp His Ala Gln Ala Ile Lys Val His Thr Ala Ala Met Lys Val
   660 665 670
   Gly Leu Arg Ile Val Tyr Gly Asn Thr Thr Ser Phe Leu Asp Val Tyr
   675 680 685
   Val Asn Gly Val Thr Pro Gly Thr Ser Lys Asp Leu Lys Val Ile Ala
   690 695 700
   Gly Pro Ile Ser Ala Ser Phe Thr Pro Phe Asp His Lys Val Val Ile
   705 710 715 720
   His Arg Gly Leu Val Tyr Asn Tyr Asp Phe Pro Glu Tyr Gly Ala Met
   725 730 735
   Lys Pro Gly Ala Phe Gly Asp Ile Gln Ala Thr Ser Leu Thr Ser Lys
   740 745 750
   Asp Leu Ile Ala Ser Thr Asp Ile Arg Leu Leu Lys Pro Ser Ala Lys
   755 760 765
   Asn Val His Val Pro Tyr Thr Gln Ala Ser Ser Gly Phe Glu Met Trp
   770 775 780
   Lys Asn Asn Ser Gly Arg Pro Leu Gln Glu Thr Ala Pro Phe Gly Cys
   785 790 795 800
   Lys Ile Ala Val Asn Pro Leu Arg Ala Val Asp Cys Ser Tyr Gly Asn
   805 810 815
   Ile Pro Ile Ser Ile Asp Ile Pro Asn Ala Ala Phe Ile Arg Thr Ser
   820 825 830
   Asp Ala Pro Leu Val Ser Thr Val Lys Cys Glu Val Ser Glu Cys Thr
   835 840 845
   Tyr Ser Ala Asp Phe Gly Gly Met Ala Thr Leu Gln Tyr Val Ser Asp
       850                 855                 860
   Arg Glu Gly Gln Cys Pro Val His Ser His Ser Ser Thr Ala Thr Leu
   865                 870                 875                 880
   Gln Glu Ser Thr Val His Val Leu Glu Lys Gly Ala Val Thr Val His
                   885                 890                 895
   Phe Ser Thr Ala Ser Pro Gln Ala Asn Phe Ile Val Ser Leu Cys Gly
               900                 905                 910
   Lys Lys Thr Thr Cys Asn Ala Glu Cys Lys Pro Pro Ala Asp His Ile
           915                 920                 925
   Val Ser Thr Pro His Lys Asn Asp Gln Glu Phe Gln Ala Ala Ile Ser
       930                 935                 940
   Lys Thr Ser Trp Ser Trp Leu Phe Ala Leu Phe Gly Gly Ala Ser Ser
   945                 950                 955                 960
   Leu Leu Ile Ile Gly Leu Met Ile Phe Ala Cys Ser Met Met Leu Thr
                   965                 970                 975
   Ser Thr Arg Arg
               980
<210> 6
   <211> 981
   <212> PRT
   <213> Sindbis virus
<400> 6
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
   85 90 95
   Glu Gln Val Trp Asp Glu Ala Asp Asp Asn Thr Ile Arg Ile Gln Thr
   100 105 110
   Ser Ala Gln Phe Gly Tyr Asp Gln Ser Gly Ala Ala Ser Ala Asn Lys
   115 120 125
   Tyr Arg Tyr Met Ser Leu Glu Gln Asp His Thr Val Glu Glu Gly Thr
   130 135 140
   Met Asp Asp Ile Lys Ile Ser Thr Ser Gly Pro Cys Arg Arg Leu Ser
   145 150 155 160
   Tyr Lys Gly Tyr Phe Leu Leu Ala Lys Cys Pro Pro Gly Asp Ser Val
   165 170 175
   Thr Val Ser Ile Val Ser Ser Asn Ser Ala Thr Ser Cys Thr Leu Ala
   180 185 190
   Arg Lys Ile Lys Pro Lys Phe Val Gly Arg Glu Lys Tyr Asp Leu Pro
   195 200 205
   Pro Val His Gly Lys Lys Ile Pro Cys Thr Val Tyr Asp Arg Leu Glu
   210 215 220
   Thr Thr Ala Gly Tyr Ile Thr Met His Arg Pro Arg Pro His Ala Tyr
   225 230 235 240
   Thr Ser Tyr Leu Glu Glu Ser Ser Gly Lys Val Tyr Ala Lys Pro Pro
   245 250 255
   Ser Gly Lys Asn Ile Thr Tyr Glu Cys Lys Cys Gly Asp Tyr Lys Thr
   260 265 270
   Gly Thr Val Ser Thr Arg Thr Glu Ile Thr Gly Cys Thr Ala Ile Lys
   275 280 285
   Gln Cys Val Ala Tyr Lys Ser Asp Gln Thr Lys Trp Val Phe Asn Ser
   290 295 300
   Pro Asp Leu Ile Arg His Asp Asp His Thr Ala Gln Gly Lys Leu His
   305 310 315 320
   Leu Pro Phe Lys Leu Ile Pro Ser Thr Cys Met Val Pro Val Ala His
   325 330 335
   Ala Pro Asn Val Ile His Gly Phe Lys His Ile Ser Leu Gln Leu Asp
               340                 345                 350
   Thr Asp His Leu Thr Leu Leu Thr Thr Arg Arg Leu Gly Ala Asn Pro
           355                 360                 365
   Glu Pro Thr Thr Glu Trp Ile Val Gly Lys Thr Val Arg Asn Phe Thr
       370                 375                 380
   Val Asp Arg Asp Gly Leu Glu Tyr Ile Trp Gly Asn His Glu Pro Val
   385                 390                 395                 400
   Arg Val Tyr Ala Gln Glu Ser Ala Pro Gly Asp Pro His Gly Trp Pro
                   405                 410                 415
   His Glu Ile Val Gln His Tyr Tyr His Arg His Pro Val Tyr Thr Ile
               420                 425                 430
   Leu Ala Val Ala Ser Ala Thr Val Ala Met Met Ile Gly Val Thr Val
           435                 440                 445
   Ala Val Leu Cys Ala Cys Lys Ala Arg Arg Glu Cys Leu Thr Pro Tyr
       450                 455                 460
   Ala Leu Ala Pro Asn Ala Val Ile Pro Thr Ser Leu Ala Leu Leu Cys
   465                 470                 475                 480
   Cys Val Arg Ser Ala Asn Ala Glu Thr Phe Thr Glu Thr Met Ser Tyr
                   485                 490                 495
   Leu Trp Ser Asn Ser Gln Pro Phe Phe Trp Val Gln Leu Cys Ile Pro
               500                 505                 510
   Leu Ala Ala Phe Ile Val Leu Met Arg Cys Cys Ser Cys Cys Leu Pro
           515                 520                 525
   Phe Leu Val Val Ala Gly Ala Tyr Leu Ala Lys Val Asp Ala Tyr Glu
       530                 535                 540
   His Ala Thr Thr Val Pro Asn Val Pro Gln Ile Pro Tyr Lys Ala Leu
   545                 550                 555                 560
   Val Glu Arg Ala Gly Tyr Ala Pro Leu Asn Leu Glu Ile Thr Val Met
                   565                 570                 575
   Ser Ser Glu Val Leu Pro Ser Thr Asn Gln Glu Tyr Ile Thr Cys Lys
               580                 585                 590
   Phe Thr Thr Val Val Pro Ser Pro Lys Ile Lys Cys Cys Gly Ser Leu
           595                 600                 605
   Glu Cys Gln Pro Ala Ala His Ala Asp Tyr Thr Cys Lys Val Phe Gly
       610                 615                 620
   Gly Val Tyr Pro Phe Met Trp Gly Gly Ala Gln Cys Phe Cys Asp Ser
   625                 630                 635                 640
   Glu Asn Ser Gln Met Ser Glu Ala Tyr Val Glu Leu Ser Ala Asp Cys
                   645                 650                 655
   Ala Ser Asp His Ala Gln Ala Ile Lys Val His Thr Ala Ala Met Lys
               660                 665                 670
   Val Gly Leu Arg Ile Val Tyr Gly Asn Thr Thr Ser Phe Leu Asp Val
           675                 680                 685
   Tyr Val Asn Gly Val Thr Pro Gly Thr Ser Lys Asp Leu Lys Val Ile
       690                 695                 700
   Ala Gly Pro Ile Ser Ala Ser Phe Thr Pro Phe Asp His Lys Val Val
   705                 710                 715                 720
   Ile His Arg Gly Leu Val Tyr Asn Tyr Asp Phe Pro Glu Tyr Gly Ala
                   725                 730                 735
   Met Lys Pro Gly Ala Phe Gly Asp Ile Gln Ala Thr Ser Leu Thr Ser
               740                 745                 750
   Lys Asp Leu Ile Ala Ser Thr Asp Ile Arg Leu Leu Lys Pro Ser Ala
           755                 760                 765
   Lys Asn Val His Val Pro Tyr Thr Gln Ala Ser Ser Gly Phe Glu Met
       770                 775                 780
   Trp Lys Asn Asn Ser Gly Arg Pro Leu Gln Glu Thr Ala Pro Phe Gly
   785                 790                 795                 800
   Cys Lys Ile Ala Val Asn Pro Leu Arg Ala Val Asp Cys Ser Tyr Gly
                   805                 810                 815
   Asn Ile Pro Ile Ser Ile Asp Ile Pro Asn Ala Ala Phe Ile Arg Thr
               820                 825                 830
   Ser Asp Ala Pro Leu Val Ser Thr Val Lys Cys Glu Val Ser Glu Cys
           835                 840                 845
   Thr Tyr Ser Ala Asp Phe Gly Gly Met Ala Thr Leu Gln Tyr Val Ser
   850 855 860
   Asp Arg Glu Gly Gln Cys Pro Val His Ser His Ser Ser Thr Ala Thr
   865 870 875 880
   Leu Gln Glu Ser Thr Val His Val Leu Glu Lys Gly Ala Val Thr Val
   885 890 895
   His Phe Ser Thr Ala Ser Pro Gln Ala Asn Phe Ile Val Ser Leu Cys
   900 905 910
   Gly Lys Lys Thr Thr Cys Asn Ala Glu Cys Lys Pro Pro Ala Asp His
   915 920 925
   Ile Val Ser Thr Pro His Lys Asn Asp Gln Glu Phe Gln Ala Ala Ile
   930 935 940
   Ser Lys Thr Ser Trp Ser Trp Leu Phe Ala Leu Phe Gly Gly Ala Ser
   945 950 955 960
   Ser Leu Leu Ile Ile Gly Leu Met Ile Phe Ala Cys Ser Met Met Leu
   965 970 975
   Thr Ser Thr Arg Arg
   980
<210> 7
   <211> 982
   <212> PRT
   <213> Sindbis virus
<400> 7
   Met Ser Ala Ala Pro Leu Val Thr Ala Met Cys Leu Leu Gly Asn Val
   1 5 10 15
   Ser Phe Pro Cys Asp Arg Pro Pro Thr Cys Tyr Thr Arg Glu Pro Ser
   20 25 30
   Arg Ala Leu Asp Ile Leu Glu Glu Asn Val Asn His Glu Ala Tyr Asp
   35 40 45
   Thr Leu Leu Asn Ala Ile Leu Arg Cys Gly Ser Ser Gly Arg Ser Lys
   50 55 60
   Arg Ser Val Ile Asp Asp Phe Thr Leu Thr Ser Pro Tyr Leu Gly Thr
   65 70 75 80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Glu
       210                 215                 220
   Gly Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
   325 330 335
   His Ala Pro Asn Val Ile His Gly Phe Lys His Ile Ser Leu Gln Leu
   340 345 350
   Asp Thr Asp His Leu Thr Leu Leu Thr Thr Arg Arg Leu Gly Ala Asn
   355 360 365
   Pro Glu Pro Thr Thr Glu Trp Ile Val Gly Lys Thr Val Arg Asn Phe
   370 375 380
   Thr Val Asp Arg Asp Gly Leu Glu Tyr Ile Trp Gly Asn His Glu Pro
   385 390 395 400
   Val Arg Val Tyr Ala Gln Glu Ser Ala Pro Gly Asp Pro His Gly Trp
   405 410 415
   Pro His Glu Ile Val Gln His Tyr Tyr His Arg His Pro Val Tyr Thr
   420 425 430
   Ile Leu Ala Val Ala Ser Ala Thr Val Ala Met Met Ile Gly Val Thr
   435 440 445
   Val Ala Val Leu Cys Ala Cys Lys Ala Arg Arg Glu Cys Leu Thr Pro
   450 455 460
   Tyr Ala Leu Ala Pro Asn Ala Val Ile Pro Thr Ser Leu Ala Leu Leu
   465 470 475 480
   Cys Cys Val Arg Ser Ala Asn Ala Glu Thr Phe Thr Glu Thr Met Ser
   485 490 495
   Tyr Leu Trp Ser Asn Ser Gln Pro Phe Phe Trp Val Gln Leu Cys Ile
   500 505 510
   Pro Leu Ala Ala Phe Ile Val Leu Met Arg Cys Cys Ser Cys Cys Leu
   515 520 525
   Pro Phe Leu Val Val Ala Gly Ala Tyr Leu Ala Lys Val Asp Ala Tyr
   530 535 540
   Glu His Ala Thr Thr Val Pro Asn Val Pro Gln Ile Pro Tyr Lys Ala
   545 550 555 560
   Leu Val Glu Arg Ala Gly Tyr Ala Pro Leu Asn Leu Glu Ile Thr Val
   565 570 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
       610                 615                 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705                 710                 715                 720
   Val Ile His Arg Gly Leu Val Tyr Asn Tyr Asp Phe Pro Glu Tyr Gly
                   725                 730                 735
   Ala Met Lys Pro Gly Ala Phe Gly Asp Ile Gln Ala Thr Ser Leu Thr
               740                 745                 750
   Ser Lys Asp Leu Ile Ala Ser Thr Asp Ile Arg Leu Leu Lys Pro Ser
           755                 760                 765
   Ala Lys Asn Val His Val Pro Tyr Thr Gln Ala Ser Ser Gly Phe Glu
       770                 775                 780
   Met Trp Lys Asn Asn Ser Gly Arg Pro Leu Gln Glu Thr Ala Pro Phe
   785                 790                 795                 800
   Gly Cys Lys Ile Ala Val Asn Pro Leu Arg Ala Val Asp Cys Ser Tyr
                   805                 810                 815
   Gly Asn Ile Pro Ile Ser Ile Asp Ile Pro Asn Ala Ala Phe Ile Arg
               820                 825                 830
   Thr Ser Asp Ala Pro Leu Val Ser Thr Val Lys Cys Glu Val Ser Glu
   835 840 845
   Cys Thr Tyr Ser Ala Asp Phe Gly Gly Met Ala Thr Leu Gln Tyr Val
   850 855 860
   Ser Asp Arg Glu Gly Gln Cys Pro Val His Ser His Ser Ser Thr Ala
   865 870 875 880
   Thr Leu Gln Glu Ser Thr Val His Val Leu Glu Lys Gly Ala Val Thr
   885 890 895
   Val His Phe Ser Thr Ala Ser Pro Gln Ala Asn Phe Ile Val Ser Leu
   900 905 910
   Cys Gly Lys Lys Thr Thr Cys Asn Ala Glu Cys Lys Pro Pro Ala Asp
   915 920 925
   His Ile Val Ser Thr Pro His Lys Asn Asp Gln Glu Phe Gln Ala Ala
   930 935 940
   Ile Ser Lys Thr Ser Trp Ser Trp Leu Phe Ala Leu Phe Gly Gly Ala
   945 950 955 960
   Ser Ser Leu Leu Ile Ile Gly Leu Met Ile Phe Ala Cys Ser Met Met
   965 970 975
   Leu Thr Ser Thr Arg Arg
   980
<210> 8
   <211> 981
   <212> PRT
   <213> Sindbis virus
<400> 8
   Met Ser Ala Ala Pro Leu Val Thr Ala Met Cys Leu Leu Gly Asn Val
   1 5 10 15
   Ser Phe Pro Cys Asp Arg Pro Pro Thr Cys Tyr Thr Arg Glu Pro Ser
   20 25 30
   Arg Ala Leu Asp Ile Leu Glu Glu Asn Val Asn His Glu Ala Tyr Asp
   35 40 45
   Thr Leu Leu Asn Ala Ile Leu Arg Cys Gly Ser Ser Gly Arg Ser Lys
   50 55 60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Glu
       210                 215                 220
   Thr Thr Ala Gly Tyr Ile Thr Met His Arg Pro Arg Pro His Ala Tyr
   225                 230                 235                 240
   Thr Ser Tyr Leu Glu Glu Ser Ser Gly Lys Val Tyr Ala Lys Pro Pro
                   245                 250                 255
   Ser Gly Lys Asn Ile Thr Tyr Glu Cys Lys Cys Gly Asp Tyr Lys Thr
               260                 265                 270
   Gly Thr Val Ser Thr Arg Thr Glu Ile Thr Gly Cys Thr Ala Ile Lys
           275                 280                 285
   Gln Cys Val Ala Tyr Lys Ser Asp Gln Thr Lys Trp Val Phe Asn Ser
       290                 295                 300
   Pro Asp Leu Ile Arg His Asp Asp His Thr Ala Gln Gly Lys Leu His
   305                 310                 315                 320
   Leu Pro Phe Lys Leu Ile Pro Ser Thr Cys Met Val Pro Val Ala His
                   325                 330                 335
   Ala Pro Asn Val Ile His Gly Phe Lys His Ile Ser Leu Gln Leu Asp
               340                 345                 350
   Thr Asp His Leu Thr Leu Leu Thr Thr Arg Arg Leu Gly Ala Asn Pro
           355                 360                 365
   Glu Pro Thr Thr Glu Trp Ile Val Gly Lys Thr Val Arg Asn Phe Thr
       370                 375                 380
   Val Asp Arg Asp Gly Leu Glu Tyr Ile Trp Gly Asn His Glu Pro Val
   385                 390                 395                 400
   Arg Val Tyr Ala Gln Glu Ser Ala Pro Gly Asp Pro His Gly Trp Pro
                   405                 410                 415
   His Glu Ile Val Gln His Tyr Tyr His Arg His Pro Val Tyr Thr Ile
               420                 425                 430
   Leu Ala Val Ala Ser Ala Thr Val Ala Met Met Ile Gly Val Thr Val
           435                 440                 445
   Ala Val Leu Cys Ala Cys Lys Ala Arg Arg Glu Cys Leu Thr Pro Tyr
       450                 455                 460
   Ala Leu Ala Pro Asn Ala Val Ile Pro Thr Ser Leu Ala Leu Leu Cys
   465                 470                 475                 480
   Cys Val Arg Ser Ala Asn Ala Glu Thr Phe Thr Glu Thr Met Ser Tyr
                   485                 490                 495
   Leu Trp Ser Asn Ser Gln Pro Phe Phe Trp Val Gln Leu Cys Ile Pro
               500                 505                 510
   Leu Ala Ala Phe Ile Val Leu Met Arg Cys Cys Ser Cys Cys Leu Pro
           515                 520                 525
   Phe Leu Val Val Ala Gly Ala Tyr Leu Ala Lys Val Asp Ala Tyr Glu
       530                 535                 540
   His Ala Thr Thr Val Pro Asn Val Pro Gln Ile Pro Tyr Lys Ala Leu
   545                 550                 555                 560
   Val Glu Arg Ala Gly Tyr Ala Pro Leu Asn Leu Glu Ile Thr Val Met
   565 570 575
   Ser Ser Glu Val Leu Pro Ser Thr Asn Gln Glu Tyr Ile Thr Cys Lys
   580 585 590
   Phe Thr Thr Val Val Pro Ser Pro Lys Ile Lys Cys Cys Gly Ser Leu
   595 600 605
   Glu Cys Gln Pro Ala Ala His Ala Asp Tyr Thr Cys Lys Val Phe Gly
   610 615 620
   Gly Val Tyr Pro Phe Met Trp Gly Gly Ala Gln Cys Phe Cys Asp Ser
   625 630 635 640
   Glu Asn Ser Gln Met Ser Glu Ala Tyr Val Glu Leu Ser Ala Asp Cys
   645 650 655
   Ala Ser Asp His Ala Gln Ala Ile Lys Val His Thr Ala Ala Met Lys
   660 665 670
   Val Gly Leu Arg Ile Val Tyr Gly Asn Thr Thr Ser Phe Leu Asp Val
   675 680 685
   Tyr Val Asn Gly Val Thr Pro Gly Thr Ser Lys Asp Leu Lys Val Ile
   690 695 700
   Ala Gly Pro Ile Ser Ala Ser Phe Thr Pro Phe Asp His Lys Val Val
   705 710 715 720
   Ile His Arg Gly Leu Val Tyr Asn Tyr Asp Phe Pro Glu Tyr Gly Ala
   725 730 735
   Met Lys Pro Gly Ala Phe Gly Asp Ile Gln Ala Thr Ser Leu Thr Ser
   740 745 750
   Lys Asp Leu Ile Ala Ser Thr Asp Ile Arg Leu Leu Lys Pro Ser Ala
   755 760 765
   Lys Asn Val His Val Pro Tyr Thr Gln Ala Ser Ser Gly Phe Glu Met
   770 775 780
   Trp Lys Asn Asn Ser Gly Arg Pro Leu Gln Glu Thr Ala Pro Phe Gly
   785 790 795 800
   Cys Lys Ile Ala Val Asn Pro Leu Arg Ala Val Asp Cys Ser Tyr Gly
   805 810 815
   Asn Ile Pro Ile Ser Ile Asp Ile Pro Asn Ala Ala Phe Ile Arg Thr
   820 825 830
   Ser Asp Ala Pro Leu Val Ser Thr Val Lys Cys Glu Val Ser Glu Cys
   835 840 845
   Thr Tyr Ser Ala Asp Phe Gly Gly Met Ala Thr Leu Gln Tyr Val Ser
   850 855 860
   Asp Arg Glu Gly Gln Cys Pro Val His Ser His Ser Ser Thr Ala Thr
   865 870 875 880
   Leu Gln Glu Ser Thr Val His Val Leu Glu Lys Gly Ala Val Thr Val
   885 890 895
   His Phe Ser Thr Ala Ser Pro Gln Ala Asn Phe Ile Val Ser Leu Cys
   900 905 910
   Gly Lys Lys Thr Thr Cys Asn Ala Glu Cys Lys Pro Pro Ala Asp His
   915 920 925
   Ile Val Ser Thr Pro His Lys Asn Asp Gln Glu Phe Gln Ala Ala Ile
   930 935 940
   Ser Lys Thr Ser Trp Ser Trp Leu Phe Ala Leu Phe Gly Gly Ala Ser
   945 950 955 960
   Ser Leu Leu Ile Ile Gly Leu Met Ile Phe Ala Cys Ser Met Met Leu
   965 970 975
   Thr Ser Thr Arg Arg
   980
<210> 9
   <211> 982
   <212> PRT
   <213> Sindbis virus
<400> 9
   Met Ser Ala Ala Pro Leu Val Thr Ala Met Cys Leu Leu Gly Asn Val
   1 5 10 15
   Ser Phe Pro Cys Asp Arg Pro Pro Thr Cys Tyr Thr Arg Glu Pro Ser
   20 25 30
   Arg Ala Leu Asp Ile Leu Glu Glu Asn Val Asn His Glu Ala Tyr Asp
   35 40 45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Glu Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Glu
       210                 215                 220
   Gly Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305 310 315 320
   His Leu Pro Phe Lys Leu Ile Pro Ser Thr Cys Met Val Pro Val Ala
   325 330 335
   His Ala Pro Asn Val Ile His Gly Phe Lys His Ile Ser Leu Gln Leu
   340 345 350
   Asp Thr Asp His Leu Thr Leu Leu Thr Thr Arg Arg Leu Gly Ala Asn
   355 360 365
   Pro Glu Pro Thr Thr Glu Trp Ile Val Gly Lys Thr Val Arg Asn Phe
   370 375 380
   Thr Val Asp Arg Asp Gly Leu Glu Tyr Ile Trp Gly Asn His Glu Pro
   385 390 395 400
   Val Arg Val Tyr Ala Gln Glu Ser Ala Pro Gly Asp Pro His Gly Trp
   405 410 415
   Pro His Glu Ile Val Gln His Tyr Tyr His Arg His Pro Val Tyr Thr
   420 425 430
   Ile Leu Ala Val Ala Ser Ala Thr Val Ala Met Met Ile Gly Val Thr
   435 440 445
   Val Ala Val Leu Cys Ala Cys Lys Ala Arg Arg Glu Cys Leu Thr Pro
   450 455 460
   Tyr Ala Leu Ala Pro Asn Ala Val Ile Pro Thr Ser Leu Ala Leu Leu
   465 470 475 480
   Cys Cys Val Arg Ser Ala Asn Ala Glu Thr Phe Thr Glu Thr Met Ser
   485 490 495
   Tyr Leu Trp Ser Asn Ser Gln Pro Phe Phe Trp Val Gln Leu Cys Ile
   500 505 510
   Pro Leu Ala Ala Phe Ile Val Leu Met Arg Cys Cys Ser Cys Cys Leu
   515 520 525
   Pro Phe Leu Val Val Ala Gly Ala Tyr Leu Ala Lys Val Asp Ala Tyr
   530 535 540
   Glu His Ala Thr Thr Val Pro Asn Val Pro Gln Ile Pro Tyr Lys Ala
   545 550 555 560
   Leu Val Glu Arg Ala Gly Tyr Ala Pro Leu Asn Leu Glu Ile Thr Val
                   565                 570                 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
       610                 615                 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705                 710                 715                 720
   Val Ile His Arg Gly Leu Val Tyr Asn Tyr Asp Phe Pro Glu Tyr Gly
                   725                 730                 735
   Ala Met Lys Pro Gly Ala Phe Gly Asp Ile Gln Ala Thr Ser Leu Thr
               740                 745                 750
   Ser Lys Asp Leu Ile Ala Ser Thr Asp Ile Arg Leu Leu Lys Pro Ser
           755                 760                 765
   Ala Lys Asn Val His Val Pro Tyr Thr Gln Ala Ser Ser Gly Phe Glu
       770                 775                 780
   Met Trp Lys Asn Asn Ser Gly Arg Pro Leu Gln Glu Thr Ala Pro Phe
   785                 790                 795                 800
   Gly Cys Lys Ile Ala Val Asn Pro Leu Arg Ala Val Asp Cys Ser Tyr
                   805                 810                 815
   Gly Asn Ile Pro Ile Ser Ile Asp Ile Pro Asn Ala Ala Phe Ile Arg
               820                 825                 830
   Thr Ser Asp Ala Pro Leu Val Ser Thr Val Lys Cys Glu Val Ser Glu
           835                 840                 845
   Cys Thr Tyr Ser Ala Asp Phe Gly Gly Met Ala Thr Leu Gln Tyr Val
       850                 855                 860
   Ser Asp Arg Glu Gly Gln Cys Pro Val His Ser His Ser Ser Thr Ala
   865                 870                 875                 880
   Thr Leu Gln Glu Ser Thr Val His Val Leu Glu Lys Gly Ala Val Thr
                   885                 890                 895
   Val His Phe Ser Thr Ala Ser Pro Gln Ala Asn Phe Ile Val Ser Leu
               900                 905                 910
   Cys Gly Lys Lys Thr Thr Cys Asn Ala Glu Cys Lys Pro Pro Ala Asp
           915                 920                 925
   His Ile Val Ser Thr Pro His Lys Asn Asp Gln Glu Phe Gln Ala Ala
       930                 935                 940
   Ile Ser Lys Thr Ser Trp Ser Trp Leu Phe Ala Leu Phe Gly Gly Ala
   945                 950                 955                 960
   Ser Ser Leu Leu Ile Ile Gly Leu Met Ile Phe Ala Cys Ser Met Met
                   965                 970                 975
   Leu Thr Ser Thr Arg Arg
               980
<210> 10
   <211> 981
   <212> PRT
   <213> Sindbis virus
<400> 10
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
   35 40 45
   Thr Leu Leu Asn Ala Ile Leu Arg Cys Gly Ser Ser Gly Arg Ser Lys
   50 55 60
   Arg Ser Val Ile Asp Asp Phe Thr Leu Thr Ser Pro Tyr Leu Gly Thr
   65 70 75 80
   Cys Ser Tyr Cys His His Thr Val Pro Cys Phe Ser Pro Val Lys Ile
   85 90 95
   Glu Gln Val Trp Asp Glu Ala Asp Asp Asn Thr Ile Arg Ile Gln Thr
   100 105 110
   Ser Ala Gln Phe Gly Tyr Asp Gln Ser Gly Ala Ala Ser Ala Asn Lys
   115 120 125
   Tyr Arg Tyr Met Ser Leu Lys Gln Asp His Thr Val Glu Glu Gly Thr
   130 135 140
   Met Asp Asp Ile Lys Ile Ser Thr Ser Gly Pro Cys Arg Arg Leu Ser
   145 150 155 160
   Tyr Lys Gly Tyr Phe Leu Leu Ala Lys Cys Pro Pro Gly Asp Ser Val
   165 170 175
   Thr Val Ser Ile Val Ser Ser Asn Ser Ala Thr Ser Cys Thr Leu Ala
   180 185 190
   Arg Lys Ile Lys Pro Lys Phe Val Gly Arg Glu Lys Tyr Asp Leu Pro
   195 200 205
   Pro Val His Gly Lys Lys Ile Pro Cys Thr Val Tyr Asp Arg Leu Glu
   210 215 220
   Thr Thr Ala Gly Tyr Ile Thr Met His Arg Pro Arg Pro His Ala Tyr
   225 230 235 240
   Thr Ser Tyr Leu Glu Glu Ser Ser Gly Lys Val Tyr Ala Lys Pro Pro
   245 250 255
   Ser Gly Lys Asn Ile Thr Tyr Glu Cys Lys Cys Gly Asp Tyr Lys Thr
   260 265 270
   Gly Thr Val Ser Thr Arg Thr Glu Ile Thr Gly Cys Thr Ala Ile Lys
   275 280 285
   Gln Cys Val Ala Tyr Lys Ser Asp Gln Thr Lys Trp Val Phe Asn Ser
       290                 295                 300
   Pro Asp Leu Ile Arg His Asp Asp His Thr Ala Gln Gly Lys Leu His
   305                 310                 315                 320
   Leu Pro Phe Lys Leu Ile Pro Ser Thr Cys Met Val Pro Val Ala His
                   325                 330                 335
   Ala Pro Asn Val Ile His Gly Phe Lys His Ile Ser Leu Gln Leu Asp
               340                 345                 350
   Thr Asp His Leu Thr Leu Leu Thr Thr Arg Arg Leu Gly Ala Asn Pro
           355                 360                 365
   Glu Pro Thr Thr Glu Trp Ile Val Gly Lys Thr Val Arg Asn Phe Thr
       370                 375                 380
   Val Asp Arg Asp Gly Leu Glu Tyr Ile Trp Gly Asn His Glu Pro Val
   385                 390                 395                 400
   Arg Val Tyr Ala Gln Glu Ser Ala Pro Gly Asp Pro His Gly Trp Pro
                   405                 410                 415
   His Glu Ile Val Gln His Tyr Tyr His Arg His Pro Val Tyr Thr Ile
               420                 425                 430
   Leu Ala Val Ala Ser Ala Thr Val Ala Met Met Ile Gly Val Thr Val
           435                 440                 445
   Ala Val Leu Cys Ala Cys Lys Ala Arg Arg Glu Cys Leu Thr Pro Tyr
       450                 455                 460
   Ala Leu Ala Pro Asn Ala Val Ile Pro Thr Ser Leu Ala Leu Leu Cys
   465                 470                 475                 480
   Cys Val Arg Ser Ala Asn Ala Glu Thr Phe Thr Glu Thr Met Ser Tyr
                   485                 490                 495
   Leu Trp Ser Asn Ser Gln Pro Phe Phe Trp Val Gln Leu Cys Ile Pro
               500                 505                 510
   Leu Ala Ala Phe Ile Val Leu Met Arg Cys Cys Ser Cys Cys Leu Pro
           515                 520                 525
   Phe Leu Val Val Ala Gly Ala Tyr Leu Ala Lys Val Asp Ala Tyr Glu
       530                 535                 540
   His Ala Thr Thr Val Pro Asn Val Pro Gln Ile Pro Tyr Lys Ala Leu
   545                 550                 555                 560
   Val Glu Arg Ala Gly Tyr Ala Pro Leu Asn Leu Glu Ile Thr Val Met
                   565                 570                 575
   Ser Ser Glu Val Leu Pro Ser Thr Asn Gln Glu Tyr Ile Thr Cys Lys
               580                 585                 590
   Phe Thr Thr Val Val Pro Ser Pro Lys Ile Lys Cys Cys Gly Ser Leu
           595                 600                 605
   Glu Cys Gln Pro Ala Ala His Ala Asp Tyr Thr Cys Lys Val Phe Gly
       610                 615                 620
   Gly Val Tyr Pro Phe Met Trp Gly Gly Ala Gln Cys Phe Cys Asp Ser
   625                 630                 635                 640
   Glu Asn Ser Gln Met Ser Glu Ala Tyr Val Glu Leu Ser Ala Asp Cys
                   645                 650                 655
   Ala Ser Asp His Ala Gln Ala Ile Lys Val His Thr Ala Ala Met Lys
               660                 665                 670
   Val Gly Leu Arg Ile Val Tyr Gly Asn Thr Thr Ser Phe Leu Asp Val
           675                 680                 685
   Tyr Val Asn Gly Val Thr Pro Gly Thr Ser Lys Asp Leu Lys Val Ile
       690                 695                 700
   Ala Gly Pro Ile Ser Ala Ser Phe Thr Pro Phe Asp His Lys Val Val
   705                 710                 715                 720
   Ile His Arg Gly Leu Val Tyr Asn Tyr Asp Phe Pro Glu Tyr Gly Ala
                   725                 730                 735
   Met Lys Pro Gly Ala Phe Gly Asp Ile Gln Ala Thr Ser Leu Thr Ser
               740                 745                 750
   Lys Asp Leu Ile Ala Ser Thr Asp Ile Arg Leu Leu Lys Pro Ser Ala
           755                 760                 765
   Lys Asn Val His Val Pro Tyr Thr Gln Ala Ser Ser Gly Phe Glu Met
       770                 775                 780
   Trp Lys Asn Asn Ser Gly Arg Pro Leu Gln Glu Thr Ala Pro Phe Gly
   785                 790                 795                 800
   Cys Lys Ile Ala Val Asn Pro Leu Arg Ala Val Asp Cys Ser Tyr Gly
   805 810 815
   Asn Ile Pro Ile Ser Ile Asp Ile Pro Asn Ala Ala Phe Ile Arg Thr
   820 825 830
   Ser Asp Ala Pro Leu Val Ser Thr Val Lys Cys Glu Val Ser Glu Cys
   835 840 845
   Thr Tyr Ser Ala Asp Phe Gly Gly Met Ala Thr Leu Gln Tyr Val Ser
   850 855 860
   Asp Arg Glu Gly Gln Cys Pro Val His Ser His Ser Ser Thr Ala Thr
   865 870 875 880
   Leu Gln Glu Ser Thr Val His Val Leu Glu Lys Gly Ala Val Thr Val
   885 890 895
   His Phe Ser Thr Ala Ser Pro Gln Ala Asn Phe Ile Val Ser Leu Cys
   900 905 910
   Gly Lys Lys Thr Thr Cys Asn Ala Glu Cys Lys Pro Pro Ala Asp His
   915 920 925
   Ile Val Ser Thr Pro His Lys Asn Asp Gln Glu Phe Gln Ala Ala Ile
   930 935 940
   Ser Lys Thr Ser Trp Ser Trp Leu Phe Ala Leu Phe Gly Gly Ala Ser
   945 950 955 960
   Ser Leu Leu Ile Ile Gly Leu Met Ile Phe Ala Cys Ser Met Met Leu
   965 970 975
   Thr Ser Thr Arg Arg
   980
<210> 11
   <211> 982
   <212> PRT
   <213> Sindbis virus
<400> 11
   Met Ser Ala Ala Pro Leu Val Thr Ala Met Cys Leu Leu Gly Asn Val
   1 5 10 15
   Ser Phe Pro Cys Asp Arg Pro Pro Thr Cys Tyr Thr Arg Glu Pro Ser
   20 25 30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Glu Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Gly Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
               275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
                   325                 330                 335
   His Ala Pro Asn Val Ile His Gly Phe Lys His Ile Ser Leu Gln Leu
               340                 345                 350
   Asp Thr Asp His Leu Thr Leu Leu Thr Thr Arg Arg Leu Gly Ala Asn
           355                 360                 365
   Pro Glu Pro Thr Thr Glu Trp Ile Val Gly Lys Thr Val Arg Asn Phe
       370                 375                 380
   Thr Val Asp Arg Asp Gly Leu Glu Tyr Ile Trp Gly Asn His Glu Pro
   385                 390                 395                 400
   Val Arg Val Tyr Ala Gln Glu Ser Ala Pro Gly Asp Pro His Gly Trp
                   405                 410                 415
   Pro His Glu Ile Val Gln His Tyr Tyr His Arg His Pro Val Tyr Thr
               420                 425                 430
   Ile Leu Ala Val Ala Ser Ala Thr Val Ala Met Met Ile Gly Val Thr
           435                 440                 445
   Val Ala Val Leu Cys Ala Cys Lys Ala Arg Arg Glu Cys Leu Thr Pro
       450                 455                 460
   Tyr Ala Leu Ala Pro Asn Ala Val Ile Pro Thr Ser Leu Ala Leu Leu
   465                 470                 475                 480
   Cys Cys Val Arg Ser Ala Asn Ala Glu Thr Phe Thr Glu Thr Met Ser
                   485                 490                 495
   Tyr Leu Trp Ser Asn Ser Gln Pro Phe Phe Trp Val Gln Leu Cys Ile
               500                 505                 510
   Pro Leu Ala Ala Phe Ile Val Leu Met Arg Cys Cys Ser Cys Cys Leu
           515                 520                 525
   Pro Phe Leu Val Val Ala Gly Ala Tyr Leu Ala Lys Val Asp Ala Tyr
       530                 535                 540
   Glu His Ala Thr Thr Val Pro Asn Val Pro Gln Ile Pro Tyr Lys Ala
   545                 550                 555                 560
   Leu Val Glu Arg Ala Gly Tyr Ala Pro Leu Asn Leu Glu Ile Thr Val
                   565                 570                 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
       610                 615                 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705                 710                 715                 720
   Val Ile His Arg Gly Leu Val Tyr Asn Tyr Asp Phe Pro Glu Tyr Gly
                   725                 730                 735
   Ala Met Lys Pro Gly Ala Phe Gly Asp Ile Gln Ala Thr Ser Leu Thr
               740                 745                 750
   Ser Lys Asp Leu Ile Ala Ser Thr Asp Ile Arg Leu Leu Lys Pro Ser
           755                 760                 765
   Ala Lys Asn Val His Val Pro Tyr Thr Gln Ala Ser Ser Gly Phe Glu
       770                 775                 780
   Met Trp Lys Asn Asn Ser Gly Arg Pro Leu Gln Glu Thr Ala Pro Phe
   785 790 795 800
   Gly Cys Lys Ile Ala Val Asn Pro Leu Arg Ala Val Asp Cys Ser Tyr
   805 810 815
   Gly Asn Ile Pro Ile Ser Ile Asp Ile Pro Asn Ala Ala Phe Ile Arg
   820 825 830
   Thr Ser Asp Ala Pro Leu Val Ser Thr Val Lys Cys Glu Val Ser Glu
   835 840 845
   Cys Thr Tyr Ser Ala Asp Phe Gly Gly Met Ala Thr Leu Gln Tyr Val
   850 855 860
   Ser Asp Arg Glu Gly Gln Cys Pro Val His Ser His Ser Ser Thr Ala
   865 870 875 880
   Thr Leu Gln Glu Ser Thr Val His Val Leu Glu Lys Gly Ala Val Thr
   885 890 895
   Val His Phe Ser Thr Ala Ser Pro Gln Ala Asn Phe Ile Val Ser Leu
   900 905 910
   Cys Gly Lys Lys Thr Thr Cys Asn Ala Glu Cys Lys Pro Pro Ala Asp
   915 920 925
   His Ile Val Ser Thr Pro His Lys Asn Asp Gln Glu Phe Gln Ala Ala
   930 935 940
   Ile Ser Lys Thr Ser Trp Ser Trp Leu Phe Ala Leu Phe Gly Gly Ala
   945 950 955 960
   Ser Ser Leu Leu Ile Ile Gly Leu Met Ile Phe Ala Cys Ser Met Met
   965 970 975
   Leu Thr Ser Thr Arg Arg
   980
<210> 12
   <211> 981
   <212> PRT
   <213> Sindbis virus
<400> 12
   Met Ser Ala Ala Pro Leu Val Thr Ala Met Cys Leu Leu Gly Asn Val
   1 5 10 15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Glu Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Thr Thr Ala Gly Tyr Ile Thr Met His Arg Pro Arg Pro His Ala Tyr
   225                 230                 235                 240
   Thr Ser Tyr Leu Glu Glu Ser Ser Gly Lys Val Tyr Ala Lys Pro Pro
                   245                 250                 255
   Ser Gly Lys Asn Ile Thr Tyr Glu Cys Lys Cys Gly Asp Tyr Lys Thr
               260                 265                 270
   Gly Thr Val Ser Thr Arg Thr Glu Ile Thr Gly Cys Thr Ala Ile Lys
               275                 280                 285
   Gln Cys Val Ala Tyr Lys Ser Asp Gln Thr Lys Trp Val Phe Asn Ser
           290                 295                 300
   Pro Asp Leu Ile Arg His Asp Asp His Thr Ala Gln Gly Lys Leu His
   305                 310                 315                 320
   Leu Pro Phe Lys Leu Ile Pro Ser Thr Cys Met Val Pro Val Ala His
                   325                 330                 335
   Ala Pro Asn Val Ile His Gly Phe Lys His Ile Ser Leu Gln Leu Asp
               340                 345                 350
   Thr Asp His Leu Thr Leu Leu Thr Thr Arg Arg Leu Gly Ala Asn Pro
           355                 360                 365
   Glu Pro Thr Thr Glu Trp Ile Val Gly Lys Thr Val Arg Asn Phe Thr
       370                 375                 380
   Val Asp Arg Asp Gly Leu Glu Tyr Ile Trp Gly Asn His Glu Pro Val
   385                 390                 395                 400
   Arg Val Tyr Ala Gln Glu Ser Ala Pro Gly Asp Pro His Gly Trp Pro
                   405                 410                 415
   His Glu Ile Val Gln His Tyr Tyr His Arg His Pro Val Tyr Thr Ile
               420                 425                 430
   Leu Ala Val Ala Ser Ala Thr Val Ala Met Met Ile Gly Val Thr Val
           435                 440                 445
   Ala Val Leu Cys Ala Cys Lys Ala Arg Arg Glu Cys Leu Thr Pro Tyr
       450                 455                 460
   Ala Leu Ala Pro Asn Ala Val Ile Pro Thr Ser Leu Ala Leu Leu Cys
   465                 470                 475                 480
   Cys Val Arg Ser Ala Asn Ala Glu Thr Phe Thr Glu Thr Met Ser Tyr
                   485                 490                 495
   Leu Trp Ser Asn Ser Gln Pro Phe Phe Trp Val Gln Leu Cys Ile Pro
               500                 505                 510
   Leu Ala Ala Phe Ile Val Leu Met Arg Cys Cys Ser Cys Cys Leu Pro
               515                 520                 525
   Phe Leu Val Val Ala Gly Ala Tyr Leu Ala Lys Val Asp Ala Tyr Glu
       530                 535                 540
   His Ala Thr Thr Val Pro Asn Val Pro Gln Ile Pro Tyr Lys Ala Leu
   545                 550                 555                 560
   Val Glu Arg Ala Gly Tyr Ala Pro Leu Asn Leu Glu Ile Thr Val Met
                   565                 570                 575
   Ser Ser Glu Val Leu Pro Ser Thr Asn Gln Glu Tyr Ile Thr Cys Lys
               580                 585                 590
   Phe Thr Thr Val Val Pro Ser Pro Lys Ile Lys Cys Cys Gly Ser Leu
           595                 600                 605
   Glu Cys Gln Pro Ala Ala His Ala Asp Tyr Thr Cys Lys Val Phe Gly
       610                 615                 620
   Gly Val Tyr Pro Phe Met Trp Gly Gly Ala Gln Cys Phe Cys Asp Ser
   625                 630                 635                 640
   Glu Asn Ser Gln Met Ser Glu Ala Tyr Val Glu Leu Ser Ala Asp Cys
                   645                 650                 655
   Ala Ser Asp His Ala Gln Ala Ile Lys Val His Thr Ala Ala Met Lys
               660                 665                 670
   Val Gly Leu Arg Ile Val Tyr Gly Asn Thr Thr Ser Phe Leu Asp Val
           675                 680                 685
   Tyr Val Asn Gly Val Thr Pro Gly Thr Ser Lys Asp Leu Lys Val Ile
       690                 695                 700
   Ala Gly Pro Ile Ser Ala Ser Phe Thr Pro Phe Asp His Lys Val Val
   705                 710                 715                 720
   Ile His Arg Gly Leu Val Tyr Asn Tyr Asp Phe Pro Glu Tyr Gly Ala
                   725                 730                 735
   Met Lys Pro Gly Ala Phe Gly Asp Ile Gln Ala Thr Ser Leu Thr Ser
               740                 745                 750
   Lys Asp Leu Ile Ala Ser Thr Asp Ile Arg Leu Leu Lys Pro Ser Ala
           755                 760                 765
   Lys Asn Val His Val Pro Tyr Thr Gln Ala Ser Ser Gly Phe Glu Met
       770                 775                 780
   Trp Lys Asn Asn Ser Gly Arg Pro Leu Gln Glu Thr Ala Pro Phe Gly
   785                 790                 795                 800
   Cys Lys Ile Ala Val Asn Pro Leu Arg Ala Val Asp Cys Ser Tyr Gly
                   805                 810                 815
   Asn Ile Pro Ile Ser Ile Asp Ile Pro Asn Ala Ala Phe Ile Arg Thr
               820                 825                 830
   Ser Asp Ala Pro Leu Val Ser Thr Val Lys Cys Glu Val Ser Glu Cys
           835                 840                 845
   Thr Tyr Ser Ala Asp Phe Gly Gly Met Ala Thr Leu Gln Tyr Val Ser
       850                 855                 860
   Asp Arg Glu Gly Gln Cys Pro Val His Ser His Ser Ser Thr Ala Thr
   865                 870                 875                 880
   Leu Gln Glu Ser Thr Val His Val Leu Glu Lys Gly Ala Val Thr Val
                   885                 890                 895
   His Phe Ser Thr Ala Ser Pro Gln Ala Asn Phe Ile Val Ser Leu Cys
               900                 905                 910
   Gly Lys Lys Thr Thr Cys Asn Ala Glu Cys Lys Pro Pro Ala Asp His
           915                 920                 925
   Ile Val Ser Thr Pro His Lys Asn Asp Gln Glu Phe Gln Ala Ala Ile
       930                 935                 940
   Ser Lys Thr Ser Trp Ser Trp Leu Phe Ala Leu Phe Gly Gly Ala Ser
   945                 950                 955                 960
   Ser Leu Leu Ile Ile Gly Leu Met Ile Phe Ala Cys Ser Met Met Leu
                   965                 970                 975
   Thr Ser Thr Arg Arg
               980
<210> 13
   <211> 982
   <212> PRT
   <213> Sindbis virus
<400> 13
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Glu Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Gly Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
                   325                 330                 335
   His Ala Pro Asn Val Ile His Gly Phe Lys His Ile Ser Leu Gln Leu
               340                 345                 350
   Asp Thr Asp His Leu Thr Leu Leu Thr Thr Arg Arg Leu Gly Ala Asn
           355                 360                 365
   Pro Glu Pro Thr Thr Glu Trp Ile Val Gly Lys Thr Val Arg Asn Phe
       370                 375                 380
   Thr Val Asp Arg Asp Gly Leu Glu Tyr Ile Trp Gly Asn His Glu Pro
   385                 390                 395                 400
   Val Arg Val Tyr Ala Gln Glu Ser Ala Pro Gly Asp Pro His Gly Trp
                   405                 410                 415
   Pro His Glu Ile Val Gln His Tyr Tyr His Arg His Pro Val Tyr Thr
               420                 425                 430
   Ile Leu Ala Val Ala Ser Ala Thr Val Ala Met Met Ile Gly Val Thr
           435                 440                 445
   Val Ala Val Leu Cys Ala Cys Lys Ala Arg Arg Glu Cys Leu Thr Pro
       450                 455                 460
   Tyr Ala Leu Ala Pro Asn Ala Val Ile Pro Thr Ser Leu Ala Leu Leu
   465                 470                 475                 480
   Cys Cys Val Arg Ser Ala Asn Ala Glu Thr Phe Thr Glu Thr Met Ser
                   485                 490                 495
   Tyr Leu Trp Ser Asn Ser Gln Pro Phe Phe Trp Val Gln Leu Cys Ile
               500                 505                 510
   Pro Leu Ala Ala Phe Ile Val Leu Met Arg Cys Cys Ser Cys Cys Leu
           515                 520                 525
   Pro Phe Leu Val Val Ala Gly Ala Tyr Leu Ala Lys Val Asp Ala Tyr
       530                 535                 540
   Glu His Ala Thr Thr Val Pro Asn Val Pro Gln Ile Pro Tyr Lys Ala
   545                 550                 555                 560
   Leu Val Glu Arg Ala Gly Tyr Ala Pro Leu Asn Leu Glu Ile Thr Val
                   565                 570                 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
       610                 615                 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705                 710                 715                 720
   Val Ile His Arg Gly Leu Val Tyr Asn Tyr Asp Phe Pro Glu Tyr Gly
                   725                 730                 735
   Ala Met Lys Pro Gly Ala Phe Gly Asp Ile Gln Ala Thr Ser Leu Thr
               740                 745                 750
   Ser Lys Asp Leu Ile Ala Ser Thr Asp Ile Arg Leu Leu Lys Pro Ser
           755                 760                 765
   Ala Lys Asn Val His Val Pro Tyr Thr Gln Ala Ser Ser Gly Phe Glu
       770                 775                 780
   Met Trp Lys Asn Asn Ser Gly Arg Pro Leu Gln Glu Thr Ala Pro Phe
   785                 790                 795                 800
   Gly Cys Lys Ile Ala Val Asn Pro Leu Arg Ala Val Asp Cys Ser Tyr
                   805                 810                 815
   Gly Asn Ile Pro Ile Ser Ile Asp Ile Pro Asn Ala Ala Phe Ile Arg
               820                 825                 830
   Thr Ser Asp Ala Pro Leu Val Ser Thr Val Lys Cys Glu Val Ser Glu
           835                 840                 845
   Cys Thr Tyr Ser Ala Asp Phe Gly Gly Met Ala Thr Leu Gln Tyr Val
       850                 855                 860
   Ser Asp Arg Glu Gly Gln Cys Pro Val His Ser His Ser Ser Thr Ala
   865                 870                 875                 880
   Thr Leu Gln Glu Ser Thr Val His Val Leu Glu Lys Gly Ala Val Thr
                   885                 890                 895
   Val His Phe Ser Thr Ala Ser Pro Gln Ala Asn Phe Ile Val Ser Leu
               900                 905                 910
   Cys Gly Lys Lys Thr Thr Cys Asn Ala Glu Cys Lys Pro Pro Ala Asp
           915                 920                 925
   His Ile Val Ser Thr Pro His Lys Asn Asp Gln Glu Phe Gln Ala Ala
       930                 935                 940
   Ile Ser Lys Thr Ser Trp Ser Trp Leu Phe Ala Leu Phe Gly Gly Ala
   945                 950                 955                 960
   Ser Ser Leu Leu Ile Ile Gly Leu Met Ile Phe Ala Cys Ser Met Met
                   965                 970                 975
   Leu Thr Ser Thr Arg Arg
               980
<210> 14
   <211> 981
   <212> PRT
   <213> Sindbis virus
<400> 14
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Glu Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Thr Thr Ala Gly Tyr Ile Thr Met His Arg Pro Arg Pro His Ala Tyr
   225                 230                 235                 240
   Thr Ser Tyr Leu Glu Glu Ser Ser Gly Lys Val Tyr Ala Lys Pro Pro
                   245                 250                 255
   Ser Gly Lys Asn Ile Thr Tyr Glu Cys Lys Cys Gly Asp Tyr Lys Thr
               260                 265                 270
   Gly Thr Val Ser Thr Arg Thr Glu Ile Thr Gly Cys Thr Ala Ile Lys
           275                 280                 285
   Gln Cys Val Ala Tyr Lys Ser Asp Gln Thr Lys Trp Val Phe Asn Ser
       290                 295                 300
   Pro Asp Leu Ile Arg His Asp Asp His Thr Ala Gln Gly Lys Leu His
   305                 310                 315                 320
   Leu Pro Phe Lys Leu Ile Pro Ser Thr Cys Met Val Pro Val Ala His
                   325                 330                 335
   Ala Pro Asn Val Ile His Gly Phe Lys His Ile Ser Leu Gln Leu Asp
               340                 345                 350
   Thr Asp His Leu Thr Leu Leu Thr Thr Arg Arg Leu Gly Ala Asn Pro
           355                 360                 365
   Glu Pro Thr Thr Glu Trp Ile Val Gly Lys Thr Val Arg Asn Phe Thr
       370                 375                 380
   Val Asp Arg Asp Gly Leu Glu Tyr Ile Trp Gly Asn His Glu Pro Val
   385                 390                 395                 400
   Arg Val Tyr Ala Gln Glu Ser Ala Pro Gly Asp Pro His Gly Trp Pro
                   405                 410                 415
   His Glu Ile Val Gln His Tyr Tyr His Arg His Pro Val Tyr Thr Ile
               420                 425                 430
   Leu Ala Val Ala Ser Ala Thr Val Ala Met Met Ile Gly Val Thr Val
           435                 440                 445
   Ala Val Leu Cys Ala Cys Lys Ala Arg Arg Glu Cys Leu Thr Pro Tyr
       450                 455                 460
   Ala Leu Ala Pro Asn Ala Val Ile Pro Thr Ser Leu Ala Leu Leu Cys
   465                 470                 475                 480
   Cys Val Arg Ser Ala Asn Ala Glu Thr Phe Thr Glu Thr Met Ser Tyr
                   485                 490                 495
   Leu Trp Ser Asn Ser Gln Pro Phe Phe Trp Val Gln Leu Cys Ile Pro
   500 505 510
   Leu Ala Ala Phe Ile Val Leu Met Arg Cys Cys Ser Cys Cys Leu Pro
   515 520 525
   Phe Leu Val Val Ala Gly Ala Tyr Leu Ala Lys Val Asp Ala Tyr Glu
   530 535 540
   His Ala Thr Thr Val Pro Asn Val Pro Gln Ile Pro Tyr Lys Ala Leu
   545 550 555 560
   Val Glu Arg Ala Gly Tyr Ala Pro Leu Asn Leu Glu Ile Thr Val Met
   565 570 575
   Ser Ser Glu Val Leu Pro Ser Thr Asn Gln Glu Tyr Ile Thr Cys Lys
   580 585 590
   Phe Thr Thr Val Val Pro Ser Pro Lys Ile Lys Cys Cys Gly Ser Leu
   595 600 605
   Glu Cys Gln Pro Ala Ala His Ala Asp Tyr Thr Cys Lys Val Phe Gly
   610 615 620
   Gly Val Tyr Pro Phe Met Trp Gly Gly Ala Gln Cys Phe Cys Asp Ser
   625 630 635 640
   Glu Asn Ser Gln Met Ser Glu Ala Tyr Val Glu Leu Ser Ala Asp Cys
   645 650 655
   Ala Ser Asp His Ala Gln Ala Ile Lys Val His Thr Ala Ala Met Lys
   660 665 670
   Val Gly Leu Arg Ile Val Tyr Gly Asn Thr Thr Ser Phe Leu Asp Val
   675 680 685
   Tyr Val Asn Gly Val Thr Pro Gly Thr Ser Lys Asp Leu Lys Val Ile
   690 695 700
   Ala Gly Pro Ile Ser Ala Ser Phe Thr Pro Phe Asp His Lys Val Val
   705 710 715 720
   Ile His Arg Gly Leu Val Tyr Asn Tyr Asp Phe Pro Glu Tyr Gly Ala
   725 730 735
   Met Lys Pro Gly Ala Phe Gly Asp Ile Gln Ala Thr Ser Leu Thr Ser
   740 745 750
   Lys Asp Leu Ile Ala Ser Thr Asp Ile Arg Leu Leu Lys Pro Ser Ala
           755                 760                 765
   Lys Asn Val His Val Pro Tyr Thr Gln Ala Ser Ser Gly Phe Glu Met
       770                 775                 780
   Trp Lys Asn Asn Ser Gly Arg Pro Leu Gln Glu Thr Ala Pro Phe Gly
   785                 790                 795                 800
   Cys Lys Ile Ala Val Asn Pro Leu Arg Ala Val Asp Cys Ser Tyr Gly
                   805                 810                 815
   Asn Ile Pro Ile Ser Ile Asp Ile Pro Asn Ala Ala Phe Ile Arg Thr
               820                 825                 830
   Ser Asp Ala Pro Leu Val Ser Thr Val Lys Cys Glu Val Ser Glu Cys
           835                 840                 845
   Thr Tyr Ser Ala Asp Phe Gly Gly Met Ala Thr Leu Gln Tyr Val Ser
       850                 855                 860
   Asp Arg Glu Gly Gln Cys Pro Val His Ser His Ser Ser Thr Ala Thr
   865                 870                 875                 880
   Leu Gln Glu Ser Thr Val His Val Leu Glu Lys Gly Ala Val Thr Val
                   885                 890                 895
   His Phe Ser Thr Ala Ser Pro Gln Ala Asn Phe Ile Val Ser Leu Cys
               900                 905                 910
   Gly Lys Lys Thr Thr Cys Asn Ala Glu Cys Lys Pro Pro Ala Asp His
           915                 920                 925
   Ile Val Ser Thr Pro His Lys Asn Asp Gln Glu Phe Gln Ala Ala Ile
       930                 935                 940
   Ser Lys Thr Ser Trp Ser Trp Leu Phe Ala Leu Phe Gly Gly Ala Ser
   945                 950                 955                 960
   Ser Leu Leu Ile Ile Gly Leu Met Ile Phe Ala Cys Ser Met Met Leu
                   965                 970                 975
   Thr Ser Thr Arg Arg
               980
<210> 15
   <211> 982
   <212> PRT
   <213> Sindbis virus
<400> 15
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Glu Gln Asp His Thr Val Glu Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Gly Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
                   325                 330                 335
   His Ala Pro Asn Val Ile His Gly Phe Lys His Ile Ser Leu Gln Leu
               340                 345                 350
   Asp Thr Asp His Leu Thr Leu Leu Thr Thr Arg Arg Leu Gly Ala Asn
           355                 360                 365
   Pro Glu Pro Thr Thr Glu Trp Ile Val Gly Lys Thr Val Arg Asn Phe
       370                 375                 380
   Thr Val Asp Arg Asp Gly Leu Glu Tyr Ile Trp Gly Asn His Glu Pro
   385                 390                 395                 400
   Val Arg Val Tyr Ala Gln Glu Ser Ala Pro Gly Asp Pro His Gly Trp
                   405                 410                 415
   Pro His Glu Ile Val Gln His Tyr Tyr His Arg His Pro Val Tyr Thr
               420                 425                 430
   Ile Leu Ala Val Ala Ser Ala Thr Val Ala Met Met Ile Gly Val Thr
           435                 440                 445
   Val Ala Val Leu Cys Ala Cys Lys Ala Arg Arg Glu Cys Leu Thr Pro
       450                 455                 460
   Tyr Ala Leu Ala Pro Asn Ala Val Ile Pro Thr Ser Leu Ala Leu Leu
   465                 470                 475                 480
   Cys Cys Val Arg Ser Ala Asn Ala Glu Thr Phe Thr Glu Thr Met Ser
                   485                 490                 495
   Tyr Leu Trp Ser Asn Ser Gln Pro Phe Phe Trp Val Gln Leu Cys Ile
               500                 505                 510
   Pro Leu Ala Ala Phe Ile Val Leu Met Arg Cys Cys Ser Cys Cys Leu
           515                 520                 525
   Pro Phe Leu Val Val Ala Gly Ala Tyr Leu Ala Lys Val Asp Ala Tyr
       530                 535                 540
   Glu His Ala Thr Thr Val Pro Asn Val Pro Gln Ile Pro Tyr Lys Ala
   545                 550                 555                 560
   Leu Val Glu Arg Ala Gly Tyr Ala Pro Leu Asn Leu Glu Ile Thr Val
                   565                 570                 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
       610                 615                 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705                 710                 715                 720
   Val Ile His Arg Gly Leu Val Tyr Asn Tyr Asp Phe Pro Glu Tyr Gly
                   725                 730                 735
   Ala Met Lys Pro Gly Ala Phe Gly Asp Ile Gln Ala Thr Ser Leu Thr
               740                 745                 750
   Ser Lys Asp Leu Ile Ala Ser Thr Asp Ile Arg Leu Leu Lys Pro Ser
           755                 760                 765
   Ala Lys Asn Val His Val Pro Tyr Thr Gln Ala Ser Ser Gly Phe Glu
       770                 775                 780
   Met Trp Lys Asn Asn Ser Gly Arg Pro Leu Gln Glu Thr Ala Pro Phe
   785                 790                 795                 800
   Gly Cys Lys Ile Ala Val Asn Pro Leu Arg Ala Val Asp Cys Ser Tyr
                   805                 810                 815
   Gly Asn Ile Pro Ile Ser Ile Asp Ile Pro Asn Ala Ala Phe Ile Arg
               820                 825                 830
   Thr Ser Asp Ala Pro Leu Val Ser Thr Val Lys Cys Glu Val Ser Glu
           835                 840                 845
   Cys Thr Tyr Ser Ala Asp Phe Gly Gly Met Ala Thr Leu Gln Tyr Val
       850                 855                 860
   Ser Asp Arg Glu Gly Gln Cys Pro Val His Ser His Ser Ser Thr Ala
   865                 870                 875                 880
   Thr Leu Gln Glu Ser Thr Val His Val Leu Glu Lys Gly Ala Val Thr
                   885                 890                 895
   Val His Phe Ser Thr Ala Ser Pro Gln Ala Asn Phe Ile Val Ser Leu
               900                 905                 910
   Cys Gly Lys Lys Thr Thr Cys Asn Ala Glu Cys Lys Pro Pro Ala Asp
           915                 920                 925
   His Ile Val Ser Thr Pro His Lys Asn Asp Gln Glu Phe Gln Ala Ala
       930                 935                 940
   Ile Ser Lys Thr Ser Trp Ser Trp Leu Phe Ala Leu Phe Gly Gly Ala
   945                 950                 955                 960
   Ser Ser Leu Leu Ile Ile Gly Leu Met Ile Phe Ala Cys Ser Met Met
                   965                 970                 975
   Leu Thr Ser Thr Arg Arg
               980
<210> 16
   <211> 981
   <212> PRT
   <213> Sindbis virus
<400> 16
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Glu Gln Asp His Thr Val Glu Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Thr Thr Ala Gly Tyr Ile Thr Met His Arg Pro Arg Pro His Ala Tyr
   225 230 235 240
   Thr Ser Tyr Leu Glu Glu Ser Ser Gly Lys Val Tyr Ala Lys Pro Pro
   245 250 255
   Ser Gly Lys Asn Ile Thr Tyr Glu Cys Lys Cys Gly Asp Tyr Lys Thr
   260 265 270
   Gly Thr Val Ser Thr Arg Thr Glu Ile Thr Gly Cys Thr Ala Ile Lys
   275 280 285
   Gln Cys Val Ala Tyr Lys Ser Asp Gln Thr Lys Trp Val Phe Asn Ser
   290 295 300
   Pro Asp Leu Ile Arg His Asp Asp His Thr Ala Gln Gly Lys Leu His
   305 310 315 320
   Leu Pro Phe Lys Leu Ile Pro Ser Thr Cys Met Val Pro Val Ala His
   325 330 335
   Ala Pro Asn Val Ile His Gly Phe Lys His Ile Ser Leu Gln Leu Asp
   340 345 350
   Thr Asp His Leu Thr Leu Leu Thr Thr Arg Arg Leu Gly Ala Asn Pro
   355 360 365
   Glu Pro Thr Thr Glu Trp Ile Val Gly Lys Thr Val Arg Asn Phe Thr
   370 375 380
   Val Asp Arg Asp Gly Leu Glu Tyr Ile Trp Gly Asn His Glu Pro Val
   385 390 395 400
   Arg Val Tyr Ala Gln Glu Ser Ala Pro Gly Asp Pro His Gly Trp Pro
   405 410 415
   His Glu Ile Val Gln His Tyr Tyr His Arg His Pro Val Tyr Thr Ile
   420 425 430
   Leu Ala Val Ala Ser Ala Thr Val Ala Met Met Ile Gly Val Thr Val
   435 440 445
   Ala Val Leu Cys Ala Cys Lys Ala Arg Arg Glu Cys Leu Thr Pro Tyr
   450 455 460
   Ala Leu Ala Pro Asn Ala Val Ile Pro Thr Ser Leu Ala Leu Leu Cys
   465 470 475 480
   Cys Val Arg Ser Ala Asn Ala Glu Thr Phe Thr Glu Thr Met Ser Tyr
   485 490 495
   Leu Trp Ser Asn Ser Gln Pro Phe Phe Trp Val Gln Leu Cys Ile Pro
   500 505 510
   Leu Ala Ala Phe Ile Val Leu Met Arg Cys Cys Ser Cys Cys Leu Pro
   515 520 525
   Phe Leu Val Val Ala Gly Ala Tyr Leu Ala Lys Val Asp Ala Tyr Glu
   530 535 540
   His Ala Thr Thr Val Pro Asn Val Pro Gln Ile Pro Tyr Lys Ala Leu
   545 550 555 560
   Val Glu Arg Ala Gly Tyr Ala Pro Leu Asn Leu Glu Ile Thr Val Met
   565 570 575
   Ser Ser Glu Val Leu Pro Ser Thr Asn Gln Glu Tyr Ile Thr Cys Lys
   580 585 590
   Phe Thr Thr Val Val Pro Ser Pro Lys Ile Lys Cys Cys Gly Ser Leu
   595 600 605
   Glu Cys Gln Pro Ala Ala His Ala Asp Tyr Thr Cys Lys Val Phe Gly
   610 615 620
   Gly Val Tyr Pro Phe Met Trp Gly Gly Ala Gln Cys Phe Cys Asp Ser
   625 630 635 640
   Glu Asn Ser Gln Met Ser Glu Ala Tyr Val Glu Leu Ser Ala Asp Cys
   645 650 655
   Ala Ser Asp His Ala Gln Ala Ile Lys Val His Thr Ala Ala Met Lys
   660 665 670
   Val Gly Leu Arg Ile Val Tyr Gly Asn Thr Thr Ser Phe Leu Asp Val
   675 680 685
   Tyr Val Asn Gly Val Thr Pro Gly Thr Ser Lys Asp Leu Lys Val Ile
   690 695 700
   Ala Gly Pro Ile Ser Ala Ser Phe Thr Pro Phe Asp His Lys Val Val
   705 710 715 720
   Ile His Arg Gly Leu Val Tyr Asn Tyr Asp Phe Pro Glu Tyr Gly Ala
   725 730 735
   Met Lys Pro Gly Ala Phe Gly Asp Ile Gln Ala Thr Ser Leu Thr Ser
   740 745 750
   Lys Asp Leu Ile Ala Ser Thr Asp Ile Arg Leu Leu Lys Pro Ser Ala
   755 760 765
   Lys Asn Val His Val Pro Tyr Thr Gln Ala Ser Ser Gly Phe Glu Met
   770 775 780
   Trp Lys Asn Asn Ser Gly Arg Pro Leu Gln Glu Thr Ala Pro Phe Gly
   785 790 795 800
   Cys Lys Ile Ala Val Asn Pro Leu Arg Ala Val Asp Cys Ser Tyr Gly
   805 810 815
   Asn Ile Pro Ile Ser Ile Asp Ile Pro Asn Ala Ala Phe Ile Arg Thr
   820 825 830
   Ser Asp Ala Pro Leu Val Ser Thr Val Lys Cys Glu Val Ser Glu Cys
   835 840 845
   Thr Tyr Ser Ala Asp Phe Gly Gly Met Ala Thr Leu Gln Tyr Val Ser
   850 855 860
   Asp Arg Glu Gly Gln Cys Pro Val His Ser His Ser Ser Thr Ala Thr
   865 870 875 880
   Leu Gln Glu Ser Thr Val His Val Leu Glu Lys Gly Ala Val Thr Val
   885 890 895
   His Phe Ser Thr Ala Ser Pro Gln Ala Asn Phe Ile Val Ser Leu Cys
   900 905 910
   Gly Lys Lys Thr Thr Cys Asn Ala Glu Cys Lys Pro Pro Ala Asp His
   915 920 925
   Ile Val Ser Thr Pro His Lys Asn Asp Gln Glu Phe Gln Ala Ala Ile
   930 935 940
   Ser Lys Thr Ser Trp Ser Trp Leu Phe Ala Leu Phe Gly Gly Ala Ser
   945 950 955 960
   Ser Leu Leu Ile Ile Gly Leu Met Ile Phe Ala Cys Ser Met Met Leu
   965 970 975
   Thr Ser Thr Arg Arg
   980
<210> 17
   <211> 982
   <212> PRT
   <213> Sindbis virus
<400> 17
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Glu Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
                   325                 330                 335
   His Ala Pro Asn Val Ile His Gly Phe Lys His Ile Ser Leu Gln Leu
               340                 345                 350
   Asp Thr Asp His Leu Thr Leu Leu Thr Thr Arg Arg Leu Gly Ala Asn
           355                 360                 365
   Pro Glu Pro Thr Thr Glu Trp Ile Val Gly Lys Thr Val Arg Asn Phe
       370                 375                 380
   Thr Val Asp Arg Asp Gly Leu Glu Tyr Ile Trp Gly Asn His Glu Pro
   385                 390                 395                 400
   Val Arg Val Tyr Ala Gln Glu Ser Ala Pro Gly Asp Pro His Gly Trp
                   405                 410                 415
   Pro His Glu Ile Val Gln His Tyr Tyr His Arg His Pro Val Tyr Thr
               420                 425                 430
   Ile Leu Ala Val Ala Ser Ala Thr Val Ala Met Met Ile Gly Val Thr
           435                 440                 445
   Val Ala Val Leu Cys Ala Cys Lys Ala Arg Arg Glu Cys Leu Thr Pro
       450                 455                 460
   Tyr Ala Leu Ala Pro Asn Ala Val Ile Pro Thr Ser Leu Ala Leu Leu
   465                 470                 475                 480
   Cys Cys Val Arg Ser Ala Asn Ala Glu Thr Phe Thr Glu Thr Met Ser
                   485                 490                 495
   Tyr Leu Trp Ser Asn Ser Gln Pro Phe Phe Trp Val Gln Leu Cys Ile
               500                 505                 510
   Pro Leu Ala Ala Phe Ile Val Leu Met Arg Cys Cys Ser Cys Cys Leu
           515                 520                 525
   Pro Phe Leu Val Val Ala Gly Ala Tyr Leu Ala Lys Val Asp Ala Tyr
       530                 535                 540
   Glu His Ala Thr Thr Val Pro Asn Val Pro Gln Ile Pro Tyr Lys Ala
   545                 550                 555                 560
   Leu Val Glu Arg Ala Gly Tyr Ala Pro Leu Asn Leu Glu Ile Thr Val
                   565                 570                 575
   Met Ser Ser Glu Val Leu Pro Ser Thr Asn Gln Glu Tyr Ile Thr Cys
               580                 585                 590
   Lys Phe Thr Thr Val Val Pro Ser Pro Lys Ile Lys Cys Cys Gly Ser
           595                 600                 605
   Leu Glu Cys Gln Pro Ala Ala His Ala Asp Tyr Thr Cys Lys Val Phe
       610                 615                 620
   Gly Gly Val Tyr Pro Phe Met Trp Gly Gly Ala Gln Cys Phe Cys Asp
   625                 630                 635                 640
   Ser Glu Asn Ser Gln Met Ser Glu Ala Tyr Val Glu Leu Ser Ala Asp
                   645                 650                 655
   Cys Ala Ser Asp His Ala Gln Ala Ile Lys Val His Thr Ala Ala Met
               660                 665                 670
   Lys Val Gly Leu Arg Ile Val Tyr Gly Asn Thr Thr Ser Phe Leu Asp
           675                 680                 685
   Val Tyr Val Asn Gly Val Thr Pro Gly Thr Ser Lys Asp Leu Lys Val
       690                 695                 700
   Ile Ala Gly Pro Ile Ser Ala Ser Phe Thr Pro Phe Asp His Lys Val
   705 710 715 720
   Val Ile His Arg Gly Leu Val Tyr Asn Tyr Asp Phe Pro Glu Tyr Gly
   725 730 735
   Ala Met Lys Pro Gly Ala Phe Gly Asp Ile Gln Ala Thr Ser Leu Thr
   740 745 750
   Ser Lys Asp Leu Ile Ala Ser Thr Asp Ile Arg Leu Leu Lys Pro Ser
   755 760 765
   Ala Lys Asn Val His Val Pro Tyr Thr Gln Ala Ser Ser Gly Phe Glu
   770 775 780
   Met Trp Lys Asn Asn Ser Gly Arg Pro Leu Gln Glu Thr Ala Pro Phe
   785 790 795 800
   Gly Cys Lys Ile Ala Val Asn Pro Leu Arg Ala Val Asp Cys Ser Tyr
   805 810 815
   Gly Asn Ile Pro Ile Ser Ile Asp Ile Pro Asn Ala Ala Phe Ile Arg
   820 825 830
   Thr Ser Asp Ala Pro Leu Val Ser Thr Val Lys Cys Glu Val Ser Glu
   835 840 845
   Cys Thr Tyr Ser Ala Asp Phe Gly Gly Met Ala Thr Leu Gln Tyr Val
   850 855 860
   Ser Asp Arg Glu Gly Gln Cys Pro Val His Ser His Ser Ser Thr Ala
   865 870 875 880
   Thr Leu Gln Glu Ser Thr Val His Val Leu Glu Lys Gly Ala Val Thr
   885 890 895
   Val His Phe Ser Thr Ala Ser Pro Gln Ala Asn Phe Ile Val Ser Leu
   900 905 910
   Cys Gly Lys Lys Thr Thr Cys Asn Ala Glu Cys Lys Pro Pro Ala Asp
   915 920 925
   His Ile Val Ser Thr Pro His Lys Asn Asp Gln Glu Phe Gln Ala Ala
   930 935 940
   Ile Ser Lys Thr Ser Trp Ser Trp Leu Phe Ala Leu Phe Gly Gly Ala
   945 950 955 960
   Ser Ser Leu Leu Ile Ile Gly Leu Met Ile Phe Ala Cys Ser Met Met
   965 970 975
   Leu Thr Ser Thr Arg Arg
   980
<210> 18
<400> 18
   000
<210> 19
<400> 19
   000
<210> 20
   <211> 488
   <212> PRT
   <213> Sindbis virus
<400> 20
   Met Ser Ala Ala Pro Leu Val Thr Ala Met Cys Leu Leu Gly Asn Val
   1               5                   10                  15
   Ser Phe Pro Cys Asp Arg Pro Pro Thr Cys Tyr Thr Arg Glu Pro Ser
               20                  25                  30
   Arg Ala Leu Asp Ile Leu Glu Glu Asn Val Asn His Glu Ala Tyr Asp
           35                  40                  45
   Thr Leu Leu Asn Ala Ile Leu Arg Cys Gly Ser Ser Gly Arg Ser Lys
       50                  55                  60
   Arg Ser Val Ile Asp Asp Phe Thr Leu Thr Ser Pro Tyr Leu Gly Thr
   65                  70                  75                  80
   Cys Ser Tyr Cys His His Thr Val Pro Cys Phe Ser Pro Val Lys Ile
                   85                  90                  95
   Glu Gln Val Trp Asp Glu Ala Asp Asp Asn Thr Ile Arg Ile Gln Thr
               100                 105                 110
   Ser Ala Gln Phe Gly Tyr Asp Gln Ser Gly Ala Ala Ser Ala Asn Lys
           115                 120                 125
   Tyr Arg Tyr Met Ser Leu Lys Gln Asp His Thr Val Lys Glu Gly Thr
       130                 135                 140
   Met Asp Asp Ile Lys Ile Ser Thr Ser Gly Pro Cys Arg Arg Leu Ser
   145                 150                 155                 160
   Tyr Lys Gly Tyr Phe Leu Leu Ala Lys Cys Pro Pro Gly Asp Ser Val
                   165                 170                 175
   Thr Val Ser Ile Val Ser Ser Asn Ser Ala Thr Ser Cys Thr Leu Ala
               180                 185                 190
   Arg Lys Ile Lys Pro Lys Phe Val Gly Arg Glu Lys Tyr Asp Leu Pro
           195                 200                 205
   Pro Val His Gly Lys Lys Ile Pro Cys Thr Val Tyr Asp Arg Leu Lys
       210                 215                 220
   Glu Thr Thr Ala Gly Tyr Ile Thr Met His Arg Pro Arg Pro His Ala
   225                 230                 235                 240
   Tyr Thr Ser Tyr Leu Glu Glu Ser Ser Gly Lys Val Tyr Ala Lys Pro
                   245                 250                 255
   Pro Ser Gly Lys Asn Ile Thr Tyr Glu Cys Lys Cys Gly Asp Tyr Lys
               260                 265                 270
   Thr Gly Thr Val Ser Thr Arg Thr Glu Ile Thr Gly Cys Thr Ala Ile
           275                 280                 285
   Lys Gln Cys Val Ala Tyr Lys Ser Asp Gln Thr Lys Trp Val Phe Asn
       290                 295                 300
   Ser Pro Asp Leu Ile Arg His Asp Asp His Thr Ala Gln Gly Lys Leu
   305                 310                 315                 320
   His Leu Pro Phe Lys Leu Ile Pro Ser Thr Cys Met Val Pro Val Ala
                   325                 330                 335
   His Ala Pro Asn Val Ile His Gly Phe Lys His Ile Ser Leu Gln Leu
               340                 345                 350
   Asp Thr Asp His Leu Thr Leu Leu Thr Thr Arg Arg Leu Gly Ala Asn
           355                 360                 365
   Pro Glu Pro Thr Thr Glu Trp Ile Val Gly Lys Thr Val Arg Asn Phe
       370                 375                 380
   Thr Val Asp Arg Asp Gly Leu Glu Tyr Ile Trp Gly Asn His Glu Pro
   385                 390                 395                 400
   Val Arg Val Tyr Ala Gln Glu Ser Ala Pro Gly Asp Pro His Gly Trp
                   405                 410                 415
   Pro His Glu Ile Val Gln His Tyr Tyr His Arg His Pro Val Tyr Thr
               420                 425                 430
   Ile Leu Ala Val Ala Ser Ala Thr Val Ala Met Met Ile Gly Val Thr
           435                 440                 445
   Val Ala Val Leu Cys Ala Cys Lys Ala Arg Arg Glu Cys Leu Thr Pro
       450                 455                 460
   Tyr Ala Leu Ala Pro Asn Ala Val Ile Pro Thr Ser Leu Ala Leu Leu
   465                 470                 475                 480
   Cys Cys Val Arg Ser Ala Asn Ala
                   485
<210> 21
   <211> 683
   <212> DNA
   <213> Human immunodeficiency virus type 1
<400> 21
   cctagaaaaa catggagcaa tcacaagtag caatacagca gctaccaatg ctgattgtgc      60
   ctggctagaa gcacaagagg aggaggaggt gggttttcca gtcacacctc aggtaccttt     120
   aagaccaatg acttacaagg cagctgtaga tcttagccac tttttaaaag aaaagggggg     180
   actggaaggg ctaattcact cccaacgaag acaagatatc cttgatctgt ggatctacca     240
   cacacaaggc tacttccctg attggcagaa ctacacacca gggccaggga tcagatatcc     300
   actgaccttt ggatggtgct acaagctagt accagttgag caagagaagg tagaagaagc     360
   caatgaagga gagaacaccc gcttgttaca ccctgtgagc ctgcatggga tggatgaccc     420
   ggagagagaa gtattagagt ggaggtttga cagccgccta gcatttcatc acatggcccg     480
   agagctgcat ccggactgta ctgggtctct ctggttagac cagatctgag cctgggagct     540
   ctctggctaa ctagggaacc cactgcttaa gcctcaataa agcttgcctt gagtgcttca     600
   agtagtgtgt gcccgtctgt tgtgtgactc tggtaactag agatccctca gaccctttta     660
   gtcagtgtgg aaaatctcta gca                                             683
<210> 22
   <211> 416
   <212> DNA
   <213> Human immunodeficiency virus type 1
<400> 22
   cctagaaaaa catggagcaa tcacaagtag caatacagca gctaccaatg ctgattgtgc 60
   ctggctagaa gcacaagagg aggaggaggt gggttttcca gtcacacctc aggtaccttt 120
   aagaccaatg acttacaagg cagctgtaga tcttagccac tttttaaaag aaaagggggg 180
   actggaaggg ctaattcact cccaacgaag acaagatctg ctttttgcct gtactgggtc 240
   tctctggtta gaccagatct gagcctggga gctctctggc taactaggga acccactgct 300
   taagcctcaa taaagcttgc cttgagtgct tcaagtagtg tgtgcccgtc tgttgtgtga 360
   ctctggtaac tagagatccc tcagaccctt ttagtcagtg tggaaaatct ctagca 416
<210> 23
   <211> 401
   <212> DNA
   <213> Human immunodeficiency virus type 1
<400> 23
   cctagaaaaa catggagcaa tcacaagtag caatacagca gctaccaatg ctgattgtgc 60
   ctggctagaa gcacaagagg aggaggaggt gggttttcca gtcacacctc aggtaccttt 120
   aagaccaatg acttacaagg cagctgtaga tcttagccac tttttactgg aagggctaat 180
   tcactcccaa cgaagacaag atctgctttt tgcctgtact gggtctctct ggttagacca 240
   gatctgagcc tgggagctct ctggctaact agggaaccca ctgcttaagc ctcaataaag 300
   cttgccttga gtgcttcaag tagtgtgtgc ccgtctgttg tgtgactctg gtaactagag 360
   atccctcaga cccttttagt cagtgtggaa aatctctagc a 401
<210> 24
<400> 24
   000
<210> 25
<400> 25
   000
<210> 26
   <211> 5
   <212> PRT
   <213> Sindbis virus
<400> 26
   Arg Ser Lys Arg Ser
   1 5
<210> 27
   <211> 4
   <212> PRT
   <213> Sindbis virus
<400> 27
   Arg Ser Lys Arg
   1
<210> 28
<400> 28
   000
<210> 29
<400> 29
   000
<210> 30
<400> 30
   000
<210> 31
<400> 31
   000
<210> 32
<400> 32
   000
<210> 33
<400> 33
   000
<210> 34
<400> 34
   000
<210> 35
   <211> 8
   <212> PRT
   <213> Artificial Sequence
<220>
   <223> a tag peptide sequence
<400> 35
   Asp Tyr Lys Asp Asp Asp Asp Lys
   1               5
<210> 36
   <211> 1374
   <212> PRT
   <213> Human herpesvirus 2
<400> 36
   Met Ala Ala Pro Ala Arg Asp Pro Pro Gly Tyr Arg Tyr Ala Ala Ala
   1               5                   10                  15
   Ile Leu Pro Thr Gly Ser Ile Leu Ser Thr Ile Glu Val Ala Ser His
   20 25 30
   Arg Arg Leu Phe Asp Phe Phe Ala Ala Val Arg Ser Asp Glu Asn Ser
   35 40 45
   Leu Tyr Asp Val Glu Phe Asp Ala Leu Leu Gly Ser Tyr Cys Asn Thr
   50 55 60
   Leu Ser Leu Val Arg Phe Leu Glu Leu Gly Leu Ser Val Ala Cys Val
   65 70 75 80
   Cys Thr Lys Phe Pro Glu Leu Ala Tyr Met Asn Glu Gly Arg Val Gln
   85 90 95
   Phe Glu Val His Gln Pro Leu Ile Ala Arg Asp Gly Pro His Pro Val
   100 105 110
   Glu Gln Pro Val His Asn Tyr Met Thr Lys Val Ile Asp Arg Arg Ala
   115 120 125
   Leu Asn Ala Ala Phe Ser Leu Ala Thr Glu Ala Ile Ala Leu Leu Thr
   130 135 140
   Gly Glu Ala Leu Asp Gly Thr Gly Ile Ser Leu His Arg Gln Leu Arg
   145 150 155 160
   Ala Ile Gln Gln Leu Ala Arg Asn Val Gln Ala Val Leu Gly Ala Phe
   165 170 175
   Glu Arg Gly Thr Ala Asp Gln Met Leu His Val Leu Leu Glu Lys Ala
   180 185 190
   Pro Pro Leu Ala Leu Leu Leu Pro Met Gln Arg Tyr Leu Asp Asn Gly
   195 200 205
   Arg Leu Ala Thr Arg Val Ala Arg Ala Thr Leu Val Ala Glu Leu Lys
   210 215 220
   Arg Ser Phe Cys Asp Thr Ser Phe Phe Leu Gly Lys Ala Gly His Arg
   225 230 235 240
   Arg Glu Ala Ile Glu Ala Trp Leu Val Asp Leu Thr Thr Ala Thr Gln
   245 250 255
   Pro Ser Val Ala Val Pro Arg Leu Thr His Ala Asp Thr Arg Gly Arg
   260 265 270
   Pro Val Asp Gly Val Leu Val Thr Thr Ala Ala Ile Lys Gln Arg Leu
   275 280 285
   Leu Gln Ser Phe Leu Lys Val Glu Asp Thr Glu Ala Asp Val Pro Val
   290 295 300
   Thr Tyr Gly Glu Met Val Leu Asn Gly Ala Asn Leu Val Thr Ala Leu
   305 310 315 320
   Val Met Gly Lys Ala Val Arg Ser Leu Asp Asp Val Gly Arg His Leu
   325 330 335
   Leu Asp Met Gln Glu Glu Gln Leu Glu Ala Asn Arg Glu Thr Leu Asp
   340 345 350
   Glu Leu Glu Ser Ala Pro Gln Thr Thr Arg Val Arg Ala Asp Leu Val
   355 360 365
   Ala Ile Gly Asp Arg Leu Val Phe Leu Glu Ala Leu Glu Arg Arg Ile
   370 375 380
   Tyr Ala Ala Thr Asn Val Pro Tyr Pro Leu Val Gly Ala Met Asp Leu
   385 390 395 400
   Thr Phe Val Leu Pro Leu Gly Leu Phe Asn Pro Ala Met Glu Arg Phe
   405 410 415
   Ala Ala His Ala Gly Asp Leu Val Pro Ala Pro Gly His Pro Glu Pro
   420 425 430
   Arg Ala Phe Pro Pro Arg Gln Leu Phe Phe Trp Gly Lys Asp His Gln
   435 440 445
   Val Leu Arg Leu Ser Met Glu Asn Ala Val Gly Thr Val Cys His Pro
   450 455 460
   Ser Leu Met Asn Ile Asp Ala Ala Val Gly Gly Val Asn His Asp Pro
   465 470 475 480
   Val Glu Ala Ala Asn Pro Tyr Gly Ala Tyr Val Ala Ala Pro Ala Gly
   485 490 495
   Pro Gly Ala Asp Met Gln Gln Arg Phe Leu Asn Ala Trp Arg Gln Arg
   500 505 510
   Leu Ala His Gly Arg Val Arg Trp Val Ala Glu Cys Gln Met Thr Ala
   515 520 525
   Glu Gln Phe Met Gln Pro Asp Asn Ala Asn Leu Ala Leu Glu Leu His
       530                 535                 540
   Pro Ala Phe Asp Phe Phe Ala Gly Val Ala Asp Val Glu Leu Pro Gly
   545                 550                 555                 560
   Gly Glu Val Pro Pro Ala Gly Pro Gly Ala Ile Gln Ala Thr Trp Arg
                   565                 570                 575
   Val Val Asn Gly Asn Leu Pro Leu Ala Leu Cys Pro Val Ala Phe Arg
               580                 585                 590
   Asp Ala Arg Gly Leu Glu Leu Gly Val Gly Arg His Ala Met Ala Pro
           595                 600                 605
   Ala Thr Ile Ala Ala Val Arg Gly Ala Phe Glu Asp Arg Ser Tyr Pro
       610                 615                 620
   Ala Val Phe Tyr Leu Leu Gln Ala Ala Ile His Gly Asn Glu His Val
   625                 630                 635                 640
   Phe Cys Ala Leu Ala Arg Leu Val Thr Gln Cys Ile Thr Ser Tyr Trp
                   645                 650                 655
   Asn Asn Thr Arg Cys Ala Ala Phe Val Asn Asp Tyr Ser Leu Val Ser
               660                 665                 670
   Tyr Ile Val Thr Tyr Leu Gly Gly Asp Leu Pro Glu Glu Cys Met Ala
           675                 680                 685
   Val Tyr Arg Asp Leu Val Ala His Val Glu Ala Leu Ala Gln Leu Val
       690                 695                 700
   Asp Asp Phe Thr Leu Pro Gly Pro Glu Leu Gly Gly Gln Ala Gln Ala
   705                 710                 715                 720
   Glu Leu Asn His Leu Met Arg Asp Pro Ala Leu Leu Pro Pro Leu Val
                   725                 730                 735
   Trp Asp Cys Asp Gly Leu Met Arg His Ala Ala Leu Asp Arg His Arg
               740                 745                 750
   Asp Cys Arg Ile Asp Ala Gly Gly His Glu Pro Val Tyr Ala Ala Ala
           755                 760                 765
   Cys Asn Val Ala Thr Ala Asp Phe Asn Arg Asn Asp Gly Arg Leu Leu
       770                 775                 780
   His Asn Thr Gln Ala Arg Ala Ala Asp Ala Ala Asp Asp Arg Pro His
   785 790 795 800
   Arg Pro Ala Asp Trp Thr Val His His Lys Ile Tyr Tyr Tyr Val Leu
   805 810 815
   Val Pro Ala Phe Ser Arg Gly Arg Cys Cys Thr Ala Gly Val Arg Phe
   820 825 830
   Asp Arg Val Tyr Ala Thr Leu Gln Asn Met Val Val Pro Glu Ile Ala
   835 840 845
   Pro Gly Glu Glu Cys Pro Ser Asp Pro Val Thr Asp Pro Ala His Pro
   850 855 860
   Leu His Pro Ala Asn Leu Val Ala Asn Thr Val Lys Arg Met Phe His
   865 870 875 880
   Asn Gly Arg Val Val Val Asp Gly Pro Ala Met Leu Thr Leu Gln Val
   885 890 895
   Leu Ala His Asn Met Ala Glu Arg Thr Thr Ala Leu Leu Cys Ser Ala
   900 905 910
   Ala Pro Asp Ala Gly Ala Asn Thr Ala Ser Thr Ala Asn Met Arg Ile
   915 920 925
   Phe Asp Gly Ala Leu His Ala Gly Val Leu Leu Met Ala Pro Gln His
   930 935 940
   Leu Asp His Thr Ile Gln Asn Gly Glu Tyr Phe Tyr Val Leu Pro Val
   945 950 955 960
   His Ala Leu Phe Ala Gly Ala Asp His Val Ala Asn Ala Pro Asn Phe
   965 970 975
   Pro Pro Ala Leu Arg Asp Leu Ala Arg Asp Val Pro Leu Val Pro Pro
   980 985 990
   Ala Leu Gly Ala Asn Tyr Phe Ser Ser Ile Arg Gln Pro Val Val Gln
   995 1000 1005
   His Ala Arg Glu Ser Ala Ala Gly Glu Asn Ala Leu Thr Tyr Ala
   1010 1015 1020
   Leu Met Ala Gly Tyr Phe Lys Met Ser Pro Val Ala Leu Tyr His
   1025 1030 1035
   Gln Leu Lys Thr Gly Leu His Pro Gly Phe Gly Phe Thr Val Val
   1040 1045 1050
   Arg Gln Asp Arg Phe Val Thr Glu Asn Val Leu Phe Ser Glu Arg
   1055 1060 1065
   Ala Ser Glu Ala Tyr Phe Leu Gly Gln Leu Gln Val Ala Arg His
   1070 1075 1080
   Glu Thr Gly Gly Gly Val Asn Phe Thr Leu Thr Gln Pro Arg Gly
   1085 1090 1095
   Asn Val Asp Leu Gly Val Gly Tyr Thr Ala Val Ala Ala Thr Gly
   1100 1105 1110
   Thr Val Arg Asn Pro Val Thr Asp Met Gly Asn Leu Pro Gln Asn
   1115 1120 1125
   Phe Tyr Leu Gly Arg Gly Ala Pro Pro Leu Leu Asp Asn Ala Ala
   1130 1135 1140
   Ala Val Tyr Leu Arg Asn Ala Val Val Ala Gly Asn Arg Leu Gly
   1145 1150 1155
   Pro Ala Gln Pro Leu Pro Val Phe Gly Cys Ala Gln Val Pro Arg
   1160 1165 1170
   Arg Ala Gly Met Asp His Gly Gln Asp Ala Val Cys Glu Phe Ile
   1175 1180 1185
   Ala Thr Pro Val Ala Thr Asp Ile Asn Tyr Phe Arg Arg Pro Cys
   1190 1195 1200
   Asn Pro Arg Gly Arg Ala Ala Gly Gly Val Tyr Ala Gly Asp Lys
   1205 1210 1215
   Glu Gly Asp Val Ile Ala Leu Met Tyr Asp His Gly Gln Ser Asp
   1220 1225 1230
   Pro Ala Arg Pro Phe Ala Ala Thr Ala Asn Pro Trp Ala Ser Gln
   1235 1240 1245
   Arg Phe Ser Tyr Gly Asp Leu Leu Tyr Asn Gly Ala Tyr His Leu
   1250 1255 1260
   Asn Gly Ala Ser Pro Val Leu Ser Pro Cys Phe Lys Phe Phe Thr
   1265 1270 1275
   Ala Ala Asp Ile Thr Ala Lys His Arg Cys Leu Glu Arg Leu Ile
   1280 1285 1290
   Val Glu Thr Gly Ser Ala Val Ser Thr Ala Thr Ala Ala Ser Asp
   1295 1300 1305
   Val Gln Phe Lys Arg Pro Pro Gly Cys Arg Glu Leu Val Glu Asp
   1310 1315 1320
   Pro Cys Gly Leu Phe Gln Glu Ala Tyr Pro Ile Thr Cys Ala Ser
   1325 1330 1335
   Asp Pro Ala Leu Leu Arg Ser Ala Arg Asp Gly Glu Ala His Ala
   1340 1345 1350
   Arg Glu Thr His Phe Thr Gln Tyr Leu Ile Tyr Asp Ala Ser Pro
   1355 1360 1365
   Leu Lys Gly Leu Ser Leu
   1370
<210> 37
   <211> 15
   <212> PRT
   <213> Human herpesvirus 2
<400> 37
   Asn Tyr Phe Ser Ser Ile Arg Gln Pro Val Val Gln His Ala Arg
   1 5 10 15
<210> 38
   <211> 15
   <212> PRT
   <213> Human herpesvirus 2
<400> 38
   Cys Glu Phe Ile Ala Thr Pro Val Ala Thr Asp Ile Asn Tyr Phe
   1 5 10 15
<210> 39
   <211> 15
   <212> PRT
   <213> Human herpesvirus 2
<400> 39
   Glu Asn Ala Leu Thr Tyr Ala Leu Met Ala Gly Tyr Phe Lys Met
   1 5 10 15
<210> 40
   <211> 15
   <212> PRT
   <213> Human herpesvirus 2
<400> 40
   His Pro Gly Phe Gly Phe Thr Val Val Arg Gln Asp Arg Phe Val
   1 5 10 15
<210> 41
   <211> 8
   <212> PRT
   <213> Artificial Sequence
<220>
   <223> a tag peptide sequence
<400> 41
   Asp Leu Tyr Asp Asp Asp Asp Lys
   1 5
<210> 42
   <211> 45
   <212> PRT
   <213> Artificial Sequence
<220>
   <223> Synthetic polypeptide
<400> 42
   Gly Pro Lys Glu Pro Phe Gln Ser Tyr Val Asp Arg Phe Tyr Lys Ser
   1 5 10 15
   Leu Arg Ala Glu Gln Thr Asp Ala Ala Val Lys Asn Trp Met Thr Gln
   20 25 30
   Thr Leu Leu Ile Gln Asn Ala Asn Pro Asp Cys Lys Leu
   35 40 45
<210> 43
   <211> 11
   <212> PRT
   <213> Artificial Sequence
<220>
   <223> Synthetic peptide
<400> 43
   Ala Ala Val Lys Asn Trp Met Thr Gln Thr Leu
   1               5                   10
<210> 44
   <211> 9
   <212> PRT
   <213> Artificial Sequence
<220>
   <223> Synthetic peptide
<400> 44
   Lys Ser Leu Tyr Asn Thr Val Cys Val
   1               5
<210> 45
   <211> 13
   <212> PRT
   <213> Artificial Sequence
<220>
   <223> Synthetic peptide
<400> 45
   Asp Arg Phe Tyr Lys Ser Leu Arg Ala Glu Gln Thr Asp
   1               5                   10